# EUROPEAN PATENT SPECIFICATION

(11) **EP 4 107 166 B1**
(45) Date of publication and mention of the grant of the patent: **27.05.2026**
(21) Application number: 21757829.3
(22) Date of filing: 19.02.2021
(51) Int. Cl.: C07H 21/02, C07H 21/04, C07D 401/14, C07D 487/04, C07D 498/20, C07D 519/00

(54) **HETEROARYL COMPOUNDS FOR TREATMENT OF COMPLEMENT FACTOR D MEDIATED DISORDERS**
HETEROARYLVERBINDUNGEN ZUR BEHANDLUNG VON DURCH KOMPLEMENTFAKTOR D VERMITTELTEN STÖRUNGEN
COMPOSÉS HÉTÉROARYLE POUR LE TRAITEMENT DE TROUBLES MÉDIÉS PAR LE FACTEUR D DU COMPLÉMENT

(30) Priority: 20.02.2020 US 202062979145 P
(43) Date of publication of application: 28.12.2022
(73) Proprietor: Achillion Pharmaceuticals, Inc., Boston, MA 02210 (US)
(72) Inventor: WILES, Jason Allan, Boston, MA 02210 (US); GADHACHANDA, Venkat Rao, Boston, MA 02210 (US)
(74) Representative: J A Kemp LLP
(86) International application number: PCT/US2021/018871
(87) International publication number: WO 2021/168320

(56) References cited:
- WO-A1-2017/035353
- WO-A1-2018/160889
- WO-A1-2020/041301
- US-A1- 2012 295 884
- US-A1- 2015 368 271
- US-A1- 2019 038 623
- US-A1- 2019 359 645

## Description

### Field of the Disclosure

This disclosure provides heteroaryl drugs and their use in treating medical disorders, such as complement-mediated disorders, including Complement Factor D mediated disorders.

### Background

The complement system is a part of the innate immune system which does not adapt to changes over the course of the host's life, but is recruited and used by the adaptive immune system. For example, it assists, or complements, the ability of antibodies and phagocytic cells to clear pathogens. This sophisticated regulatory pathway allows rapid reaction to pathogenic organisms while protecting host cells from destruction. Over thirty proteins and protein fragments make up the complement system. These proteins act through opsonization (enhancing phagocytosis of antigens), chemotaxis (attracting macrophages and neutrophils), cell lysis (rupturing membranes of foreign cells), and agglutination (clustering and binding of pathogens together).

The complement system has three pathways: classical, alternative, and lectin. Complement Factor D plays an early and central role in activation of the alternative pathway of the complement cascade. Activation of the alternative complement pathway is initiated by spontaneous hydrolysis of a thioester bond within the C3 protein to produce C3(H₂O), which associates with Factor B to form the C3(H₂O)B complex. Complement Factor D acts to cleave Factor B within the C3(H₂O)B complex to form Ba and Bb. The Bb fragment remains associated with C3(H₂O) to form the alternative pathway C3 convertase C3(H₂O)Bb. Additionally, C3b generated by any of the C3 convertases also associates with Factor B to form C3bB, which Factor D cleaves to generate the later stage alternative pathway C3 convertase C3bBb. This latter form of the alternative pathway C3 convertase may provide important downstream amplification within all three of the defined complement pathways, leading ultimately to the recruitment and assembly of additional factors in the complement cascade pathway, including the cleavage of C5 to C5a and C5b. C5b acts in the assembly of factors C6, C7, C8, and C9 into the membrane attack complex, which can destroy pathogenic cells by lysing the cell.

The dysfunction of or excessive activation of complement has been linked to certain autoimmune, inflammatory, and neurodegenerative diseases, as well as ischemia-reperfusion injury and cancer. For example, activation of the alternative pathway of the complement cascade contributes to the production of C3a and C5a, both potent anaphylatoxins, which also have roles in a number of inflammatory disorders. Therefore, in some instances, it is desirable to decrease the response of the complement pathway, including the alternative complement pathway. Some examples of disorders mediated by the complement pathway include age-related macular degeneration (AMD), paroxysmal nocturnal hemoglobinuria (PNH), multiple sclerosis, and rheumatoid arthritis.

Additional complement-mediated disorders include those classified under component 3 glomerulopathy (C3G). C3G is a recently-defined entity comprised of dense deposit disease (DDD) and C3 glomerulonephritis (C3GN) which encompasses a population of chronic kidney diseases wherein elevated activity of the alternative complement pathway and terminal complement pathway results in glomerular deposits made solely of complement C3 and no immunoglobulin (Ig).

Immune-complex membranoproliferative glomerulonephritis (IC-MPGN) is a renal disease which shares many clinical, pathologic, genetic and laboratory features with C3G, and therefore can be considered a sister disease of C3G. In the majority of patients with IC-MPGN, an underlying disease or disorder-most commonly infections, autoimmune diseases, or monoclonal gammopathies-are identified to which the renal disease is secondary. Patients with idiopathic IC-MPGN can have low C3 and normal C4 levels, similar to those observed in C3G, as well as many of the same genetic or acquired factors that are associated with abnormal alternative pathway activity. Although there are current hypotheses suggesting that the majority of IC-MPGN is attributable to over activity of the classical pathway, those patients with a low C3 and a normal C4 are likely to have significant overactivity of the alternative pathway. IC-MPGN patients with a low C3 and a normal C4 may benefit from alternative pathway inhibition.

Other disorders that have been linked to the complement cascade include atypical hemolytic uremic syndrome (aHUS), hemolytic uremic syndrome (HUS), abdominal aortic aneurysm, hemodialysis complications, hemolytic anemia, or hemodialysis, neuromyelitis optica (NMO), myasthenia gravis (MG), fatty liver, nonalcoholic steatohepatitis (NASH), liver inflammation, cirrhosis, liver failure, dermatomyositis, and amyotrophic lateral sclerosis.

Factor D is an attractive target for inhibition or regulation of the complement cascade due to its early and essential role in the alternative complement pathway, and for its potential role in signal amplification within the classical and lectin complement pathways. Inhibition of Factor D effectively interrupts the pathway and attenuates the formation of the membrane attack complex.

While initial attempts have been made to develop inhibitors of Factor D, there are currently no clinically approved small molecule Factor D inhibitors. Examples of Factor D inhibitor compounds are described in the following disclosures.

US Pat. No. 6,653,340 (BioCryst Pharmaceuticals, Inc.), titled "Compounds useful in the complement, coagulate and kallikrein pathways and method for their preparation" describes fused bicyclic ring compounds that are inhibitors of Factor D. US Patent Application 2019-0142802 (BioCryst Pharmaceuticals, Inc.) describes benzopyrazole compounds for the treatment of aberrant complement disorders. Additional patents assigned to BioCryst for the treatment and prevention of complement disorders include granted US Pat. No. 10,125,102 and US Application Nos. 2018-0362458 and 2019-0345135.

Novartis PCT patent publication WO2012/093101 titled "Indole compounds or analogues thereof useful for the treatment of age-related macular degeneration" describes certain Factor D inhibitors. Additional Factor D inhibitors are described in Novartis PCT patent publications WO2012093101, WO2013/164802, WO2013/192345, WO2014/002051, WO2014/002052, WO2014/002053, WO2014/002054, WO2014/002057, WO2014/002058, WO2014/002059, WO2014/005150, WO2014/009833, WO2014/143638, WO2015/009616, WO2015/009977, WO2015/066241, and WO2016088082.

Lifesci Pharmaceuticals PCT patent publication WO2017/098328 titled "Therapeutic Inhibitory Compounds" describes various Factor D inhibitors with variations in the central core heterocyclic ring. PCT patent publication WO2018/015818 is also titled "Therapeutic Inhibitory Compounds" and describes Factor D inhibitors without a cyclic central core. PCT patent publication WO2018/229543 is also titled "Therapeutic Inhibitory Compounds" and describes Factor D inhibitors with a bi-cyclic central core.

Bristol-Myers Squibb PCT patent publication WO2004/045518 titled "Open chain prolyl urea-related modulators of androgen receptor function" describes open chain prolyl urea and thiourea related compounds for the treatment of androgen receptor-associated conditions, such as age-related diseases, for example, sarcopenia.

Alexion Pharmaceuticals PCT patent publication WO1995/029697 titled "Methods and compositions for the treatment of glomerulonephritis and other inflammatory diseases" discloses antibodies directed to C5 of the complement pathway for the treatment of glomerulonephritis and inflammatory conditions involving pathologic activation of the complement system. Alexion Pharmaceutical's anti-C5 antibody eculizumab (Soliris^{®}) is currently the only complement-specific antibody on the market, and is the first and only approved treatment for paroxysmal nocturnal hemoglobinuria (PNH).

Additional complement factor D inhibitors are described in International Publication Nos. WO2019/227102; WO2019/028284; WO2018/160889; WO2018/160891; WO2018/160892; WO2017/035348; WO2017/035349; WO 2017/035351; WO 2017/035352; WO 2017/035353; WO 2017/035355; WO2017/035357; WO2017/035360; WO2017/035361; WO2017//035362; WO2017/035415; WO2017/035401; WO2017/035405; WO2017/035413; WO2017/035409; WO2017/035411; WO2017/035417; WO2017/035408 WO2015/130784; WO2015/130795; WO2015/130806; WO2015/130830; WO2015/130838; WO2015/130842; WO2015/130845; and WO2015/130854.

WO2020/041301 discloses pharmaceutical compounds for the treatment of Complement Factor D medical disorders. US2019/038623 relates to the treatment of paroxysmal nocturnal hemoglobinuria.

Given the wide variety of medical disorders that are caused by detrimental immune or inflammatory responses, new compounds are needed for medical treatment.

### Summary

The invention provides a compound of Formula: 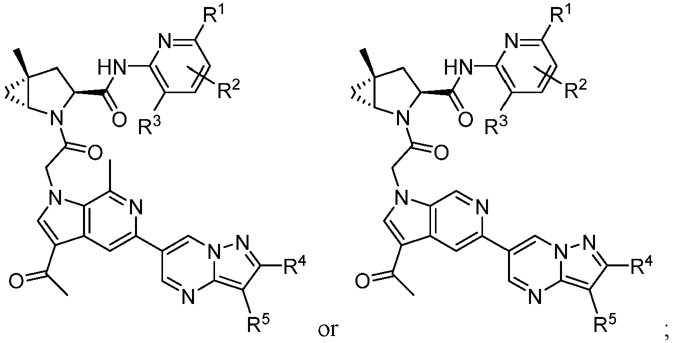 or a pharmaceutically acceptable salt thereof; wherein:
R¹ is selected from the group consisting of: halogen and C₁-C₂haloalkyl (for example, CF₃, CF₂H, CFH₂, CF₂CF₃ or CH₂CF₃);
R² is selected from the group consisting of: hydrogen, halogen, C₁-C₃alkyl, C₁-C₃haloalkyl, -COOR⁹, - CONR⁹R¹⁰, cyano, C₂-C₆alkanoyl, C₁-C₆alkoxy, -C₀-C₄alkylNR⁹R¹⁰, -C₀-C₄alkylOR⁹, and C₁-C₆haloalkoxy;
R³ is selected from C₁-C₃alkyl (for example methyl), halogen, and C₁-C₃haloalkyl,
R⁴ and R⁵ are independently selected from the group consisting of: hydrogen, halogen, C₁-C₃alkyl, C₁-C₃haloalkyl, -COOR⁹, -CONR⁹R¹⁰, cyano, C₂-C₆alkanoyl, C₁-C₆alkoxy, -C₀-C₄alkylNR⁹R¹⁰, -C₀-C₄alkylOR⁹, and C₁-C₆haloalkoxy, and
each R⁹ and R¹⁰ are independently selected from hydrogen and C₁-C₄alkyl.

In one embodiment, the compound of is 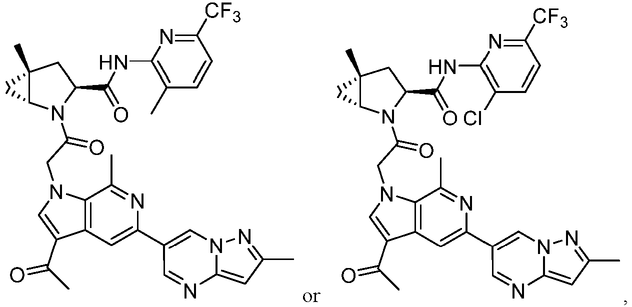 or a pharmaceutically acceptable salt thereof.

The invention also provides a compound selected from: 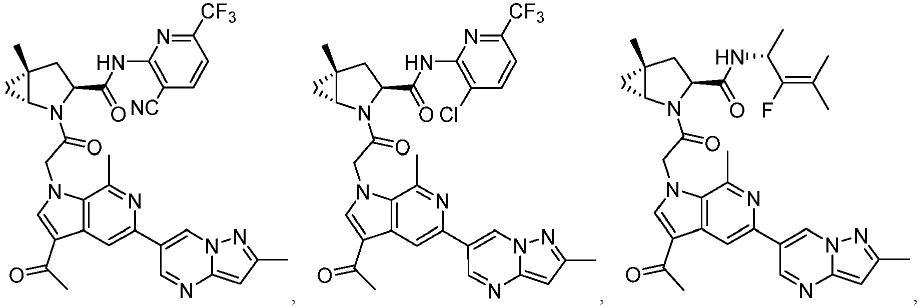 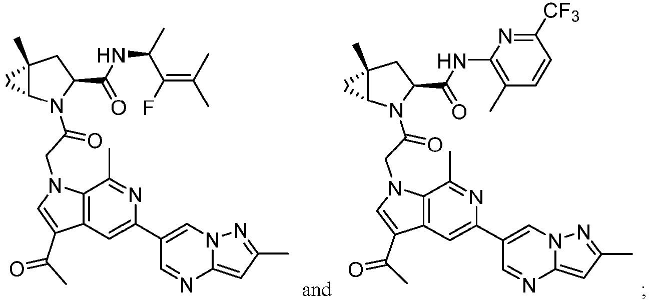 or a pharmaceutically acceptable salt thereof.

The invention also provides a pharmaceutical composition comprising a compound as described above or a pharmaceutically acceptable salt thereof, and a pharmaceutically acceptable carrier.

The invention also provides a compound as described above or a pharmaceutically acceptable salt thereof for use in treating a complement factor D mediated disorder in a subject in need thereof, wherein the disorder is selected from acute respiratory distress syndrome, age-related macular degeneration, arthritis, asthma, Alzheimer's dementia, amyotrophic lateral sclerosis, antibody-mediated transplant rejection, antineutrophil cytoplasm antibody-associated vasculitis, antiphospholipid syndrome, atypical or typical hemolytic uremic syndrome, cardiovascular disease, cold agglutinin disease, complement 3 glomerulopathy, chronic obstructive pulmonary disease, cirrhosis, Crohn's disease, C3 glomerulonephritis, diabetic retinopathy, dermatomyositis, dermatitis, epidermolysis bullosa acquisita, fatty liver, focal segmental glomerulosclerosis, geographic atrophy, glomerulonephritis, graft versus host disease, Guillain Barre syndrome, hemolytic anemia, hidradenitis suppurativa, IgA nephropathy, ischemia/reperfusion injury, liver failure, liver inflammation, lupus nephritis, membrane proliferative glomerulonephritis, multifocal motor neuropathy, multiple sclerosis, myasthenia gravis, neuromyelitis optica, nonalcoholic steatohepatitis, ocular disorder, ophthalmic disease, pancreatitis, paroxysmal nocturnal hemoglobinuria, pemphigoid, pemphigus vulgaris, pre-eclampsia, reduced glomerular filtration rate, renovascular disorder, respiratory disease, retinal detachment, rheumatoid arthritis, scleroderma, sepsis, Shiga toxin E. coli-related hemolytic uremic syndrome, spinal cord injury, sickle cell disease, traumatic brain injury, and ulcerative colitis.

### Detailed Description

### TERMINOLOGY

Compounds are described using standard nomenclature. Unless defined otherwise, all technical and scientific terms used herein have the same meaning as is commonly understood by one of skill in the art to which this disclosure belongs.

The compounds in any of the Formulas described herein include enantiomers, mixtures of enantiomers, diastereomers, tautomers, racemates and other isomers, such as rotamers, as if each is specifically described, unless otherwise indicated or otherwise excluded by context.

The terms "a" and "an" do not denote a limitation of quantity, but rather denote the presence of at least one of the referenced item. The term "or" means "and/or". Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of examples, or exemplary language (e.g., "such as"), is intended merely to better illustrate the disclosure and does not pose a limitation on the scope of the disclosure unless otherwise claimed. Unless defined otherwise, technical and scientific terms used herein have the same meaning as is commonly understood by one of skill in the art to which this disclosure belongs.

The present disclosure includes compounds as described herein with at least one desired isotopic substitution of an atom, at an amount above the natural abundance of the isotope, i.e., enriched.

Examples of isotopes that can be incorporated into compounds of the disclosure include isotopes of hydrogen, carbon, nitrogen, oxygen, phosphorous, fluorine, and chlorine, such as ²H, ³H, ¹¹C, ¹³C, ¹⁴C, ¹⁵N, ¹⁷O, ¹⁸O, ¹⁸F ³¹P, ³²P, ³⁵S, ³⁶CI, and ¹²⁵I respectively. In one embodiment, isotopically labelled compounds can be used in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F labeled compound may be particularly desirable for PET or SPECT studies. Isotopically labeled compounds of this disclosure and prodrugs thereof can generally be prepared by carrying out the procedures disclosed in the schemes or in the examples and preparations described below by substituting a readily available isotopically labeled reagent for a non-isotopically labeled reagent.

By way of general example and without limitation, isotopes of hydrogen, for example, deuterium (²H) and tritium (³H) may optionally be used anywhere in described structures that achieves the desired result. Alternatively or in addition, isotopes of carbon, e.g., ¹³C and ¹⁴C, may be used. In one embodiment, the isotopic substitution is replacing hydrogen with a deuterium at one or more locations on the molecule to improve the performance of the drug, for example, the pharmacodynamics, pharmacokinetics, biodistribution, half-life, stability, AUC, Tmax, Cmax, etc. For example, the deuterium can be bound to carbon in a location of bond breakage during metabolism (an α-deuterium kinetic isotope effect) or next to or near the site of bond breakage (a β-deuterium kinetic isotope effect). In certain embodiments where a molecule is drawn with a CH₃, the CH₃ group may be replaced with CD₃, CHD₂, or CH₂D. In certain embodiments where a molecule is drawn with a CD₃, the CD₃ group may be replaced with CHD₂ or CH₂D.

Isotopic substitutions, for example deuterium substitutions, can be partial or complete. Partial deuterium substitution means that at least one hydrogen is substituted with deuterium. In certain embodiments, the isotope is 80, 85, 90, 95 or 99% or more enriched in an isotope at any location of interest. In certain embodiments deuterium is 80, 85, 90, 95 or 99% enriched at a desired location. Unless otherwise stated, the enrichment at any point is above natural abundance, and in an embodiment is enough to alter a detectable property of the drug in a human.

The compound(s) of the present disclosure may form a solvate with solvents (including water). Therefore, in one embodiment, the disclosure includes a solvated form of the active compound. The term "solvate" refers to a molecular complex of a compound of the present disclosure (including a salt thereof) with one or more solvent molecules. Non-limiting examples of solvents are water, ethanol, dimethyl sulfoxide, acetone, and other common organic solvents. The term "hydrate" refers to a molecular complex comprising a compound of the disclosure and water. Pharmaceutically acceptable solvates in accordance with the disclosure include those wherein the solvent of crystallization may be isotopically substituted, *e.g*., D₂O, d₆-acetone, and d₆-DMSO. A solvate can be in a liquid or solid form.

A dash ("-") that is not between two letters or symbols is used to indicate a point of attachment for a substituent. For example, -(C=O)NH₂ is attached through carbon of the keto (C=O) group.

"Alkyl" is a branched, straight chain, or cyclic saturated aliphatic hydrocarbon group. In one embodiment, the alkyl contains from 1 to about 12 carbon atoms, more generally from 1 to about 6 carbon atoms or from 1 to about 4 carbon atoms. In one embodiment, the alkyl contains from 1 to about 8 carbon atoms. In certain embodiments, the alkyl is C₁-C₂, C₁-C₃, C₁-C₄, C₁-C₅ or C₁-C₆. The specified ranges as used herein indicate an alkyl group having each member of the range described as an independent species. For example, the term C₁-C₆ alkyl as used herein indicates a straight or branched alkyl group having from 1, 2, 3, 4, 5, or 6 carbon atoms and is intended to mean that each of these is described as an independent species. For example, the term C₁-C₄alkyl as used herein indicates a straight or branched alkyl group having from 1, 2, 3, or 4 carbon atoms and is intended to mean that each of these is described as an independent species. When C₀-Cₙ alkyl is used herein in conjunction with another group, for example, (C₃₋C₇cycloalkyl)C₀-C₄ alkyl, or -C₀-C₄alkyl(C₃-C₇cycloalkyl), the indicated group, in this case cycloalkyl, is either directly bound by a single covalent bond (C₀alkyl), or attached by an alkyl chain in this case 1, 2, 3, or 4 carbon atoms. Alkyls can also be attached via other groups such as heteroatoms as in -O-C₀-C₄alkyl(C₃-C₇cycloalkyl). Examples of alkyl include, but are not limited to, methyl, ethyl, n-propyl, isopropyl, n-butyl, isobutyl, sec-butyl, t-butyl, n-pentyl, isopentyl, *tert*-pentyl, neopentyl, n-hexyl, 2-methylpentane, 3-methylpentane, 2,2-dimethylbutane, 2,3-dimethylbutane, and hexyl.

When a term is used that includes "alk" it should be understood that "cycloalkyl" or "carbocyclic" can be considered part of the definition, unless unambiguously excluded by the context. For example and without limitation, the terms alkyl, alkenyl, alkynyl, alkoxy, alkanoyl, alkenyloxy, haloalkyl, etc. can all be considered to include the cyclic forms of alkyl, unless unambiguously excluded by context.

"Alkanoyl" is an alkyl group as defined above covalently bound through a carbonyl (C=O) bridge. The carbonyl carbon is included in the number of carbons, that is C₂alkanoyl is a CH₃(C=O)- group. In one embodiment, the alkanoyl group is optionally substituted as described

"Haloalkyl" indicates both branched and straight-chain alkyl groups substituted with 1 or more halogen atoms, up to the maximum allowable number of halogen atoms. Examples of haloalkyl include, but are not limited to, trifluoromethyl, monofluoromethyl, difluoromethyl, 2-fluoroethyl, and penta-fluoroethyl.

"Haloalkoxy" indicates a haloalkyl group as defined herein attached through an oxygen bridge (oxygen of an alcohol radical).

"Halo" or "halogen" indicates independently, any of fluoro, chloro, bromo or iodo.

A "dosage form" means a unit of administration of an active agent. Examples of dosage forms include tablets, capsules, injections, suspensions, liquids, emulsions, implants, particles, spheres, creams, ointments, suppositories, inhalable forms, transdermal forms, buccal, sublingual, topical, gel, mucosal, and the like. A "dosage form" can also include an implant, for example an optical implant.

"Pharmaceutical compositions" are compositions comprising at least one active agent, and at least one other substance, such as a carrier. "Pharmaceutical combinations" are combinations of at least two active agents which may be combined in a single dosage form or provided together in separate dosage forms with instructions that the active agents are to be used together to treat any disorder described herein.

A "pharmaceutically acceptable salt" is a derivative of the disclosed compound in which the parent compound is modified by making inorganic and organic, pharmaceutically acceptable, acid or base addition salts thereof. The salts of the present compounds can be synthesized from a parent compound that contains a basic or acidic moiety by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, nonaqueous media like ether, ethyl acetate, ethanol, isopropanol, or acetonitrile are typical, where practicable. Salts of the present compounds further include solvates of the compounds and of the compound salts.

Examples of pharmaceutically acceptable salts include, but are not limited to, mineral or organic acid salts of basic residues such as amines; alkali or organic salts of acidic residues such as carboxylic acids; and the like. The pharmaceutically acceptable salts include salts which are acceptable for human consumption and the quaternary ammonium salts of the parent compound formed, for example, from inorganic or organic acids. Examples of such salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)₁₋₄-COOH, and the like, or using a different acid that produces the same counterion. Lists of additional suitable salts may be found, e.g., in Remington's Pharmaceutical Sciences, 17th ed., Mack Publishing Company, Easton, Pa., p. 1418 (1985).

The term "carrier" applied to pharmaceutical compositions/combinations of the disclosure refers to a diluent, excipient, or vehicle with which an active compound is provided.

A "pharmaceutically acceptable excipient" means an excipient that is useful in preparing a pharmaceutical composition/combination that is generally safe, acceptable for human consumption, and neither biologically nor otherwise inappropriate for administration to a host, typically a human. In one embodiment, an excipient is used that is acceptable for veterinary use.

A "patient" or "host" or "subject" is a human or non-human animal in need of treatment or prevention of any of the disorders as specifically described herein, including but not limited to by modulation of the complement Factor D pathway or with a condition that is treatable with one of the compounds described herein. Typically the host is a human. A "patient" or "host" or "subject" also refers to for example, a mammal, primate (e.g., human), cows, sheep, goat, horse, dog, cat, rabbit, rat, mice, bird and the like.

"Providing a compound with at least one additional active agent," for example, in one embodiment can mean that the compound and the additional active agent(s) are provided simultaneously in a single dosage form, provided concomitantly in separate dosage forms, or provided in separate dosage forms for administration. In one embodiment, the compound administrations are separated by some amount of time that is within the time in which both the compound and the at least one additional active agent are within the blood stream of a patient. In certain embodiments the compound and the additional active agent need not be prescribed for a patient by the same medical care worker. In certain embodiments the additional active agent or agents need not require a prescription. Administration of the compound or the at least one additional active agent can occur via any appropriate route, for example, oral tablets, oral capsules, oral liquids, inhalation, injection, suppositories, parenteral, sublingual, buccal, intravenous, intraaortal, transdermal, polymeric controlled delivery, non-polymeric controlled delivery, nano or microparticles, liposomes, and/or topical contact. In one embodiment, the instructions for administration in a form of combination therapy is provided in the drug labeling.

A "therapeutically effective amount" of a pharmaceutical composition/combination of this disclosure means an amount effective, when administered to a host, provides a therapeutic benefit such as an amelioration of symptoms or reduction or diminution of the disease itself. In one embodiment, a therapeutically effective amount is an amount sufficient to prevent a significant increase or will significantly reduce the detectable level of complement Factor D in the patient's blood, serum, or tissues.

### N-Oxides

Any of the active compounds can be provided in its N-oxide form to a patient in need thereof. An N-oxide of an active compound or a precursor of the active compound may be used in a manufacturing scheme. The N-oxide may be a metabolite of administration of one of the active compounds herein, and may have independent activity. The N-oxide can be formed by treating the compound of interest with an oxidizing agent, for example a suitable peroxyacid or peroxide, to generate an N-oxide compound. For example, a heteroaryl group, for example a pyridyl group, can be treated with an oxidizing agent such as sodium percarbonate in the presence of a rhenium-based catalyst under mild reaction conditions to generate an N-oxide compound. A person skilled in the art will understand that appropriate protecting groups may be necessary to carry out the chemistry. See, Jain, S.L. et al., "Rhenium-Catalyzed Highly Efficient Oxidations of Tertiary Nitrogen Compounds to N-Oxides Using Sodium Percarbonate as Oxygen Source, Synlett, 2261-2663, 2006.

### Embodiments of "alkyl"

In one embodiment, "alkyl" is a C₁-C₁₀alkyl, C₁-C₉alkyl, C₁-C₈alkyl, C₁-C₇alkyl, C₁-C₆alkyl, C₁-C₅alkyl, C₁-C₄alkyl, C₁-C₃alkyl, or C₁-C₂alkyl.

In one embodiment, "alkyl" has one carbon.

In one embodiment, "alkyl" has two carbons.

In one embodiment, "alkyl" has three carbons.

In one embodiment, "alkyl" has four carbons.

In one embodiment, "alkyl" has five carbons.

In one embodiment, "alkyl" has six carbons.

Non-limiting examples of "alkyl" include: methyl, ethyl, propyl, butyl, pentyl, and hexyl.

Additional non-limiting examples of "alkyl" include: isopropyl, isobutyl, isopentyl, and isohexyl.

Additional non-limiting examples of "alkyl" include: *sec*-butyl, *sec*-pentyl, and *sec*-hexyl.

Additional non-limiting examples of "alkyl" include: *tert*-butyl, *tert*-pentyl, and *tert*-hexyl.

Additional non-limiting examples of "alkyl" include: neopentyl, 3-pentyl, and active pentyl.

In an alternative embodiment, the "alkyl" group is optionally substituted.

In an alternative embodiment, the "alkenyl" group is optionally substituted.

In an alternative embodiment, the "alkynyl" group is optionally substituted.

### Embodiments of "haloalkyl"

In one embodiment, "haloalkyl" is a C₁-C₁₀haloalkyl, C₁-C₉haloalkyl, C₁-C₈haloalkyl, C₁-C₇haloalkyl, C₁-C₆haloalkyl, C₁-C₅haloalkyl, C₁-C₄haloalkyl, C₁-C₃haloalkyl, and C₁-C₂haloalkyl.

In one embodiment, "haloalkyl" has one carbon.

In one embodiment, "haloalkyl" has one carbon and one halogen.

In one embodiment, "haloalkyl" has one carbon and two halogens.

In one embodiment, "haloalkyl" has one carbon and three halogens.

In one embodiment, "haloalkyl" has two carbons.

In one embodiment, "haloalkyl" has three carbons.

In one embodiment, "haloalkyl" has four carbons.

In one embodiment, "haloalkyl" has five carbons.

In one embodiment, "haloalkyl" has six carbons.

Non-limiting examples of "haloalkyl" include:

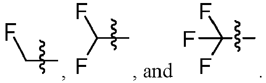

Additional non-limiting examples of "haloalkyl" include:

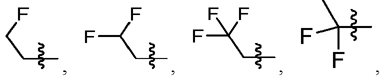

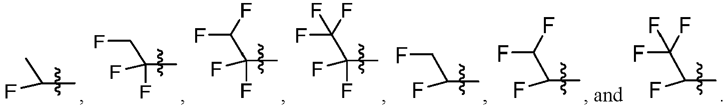

Additional non-limiting examples of "haloalkyl" include:

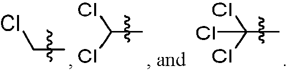

Additional non-limiting examples of "haloalkyl" include:

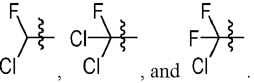

### Compounds

A compound of the following Formula is provided: 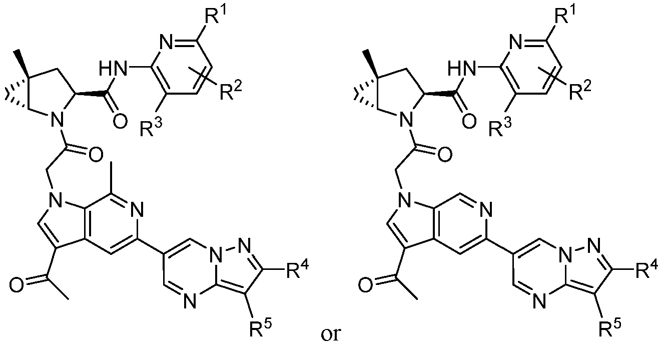 or a pharmaceutically acceptable salt thereof; wherein:
R¹ is selected from the group consisting of halogen and C₁-C₂haloalkyl;
R² is selected from the group consisting of hydrogen, halogen, C₁-C₃alkyl, C₁-C₃haloalkyl, -COOR⁹, - CONR⁹R¹⁰, cyano, C₂-C₆alkanoyl, C₁-C₆alkoxy, -C₀-C₄alkylNR⁹R¹⁰, -C₀-C₄alkylOR⁹, and C₁-C₆haloalkoxy;
R³ is C₁-C₃alkyl, halogen, or C₁-C₃haloalkyl;
R⁴ and R⁵ are independently selected from the group consisting of hydrogen, halogen, C₁-C₃alkyl, C₁-C₃haloalkyl, -COOR⁹, -CONR⁹R¹⁰, cyano, C₂-C₆alkanoyl, C₁-C₆alkoxy, -C₀-C₄alkylNR⁹R¹⁰, -C₀-C₄alkylOR⁹, and C₁-C₆haloalkoxy; and
each R⁹ and R¹⁰ are independently selected from the group consisting of hydrogen and C₁-C₄alkyl.

In certain embodiments the compound is selected from: 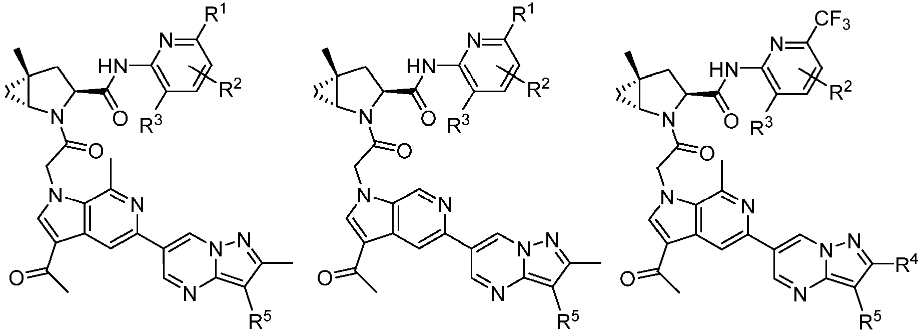 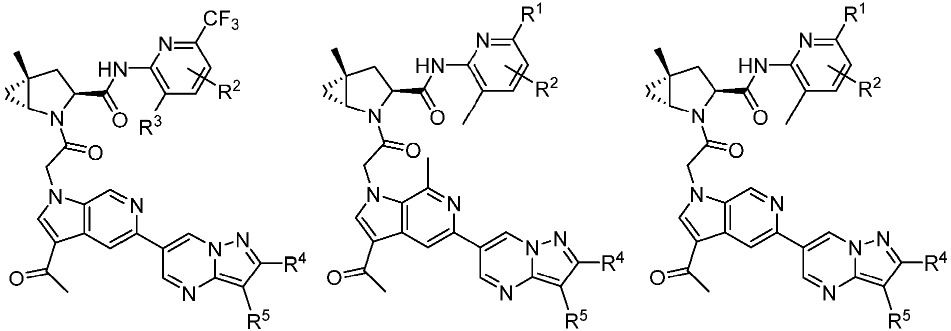 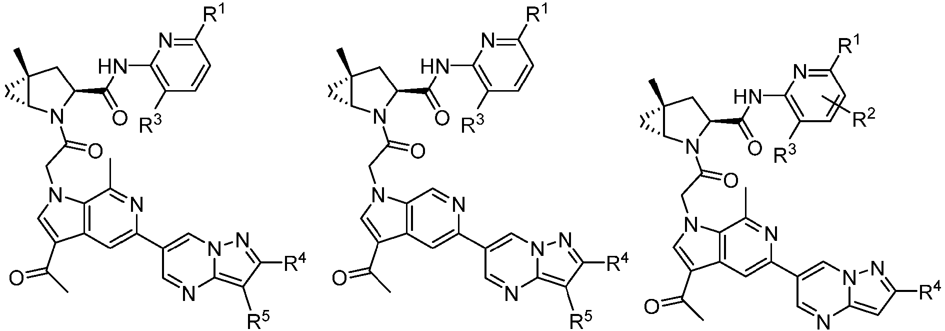 and 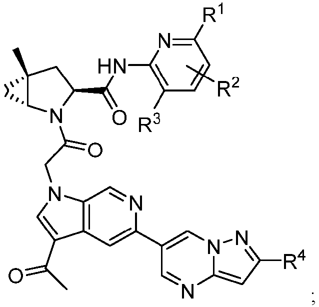 or a pharmaceutically acceptable salt thereof.

In certain embodiments the compound of is selected from: 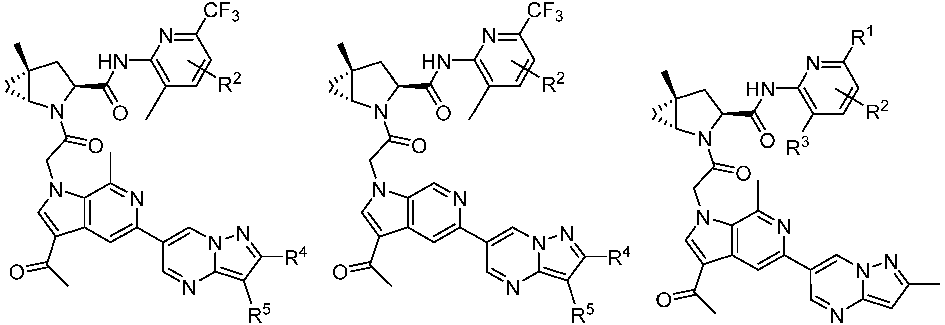 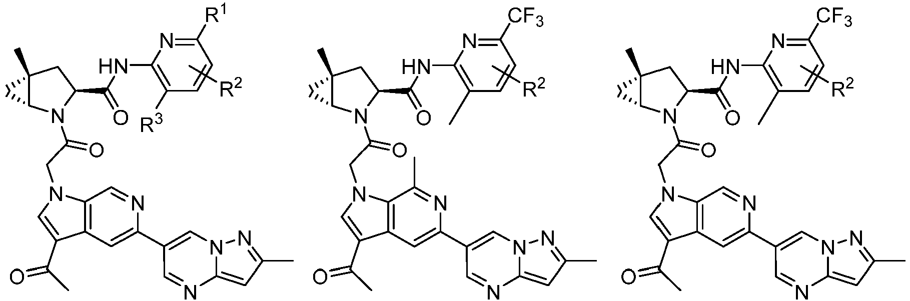 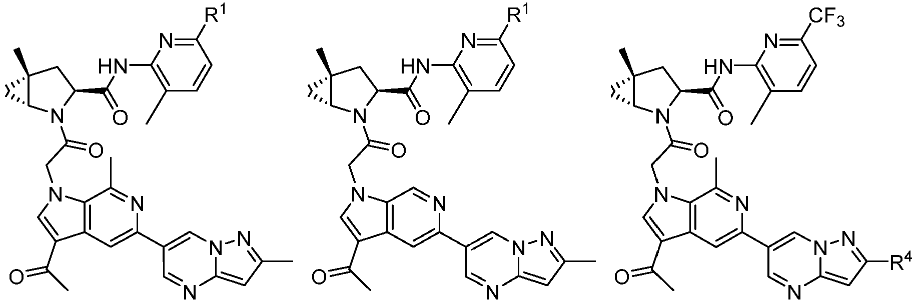 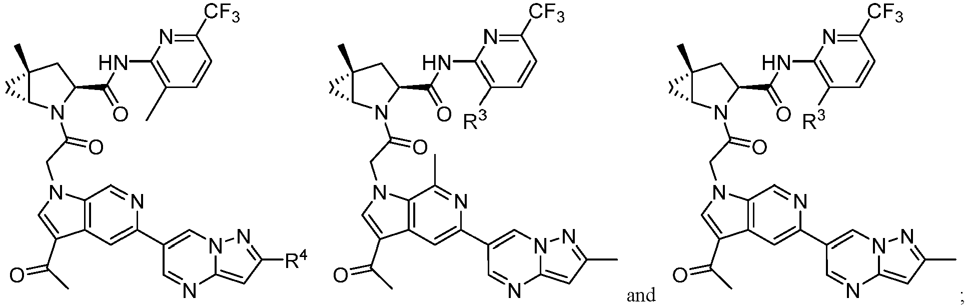 or a pharmaceutically acceptable salt thereof.

In certain embodiments the compound is selected from: 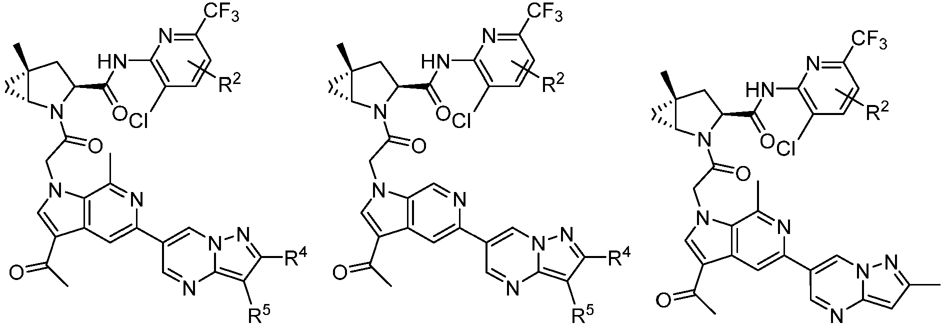 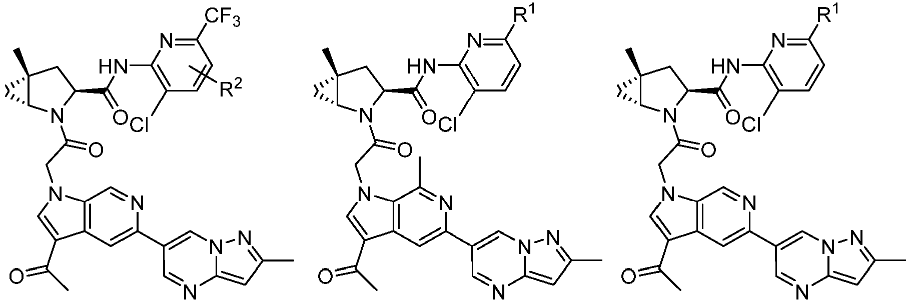 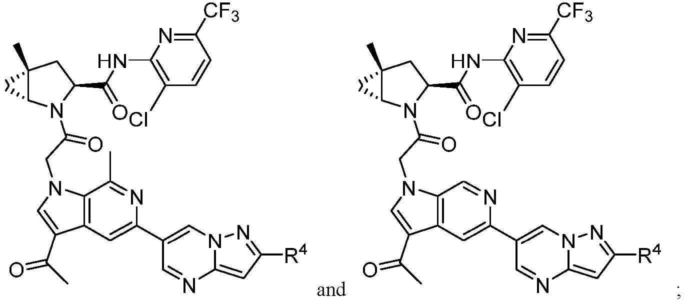 or a pharmaceutically acceptable salt thereof.

In one embodimeht, the compound is selected from: 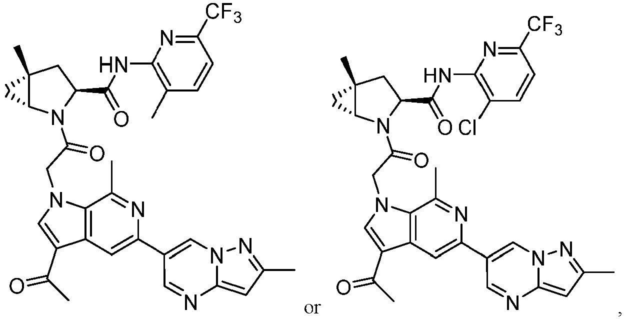 or a pharmaceutically acceptable salt thereof.

In one embodiment, the compound is selected from 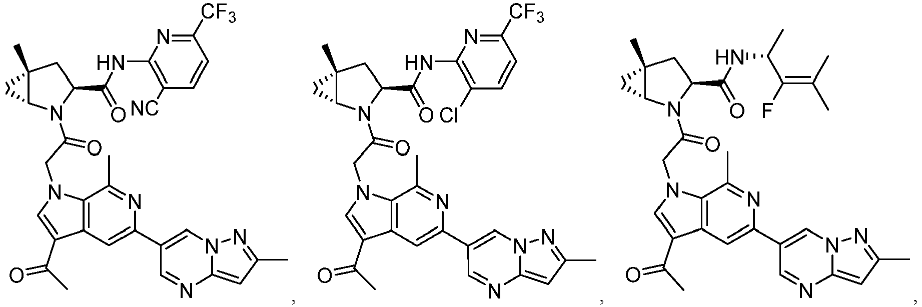 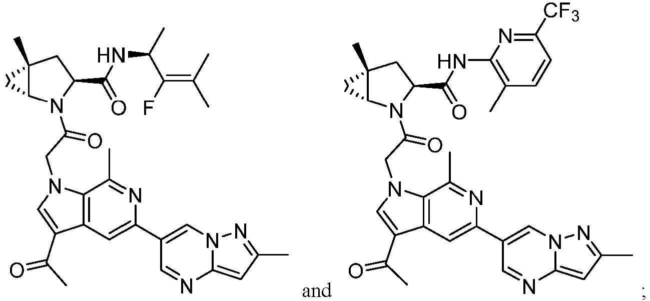 or a pharmaceutically acceptable salt thereof.

### Additional embodiments of the present disclosure

In one embodiment, R¹ is halogen.

In one embodiment, R¹ is bromine.

In one embodiment, R¹ is C₁-C₂haloalkyl.

In one embodiment, R¹ is -CF₃.

In one embodiment, R¹ is -CH₂F, -CHF₂, -CH₂CF₃, or -CF₂CF₃.

Any one of embodiments 1-5 wherein R² is hydrogen.

Any one of embodiments 1-5 wherein R² is halogen.

Any one of embodiments 1-5 wherein R² is C₁-C₃alkyl.

Any one of embodiments 1-5 wherein R² is C₁-C₃haloalkyl.

Any one of embodiments 1-5 wherein R² is -COOR⁹ or -CONR⁹R¹⁰.

Any one of embodiments 1-5 wherein R² is -C₀-C₄alkylNR⁹R¹⁰, -C₀-C₄alkylOR⁹, or C₁-C₆haloalkoxy.

Any one of embodiments 1-11 wherein R³ is C₁-C₃alkyl.

Any one of embodiments 1-11 wherein R³ is methyl.

Any one of embodiments 1-11 wherein R³ is ethyl, n-propyl, i-propyl, or cyclopropyl.

Any one of embodiments 1-11 wherein R³ is halogen.

Any one of embodiments 1-11 wherein R³ is chloro.

Any one of embodiments 1-11 wherein R³ is bromo.

Any one of embodiments 1-11 wherein R³ is C₁-C₃haloalkyl.

Any one of embodiments 1-11 wherein R³ is -CF₃.

Any one of embodiments 1-11 wherein R³ is -CH₂F, -CHF₂, -CH₂CF₃, or -CF₂CF₃.

Any one of embodiments 1-20 wherein R⁴ is hydrogen.

Any one of embodiments 1-20 wherein R⁴ is halogen.

Any one of embodiments 1-20 wherein R⁴ is C₁-C₃alkyl.

Any one of embodiments 1-20 wherein R⁴ is C₁-C₃haloalkyl.

Any one of embodiments 1-20 wherein R⁴ is -COOR⁹ or -CONR⁹R¹⁰.

Any one of embodiments 1-20 wherein R⁴ is -C₀-C₄alkylNR⁹R¹⁰, -C₀-C₄alkylOR⁹, or C₁-C₆haloalkoxy.

Any one of embodiments 1-26 wherein R⁵ is hydrogen.

Any one of embodiments 1-26 wherein R⁵ is halogen.

Any one of embodiments 1-26 wherein R⁵ is C₁-C₃alkyl.

Any one of embodiments 1-26 wherein R⁵ is C₁-C₃haloalkyl.

Any one of embodiments 1-26 wherein R⁵ is -COOR⁹ or -CONR⁹R¹⁰.

Any one of embodiments 1-26 wherein R⁵ is -C₀-C₄alkylNR⁹R¹⁰, -C₀-C₄alkylOR⁹, or C₁-C₆haloalkoxy.

Any one of embodiments 1-26 wherein R⁹ and R¹⁰ are both hydrogen.

Any one of embodiments 1-26 wherein one of R⁹ and R¹⁰ are hydrogen.

Any one of embodiments 1-26 wherein R⁹ and R¹⁰ are both C₁-C₄alkyl.

In certain embodiments R¹ is halogen.

In certain embodiments R¹ is bromine.

In certain embodiments R¹ is C₁-C₂haloalkyl.

In certain embodiments R¹ is -CF₃.

In certain embodiments R¹ is -CH₂F, -CHF₂, -CH₂CF₃, or -CF₂CF₃.

In certain embodiments R² is hydrogen.

In certain embodiments R² is halogen.

In certain embodiments R² is C₁-C₃alkyl.

In certain embodiments R² is C₁-C₃haloalkyl.

In certain embodiments R² is -COOR⁹ or -CONR⁹R¹⁰.

In certain embodiments R² is -C₀-C₄alkylNR⁹R¹⁰, -C₀-C₄alkylOR⁹, or C₁-C₆haloalkoxy.

In certain embodiments R³ is C₁-C₃alkyl.

In certain embodiments R³ is methyl.

In certain embodiments R³ is ethyl, n-propyl, i-propyl, or cyclopropyl.

In certain embodiments R³ is halogen.

In certain embodiments R³ is chloro.

In certain embodiments R³ is bromo.

In certain embodiments R³ is C₁-C₃haloalkyl.

In certain embodiments R³ is -CF₃.

In certain embodiments R³ is -CH₂F, -CHF₂, -CH₂CF₃, or -CF₂CF₃.

In certain embodiments R⁴ is hydrogen.

In certain embodiments R⁴ is halogen.

In certain embodiments R⁴ is C₁-C₃alkyl.

In certain embodiments R⁴ is methyl and R⁵ is hydrogen.

In certain embodiments R⁴ is C₁-C₃haloalkyl.

In certain embodiments R⁴ is -COOR⁹ or -CONR⁹R¹⁰.

In certain embodiments R⁴ is -C₀-C₄alkylNR⁹R¹⁰, -C₀-C₄alkylOR⁹, or C₁-C₆haloalkoxy.

In certain embodiments R⁵ is hydrogen.

In certain embodiments R⁵ is halogen.

In certain embodiments R⁵ is C₁-C₃alkyl.

In certain embodiments R⁵ is C₁-C₃haloalkyl.

In certain embodiments R⁵ is -COOR⁹ or -CONR⁹R¹⁰.

In certain embodiments R⁵ is -C₀-C₄alkylNR⁹R¹⁰, -C₀-C₄alkylOR⁹, or C₁-C₆haloalkoxy.

In certain embodiments R⁹ and R¹⁰ are both hydrogen.

In certain embodiments one of R⁹ and R¹⁰ are hydrogen.

In certain embodiments R⁹ and R¹⁰ are both C₁-C₄alkyl.

In certain non-limiting embodiments, compounds are provided that have minimal effect on BSEP (bile salt export pump protein) (e.g., with an IC₅₀ of greater than about 20, 30, 40, 50, 60, 75 or 100 µM or greater), or with a therapeutic index of BSEP relative to complement D inhibition (e.g., IC₅₀ inhibition of BSEP/IC₅₀ inhibition of complement D inhibitor), of about at least 50, 100, 200, 300, 400, 500, 750 or 1000 or greater). BSEP inhibition correlates with cholestatic drug-induced liver injury.

In some embodiments, the compound of the present disclosure exhibits reduced hydrolysis of the amide bond between the pyrrolidine and the pyridine *in vivo,* for example, by including a proline that has a cis-substituent relative to the proline-carbonyl bond directed toward the B-ring.

Additionally the R¹, R², and R³ substituents may decrease the potential for formation of reactive metabolites and/or increase potency.

### PHARMACEUTICAL PREPARATIONS

Active compounds described herein can be administered to a host in need thereof as the neat chemical, but are more typically administered as a pharmaceutical composition that includes an effective amount for a host, typically a human, in need of such treatment of an active compound as described herein or its pharmaceutically acceptable saltthereof. Thus, in one embodiment, the disclosure provides pharmaceutical compositions comprising an effective amount of compound or pharmaceutically acceptable saltthereof together with at least one pharmaceutically acceptable carrier for any of the uses described herein. The pharmaceutical composition may contain a compound or salt as the only active agent, or, in an alternative embodiment, the compound and at least one additional active agent.

An effective amount of an active compound as described herein, or the active compound described herein in combination or alternation with, or preceded by, concomitant with or followed by another active agent, can be used in an amount sufficient to (a) inhibit the progression of a disorder mediated by the complement pathway, including an inflammatory, immune, including an autoimmune, disorder or complement Factor D related disorder; (b) cause a regression of an inflammatory, immune, including an autoimmune, disorder or complement Factor D related disorder; (c) cause a cure of an inflammatory, immune, including an autoimmune, disorder or complement Factor D related disorder; or inhibit or prevent the development of an inflammatory, immune, including an autoimmune, disorder or complement Factor D related disorder. Accordingly, an effective amount of an active compound or its salt or composition described herein will provide a sufficient amount of the active agent when administered to a patient to provide a clinical benefit.

The exact amount of the active compound or pharmaceutical composition described herein to be delivered to the host, typically a human, in need thereof, will be determined by the health care provider to achieve the desired clinical benefit.

In certain embodiments the pharmaceutical composition is in a dosage form that contains from about 0.1 mg to about 2000 mg, from about 10 mg to about 1000 mg, from about 100 mg to about 800 mg, or from about 200 mg to about 600 mg of the active compound and optionally from about 0.1 mg to about 2000 mg, from about 10 mg to about 1000 mg, from about 100 mg to about 800 mg, or from about 200 mg to about 600 mg of an additional active agent in a unit dosage form. Examples are dosage forms with at least about 0.5, 1, 1.5, 2, 2.5, 3, 3.5, 4, 4.5, 5, 10, 15, 20, 25, 50, 75, 100, 150, 200, 250, 300, 350, 400, 450, 500, 550, 600, 650, 700, 750, 800, 900, 1000, 1100, 1200, 1250, 1300, 1400, 1500, or 1600 mg of active compound, or its salt. In one embodiment, the dosage form has at least about 1mg, 5 mg, 10 mg, 25 mg, 50 mg, 75 mg, 100 mg, 200 mg, 400 mg, 500 mg, 600 mg, 1000mg, 1200 mg, or 1600 mg of active compound, or its salt. The amount of active compound in the dosage form is calculated without reference to the salt. The dosage form can be administered, for example, once a day (q.d.), twice a day (b.i.d.), three times a day (t.i.d.), four times a day (q.i.d.), once every other day (Q2d), once every third day (Q3d), as needed, or any dosage schedule that provides treatment of a disorder described herein.

Compounds disclosed herein or used as described herein may be administered orally, topically, parenterally, by inhalation or spray, sublingually, via implant, including ocular implant, transdermally, via buccal administration, rectally, as an ophthalmic solution, injection, including ocular injection, intravenous, intra-aortal, intracranial, subdermal, intraperitoneal, subcutaneous, transnasal, sublingual, intrathecal, or rectal or by other means, in dosage unit formulations containing conventional pharmaceutically acceptable carriers. For ocular delivery, the compound can be administered, as desired, for example, as a solution, suspension, or other formulation via intravitreal, intrastromal, intracameral, sub-tenon, sub-retinal, retro-bulbar, peribulbar, suprachorodial, subchorodial, chorodial, conjunctival, subconjunctival, episcleral, periocular, transscleral, retrobulbar, posterior juxtascleral, circumcorneal, or tear duct injections, or through a mucus, mucin, or a mucosal barrier, in an immediate or controlled release fashion or via an ocular device, injection, or topically administered formulation, for example a solution or suspension provided as an eye drop.

The pharmaceutical composition may be formulated as any pharmaceutically useful form, e.g., as an aerosol, a cream, a gel, a gel cap, a pill, a microparticle, a nanoparticle, an injection or infusion solution, a capsule, a tablet, a syrup, a transdermal patch, a subcutaneous patch, a dry powder, an inhalation formulation, in a medical device, suppository, buccal, or sublingual formulation, parenteral formulation, or an ophthalmic solution or suspension. Some dosage forms, such as tablets and capsules, are subdivided into suitably sized unit doses containing appropriate quantities of the active components, e.g., an effective amount to achieve the desired purpose.

Pharmaceutical compositions, and methods of manufacturing such compositions, suitable for administration as contemplated herein are known in the art. Examples of known techniques include, for example, US Pat. Nos. 4,983,593, 5,013,557, 5,456,923, 5,576,025, 5,723,269, 5,858,411, 6,254,889, 6,303,148, 6,395,302, 6,497,903, 7,060,296, 7,078,057, 7,404,828, 8,202,912, 8,257,741, 8,263,128, 8,337,899, 8,431,159, 9,028,870, 9,060,938, 9,211,261, 9,265,731, 9,358,478, and 9,387,252.

The pharmaceutical compositions contemplated here can optionally include a carrier. Carriers must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound. Classes of carriers include, but are not limited to binders, buffering agents, coloring agents, diluents, disintegrants, emulsifiers, fillers, flavorants, glidents, lubricants, pH modifiers, preservatives, stabilizers, surfactants, solubilizers, tableting agents, and wetting agents. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin; talc, and vegetable oils. Examples of other matrix materials, fillers, or diluents include lactose, mannitol, xylitol, microcrystalline cellulose, calcium diphosphate, and starch. Examples of surface active agents include sodium lauryl sulfate and polysorbate 80. Examples of drug complexing agents or solubilizers include the polyethylene glycols, caffeine, xanthene, gentisic acid and cyclodextrins. Examples of disintegrants include sodium starch glycolate, sodium alginate, carboxymethyl cellulose sodium, methyl cellulose, colloidal silicon dioxide, and croscarmellose sodium. Examples of binders include methyl cellulose, microcrystalline cellulose, starch, and gums such as guar gum, and tragacanth. Examples of lubricants include magnesium stearate and calcium stearate. Examples of pH modifiers include acids such as citric acid, acetic acid, ascorbic acid, lactic acid, aspartic acid, succinic acid, phosphoric acid, and the like; bases such as sodium acetate, potassium acetate, calcium oxide, magnesium oxide, trisodium phosphate, sodium hydroxide, calcium hydroxide, aluminum hydroxide, and the like, and buffers generally comprising mixtures of acids and the salts of said acids. Optional other active agents may be included in a pharmaceutical composition, which do not substantially interfere with the activity of the compound of the present disclosure.

In certain embodiments, the pharmaceutical composition for administration further includes a compound as disclosed herein or a salt thereof and optionally comprises one or more of a phosphoglyceride; phosphatidylcholine; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohol such as polyethylene glycol (PEG); polyoxyethylene-9-lauryl ether; a surface active fatty acid, such as palmitic acid or oleic acid; fatty acid; fatty acid monoglyceride; fatty acid diglyceride; fatty acid amide; sorbitan trioleate (Span^{®}85) glycocholate; sorbitan monolaurate (Span^{®}20); polysorbate 20 (Tween^{®}20); polysorbate 60 (Tween^{®}60); polysorbate 65 (Tween^{®}65); polysorbate 80 (Tween^{®}80); polysorbate 85 (Tween^{®}85); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebroside; dicetylphosphate; dipalmitoylphosphatidylglycerol; stearylamine; dodecylamine; hexadecyl-amine; acetyl palmitate; glycerol ricinoleate; hexadecyl sterate; isopropyl myristate; tyloxapol; poly(ethylene glycol)5000-phosphatidylethanolamine; poly(ethylene glycol)400-monostearate; phospholipid; synthetic and/or natural detergent having high surfactant properties; deoxycholate; cyclodextrin; chaotropic salt; ion pairing agent; glucose, fructose, galactose, ribose, lactose, sucrose, maltose, trehalose, cellbiose, mannose, xylose, arabinose, glucoronic acid, galactoronic acid, mannuronic acid, glucosamine, galatosamine, and neuramic acid; pullulan, cellulose, microcrystalline cellulose, hydroxypropyl methylcellulose (HPMC), hydroxycellulose (HC), methylcellulose (MC), dextran, cyclodextran, glycogen, hydroxyethylstarch, carageenan, glycon, amylose, chitosan, N,O-carboxylmethylchitosan, algin and alginic acid, starch, chitin, inulin, konjac, glucommannan, pustulan, heparin, hyaluronic acid, curdlan, and xanthan, mannitol, sorbitol, xylitol, erythritol, maltitol, and lactitol, a pluronic polymer, polyethylene, polycarbonate (e.g. poly(1,3-dioxan-2one)), polyanhydride (e.g. poly(sebacic anhydride)), polypropylfumerate, polyamide (e.g. polycaprolactam), polyacetal, polyether, polyester (e.g., polylactide, polyglycolide, polylactide-co-glycolide, polycaprolactone, polyhydroxyacid (e.g. poly((β-hydroxyalkanoate))), poly(orthoester), polycyanoacrylate, polyvinyl alcohol, polyurethane, polyphosphazene, polyacrylate, polymethacrylate, polyurea, polystyrene, and polyamine, polylysine, polylysine-PEG copolymer, and poly(ethyleneimine), poly(ethylene imine)-PEG copolymer, glycerol monocaprylocaprate, propylene glycol, Vitamin E TPGS (also known as d-α-Tocopheryl polyethylene glycol 1000 succinate), gelatin, titanium dioxide, polyvinylpyrrolidone (PVP), hydroxypropyl methyl cellulose (HPMC), hydroxypropyl cellulose (HPC), methyl cellulose (MC), block copolymers of ethylene oxide and propylene oxide (PEO/PPO), polyethyleneglycol (PEG), sodium carboxymethylcellulose (NaCMC), hydroxypropylmethyl cellulose acetate succinate (HPMCAS).

In some embodiments, the pharmaceutical preparation may include polymers for controlled delivery of the described compounds, including, but not limited to pluronic polymers, polyesters (e.g., polylactic acid, poly(lactic-co-glycolic acid), polycaprolactone, polyvalerolactone, poly(1,3-dioxan-2one)); polyanhydrides (e.g., poly(sebacic anhydride)); polyethers (e.g., polyethylene glycol); polyurethanes; polymethacrylates; polyacrylates; and polycyanoacrylates. In some embodiments, polymers may be modified with polyethylene glycol (PEG), with a carbohydrate, and/or with acyclic polyacetals derived from polysaccharides. See, e.g., Papisov, 2001, ACS Symposium Series, 786:301.

The compounds of the present disclosure can be formulated as particles. In one embodiment, the particles are or include microparticles. In an alternative embodiment, the particles are or include nanoparticles.

In an additional alternative embodiment, common techniques for preparing particles include, but are not limited to, solvent evaporation, solvent removal, spray drying, phase inversion, coacervation, and low temperature casting. Suitable methods of particle formulation are briefly described below. Pharmaceutically acceptable excipients, including pH modifying agents, disintegrants, preservatives, and antioxidants, can optionally be incorporated into the particles during particle formation.

In one embodiment, the particles are derived through a solvent evaporation method. In this method, a compound described herein (or polymer matrix and one or more compounds described herein) is dissolved in a volatile organic solvent, such as methylene chloride. The organic solution containing a compound described herein is then suspended in an aqueous solution that contains a surface active agent such as poly(vinyl alcohol). The resulting emulsion is stirred until most of the organic solvent evaporated, leaving solid nanoparticles or microparticles. The resulting nanoparticles or microparticles are washed with water and dried overnight in a lyophilizer. Nanoparticles with different sizes and morphologies can be obtained by this method.

Pharmaceutical compositions which contain labile polymers, such as certain polyanhydrides, may degrade during the fabrication process due to the presence of water. For these polymers, methods which are performed in completely or substantially anhydrous organic solvents can be used to make the particles.

Solvent removal can also be used to prepare particles from a compound that is hydrolytically unstable. In this method, the compound (or polymer matrix and one or more compounds) is dispersed or dissolved in a volatile organic solvent such as methylene chloride. This mixture is then suspended by stirring in an organic oil (such as silicon oil) to form an emulsion. Solid particles form from the emulsion, which can subsequently be isolated from the supernatant. The external morphology of spheres produced with this technique is highly dependent on the identity of the drug.

In one embodiment, an active compound as described herein is administered to a patient in need thereof as particles formed by solvent removal. In another embodiment, the present disclosure provides particles formed by solvent removal comprising a compound of the present disclosure and one or more pharmaceutically acceptable excipients as defined herein. In another embodiment, the particles formed by solvent removal comprise a compound of the present disclosure and an additional therapeutic agent. In a further embodiment, the particles formed by solvent removal comprise a compound of the present disclosure, an additional therapeutic agent, and one or more pharmaceutically acceptable excipients. In another embodiment, any of the described particles formed by solvent removal can be formulated into a tablet and then coated to form a coated tablet. In an alternative embodiment, the particles formed by solvent removal are formulated into a tablet but the tablet is uncoated.

In one embodiment, the particles are derived by spray drying. In this method, a compound (or polymer matrix and one or more compounds) is dissolved in an organic solvent such as methylene chloride. The solution is pumped through a micronizing nozzle driven by a flow of compressed gas, and the resulting aerosol is suspended in a heated cyclone of air, allowing the solvent to evaporate from the micro droplets, forming particles. Microparticles and nanoparticles can be obtained using this method.

In one embodiment, an active compound as described herein is administered to a patient in need thereof as a spray dried dispersion (SDD). In another embodiment, the present disclosure provides a spray dried dispersion (SDD) comprising a compound of the present disclosure and one or more pharmaceutically acceptable excipients as defined herein. In another embodiment, the SDD comprises a compound of the present disclosure and an additional therapeutic agent. In a further embodiment, the SDD comprises a compound of the present disclosure, an additional therapeutic agent, and one or more pharmaceutically acceptable excipients. In another embodiment, any of the described spray dried dispersions can be coated to form a coated tablet. In an alternative embodiment, the spray dried dispersion is formulated into a tablet but is uncoated.

Particles can be formed from the active compound as described herein using a phase inversion method. In this method, the compound (or polymer matrix and one or more active compounds) is dissolved in a suitable solvent, and the solution is poured into a strong non-solvent for the compound to spontaneously produce, under favorable conditions, microparticles or nanoparticles. The method can be used to produce nanoparticles in a wide range of sizes, including, for example, from nanoparticles to microparticles, typically possessing a narrow particle size distribution.

In one embodiment, an active compound as described herein is administered to a patient in need thereof as particles formed by phase inversion. In another embodiment, the present disclosure provides particles formed by phase inversion comprising a compound of the present disclosure and one or more pharmaceutically acceptable excipients as defined herein. In another embodiment, the particles formed by phase inversion comprise a compound of the present disclosure and an additional therapeutic agent. In a further embodiment, the particles formed by phase inversion comprise a compound of the present disclosure, an additional therapeutic agent, and one or more pharmaceutically acceptable excipients. In another embodiment, any of the described particles formed by phase inversion can be formulated into a tablet and then coated to form a coated tablet. In an alternative embodiment, the particles formed by phase inversion are formulated into a tablet but the tablet is uncoated.

Techniques for particle formation using coacervation are known in the art, for example, as described in GB-B-929 406; GB-B-929 40 1; and U.S. Patent Nos. 3,266,987, 4,794,000, and 4,460,563. Coacervation involves the separation of a compound (or polymer matrix and one or more compounds) solution into two immiscible liquid phases. One phase is a dense coacervate phase, which contains a high concentration of the compound, while the second phase contains a low concentration of the compound. Within the dense coacervate phase, the compound forms nanoscale or microscale droplets, which harden into particles. Coacervation may be induced by a temperature change, addition of a non-solvent or addition of a micro-salt (simple coacervation), or by the addition of another polymer thereby forming an interpolymer complex (complex coacervation).

In one embodiment, an active compound as described herein is administered to a patient in need thereof as particles formed by coacervation. In another embodiment, the present disclosure provides particles formed by coacervation comprising a compound of the present disclosure and one or more pharmaceutically acceptable excipients as defined herein. In another embodiment, the particles formed by coacervation comprise a compound of the present disclosure and an additional therapeutic agent. In a further embodiment, the particles formed by coacervation comprise a compound of the present disclosure, an additional therapeutic agent, and one or more pharmaceutically acceptable excipients. In another embodiment, any of the described particles formed by coacervation can be formulated into a tablet and then coated to form a coated tablet. In an alternative embodiment, the particles formed by coacervation are formulated into a tablet but the tablet is uncoated.

Methods for very low temperature casting of controlled release microspheres are described in U.S. Patent No. 5,019,400 to Gombotz et al. In this method, the compound is dissolved in a solvent. The mixture is then atomized into a vessel containing a liquid non-solvent at a temperature below the freezing point of the drug solution which freezes the compound droplets. As the droplets and non-solvent for the compound are warmed, the solvent in the droplets thaws and is extracted into the non-solvent, hardening the microspheres.

In one embodiment, a compound of the present disclosure is administered to a patient in need thereof as particles formed by low temperature casting. In another embodiment, the present disclosure provides particles formed by low temperature casting comprising a compound of the present disclosure and one or more pharmaceutically acceptable excipients as defined herein. In another embodiment, the particles formed by low temperature casting comprise a compound of the present disclosure and an additional therapeutic agent. In a further embodiment, the particles formed by low temperature casting comprise a compound of the present disclosure, an additional therapeutic agent, and one or more pharmaceutically acceptable excipients. In another embodiment, any of the described particles formed by low temperature casting can be formulated into a tablet and then coated to form a coated tablet. In an alternative embodiment, the particles formed by low temperature casting are formulated into a tablet but the tablet is uncoated.

In one aspect of the present disclosure, an effective amount of an active compound as described herein is incorporated into a nanoparticle, e.g. for convenience of delivery and/or extended release delivery. The use of materials in nanoscale provides one the ability to modify fundamental physical properties such as solubility, diffusivity, blood circulation half-life, drug release characteristics, and/or immunogenicity. A number of nanoparticle-based therapeutic and diagnostic agents have been developed for the treatment of cancer, diabetes, pain, asthma, allergy, and infections. These nanoscale agents may provide more effective and/or more convenient routes of administration, lower therapeutic toxicity, extend the product life cycle, and ultimately reduce health-care costs. As therapeutic delivery systems, nanoparticles can allow targeted delivery and controlled release.

In addition, nanoparticle-based compound delivery can be used to release compounds at a sustained rate and thus lower the frequency of administration, deliver drugs in a targeted manner to minimize systemic side effects, or deliver two or more drugs simultaneously for combination therapy to generate a synergistic effect and suppress drug resistance. A number of nanotechnology-based therapeutic products have been approved for clinical use. Among these products, liposomal drugs and polymer-based conjugates account for a large proportion of the products. *See,* Zhang, L., et al., Nanoparticles in Medicine: Therapeutic Applications and Developments, Clin. Pharm. and Ther., 83(5):761-769, 2008.

Methods for producing nanoparticles are known in the art. For example, see Muller, R.H., et al., Solid lipid nanoparticles (SLN) for controlled drug delivery - a review of the state of the art, Eur. H. Pharm. Biopharm., 50:161-177, 2000; US Pat. No. 8,691,750 to Consien et al.; WO 2012/145801 to Kanwar. US Pat. No. 8,580,311 to Armes, S. et al.; Petros, R.A. and DeSimone, J.M., Strategies in the design of nanoparticles for therapeutic applications, Nature Reviews/Drug Discovery, vol. 9:615-627, 2010; US Pat. No. 8,465,775; US Pat. No. 8,444,899; US Pat. No. 8,420,124; US Pat. No. 8,263,129; US Pat. No. 8,158,728; 8,268,446; Pellegrino et al., 2005, Small, 1:48; Murray et al., 2000, Ann. Rev. Mat. Sci., 30:545; and Trindade et al., 2001, Chem. Mat., 13:3843. Additional methods have been described in the literature (see, e.g., Doubrow, Ed., "Microcapsules and Nanoparticles in Medicine and Pharmacy," CRC Press, Boca Raton, 1992; Mathiowitz et al., 1987, J. Control. Release, 5:13; Mathiowitz et al., 1987, Reactive Polymers, 6:275; and Mathiowitz et al., 1988, J. Appl. Polymer Sci., 35:755; U.S. Pat. Nos. 5,578,325 and 6,007,845; P. Paolicelli et al., "Surface-modified PLGA-based Nanoparticles that can Efficiently Associate and Deliver Virus-like Particles" Nanomedicine, 5(6):843-853 (2010)), U.S. Pat. No. 5,543,158 to Gref et al., or WO publication WO2009/051837 by Von Andrian et al. Zauner et al., 1998, Adv. Drug Del. Rev., 30:97; and Kabanov et al., 1995, Bioconjugate Chem., 6:7;(PEI; Boussif et al., 1995, Proc. Natl. Acad. Sci., USA, 1995, 92:7297), and poly(amidoamine) dendrimers (Kukowska-Latallo et al., 1996, Proc. Natl. Acad. Sci., USA, 93:4897; Tang et al., 1996, Bioconjugate Chem., 7:703; and Haensler et al., 1993, Bioconjugate Chem., 4:372; Putnam et al., 1999, Macromolecules, 32:3658; Barrera et al., 1993, J. Am. Chem. Soc., 115:11010; Kwon et al., 1989, Macromolecules, 22:3250; Lim et al., 1999, J. Am. Chem. Soc., 121:5633; and Zhou et al., 1990, Macromolecules, 23:3399). Examples of these polyesters include poly(L-lactide-co-L-lysine) (Barrera et al., 1993, J. Am. Chem. Soc., 115:11010), poly(serine ester) (Zhou et al., 1990, Macromolecules, 23:3399), poly(4-hydroxy-L-proline ester) (Putnam et al., 1999, Macromolecules, 32:3658; and Lim et al., 1999, J. Am. Chem. Soc., 121:5633), and poly(4-hydroxy-L-proline ester) (Putnam et al., 1999, Macromolecules, 32:3658; and Lim et al., 1999, J. Am. Chem. Soc., 121:5633; U.S. Pat. No. 6,123,727; U.S. Pat. No. 5,804,178; U.S. Pat. No. 5,770,417; U.S. Pat. No. 5,736,372; U.S. Pat. No. 5,716,404; U.S. Pat. No. 6,095,148; U.S. Pat. No. 5,837,752; U.S. Pat. No. 5,902,599; U.S. Pat. No. 5,696,175; U.S. Pat. No. 5,514,378; U.S. Pat. No. 5,512,600; U.S. Pat. No. 5,399,665; U.S. Pat. No. 5,019,379; U.S. Pat. No. 5,010,167; U.S. Pat. No. 4,806,621; U.S. Pat. No. 4,638,045; and U.S. Pat. No. 4,946,929; Wang et al., 2001, J. Am. Chem. Soc., 123:9480; Lim et al., 2001, J. Am. Chem. Soc., 123:2460; Langer, 2000, Acc. Chem. Res., 33:94; Langer, 1999, J. Control. Release, 62:7; and Uhrich et al., 1999, Chem. Rev., 99:3181; Concise Encyclopedia of Polymer Science and Polymeric Amines and Ammonium Salts, Ed. by Goethals, Pergamon Press, 1980; Principles of Polymerization by Odian, John Wiley & Sons, Fourth Edition, 2004; Contemporary Polymer Chemistry by Allcock et al., Prentice-Hall, 1981; Deming et al., 1997, Nature, 390:386; and in U.S. Pat. Nos. 6,506,577, 6,632,922, 6,686,446, and 6,818,732; C. Astete et al., "Synthesis and characterization of PLGA nanoparticles" J. Biomater. Sci. Polymer Edn, Vol. 17, No. 3, pp. 247-289 (2006); K. Avgoustakis "Pegylated Poly(Lactide) and Poly(Lactide-Co-Glycolide) Nanoparticles: Preparation, Properties and Possible Applications in Drug Delivery" Current Drug Delivery 1:321-333 (2004); C. Reis et al., "Nanoencapsulation I. Methods for preparation of drug-loaded polymeric nanoparticles" Nanomedicine 2:8-21 (2006); P. Paolicelli et al., "Surface-modified PLGA-based Nanoparticles that can Efficiently Associate and Deliver Virus-like Particles" Nanomedicine, 5(6):843-853 (2010); U.S. Pat. No. 6,632,671 to Unger Oct. 14, 2003.

In one embodiment, the polymeric particle is between about 0.1 nm to about 10000 nm, between about 1 nm to about 1000 nm, between about 10 nm and 1000 nm, between about 1 and 100 nm, between about 1 and 10 nm, between about 1 and 50 nm, between about 100 nm and 800 nm, between about 400 nm and 600 nm, or about 500 nm. In one embodiment, the micro-particles are no more than about 0.1 nm, 0.5 nm, 1.0 nm, 5.0 nm, 10 nm, 25 nm, 50 nm, 75 nm, 100 nm, 150 nm, 200 nm, 250 nm, 300 nm, 400 nm, 450 nm, 500 nm, 550 nm, 600 nm, 650 nm, 700 nm, 750 nm, 800 nm, 850 nm, 900 nm, 950 nm, 1000 nm, 1250 nm, 1500 nm, 1750 nm, or 2000 nm. In some embodiments, a compound described herein may be covalently coupled to a polymer used in the nanoparticle, for example a polystyrene particle, PLGA particle, PLA particle, or other nanoparticle.

The pharmaceutical compositions can be formulated for oral administration. These compositions can contain any amount of active compound that achieves the desired result, for example, between 0.1 and 99 weight % (wt.%) of the compound and usually at least about 5 wt.% of the compound. Some embodiments contain at least about 10%, 15%, 20%, 25 wt.% to about 50 wt. % or from about 5 wt.% to about 75 wt.% of the compound.

Pharmaceutical compositions suitable for rectal administration are typically presented as unit dose suppositories. These may be prepared by admixing the active compound with one or more conventional solid carriers, for example, cocoa butter, and then shaping the resulting mixture.

Pharmaceutical compositions suitable for topical application to the skin preferably take the form of an ointment, cream, lotion, paste, gel, spray, aerosol, or oil. Carriers which may be used include petroleum jelly, lanoline, polyethylene glycols, alcohols, transdermal enhancers, and combinations of two or more thereof.

Pharmaceutical compositions suitable for transdermal administration may be presented as discrete patches adapted to remain in intimate contact with the epidermis of the recipient for a prolonged period of time. Pharmaceutical compositions suitable for transdermal administration may also be delivered by iontophoresis (*see,* for example, Pharmaceutical Research 3 (6):318 (1986)) and typically take the form of an optionally buffered aqueous solution of the active compound. In one embodiment, microneedle patches or devices are provided for delivery of drugs across or into biological tissue, particularly the skin. The microneedle patches or devices permit drug delivery at clinically relevant rates across or into skin or other tissue barriers, with minimal or no damage, pain, or irritation to the tissue.

Pharmaceutical compositions suitable for administration to the lungs can be delivered by a wide range of passive breath driven and active power driven single/-multiple dose dry powder inhalers (DPI). The devices most commonly used for respiratory delivery include nebulizers, metered-dose inhalers, and dry powder inhalers. Several types of nebulizers are available, including jet nebulizers, ultrasonic nebulizers, and vibrating mesh nebulizers. Selection of a suitable lung delivery device depends on parameters, such as nature of the drug and its formulation, the site of action, and pathophysiology of the lung.

Additional non-limiting examples of inhalation drug delivery devices and methods include, for example, US Pat. No. 7,383,837 titled "Inhalation device" (SmithKline Beecham Corporation); WO/2006/033584 titled "Powder inhaler" (Glaxo SmithKline Pharmaceuticals SA); WO/2005/044186 titled "Inhalable pharmaceutical formulations employing desiccating agents and methods of administering the same" (Glaxo Group Ltd and SmithKline Beecham Corporation); US9,095,670 titled "Inhalation device and method of dispensing medicament", US Pat. No. 8,205,611 titled "Dry powder inhaler" (AstraZeneca AB); WO/2013/038170 titled "Inhaler" (AstraZeneca AB and AstraZeneca UK Ltd.); US/2014/0352690 titled "Inhalation Device with Feedback System", US Pat. No. 8,910,625 and US/2015/0165137 titled "Inhalation Device for Use in Aerosol Therapy" (Vectura GmbH); US Pat. No. 6,948,496 titled "Inhalers", US/2005/0152849 titled "Powders comprising anti-adherent materials for use in dry powder inhalers", US Pat. No. 6,582,678, US Pat. No. 8,137,657, US/2003/0202944, and US/2010/0330188 titled "Carrier particles for use in dry powder inhalers", US Pat. No. 6,221,338 titled "Method of producing particles for use in dry powder inhalers", US Pat. No. 6,989,155 titled "Powders", US/2007/0043030 titled "Pharmaceutical compositions for treating premature ejaculation by pulmonary inhalation", US Pat. No. 7,845,349 titled "Inhaler", US/2012/0114709 and US Pat. No. 8,101,160 titled "Formulations for Use in Inhaler Devices", US/2013/0287854 titled "Compositions and Uses", US/2014/0037737 and US Pat. No. 8,580,306 titled "Particles for Use in a Pharmaceutical Composition", US/2015/0174343 titled "Mixing Channel for an Inhalation Device", US Pat. No. 7,744,855 and US/2010/0285142 titled "Method of making particles for use in a pharmaceutical composition", US Pat. No. 7,541,022, US/2009/0269412, and US/2015/0050350 titled "Pharmaceutical formulations for dry powder inhalers" (Vectura Limited).

Many methods and devices for drug delivery to the eye are known in the art. Non-limiting examples are described in the following patents and patent applications. Examples are US Pat. No. 8,192,408 titled "Ocular trocar assembly" (Psivida Us, Inc.); US Pat. No. 7,585,517 titled "Transcleral delivery" (Macusight, Inc.); US Pat. No. 5,710,182 and US Pat. No. 5,795,913 titled "Ophthalmic composition" (Santen OY); US Pat. No. 8,663,639 titled "Formulations for treating ocular diseases and conditions", US Pat. No. 8,486,960 titled "Formulations and methods for vascular permeability-related diseases or conditions", US Pat. No. 8,367,097 and US Pat. No. 8,927,005 titled "Liquid formulations for treatment of diseases or conditions", US Pat. No. 7,455,855 titled "Delivering substance and drug delivery system using the same" (Santen Pharmaceutical Co., Ltd.); WO/2011/050365 titled "Conformable Therapeutic Shield For Vision and Pain" and WO/2009/145842 titled "Therapeutic Device for Pain Management and Vision" (Forsight Labs, LLC); US Pat. No. 9,066,779 and US Pat. No. 8,623,395 titled "Implantable therapeutic device", WO/2014/160884 titled "Ophthalmic Implant for Delivering Therapeutic Substances", US Pat. No. 8,399,006, US Pat. No. 8,277,830, US Pat. No. 8,795,712, US Pat. No. 8,808,727, US Pat. No. 8,298,578, and WO/2010/088548 titled "Posterior segment drug delivery", WO/2014/152959 and US20140276482 titled "Systems for Sustained Intraocular Delivery of Low Solubility Compounds from a Port Delivery System Implant", US Pat. No. 8,905,963 and US Pat. No. 9,033,911 titled "Injector apparatus and method for drug delivery", WO/2015/057554 titled "Formulations and Methods for Increasing or Reducing Mucus", US Pat. No. 8,715,712 and US Pat. No. 8,939,948 titled "Ocular insert apparatus and methods", WO/2013/116061 titled "Insertion and Removal Methods and Apparatus for Therapeutic Devices", WO/2014/066775 titled "Ophthalmic System for Sustained Release of Drug to the Eye", WO/2015/085234 and WO/2012/019176 titled "Implantable Therapeutic Device", WO/2012/065006 titled "Methods and Apparatus to determine Porous Structures for Drug Delivery", WO/2010/141729 titled "Anterior Segment Drug Delivery", WO/2011/050327 titled "Corneal Denervation for Treatment of Ocular Pain", WO/2013/022801 titled "Small Molecule Delivery with Implantable Therapeutic Device", WO/2012/019047 titled "Subconjunctival Implant for Posterior Segment Drug Delivery", WO/2012/068549 titled "Therapeutic Agent Formulations for Implanted Devices", WO/2012/019139 titled " Combined Delivery Methods and Apparatus", WO/2013/040426 titled "Ocular Insert Apparatus and Methods", WO/2012/019136 titled "Injector Apparatus and Method for Drug Delivery", WO/2013/040247 titled "Fluid Exchange Apparatus and Methods" (ForSight Vision4, Inc.).

Additional non-limiting examples of how to deliver the active compounds are provided in WO/2015/085251 titled "Intracameral Implant for Treatment of an Ocular Condition" (Envisia Therapeutics, Inc.); WO/2011/008737 titled "Engineered Aerosol Particles, and Associated Methods", WO/2013/082111 titled "Geometrically Engineered Particles and Methods for Modulating Macrophage or Immune Responses", WO/2009/132265 titled "Degradable compounds and methods of use thereof, particularly with particle replication in non-wetting templates", WO/2010/099321 titled "Interventional drug delivery system and associated methods", WO/2008/100304 titled "Polymer particle composite having high fidelity order, size, and shape particles", WO/2007/024323 titled "Nanoparticle fabrication methods, systems, and materials" (Liquidia Technologies, Inc. and the University of North Carolina at Chapel Hill); WO/2010/009087 titled "Iontophoretic Delivery of a Controlled-Release Formulation in the Eye", (Liquidia Technologies, Inc. and Eyegate Pharmaceuticals, Inc.) and WO/2009/132206 titled "Compositions and Methods for Intracellular Delivery and Release of Cargo", WO/2007/133808 titled "Nano-particles for cosmetic applications", WO/2007/056561 titled "Medical device, materials, and methods", WO/2010/065748 titled "Method for producing patterned materials", WO/2007/081876 titled "Nanostructured surfaces for biomedical/biomaterial applications and processes thereof" (Liquidia Technologies, Inc.).

Additional non-limiting examples of methods and devices for drug delivery to the eye include, for example, WO2011/106702 and US Pat. No. 8,889,193 titled "Sustained delivery of therapeutic agents to an eye compartment", WO2013/138343 and US Pat. No. 8,962,577 titled "Controlled release formulations for the delivery of HIF-1 inhibitors", WO/2013/138346 and US2013/0272994 titled "Non-Linear Multiblock Copolymer-Drug Conjugates for the Delivery of Active Agents", WO2005/072710 and US Pat. No. 8,957,034 titled "Drug and Gene Carrier Particles that Rapidly Move Through Mucus Barriers", WO2008/030557, US2010/0215580, US2013/0164343 titled "Compositions and Methods for Enhancing Transport Through Mucous", WO2012/061703, US2012/0121718, and US2013/0236556 titled "Compositions and Methods Relating to Reduced Mucoadhesion", WO2012/039979 and US2013/0183244 titled "Rapid Diffusion of Large Polymeric Nanoparticles in the Mammalian Brain", WO2012/109363 and US2013/0323313 titled "Mucus Penetrating Gene Carriers", WO 2013/090804 and US2014/0329913 titled "Nanoparticles with enhanced mucosal penetration or decreased inflammation", WO2013/110028 titled "Nanoparticle formulations with enhanced mucosal penetration", WO2013/166498 and US2015/0086484 titled "Lipid-based drug carriers for rapid penetration through mucus linings" (The Johns Hopkins University); WO2013/166385 titled "Pharmaceutical Nanoparticles Showing Improved Mucosal Transport", US2013/0323179 titled "Nanocrystals, Compositions, And Methods that Aid Particle Transport in Mucus" (The Johns Hopkins University and Kala Pharmaceuticals, Inc.); WO/2015/066444 titled "Compositions and methods for ophthalmic and/or other applications", WO/2014/020210 and WO/2013/166408 titled "Pharmaceutical nanoparticles showing improved mucosal transport" (Kala Pharmaceuticals, Inc.); US Pat. No. 9,022,970 titled "Ophthalmic injection device including dosage control device", WO/2011/153349 titled "Ophthalmic compositions comprising pbo-peo-pbo block copolymers", WO/2011/140203 titled "Stabilized ophthalmic galactomannan formulations", WO/2011/068955 titled "Ophthalmic emulsion", WO/2011/037908 titled "Injectable aqueous ophthalmic composition and method of use therefor", US2007/0149593 titled "Pharmaceutical Formulation for Delivery of Receptor Tyrosine Kinase Inhibiting (RTKi) Compounds to the Eye", US Pat. No. 8,632,809 titled "Water insoluble polymer matrix for drug delivery" (Alcon, Inc.).

Additional non-limiting examples of drug delivery devices and methods include, for example, US2009/0203709 titled "Pharmaceutical Dosage Form For Oral Administration Of Tyrosine Kinase Inhibitor" (Abbott Laboratories); US2005/0009910 titled "Delivery of an active drug to the posterior part of the eye via subconjunctival or periocular delivery of a prodrug", US2013/0071349 titled "Biodegradable polymers for lowering intraocular pressure", US Pat. No. 8,481,069 titled "Tyrosine kinase microspheres", US Pat. No. 8,465,778 titled "Method of making tyrosine kinase microspheres", US Pat. No. 8,409,607 titled "Sustained release intraocular implants containing tyrosine kinase inhibitors and related methods", US Pat. No. 8,512,738 and US Pat. No. 2014/0031408 titled "Biodegradable intravitreal tyrosine kinase implants", US2014/0294986 titled "Microsphere Drug Delivery System for Sustained Intraocular Release", US Pat. No. 8,911,768 titled "Methods For Treating Retinopathy With Extended Therapeutic Effect" (Allergan, Inc.); US Pat. No. 6,495,164 titled "Preparation of injectable suspensions having improved injectability" (Alkermes Controlled Therapeutics, Inc.); WO 2014/047439 titled "Biodegradable Microcapsules Containing Filling Material" (Akina, Inc.); WO 2010/132664 titled "Compositions And Methods For Drug Delivery" (Baxter); US2012/0052041 titled "Polymeric nanoparticles with enhanced drug-loading and methods of use thereof" (The Brigham and Women's Hospital, Inc.); US2014/0178475, US2014/0248358, and US2014/0249158 titled "Therapeutic Nanoparticles Comprising a Therapeutic Agent and Methods of Making and Using Same" (BIND Therapeutics, Inc.); US Pat. No. 5,869,103 titled "Polymer microparticles for drug delivery" (Danbiosyst UK Ltd.); US Pat. No. 8,628,801 titled "Pegylated Nanoparticles" (Universidad de Navarra); US2014/0107025 titled "Ocular drug delivery system" (Jade Therapeutics, LLC); US Pat. No. 6,287,588 titled "Agent delivering system comprised of microparticle and biodegradable gel with an improved releasing profile and methods of use thereof", US Pat. No. 6,589,549 titled "Bioactive agent delivering system comprised of microparticles within a biodegradable to improve release profiles" (Macromed, Inc.); US Pat. No. 6,007,845 and US Pat. No. 5,578,325 titled "Nanoparticles and microparticles of non-linear hydrophilic-hydrophobic multiblock copolymers" (Massachusetts Institute of Technology); US2004/0234611, US2008/0305172, US2012/0269894, and US2013/0122064 titled "Ophthalmic depot formulations for periocular or subconjunctival administration (Novartis Ag); US Pat. No. 6,413,539 titled "Block polymer" (Poly-Med, Inc.); US2007/0071756 titled "Delivery of an agent to ameliorate inflammation" (Peyman); US2008/0166411 titled "Injectable Depot Formulations And Methods For Providing Sustained Release Of Poorly Soluble Drugs Comprising Nanoparticles" (Pfizer, Inc.); US Pat. No. 6,706,289 titled "Methods and compositions for enhanced delivery of bioactive molecules" (PR Pharmaceuticals, Inc.); and US Pat. No. 8,663,674 titled "Microparticle containing matrices for drug delivery" (Surmodics).

### USES OF ACTIVE COMPOUNDS FOR TREATMENT OF SELECTED DISORDERS

In one aspect, an effective amount of an active compound or its salt or composition as described herein is used to treat a medical disorder which is an inflammatory or immune condition, a disorder mediated by the complement cascade (including a dysfunctional cascade) including a complement-mediated disease or disorder including a complement factor D-related disorder or alternative complement pathway-related disorder, a disorder or abnormality of a cell that adversely affects the ability of the cell to engage in or respond to normal complement activity, or an undesired complement-mediated response to a medical treatment, such as surgery or other medical procedure or a pharmaceutical or biopharmaceutical drug administration, a blood transfusion, or other allogenic tissue or fluid administration.

A complement-mediated disease or disorder is a disease or disorder in which the amount or activity of complement is such as to cause disease or disorder in an individual. In some embodiments, the complement-mediated disease or disorder is selected from the group consisting of autoimmune disease, cancer, hematological disease, infectious disease, inflammatory disease, ischemia-reperfusion injury, neurodegenerative disease, neurodegenerative disorder, ocular disease, renal disease, transplant rejection, vascular disease, and vasculitis disease. In some embodiments, the complement-mediated disease or disorder is an autoimmune disease. In some embodiments, the complement-mediated disease or disorder is cancer. In some embodiments, the complement-mediated disease or disorder is an infectious disease. In some embodiments, the complement-mediated disease or disorder is an inflammatory disease. In some embodiments, the complement-mediated disease or disorder is a hematological disease. In some embodiments, the complement-mediated disease or disorder is an ischemia-reperfusion injury. In some embodiments, the complement-mediated disease or disorder is ocular disease. In some embodiments, the complement-mediated disease or disorder is a renal disease. In some embodiments, the complement-mediated disease or disorder is transplant rejection. In some embodiments, the complement-mediated disease or disorder is antibody-mediated transplant rejection. In some embodiments, the complement-mediated disease or disorder is a vascular disease. In some embodiments, the complement-mediated disease or disorder is a vasculitis disorder. In some embodiments, the complement-mediated disease or disorder is a neurodegenerative disease or disorder. In some embodiments, the complement-mediated disease is a neurodegenerative disease. In some embodiments, the complement-mediated disorder is a neurodegenerative disorder. In some embodiments, the complement-mediated disease or disorder is a tauopathy.

In some embodiments, a compound as disclosed herein, or a pharmaceutically acceptable salt thereof, optionally in a pharmaceutically acceptable composition, is provided for use in a method for the treatment of C3 glomerulonephritis (C3G), the method comprising the administration of an effective amount of the compound, salt or composition to a host . In some embodiments, a compound as disclosed herein, or a pharmaceutically acceptable salt thereof, optionally in a pharmaceutically acceptable composition, is provided for use in a method for the treatment of paroxysmal nocturnal hemoglobinuria (PNH), the methodcomprising the administration of an effective amount of the compound, salt or composition. In another embodiment, a compound as disclosed herein, or a pharmaceutically acceptable salt thereof, optionally in a pharmaceutically acceptable composition, is provided for use in a method for the treatment of wet or dry age-related macular degeneration (AMD) in a host, the method comprising the administration of an effective amount of the compound, salt or composition. In another embodiment, a compound as disclosed herein, or a pharmaceutically acceptable salt thereof, optionally in a pharmaceutically acceptable composition, is provided for use in a method for the treatment of rheumatoid arthritis in a host, the method comprising the administration of an effective amount of the compound, salt or composition. In another embodiment, a compound as disclosed herein, or a pharmaceutically acceptable salt thereof, optionally in a pharmaceutically acceptable composition, is provided for use in a method for the treatment of multiple sclerosis in a host , the method comprising the administration of an effective amount of the compound, salt or composition.

The active compound or its pharmaceutically acceptable saltthereof, optionally in a pharmaceutically acceptable composition, as disclosed herein is also useful for administration in combination (in the same or a different dosage form) or alternation with a second pharmaceutical agent for use in ameliorating or reducing a side effect of the second pharmaceutical agent. For example, in some embodiments, the active compound may be used in combination with an adoptive cell transfer therapy to reduce an inflammatory response associated with such therapy, for example, a cytokine mediated response such as cytokine response syndrome. In some embodiments, the adoptive cell transfer therapy is a chimeric antigen receptor T-Cell (CAR T) or a dendritic cell used to treat a hematologic or solid tumor, for example, a B-cell related hematologic cancer. In some embodiments, the hematologic or solid tumor is acute lymphoblastic leukemia (ALL), acute myeloid leukemia (AML), non-Hodgkin's lymphoma, chronic lymphocytic leukemia (CLL), pancreatic cancer, glioblastoma, or a cancer that expresses CD19. In some embodiments, the associated inflammatory response is a cytokine mediated response.

One embodiment includes the administration of an effective amount of an active compound or a pharmaceutically acceptable salt thereof, optionally in a pharmaceutically acceptable composition to a host to treat an ocular, pulmonary, gastrointestinal, or other disorder that can benefit from topical or local delivery.

Any of the compounds described herein can be administered to the eye in any desired form of administration, including via intravitreal, intrastromal, intracameral, sub-tenon, sub-retinal, retro-bulbar, peribulbar, suprachorodial, choroidal, subchoroidal, conjunctival, subconjunctival, episcleral, posterior juxtascleral, scleral, circumcorneal, and tear duct injections, or through a mucus, mucin, or a mucosal barrier, in an immediate or controlled release fashion. In certain embodiments, the active compound includes a lipophilic group, such as a lipophilic acyl group, which is delivered to the eye in a polymeric drug delivery system such as polylactic acid, polylactide-co-glycolide, polyglycolide or other erodible polymer, or a combination thereof, or in another type of lipophilic material for ocular delivery. In some embodiments, the lipophilic active molecule is more soluble in the polymeric or other form of delivery system than in ocular fluid.

In other embodiments of the disclosure, an active compound provided herein can be used to treat or prevent a disorder in a host mediated by complement factor D, or by an excessive or detrimental amount of the complement-C3 amplification loop of the complement pathway. As examples, the disclosure includes methods to treat or prevent complement associated disorders that are induced by antibody-antigen interactions, a component of an immune or autoimmune disorder or by ischemic injury. The disclosure also provides methods to decrease inflammation or an immune response, including an autoimmune response, where mediated or affected by factor D.

In some embodiments, the disorder is selected from fatty liver and conditions stemming from fatty liver, such as nonalcoholic steatohepatitis (NASH), liver inflammation, cirrhosis and liver failure. In some embodiments of the present disclosure, an active compound or its salt or composition as described herein is provided for use in a method for treating fatty liver disease in a host, the method comprising administering an effective amount of the active compound or its salt or composition as described herein.

In another embodiment, an active compound or its salt or composition as described herein is used to modulate an immune response prior to or during surgery or other medical procedure. One non-limiting example is use in connection with acute or chronic graft versus host disease, which is a common complication as a result of allogeneic tissue transplant, and can also occur as a result of a blood transfusion.

In some embodiments, the present disclosure provides an active compound or its salt or composition as described herein for use in a method of treating or preventing dermatomyositis, the method comprisingadministering to a subject in need thereof an effective amount of the active compound or its salt or composition as described herein.

In some embodiments, the present disclosure provides an active compound or its salt or composition as described herein for use in a method of treating or preventing amyotrophic lateral sclerosis, the method comprising administering to a subject in need thereof an effective amount of the active compound or its salt or composition as described herein.

In some embodiments, the present disclosure provides an active compound or its salt or composition as described herein for use in a method of treating or preventing abdominal aortic aneurysm, hemodialysis complications, hemolytic anemia, or hemodialysis, the method comprising administering to a subject in need thereof an effective amount of the active compound or its salt or composition as described herein.

In one embodiment, an active compound or its salt or composition as described herein is provided for use in a method for the treatment or prevention of cytokine or inflammatory reactions in response to the administration of pharmaceutical or biotherapeutic (e.g. CAR T-cell therapy or monoclonal antibody therapy) in a host, the method comprising administering an effective amount of the active compound or its salt or composition as described herein. Various types of cytokine or inflammatory reactions may occur in response to a number of factors, such as the administrations of biotherapeutics. In some embodiments, the cytokine or inflammatory reaction is cytokine release syndrome. In some embodiments, the cytokine or inflammatory reaction is tumor lysis syndrome (which also leads to cytokine release). Symptoms of cytokine release syndrome range from fever, headache, and skin rashes to bronchospasm, hypotension and even cardiac arrest. Severe cytokine release syndrome is described as cytokine storm, and can be fatal.

Fatal cytokine storms have been observed in response to infusion with several monoclonal antibody therapeutics. See, Abramowicz D, et al. "Release of tumor necrosis factor, interleukin-2, and gamma-interferon in serum after injection of OKT3 monoclonal antibody in kidney transplant recipients" Transplantation (1989) 47(4):606-8; Chatenoud L, et al. "In vivo cell activation following OKT3 administration. Systemic cytokine release and modulation by corticosteroids" Transplantation (1990) 49(4):697-702; and Lim LC, Koh LP, and Tan P. "Fatal cytokine release syndrome with chimeric anti-CD20 monoclonal antibody rituximab in a 71-year-old patient with chronic lymphocytic leukemia" J. Clin Oncol. (1999) 17(6):1962-3.

Also contemplated herein, is the use of an active compound or its salt or composition as described herein to mediate an adverse immune response in patients receiving bi-specific T-cell engagers (BiTE). A bi-specific T-cell engager directs T-cells to target and bind with a specific antigen on the surface of a cancer cell. For example, Blinatumomab (Amgen), a BiTE has recently been approved as a second line therapy in Philadelphia chromosome-negative relapsed or refractory acute lymphoblastic leukemia. Blinatumomab is given by continuous intravenous infusion in 4-week cycles. The use of BiTE agents has been associated with adverse immune responses, including cytokine release syndrome. The most significantly elevated cytokines in the CRS associated with ACT include IL-10, IL-6, and IFN-γ (Klinger et al., Immunopharmacologic response of patients with B-lineage acute lymphoblastic leukemia to continuous infusion of T cell-engaging CD19/CD3-bispecific BiTE antibody blinatumomab. Blood (2012) 119:6226-6233).

In another embodiment, the disorder is episcleritis, idiopathic episcleritis, anterior episcleritis, or posterior episcleritis. In some embodiments, the disorder is idiopathic anterior uveitis, HLA-B27 related uveitis, herpetic keratouveitis, Posner Schlossman syndrome, Fuch's heterochromic iridocyclitis, or cytomegalovirus anterior uveitis.

In some embodiments, the present disclosure provides an active compound or its salt or composition as described herein for use in a method of treating or preventing a C3 glomerulopathy, the method comprising administering to a subject in need thereof an effective amount of the active compound or its salt or composition as described herein. In some embodiments, the disorder is selected from dense deposit disease (DDD) and C3 glomerulonephritis (C3GN).

In some embodiments, the present disclosure provides an active compound or its salt or composition as described herein for use in a method of treating or preventing a IC-MPGN, the method comprising administering to a subject in need thereof an effective amount of the active compound or its salt or composition as described herein.

In some embodiments, the present disclosure provides an active compound or its salt or composition as described herein for use in a method of treating or preventing a paroxysmal nocturnal hemoglobinuria (PNH), the method comprising administering to a subject in need thereof an effective amount of the active compound or its salt or composition as described herein.

In some embodiments, the present disclosure provides an active compound or its salt or composition as described herein for use in a method of treating or preventing age-related macular degeneration (AMD), the method comprising administering to a subject in need thereof an effective amount of the active compound or its salt or composition as described herein.

In some embodiments, the present disclosure provides an active compound or its salt or composition as described herein for use in a method of treating or preventing rheumatoid arthritis, the method comprising administering to a subject in need thereof an effective amount of the active compound or its salt or composition as described herein.

In some embodiments, the present disclosure provides an active compound or its salt or composition as described herein for use in a method of treating or preventing multiple sclerosis, the method comprisingadministering to a subject in need thereof an effective amount of the active compound or its salt or composition as described herein.

In some embodiments, the present disclosure provides an active compound or its salt or composition as described herein for use in a method of treating or preventing myasthenia gravis, the method comprising administering to a subject in need thereof an effective amount of the active compound or its salt or composition as described herein.

In some embodiments, the present disclosure provides an active compound or its salt or composition as described herein for use in a method of treating or preventing atypical hemolytic uremic syndrome (aHUS), the method comprising administering to a subject in need thereof an effective amount of the active compound or its salt or composition as described herein.

In some embodiments, the present disclosure provides an active compound or its salt or composition as described herein for use in a method of treating or preventing neuromyelitis optica (NMO), the method comprisingadministering to a subject in need thereof an effective amount of the active compound or its salt or composition as described herein.

In yet another embodiment, the present disclosure provides an active compound or its salt or composition as described herein for use in a method of treating or preventing a disorder as described below, the method comprising administering to a subject in need thereof an effective amount of the active compound or its salt or composition as described herein, wherein the disorder may be selected from:
vitritis, sarcoidosis, syphilis, tuberculosis, or Lyme disease; retinal vasculitis, Eales disease, tuberculosis, syphilis, or toxoplasmosis; neuroretinitis, viral retinitis, or acute retinal necrosis;
varicella zoster virus, herpes simplex virus, cytomegalovirus, Epstein-Barr virus, lichen planus, or Dengue-associated disease (e.g., hemorrhagic Dengue Fever); Masquerade syndrome, contact dermatitis, trauma induced inflammation, UVB induced inflammation, eczema, granuloma annulare, or acne.

In an additional embodiment, the disorder is selected from: acute myocardial infarction, aneurysm, cardiopulmonary bypass, dilated cardiomyopathy, complement activation during cardiopulmonary bypass operations, coronary artery disease, restenosis following stent placement, or percutaneous transluminal coronary angioplasty (PTCA); antibody-mediated transplant rejection, anaphylactic shock, anaphylaxis, allogenic transplant, humoral and vascular transplant rejection, graft dysfunction, graft-versus-host disease, Graves' disease, adverse drug reactions, or chronic graft vasculopathy; allergic bronchopulmonary aspergillosis, allergic neuritis, drug allergy, radiation- induced lung injury, eosinophilic pneumonia, radiographic contrast media allergy, bronchiolitis obliterans, or interstitial pneumonia; parkinsonism-dementia complex, sporadic frontotemporal dementia, frontotemporal dementia with Parkinsonism linked to chromosome 17, frontotemporal lobar degeneration, tangle only dementia, cerebral amyloid angiopathy, cerebrovascular disorder, certain forms of frontotemporal dementia, chronic traumatic encephalopathy (CTE), PD with dementia (PDD), argyrophilic grain dementia, dementia pugilistica, dementia with Lewy Bodies (DLB), or multi-infarct dementia; Creutzfeldt-Jakob disease, Huntington's disease, multifocal motor neuropathy (MMN), prion protein cerebral amyloid angiopathy, polymyositis, postencephalitic parkinsonism, subacute sclerosing panencephalitis, non-Guamanian motor neuron disease with neurofibrillary tangles, neural regeneration, and diffuse neurofibrillary tangles with calcification.

In some embodiments, the disorder is selected from: atopic dermatitis, dermatitis, dermatomyositis bullous pemphigoid, scleroderma, sclerodermatomyositis, psoriatic arthritis, pemphigus vulgaris, Discoid lupus erythematosus, cutaneous lupus, chilblain lupus erythematosus, or lupus erythematosus-lichen planus overlap syndrome; cryoglobulinemic vasculitis, mesenteric/enteric vascular disorder, peripheral vascular disorder, antineutrophil cytoplasm antibody (ANCA)-associated vasculitis (AAV), IL-2 induced vascular leakage syndrome, or immune complex vasculitis, angioedema, low platelets (HELLP) syndrome, sickle cell disease, platelet refractoriness, red cell casts, or typical or infectious hemolytic uremic syndrome (tHUS); hematuria, hemorrhagic shock, drug-induced thrombocytopenia, autoimmune hemolytic anemia (AIHA), azotemia, blood vessel and/or lymph vessel inflammation, rotational atherectomy, or delayed hemolytic transfusion reaction; British type amyloid angiopathy, Buerger's disease, bullous pemphigoid, C1q nephropathy, cancer, and catastrophic antiphospholipid syndrome.

In another embodiment, the disorder is selected from: wet (exudative) AMD, dry (non-exudative) AMD, chorioretinal degeneration, choroidal neovascularization (CNV), choroiditis, loss of RPE function, loss of vision (including loss of visual acuity or visual field), loss of vision from AMD, retinal damage in response to light exposure, retinal degeneration, retinal detachment, retinal dysfunction, retinal neovascularization (RNV), retinopathy of prematurity, pathological myopia, or RPE degeneration; pseudophakic bullous keratopathy, symptomatic macular degeneration related disorder, optic nerve degeneration, photoreceptor degeneration, cone degeneration, loss of photoreceptor cells, pars planitis, scleritis, proliferative vitreoretinopathy, or formation of ocular drusen; chronic urticaria, Churg-Strauss syndrome, cold agglutinin disease (CAD), corticobasal degeneration (CBD), cryoglobulinemia, cyclitis, damage of the Bruch's membrane, Degos disease, diabetic angiopathy, elevated liver enzymes, endotoxemia, epidermolysis bullosa, or epidermolysis bullosa acquisita, essential mixed cryoglobulinemia, excessive blood urea nitrogen-BUN, focal segmental glomerulosclerosis, Gerstmann-Straussler-Scheinker disease, giant cell arteritis, gout, Hallervorden-Spatz disease, Hashimoto's thyroiditis, Henoch-Schonlein purpura nephritis, abnormal urinary sediments, hepatitis, hepatitis A, hepatitis B, hepatitis C or human immunodeficiency virus (HIV), a viral infection more generally, for example, selected from Flaviviridae, Retroviruses, Coronaviridae (including SARS-CoV-2), Poxviridae, Adenoviridae, Herpesviridae, Caliciviridae, Reoviridae, Picornaviridae, Togaviridae, Orthomyxoviridae, Rhabdoviridae, or Hepadnaviridae; Neisseria meningitidis, shiga toxin E. coli-related hemolytic uremic syndrome (STEC-HUS), hemolytic uremic syndrome (HUS); Streptococcus, and poststreptococcal glomerulonephritis.

In a further embodiment, the disorder is selected from: hyperlipidemia, hypertension, hypoalbuminemia, hypobolemic shock, hypocomplementemic urticarial vasculitis syndrome, hypophosphastasis, hypovolemic shock, idiopathic pneumonia syndrome, or idiopathic pulmonary fibrosis; inclusion body myositis, intestinal ischemia, iridocyclitis, iritis, juvenile chronic arthritis, Kawasaki's disease (arteritis), or lipiduria; membranoproliferative glomerulonephritis (MPGN) I, microscopic polyangiitis, mixed cryoglobulinemia, molybdenum cofactor deficiency (MoCD) type A, pancreatitis, panniculitis, Pick's disease, polyarteritis nodosa (PAN), progressive subcortical gliosis, proteinuria, reduced glomerular filtration rate (GFR), or renovascular disorder; multiple organ failure, multiple system atrophy (MSA), myotonic dystrophy, Niemann-Pick disease type C, chronic demyelinating diseases, or progressive supranuclear palsy; spinal cord injury, spinal muscular atrophy, spondyloarthropathies, Reiter's syndrome, spontaneous fetal loss, recurrent fetal loss, pre-eclampsia, synucleinopathy, Takayasu's arteritis, post-partum thryoiditis, thyroiditis, Type I cryoglobulinemia, Type II mixed cryoglobulinemia, Type III mixed cryoglobulinemia, ulcerative colitis, uremia, urticaria, venous gas embolus (VGE), or Wegener's granulomatosis; von Hippel-Lindau disease, histoplasmosis of the eye, hard drusen, soft drusen, pigment clumping, or photoreceptor and/or retinal pigmented epithelia (RPE) loss,.

In some embodiments, an active compound or its salt or composition as described herein is useful for treating or preventing a disorder selected from autoimmune oophoritis, endometriosis, autoimmune orchitis, Ord's thyroiditis, autoimmune enteropathy, coeliac disease, Hashimoto's encephalopathy, antiphospholipid syndrome (APLS) (Hughes syndrome), aplastic anemia, autoimmune lymphoproliferative syndrome (Canale-Smith syndrome), autoimmune neutropenia, Evans syndrome, pernicious anemia, pure red cell aplasia, thrombocytopenia, adipose dolorosa (Dercum's disease), adult onset Still's disease, ankylosing spondylitis, CREST syndrome, drug-induced lupus, eosinophilic fasciitis (Shulman's syndrome), Felty syndrome, IgG4-related disease, mixed connective tissue disease (MCTD), palindromic rheumatism (Hench-Rosenberg syndrome), Parry-Romberg syndrome, Parsonage-Turner syndrome, relapsing polychondritis (Meyenburg-Altherr-Uehlinger syndrome), retroperitonial fibrosis, rheumatic fever, Schnitzler syndrome, fibromyalgia, neuromyotonia (Isaac's disease), paraneoplastic degeneration, autoimmune inner ear disease, Meniere's disease, interstitial cystitis, autoimmune pancreatitis, zika virus-related disorders, chikungunya virus-related disorders, subacute bacterial endocarditis (SBE), IgA nephropathy, IgA vasculitis, polymyalgia rheumatic, rheumatoid vasculitis, alopecia areata, autoimmune progesterone dermatitis, dermatitis herpetiformis, erythema nodosum, gestational pemphigoid, hidradenitis suppurativa, lichen sclerosus, linear IgA disease (LAD), morphea, myositis, pityriasis lichenoides et varioliformis acuta, vitiligo post-myocardial infarction syndrome (Dressler's syndrome), post-pericardiotomy syndrome, autoimmune retinopathy, Cogan syndrome, Graves opthalmopathy, ligneous conjunctivitis, Mooren's ulcer, opsoclonus myoclonus syndrome, optic neuritis, retinocochleocerebral vasculopathy (Susac's syndrome), sympathetic opthalmia, Tolosa-Hunt syndrome, interstitial lung disease, antisynthetase syndrome, Addison's disease, autoimmune polyendocrine syndrome (APS) type I, autoimmune polyendocrine syndrome (APS) type II, autoimmune polyendocrine syndrome (APS) type III, disseminated sclerosis (multiple sclerosis, pattern II), rapidly progressing glomerulonephritis (RPGN), juvenile rheumatoid arthritis, enthesitis-related arthritis, reactive arthritis (Reiter's syndrome), autoimmune hepatitis or lupoid hepatitis, primary biliary cirrhosis (PBS), primary sclerosing cholangitis, microscopic colitis, latent lupus (undifferentiated connective tissue disease (UCTD)), acute disseminated encephalomyelitis (ADEM), acute motor axonal neuropathy, anti-n-methyl-D-aspartate receptor encephalitis, Balo concentric sclerosis (Schilders disease), Bickerstaff's encephalitis, chronic inflammatory demyelinating polyneuropathy, idiopathic inflammatory demyelinating disease, Lambert-Eaton mysathenic syndrome, Oshtoran syndrome, pediatric autoimmune neuropsychiatric disorder associated with streptococcus (PANDAS), progressive inflammatory neuropathy, restless leg syndrome, stiff person syndrome, Sydenhem syndrome, transverse myelitis, lupus vasculitis, leukocytoclastic vasculitis, Microscopic Polyangiitis, polymyositis and ischemic-reperfusion injury of the eye.

Examples of eye disorders that may be treated according to the compositions and methods disclosed herein include amoebic keratitis, fungal keratitis, bacterial keratitis, viral keratitis, onchorcercal keratitis, bacterial keratoconjunctivitis, viral keratoconjunctivitis, corneal dystrophic diseases, Fuchs' endothelial dystrophy, Sjogren's syndrome, Stevens-Johnson syndrome, autoimmune dry eye diseases, environmental dry eye diseases, corneal neovascularization diseases, post-corneal transplant rejection prophylaxis and treatment, autoimmune uveitis, infectious uveitis, posterior uveitis (including toxoplasmosis), pan-uveitis, an inflammatory disease of the vitreous or retina, endophthalmitis prophylaxis and treatment, macular edema, macular degeneration, age related macular degeneration, proliferative and non-proliferative diabetic retinopathy, hypertensive retinopathy, an autoimmune disease of the retina, primary and metastatic intraocular melanoma, other intraocular metastatic tumors, open angle glaucoma, closed angle glaucoma, pigmentary glaucoma, and combinations thereof.

In a further embodiment, the disorder is selected from glaucoma, diabetic retinopathy, blistering cutaneous diseases (including bullous pemphigoid, pemphigus, and epidermolysis bullosa), ocular cicatrical pemphigoid, uveitis, adult macular degeneration, diabetic retinopa retinitis pigmentosa, macular edema, diabetic macular edema, Behçet's uveitis, multifocal choroiditis, Vogt-Koyangi-Harada syndrome, imtermediate uveitis, birdshot retino-chorioditis, sympathetic ophthalmia, ocular dicatricial pemphigoid, ocular pemphigus, nonartertic ischemic optic neuropathy, postoperative inflammation, and retinal vein occlusion, or central retinal vein occlusion (CVRO).

In some embodiments, complement mediated diseases include ophthalmic diseases (including early or neovascular age-related macular degeneration and geographic atrophy), autoimmune diseases (including arthritis, rheumatoid arthritis), respiratory diseases, and cardiovascular diseases. In other embodiments, the compounds of the disclosure are suitable for use in the treatment of diseases and disorders associated with fatty acid metabolism, including obesity and other metabolic disorders.

Disorders that may be treated or prevented by an active compound or its salt or composition as described herein also include, but are not limited to: hereditary angioedema, capillary leak syndrome, hemolytic uremic syndrome (HUS), neurological disorders, Guillain Barre Syndrome, diseases of the central nervous system and other neurodegenerative conditions, glomerulonephritis (including membrane proliferative glomerulonephritis), SLE nephritis, proliferative nephritis, liver fibrosis, tissue regeneration and neural regeneration, or Barraquer-Simons Syndrome; inflammatory effects of sepsis, systemic inflammatory response syndrome (SIRS), disorders of inappropriate or undesirable complement activation, interleukin-2 induced toxicity during IL-2 therapy, inflammatory disorders, inflammation of autoimmune diseases, system lupus erythematosus (SLE), lupus nephritides, arthritis, immune complex disorders and autoimmune diseases, systemic lupus, or lupus erythematosus, ischemia/ reperfusion injury (I/R injury), myocardial infarction, myocarditis, post-ischemic reperfusion conditions, balloon angioplasty, atherosclerosis, post-pump syndrome in cardiopulmonary bypass or renal bypass, renal ischemia, mesenteric artery reperfusion after aortic reconstruction, antiphospholipid syndrome, autoimmune heart disease, ischemia-reperfusion injuries, obesity, or diabetes; Alzheimer's dementia, stroke, schizophrenia, traumatic brain injury, trauma, Parkinson's disease, epilepsy, transplant rejection, prevention of fetal loss, biomaterial reactions (e.g., in hemodialysis, inplants), hyperacute allograft rejection, xenograft rejection, transplantation, psoriasis, burn injury, thermal injury including burns or frostbite, or crush injury; asthma, allergy, acute respiratory distress syndrome (ARDS), cystic fibrosis, adult respiratory distress syndrome, dyspnea, hemoptysis, chronic obstructive pulmonary disease (COPD), emphysema, pulmonary embolisms and infarcts, pneumonia, fibrogenic dust diseases, inert dusts and minerals (e.g., silicon, coal dust, beryllium, and asbestos), pulmonary fibrosis, organic dust diseases, chemical injury (due to irritant gases and chemicals, e.g., chlorine, phosgene, sulfur dioxide, hydrogen sulfide, nitrogen dioxide, ammonia, and hydrochloric acid), smoke injury, thermal injury (e.g., burn, freeze), bronchoconstriction, hypersensitivity pneumonitis, parasitic diseases, Goodpasture's Syndrome (anti-glomerular basement membrane nephritis), pulmonary vasculitis, Pauciimmune vasculitis, and immune complex- associated inflammation.

In some embodiments, an active compound or its salt or composition as described herein is provided for use in a method for the treatment of sickle cell in a host, the method comprising the administration of an effective amount of the active compound or its salt or composition as described herein. In some embodiments, an active compound or its salt or composition as described herein is provided for use in a method for the treatment of immunothrombocytopenic purpura (ITP), thrombotic thrombocytopenic purpura (TTP), or idiopathic thrombocytopenic purpura (ITP) in a host, the method comprising the administration of an effective amount of the active compound or its salt or composition as described herein. In some embodiments, an active compound or its salt or composition as described herein is provided for use in a method for the treatment of ANCA-vasculitis in a host , the method comprising the administration of an effective amount of the active compound or its salt or composition as described herein. In some embodiments, an active compound or its salt or composition as described herein is provided for use in a method for the treatment of IgA nephropathy in a host, the method comprising the administration of an effective amount of the active compound or its salt or composition as described herein. In some embodiments, an active compound or its salt or composition as described herein is provided for use in a method for the treatment of rapidly progressing glomerulonephritis (RPGN), in a host, the method comprising the administration of an effective amount of the active compound or its salt or composition as described herein. In some embodiments, an active compound or its salt or composition as described herein is provided for use in a method for the treatment of lupus nephritis in a host, the method comprising the administration of an effective amount of the active compound or its salt or composition as described herein. In some embodiments, an active compound or its salt or composition as described herein is provided for use in a method for the treatment of hemorraghic dengue fever in a host, the method comprising the administration of an effective amount of the active compound or its salt or composition as described herein.

In an additional alternative embodiment, an active compound or its salt or composition as described herein is used in the treatment of an autoimmune disorder.

The complement pathway enhances the ability of antibodies and phagocytic cells to clear microbes and damaged cells from the body. It is part of the innate immune system and in healthy individuals is an essential process. Inhibiting the complement pathway will decrease the body's immune system response. Therefore, it is an object of the present disclosure to treat autoimmune disorders by administering an effective dose of an active compound or its salt or composition as described herein to a subject in need thereof.

In some embodiments, the autoimmune disorder is caused by activity of the complement system. In some embodiments, the autoimmune disorder is caused by activity of the alternative complement pathway. In some embodiments, the autoimmune disorder is caused by activity of the classical complement pathway. In another embodiment, the autoimmune disorder is caused by a mechanism of action that is not directly related to the complement system, such as the over-proliferation of T-lymphocytes or the over-production of cytokines.

Non-limiting examples of autoimmune disorders include: lupus, allograft rejection, autoimmune thyroid diseases (such as Graves' disease and Hashimoto's thyroiditis), autoimmune uveoretinitis, giant cell arteritis, inflammatory bowel diseases (including Crohn's disease, ulcerative colitis, regional enteritis, granulomatous enteritis, distal ileitis, regional ileitis, and terminal ileitis), diabetes, multiple sclerosis, pernicious anemia, psoriasis, rheumatoid arthritis, sarcoidosis, and scleroderma.

In some embodiments, an active compound or its salt or composition as described herein is used in the treatment of lupus. Non-limiting examples of lupus include lupus erythematosus, cutaneous lupus, discoid lupus erythematosus, chilblain lupus erythematosus, or lupus erythematosus-lichen planus overlap syndrome.

Lupus erythematosus is a general category of disease that includes both systemic and cutaneous disorders. The systemic form of the disease can have cutaneous as well as systemic manifestations. However, there are also forms of the disease that are only cutaneous without systemic involvement. For example, SLE is an inflammatory disorder of unknown etiology that occurs predominantly in women, and is characterized by articular symptoms, butterfly erythema, recurrent pleurisy, pericarditis, generalized adenopathy, and splenomegaly, as well as CNS involvement and progressive renal failure. The sera of most patients (over 98%) contain antinuclear antibodies, including anti-DNA antibodies. High titers of anti-DNA antibodies are essentially specific for SLE. Conventional treatment for this disease has been the administration of corticosteroids or immunosuppressants.

There are three forms of cutaneous lupus: chronic cutaneous lupus (also known as discoid lupus erythematosus or DLE), subacute cutaneous lupus, and acute cutaneous lupus. DLE is a disfiguring chronic disorder primarily affecting the skin with sharply circumscribed macules and plaques that display erythema, follicular plugging, scales, telangiectasia and atrophy. The condition is often precipitated by sun exposure, and the early lesions are erythematous, round scaling papules that are 5 to 10 mm in diameter and display follicular plugging. DLE lesions appear most commonly on the cheeks, nose, scalp, and ears, but they may also be generalized over the upper portion of the trunk, extensor surfaces of the extremities, and on the mucous membranes of the mouth. If left untreated, the central lesion atrophies and leaves a scar. Unlike SLE, antibodies against double-stranded DNA (e.g., DNA-binding test) are almost invariably absent in DLE.

Multiple Sclerosis (MS) is an autoimmune demyelinating disorder that is believed to be T lymphocyte dependent. MS generally exhibits a relapsing-remitting course or a chronic progressive course. The etiology of MS is unknown; however, viral infections, genetic predisposition, environment, and autoimmunity all appear to contribute to the disorder. Lesions in MS patients contain infiltrates of predominantly T lymphocyte mediated microglial cells and infiltrating macrophages. CD4⁺ T lymphocytes are the predominant cell type present at these lesions. The hallmark of the MS lesion is plaque, an area of demyelination sharply demarcated from the usual white matter seen in MRI scans. Histological appearance of MS plaques varies with different stages of the disease. In active lesions, the blood-brain barrier is damaged, thereby permitting extravasation of serum proteins into extracellular spaces. Inflammatory cells can be seen in perivascular cuffs and throughout white matter. CD4⁺ T-cells, especially Th1, accumulate around postcapillary venules at the edge of the plaque and are also scattered in the white matter. In active lesions, up-regulation of adhesion molecules and markers of lymphocyte and monocyte activation, such as IL2-R and CD26 have also been observed. Demyelination in active lesions is not accompanied by destruction of oligodendrocytes. In contrast, during chronic phases of the disease, lesions are characterized by a loss of oligodendrocytes and hence, the presence of myelin oligodendrocyte glycoprotein (MOG) antibodies in the blood.

Diabetes can refer to either type 1 or type 2 diabetes. In some embodiments, an active compound or its salt or composition as described herein is provided at an effective dose to treat a patient with type 1 diabetes. In some embodiments, an active compound or its salt or composition as described herein is provided at an effective dose to treat a patient with type 2 diabetes.
Type 1 diabetes is an autoimmune disease. An autoimmune disease results when the body's system for fighting infection (the immune system) attacks a part of the body. In the case of diabetes type 1, the pancreas then produces little or no insulin.

In certain aspects, an effective amount of an active compound described herein, or its pharmaceutically acceptable salt, is used to treat a medical disorder of the central nervous system (CNS) or peripheral nervous system disorders involving complement activation. In some embodiments, the CNS disorder is an acquired brain or spinal cord injury, including, but not limited to, ischaemic-reperfusion injury or stroke, traumatic brain injury (TBI) and spinal cord injury (SCI). In some embodiments, the disorder is a neurodegeneration disorder. In some embodiments, the disorder is a neuroinflammation disorder.

In certain aspects, active compound described herein, or it pharmaceutically acceptable salt is used to treat Alzheimer's disease (AD). AD is characterized by two hallmark pathologies; amyloid-β (Aβ) plaques and neurofibrillary tangles comprising hyperphosphorylated tau. Recent studies have implicated complement in AD pathogenesis, including genome-wide association studies identifying single nucleotide polymorphisms (SNPs) associated with risk of late-onset AD in genes encoding complement proteins Clusterin (CLU) and CR1 (CR1). See Carpanini et al., Therapeutic Inhibition of the Complement System in Diseases of the Central Nervous System, Front. Immunol., 04 March 2019. Biomarker studies have also identified complement proteins and activation products in plasma and/or CSF that distinguish AD from controls and predict risk of progression to AD. (Id.)

In certain aspects, an effective amount of an active compound described herein, or of its pharmaceutically acceptable salt, is used to treat certain forms of frontotemporal dementia including, but not limited to, Pick's disease, sporadic Frontotemporal dementia and Frontotemporal dementia with Parkinsonism linked to chromosome 17, Progressive supranuclear palsy (PSP), Corticobasal degeneration (CBD), and Subacute sclerosing panencephalitis.

In certain aspects, an effective amount of an active compound described herein, or of its pharmaceutically acceptable salt, is used to treat multiple sclerosis (MS). Multiple sclerosis (MS) is the most common cause of neurological disability in young adults in northern European-Caucasian populations, with an approximate lifetime risk of one in 400. C3 has been shown to be deposited in the brains of MS patients. TCC has been shown to be in association with capillary endothelial cells, predominantly within plaques and adjacent white matter. Localization of C activation to areas of active myelin destruction has also been shown, with TCC deposited exclusively in such areas. C3d has been shown to be deposited in association with short segments of disrupted myelin in plaques with low-grade active demyelination and provides evidence for a C contribution to disease progression as well as acute inflammation. See Ingram et al., Complement in multiple sclerosis: its role in disease and potential as a biomarker. Clin Exp Immunol. 2009 Feb;155(2):128-39.

In certain aspects, an active compound described herein, or its pharmaceutically acceptable salt, is used to treat neuromyelitis optica (NMO). Neuromyelitis optica (NMO) is an inflammatory demyelinating disease affecting predominantly the optic nerves and spinal cord. Traditionally seen as a variant of MS, it has been redefined recently according to new criteria using a combination of phenotypic subtyping along with a newly developed biomarker of disease, NMO-immunoglobulin G (IgG) (reported sensitivity of 58-76% and specificity of 85-99% for NMO). NMO patients have higher levels of C3a and anti-C1q antibodies than healthy controls. C3a levels correlated with disease activity, neurological disability and aquaporin-4 IgG. Nytrova et al., Complement activation in patients with neuromyelitis optica. J Neuroimmunol. 2014 Sep 15;274(1-2):185-91.

In certain aspects, an active compound described herein, or its pharmaceutically acceptable salt, is used to treat amyotrophic lateral sclerosis (ALS). ALS is caused by progressive loss of upper and lower (α) motor neurons resulting in denervation of neuromuscular junctions in the peripheral nervous system, progressive muscle weakness, atrophy, spasticity, respiratory failure, and ultimately paralysis and death. Recent studies have shown increased C1q protein in motor cortex and spinal cord of ALS post-mortem tissue; C3 activation fragments and TCC in areas of pathology; C4d and TCC staining of degenerating neurons and glia in ALS motor cortex and spinal cord, and C5aR1 upregulation in areas of pathology. C3d and C4d have been found on oligodendroglia and degenerating neurites, surrounded by CR4-positive microglia, in spinal cord and motor cortex, and C1q, C3, and TCC have been shown to be present on motor end-plates in intercostal muscles in ALS donors even early in the disease process. See Carpanini et al., Therapeutic Inhibition of the Complement System in Diseases of the Central Nervous System, Front. Immunol., 04 March 2019.

In certain aspects, an active compound described herein, or its pharmaceutically acceptable salt, is used to treat Parkinson's disease (PD). PD is characterized by loss of dopaminergic neurons in the substantia nigra and deposits of the protein α-synuclein that form the pathological hallmarks of the disease, Lewy bodies. Patients present with resting tremor, bradykinesia, and rigidity. Complement activation has been associated with α-synuclein and Lewy bodies in Parkinson's disease; in vitro studies have demonstrated that the disease-associated splice variant α-synuclein 112, but not the full-length protein, causes activation of complement. In vivo, C3d, C4d, C7 and C9 localization in Lewy bodies has been reported. More recently, deposition of iC3b and C9 in Lewy bodies and melanized neurons has been reported, and iC3b immunoreactivity has been shown to be increased with normal ageing and was further elevated in PD vs. age-matched controls. Furthermore, correlation between the ratios of C3/Aβ42 or FH/Aβ42 in CSF and severity of Parkinson's disease motor and cognitive symptoms has been shown. See Carpanini et al., Therapeutic Inhibition of the Complement System in Diseases of the Central Nervous System, Front. Immunol., 04 March 2019. In some embodiments, the subject to be treated suffers from Parkinson's Disease with dementia (PDD).

In certain aspects, an active compound described herein, or its pharmaceutically acceptable salt, is used to treat Huntington's disease (HD). HD is an autosomal dominant, inherited neurodegenerative disease characterized by progressive motor symptoms, psychiatric disturbances, and dementia. It is caused by expansion of a three-base-pair (CAG) repeat (39-121 repeats vs. normal range 8-39 repeats) in exon 1 of the HTT gene that translates into a polyglutamine tract at the N-terminus of the protein. This results in a polyglutamine length-dependent misfolding and accumulation of huntingtin protein in the striatum and cortex (layers 3, 5, and 6) followed by neuronal loss in these areas which spreads to the hippocampus. It has been shown that neurons, astrocytes, and myelin sheaths in the HD caudate and striatum were immunoreactive for C1q, C4, C3 and neo-epitopes in iC3b and TCC. Expression of mRNA encoding early complement components C1q (c-chain), C1r, C3, and C4, complement regulators C1INH, Clusterin, MCP, DAF and CD59, and complement receptors C3a and C5a have been shown to be upregulated in the HD striatum. See Carpanini et al., Therapeutic Inhibition of the Complement System in Diseases of the Central Nervous System, Front. Immunol., 04 March 2019.

In certain aspects, an active compound described herein, or its pharmaceutically acceptable salt, is used to treat argyrophilic grain dementia, British type amyloid angiopathy, cerebral amyloid angiopathy, Creutzfeldt-Jakob disease, dementia pugilistica, diffuse neurofibrillary tangles with calcification, Down's syndrome, frontotemporal lobar degeneration, Gerstmann-Straussler-Scheinker disease, Hallervorden-Spatz disease, inclusion body myositis, multiple system atrophy (MSA), myotonic dystrophy, Niemann-Pick disease type C, non-Guamanian motor neuron disease with neurofibrillary tangles, postencephalitic parkinsonism, prion protein cerebral amyloid angiopathy, progressive subcortical gliosis, progressive supranuclear palsy, subacute sclerosing panencephalitis, Tangle only dementia, multi-infarct dementia, ischemic stroke, chronic traumatic encephalopathy (CTE), traumatic brain injury (TBI), and stroke.

In certain aspects, an active compound described herein, or its pharmaceutically acceptable salt, is used to treat a hereditary motor and sensory neuropathy (HMSN). In some embodiments, the hereditary and sensory neuropathy is Charcot-Marie-Tooth (CMT) disease. In some embodiments, the HSMN is Charcot-Marie-Tooth disease type 1A or type 1B. In some embodiments, the HSMN is Charcot-Marie-Tooth disease type 2. In some embodiments, the HSMN is Dejerine-Sottas disease (Charcot-Marie-Tooth type 3). In some embodiments, the HSMN is Refsum disease. In some embodiments, the HSMN is Charcot-Marie-Tooth with pyramidal features. In some embodiments, the HSMN is Charcot-Marie-Tooth type 6. In some embodiments, the HSMN is HMSN+retinitis pigmentosa.

In some embodiments, an active compound as described herein is used to treat Churg-Strauss syndrome.

In some embodiments, an active compound as described herein is used to treat a peripheral artery disease (PAD).

In certain aspects, an effective amount of active compound described herein, or its pharmaceutically acceptable salt, is used to treat myasthenia gravis with CNS involvement.

In certain aspects, an effective amount of active compound described herein, or its pharmaceutically acceptable salt, is used to treat dementia with Lewy bodies.

In certain aspects, an active compound described herein, or its pharmaceutically acceptable salt, is used to treat an individual suffering from prion disease.

In certain aspects, an active compound described herein, or its pharmaceutically acceptable salt, is used to treat Behçet's Disease.

In certain aspects, an active compound described herein, or its pharmaceutically acceptable salt is used to treat congenital myasthenia.

In certain aspects, an active compound described herein, or its pharmaceutically acceptable salt, is used to treat subacute sclerosing panencephalitis (SSPE).

In certain aspects, an active compound described herein, or its pharmaceutically acceptable salt, is used to treat Guillain-Barré syndrome.

In certain aspects, the CNS disorder to be treated is a demyelinating disease, including, but not limited to, demyelinating myelinoclastic diseases and demyelinating leukostrophic disease.

In certain aspects, the disorder to be treated is a demyelinating myelonoclastic disease including, but not limited to, multiple sclerosis, neuromyelitis optica, neuromyelitis optica spectrum of disorders (NMOSD), idiopathic inflammatory demyelinating diseases (IIDD), anti-NMDA receptor encephalitis, acute disseminated encephalomyelitis, anti-MOG autoimmune encephalomyelitis, chronic relapsing inflammatory optic neuritis (CRION), acute disseminated encephalomyelitis (ADEM), immune-mediated encephalomyelitis, progressive multifocal leukoencephalopathy (PML); McDonalds-positive multiple sclerosis, acute hemorrhagic leukoencephalitis, Rasmussen's Encephalitis, Marburg multiple sclerosis, pseudotumefactive and tumefactive multiple sclerosis, Balo concentric sclerosis, diffuse myelinoclastic sclerosis, solitary sclerosis, multiple sclerosis with cavitary lesions, myelocortical multiple sclerosis (MCMS), atypical optic-spinal multiple sclerosis, pure spinal multiple sclerosis, HLA DRB3*02:02 multiple sclerosis, autoimmune GFAP astrocytopathy, Chronic inflammatory demyelinating polyneuropathy (CIDP), Guillain-Barré syndrome, progressive inflammatory neuropathy, Lewis-Sumner Syndrome, combined central and peripheral demyelination (CCPD), Bickerstaff brainstem encephalitis, Fisher syndrome, trigeminal neuralgia, NMDAR anti-NMDA receptor encephalitis, primary progressive MS (PPMS), OPA1 variant multiple sclerosis, KIR4.1 multiple sclerosis, aquaporine-related multiple sclerosis, chronic cerebrospinal venous insufficiency (CCSVI or CCVI), diffuse sclerosis, and Schilder's disease,

In certain aspects, the disorder to be treated is a demyelinating leukostrophic disease including, but not limited to, myelitis, central pontine myelinolysis (CPM), extrapontine myelinolysis, tabes dorsalis, progressive multifocal leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, megalencephalic leukoencephalopathy with subcortical cysts 1, hypertensive leukoencephalopathy, Metachromatic leukodystrophy, Krabbe disease, Canavan disease, X-linked adrenoleukodystrophy, Alexander disease, cerebrotendineous xanthomatosis, Pelizaeus-Merzbacher disease, and Refsum disease.

In some embodiments, an active compound as described herein is used to treat Buerger's disease, also known as thromboangiitis obliterans.

In some embodiments, an active compound as described herein is used to treat giant cell arteritis.

In some embodiments, an active compound as described herein is used to treat Raynaud's disease.

In certain aspects, the disorder to be treated is a demyelinating disease of the peripheral nervous system, including, but not limited to, Guillain-Barré syndrome and its chronic counterpart, chronic inflammatory demyelinating polyneuropathy, anti-MAG peripheral neuropathy, Charcot-Marie-Tooth disease and its counterpart Hereditary neuropathy with liability to pressure palsy, Copper deficiency-associated conditions (peripheral neuropathy, myelopathy, and rarely optic neuropathy), and progressive inflammatory neuropathy.

In certain aspects, the disorder to be treated is a neurological inflammatory disorder. In some embodiments, the disorder to be treated is cranial arteritis; giant cell arteritis; Holmes-Adie syndrome; inclusion body myositis (IBM); meningitis; neurologic paraneoplastic syndrome including, but not limited to, Lambert-Eaton myasthenic syndrome, stiff-person syndrome, encephalomyelitis (inflammation of the brain and spinal cord), myasthenia gravis, cerebellar degeneration, limbic and/or brainstem encephalitis, neuromyotonia, and opsoclonus (involving eye movement) and sensory neuropathy; polymyositis; transverse myelitis; vasculitis including temporal arteritis; arachnoiditis; Kinsbourne syndrome or opsoclonus myoclonus syndrome (OMS); and Saint Vitus Dance or sydenham chorea (SD) disease.

In some embodiments, an active compound or its salt or composition as described herein is used to treat transverse myelitis.

In certain aspects, the disorder to be treated is a peripheral neuropathy. In some embodiments, the peripheral neuropathy is a mononeuropathy. In some embodiments, the neuropathy is a polyneuropathy. In some embodiments, the polyneuropathy is distal axonopathy, diabetic neuropathy, a demyelinating polyneuropathy, small fiber peripheral neuropathy, mononeuritis multiplex, polyneuritis multiplex, autonomic neuropathy, or neuritis.

In some embodiments, an active compound or its salt or composition as described herein is used to treat an autoimmune vascular disease. In some embodiments, the autoimmune vascular disease is vasculitis. In some embodiments, the vasculitis includes, but is not limited to, autoimmune inflammatory vasculitis, Cutaneous small-vessel vasculitis, Granulomatosis with polyangiitis , Eosinophilic granulomatosis with polyangiitis, Behçet's disease, Kawasaki disease, Buerger's disease, and "Limited" granulomatosis with polyangiitis vasculitis.

In some embodiments, an active compound or its salt or composition as described herein is used to treat an arteritis. In some embodiments, the arteritis includes, but is not limited to, giant cell arteritis, Takayasu arteritis, temporal arteritis, and polyarteritis nodosa.

In some embodiments, the complement-mediated disease or disorder comprises transplant rejection. In some embodiments, the complement-mediated disease or disorder is antibody-mediated transplant rejection.

In certain aspects, an active compound or its salt or composition as described herein is used to treat a proliferative disorder, including, but not limited to, cancer. Targeted cancers suitable for administration of an active compound or its salt described herein include, but are not limited to, estrogen-receptor positive cancer, HER2-negative advanced breast cancer, late-line metastatic breast cancer, liposarcoma, non-small cell lung cancer, liver cancer, ovarian cancer, glioblastoma, refractory solid tumors, retinoblastoma positive breast cancer as well as retinoblastoma positive endometrial, vaginal and ovarian cancers and lung and bronchial cancers, adenocarcinoma of the colon, adenocarcinoma of the rectum, central nervous system germ cell tumors, teratomas, estrogen receptor-negative breast cancer, estrogen receptor-positive breast cancer, familial testicular germ cell tumors, HER2-negative breast cancer, HER2-positive breast cancer, male breast cancer, ovarian immature teratomas, ovarian mature teratoma, ovarian monodermal and highly specialized teratomas, progesterone receptor-negative breast cancer, progesterone receptor-positive breast cancer, recurrent breast cancer, recurrent colon cancer, recurrent extragonadal germ cell tumors, recurrent extragonadal non-seminomatous germ cell tumor, recurrent extragonadal seminomas, recurrent malignant testicular germ cell tumors, recurrent melanomas, recurrent ovarian germ cell tumors, recurrent rectal cancer, stage III extragonadal non-seminomatous germ cell tumors, stage III extragonadal seminomas, stage III malignant testicular germ cell tumors, stage III ovarian germ cell tumors, stage IV breast cancers, stage IV colon cancers, stage IV extragonadal non-seminomatous germ cell tumors, stage IV extragonadal seminoma, stage IV melanomas, stage IV ovarian germ cell tumors, stage IV rectal cancers, testicular immature teratomas, testicular mature teratomas. In particular embodiments, the targeted cancers included estrogen-receptor positive, HER2-negative advanced breast cancer, late-line metastatic breast cancer, liposarcoma, non-small cell lung cancer, liver cancer, ovarian cancer, glioblastoma, refractory solid tumors, retinoblastoma positive breast cancer as well as retinoblastoma positive endometrial, vaginal and ovarian cancers and lung and bronchial cancers, metastatic colorectal cancer, metastatic melanoma with CDK4 mutation or amplification, or cisplatin-refractory, unresectable germ cell tumors, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, fibrosarcoma, myxosarcoma, chondrosarcoma, osteosarcoma, chordoma, malignant fibrous histiocytoma, hemangiosarcoma, angiosarcoma, lymphangiosarcoma, Mesothelioma, leiomyosarcoma, rhabdomyosarcoma, squamous cell carcinoma; epidermoid carcinoma, malignant skin adnexal tumors, adenocarcinoma, hepatoma, hepatocellular carcinoma, renal cell carcinoma, hypernephroma, cholangiocarcinoma, transitional cell carcinoma, choriocarcinoma, seminoma, embryonal cell carcinoma, glioma anaplastic; glioblastoma multiforme, neuroblastoma, medulloblastoma, malignant meningioma, malignant schwannoma, neurofibrosarcoma, parathyroid carcinoma, medullary carcinoma of thyroid, bronchial carcinoid, pheochromocytoma, Islet cell carcinoma, malignant carcinoid, malignant paraganglioma, melanoma, Merkel cell neoplasm, cystosarcoma phylloide, salivary cancers, thymic carcinomas, bladder cancer, and Wilms tumor, a blood disorder or a hematologic malignancy, including, but not limited to, myeloid disorder, lymphoid disorder, leukemia, lymphoma, myelodysplastic syndrome (MDS), myeloproliferative disease (MPD), mast cell disorder, and myeloma (e.g., multiple myeloma), among others, T-cell or NK-cell lymphoma, for example, but not limited to: peripheral T-cell lymphoma; anaplastic large cell lymphoma, for example anaplastic lymphoma kinase (ALK) positive, ALK negative anaplastic large cell lymphoma, or primary cutaneous anaplastic large cell lymphoma; angioimmunoblastic lymphoma; cutaneous T-cell lymphoma, for example mycosis fungoides, Sézary syndrome, primary cutaneous anaplastic large cell lymphoma, primary cutaneous CD30+ T-cell lymphoproliferative disorder; primary cutaneous aggressive epidermotropic CD8+ cytotoxic T-cell lymphoma; primary cutaneous gamma-delta T-cell lymphoma; primary cutaneous small/medium CD4+ T-cell lymphoma, and lymphomatoid papulosis; Adult T-cell Leukemia/Lymphoma (ATLL); Blastic NK-cell Lymphoma; Enteropathy-type T-cell lymphoma; Hematosplenic gamma-delta T-cell Lymphoma; Lymphoblastic Lymphoma; Nasal NK/T-cell Lymphomas; Treatment-related T-cell lymphomas; for example lymphomas that appear after solid organ or bone marrow transplantation; T-cell prolymphocytic leukemia; T-cell large granular lymphocytic leukemia; Chronic lymphoproliferative disorder of NK-cells; Aggressive NK cell leukemia; Systemic EBV+ T-cell lymphoproliferative disease of childhood (associated with chronic active EBV infection); Hydroa vacciniforme-like lymphoma; Adult T-cell leukemia/ lymphoma; Enteropathy-associated T-cell lymphoma; Hepatosplenic T-cell lymphoma; or Subcutaneous panniculitis-like T-cell lymphoma.

In some embodiments, the methods described herein can be used to treat a host, for example a human, with a lymphoma or lymphocytic or myelocytic proliferation disorder or abnormality. For example, the methods as described herein can be administered to a host with a Hodgkin Lymphoma or a Non-Hodgkin Lymphoma. For example, the host can have a Non-Hodgkin Lymphoma such as, but not limited to: an AIDS-Related Lymphoma; Anaplastic Large-Cell Lymphoma; Angioimmunoblastic Lymphoma; Blastic NK-Cell Lymphoma; Burkitt's Lymphoma; Burkitt-like Lymphoma (Small Non-Cleaved Cell Lymphoma); Chronic Lymphocytic Leukemia/Small Lymphocytic Lymphoma; Cutaneous T-Cell Lymphoma; Diffuse Large B-Cell Lymphoma; Enteropathy-Type T-Cell Lymphoma; Follicular Lymphoma; Hepatosplenic Gamma-Delta T-Cell Lymphoma; Lymphoblastic Lymphoma; Mantle Cell Lymphoma; Marginal Zone Lymphoma; Nasal T-Cell Lymphoma; Pediatric Lymphoma; Peripheral T-Cell Lymphomas; Primary Central Nervous System Lymphoma; T-Cell Leukemias; Transformed Lymphomas; Treatment-Related T-Cell Lymphomas; or Waldenstrom's Macroglobulinemia, a Hodgkin Lymphoma, such as, but not limited to: Nodular Sclerosis Classical Hodgkin's Lymphoma (CHL); Mixed Cellularity CHL; Lymphocyte-depletion CHL; Lymphocyte-rich CHL; Lymphocyte Predominant Hodgkin Lymphoma; or Nodular Lymphocyte Predominant HL, a specific B-cell lymphoma or proliferative disorder such as, but not limited to: multiple myeloma; Diffuse large B cell lymphoma; Follicular lymphoma; Mucosa-Associated Lymphatic Tissue lymphoma (MALT); Small cell lymphocytic lymphoma; Mediastinal large B cell lymphoma; Nodal marginal zone B cell lymphoma (NMZL); Splenic marginal zone lymphoma (SMZL); Intravascular large B-cell lymphoma; Primary effusion lymphoma; or Lymphomatoid granulomatosis; B-cell prolymphocytic leukemia; Hairy cell leukemia; Splenic lymphoma/leukemia, unclassifiable; Splenic diffuse red pulp small B-cell lymphoma; Hairy cell leukemia-variant; Lymphoplasmacytic lymphoma; Heavy chain diseases, for example, Alpha heavy chain disease, Gamma heavy chain disease, Mu heavy chain disease; Plasma cell myeloma; Solitary plasmacytoma of bone; Extraosseous plasmacytoma; Primary cutaneous follicle center lymphoma; T cell/histiocyte rich large B-cell lymphoma; DLBCL associated with chronic inflammation; Epstein-Barr virus (EBV)+ DLBCL of the elderly; Primary mediastinal (thymic) large B-cell lymphoma; Primary cutaneous DLBCL, leg type; ALK+ large B-cell lymphoma; Plasmablastic lymphoma; Large B-cell lymphoma arising in HHV8-associated multicentric; Castleman disease; B-cell lymphoma, unclassifiable, with features intermediate between diffuse large B-cell lymphoma; or B-cell lymphoma, unclassifiable, with features intermediate between diffuse large B-cell lymphoma and classical Hodgkin lymphoma, a leukemia, for example, an acute or chronic leukemia of a lymphocytic or myelogenous origin, such as, but not limited to: Acute lymphoblastic leukemia (ALL); Acute myelogenous leukemia (AML); Chronic lymphocytic leukemia (CLL); Chronic myelogenous leukemia (CML); juvenile myelomonocytic leukemia (JMML); hairy cell leukemia (HCL); acute promyelocytic leukemia (a subtype of AML); large granular lymphocytic leukemia; or Adult T-cell chronic leukemia. In some embodiments, the patient has an acute myelogenous leukemia, for example an undifferentiated AML (M0); myeloblastic leukemia (M1; with/without minimal cell maturation); myeloblastic leukemia (M2; with cell maturation); promyelocytic leukemia (M3 or M3 variant [M3V]); myelomonocytic leukemia (M4 or M4 variant with eosinophilia [M4E]); monocytic leukemia (M5); erythroleukemia (M6); or megakaryoblastic leukemia (M7), small cell lung cancer, retinoblastoma, HPV positive malignancies like cervical cancer and certain head and neck cancers, MYC amplified tumors such as Burkitts' Lymphoma, and triple negative breast cancer; certain classes of sarcoma, certain classes of non-small cell lung carcinoma, certain classes of melanoma, certain classes of pancreatic cancer, certain classes of leukemia, certain classes of lymphoma, certain classes of brain cancer, certain classes of colon cancer, certain classes of prostate cancer, certain classes of ovarian cancer, certain classes of uterine cancer, certain classes of thyroid and other endocrine tissue cancers, certain classes of salivary cancers, certain classes of thymic carcinomas, certain classes of kidney cancers, certain classes of bladder cancers, and certain classes of testicular cancers.

In certain aspects, an active compound or its salt as described herein can be used to preserve or prevent damage to an organ or blood product. For example, an active compound or its salt described herein can be used to prevent damage to an organ, tissue, cell product, or blood product, that has been harvested for transplantation. In some embodiments, the organ is the heart, kidney, pancreas, lung, liver, or intestine. In some embodiments, the tissue is derived from the cornea, bone, tendon, muscle, heart valve, nerve, artery or vein, or the skin. In some embodiments, the blood product is whole blood, plasma, red blood cells, or reticulocytes.

In some embodiments, an active compound or its salt or composition as described herein prevents or delays the onset of at least one symptom of a complement-mediated disease or disorder in an individual. In some embodiment, an active compound or its salt or composition as described herein reduces or eliminates at least one symptom of a complement-mediated disease or disorder in an individual. Examples of symptoms include, but are not limited to, symptoms associated with autoimmune disease, cancer, hematological disease, infectious disease, inflammatory disease, ischemia-reperfusion injury, neurodegenerative disease, neurodegenerative disorder, renal disease, transplant rejection, ocular disease, vascular disease, or a vasculitis disorder. The symptom can be a neurological symptom, for example, impaired cognitive function, memory impairment, loss of motor function, etc. The symptom can also be the activity of factor D protein in a cell, tissue, or fluid of an individual. The symptom can also be the extent of complement activation in a cell, tissue, or fluid of an individual.

In some embodiments, administering an active compound or its salt or composition as described herein to an individual modulates complement activation in a cell, tissue, or fluid of an individual. In some embodiments, administration of an active compound or its salt or composition as described herein to an individual inhibits complement activation in a cell, tissue, or fluid of an individual. For example, in some embodiments, an active compound or its salt or composition as described herein, when administered in one or more doses as monotherapy or in combination therapy to an individual having a complement-mediated disease or disorder, inhibits complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the compounds described herein.

In some embodiments, an active compound or its salt or composition as described herein reduces C3 deposition onto red blood cells; for example, in some embodiments, an an active compound or its salt or composition as described herein reduces deposition of C3b, iC3b, etc., onto RBCs. In some embodiments, an active compound or its salt or composition as described herein inhibits complement-mediated red blood cell lysis.

In some embodiments, an active compound or its salt or composition as described herein reduces C3 deposition onto platelets; for example, in some embodiments, an active compound or its salt or composition as described herein reduces deposition of C3b, iC3b, etc., onto platelets.

In some embodiments, administering an active compound or its salt or composition as described herein results in an outcome selected from the group consisting of: (a) a reduction in complement activation; (b) an improvement in cognitive function; (c) a reduction in neuron loss; (d) a reduction in phospho-Tau levels in neurons; (e) a reduction in glial cell activation; (f) a reduction in lymphocyte infiltration; (g) a reduction in macrophage infiltration; (h) a reduction in antibody deposition, (i) a reduction in glial cell loss; (j) a reduction in oligodendrocyte loss; (k) a reduction in dendritic cell infiltration; (1) a reduction in neutrophil infiltration; (m) a reduction in red blood cell lysis; (n) a reduction in red blood cell phagocytosis; (o) a reduction in platelet phagocytosis; (p) a reduction in platelet lysis; (q) an improvement in transplant graft survival; (r) a reduction in macrophage mediated phagocytosis; (s) an improvement in vision; (t) an improvement in motor control; (u) an improvement in thrombus formation; (v) an improvement in clotting; (w) an improvement in kidney function; (x) a reduction in antibody mediated complement activation; (y) a reduction in autoantibody mediated complement activation; (z) an improvement in anemia; (aa) reduction of demyelination; (ab) reduction of eosinophilia; (ac) a reduction of C3 deposition on red blood cells (e.g., a reduction of deposition of C3b, iC3b, etc., onto RBCs); and (ad) a reduction in C3 deposition on platelets (e.g., a reduction of deposition of C3b, iC3b, etc., onto platelets); and (ae) a reduction of anaphylatoxin toxin production; (af) a reduction in autoantibody mediated blister formation; (ag) a reduction in autoantibody induced pruritis; (ah) a reduction in autoantibody induced erythematosus; (ai) a reduction in autoantibody mediated skin erosion; (aj) a reduction in red blood cell destruction due to transfusion reactions; (ak) a reduction in red blood cell lysis due to alloantibodies; (al) a reduction in hemolysis due to transfusion reactions; (am) a reduction in allo-antibody mediated platelet lysis; (an) a reduction in platelet lysis due to transfusion reactions; (ao) a reduction in mast cell activation; (ap) a reduction in mast cell histamine release; (aq) a reduction in vascular permeability; (ar) a reduction in edema; (as) a reduction in complement deposition on transplant graft endothelium; (at) a reduction of anaphylatoxin generation in transplant graft endothelium; (au) a reduction in the separation of the dermal-epidermal junction; (av) a reduction in the generation of anaphylatoxins in the dermal-epidermal junction; (aw) a reduction in alloantibody mediated complement activation in transplant graft endothelium; (ax) a reduction in antibody mediated loss of the neuromuscular junction; (ay) a reduction in complement activation at the neuromuscular junction; (az) a reduction in anaphylatoxin generation at the neuromuscular junction; (ba) a reduction in complement deposition at the neuromuscular junction; (bb) a reduction in paralysis; (bc) a reduction in numbness; (bd) increased bladder control; (be) increased bowel control; (bf) a reduction in mortality associated with autoantibodies; and (bg) a reduction in morbidity associated with autoantibodies.

In some embodiments, an active compound or its salt or composition as described herein, when administered in one or more doses as monotherapy or in combination therapy to an individual having a complement-mediated disease or disorder, is effect to achieve a reduction of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: (a) complement activation; (b) decline in cognitive function; (c) neuron loss; (d) phospho-Tau levels in neurons; (e) glial cell activation; (f) lymphocyte infiltration; (g) macrophage infiltration; (h) antibody deposition, (i) glial cell loss; (j) oligodendrocyte loss; (k) dendritic cell infiltration; (1) neutrophil infiltration; (m) red blood cell lysis; (n) red blood cell phagocytosis; (o) platelet phagocytosis; (p) platelet lysis; (q) transplant graft rejection; (r) macrophage mediated phagocytosis; (s) vision loss; (t) antibody mediated complement activation; (u) autoantibody mediated complement activation; (v) demyelination; (w) eosinophilia; compared to the level or degree of the outcome in the individual before treatment with the active compound.

In some embodiments, an active compound or its salt or composition as described herein, when administered in one or more doses as monotherapy or in combination therapy to an individual having a complement-mediated disease or disorder, is effect to achieve an improvement of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: a) cognitive function; b) transplant graft survival; c) vision; d) motor control; e) thrombus formation; f) clotting; g) kidney function; and h) hematocrit (red blood cell count), compared to the level or degree of the outcome in the individual before treatment with the active compound.

In some embodiments, administering an active compound or its salt or composition as described herein to an individual reduces complement activation in the individual. For example, in some embodiments, an active compound or its salt or composition as described herein, when administered in one or more doses as monotherapy or in combination therapy to an individual having a complement-mediated disease or disorder, reduces complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the active compound or its salt.

In some embodiments, administering an active compound or its salt or composition as described herein improves cognitive function in the individual. For example, in some embodiments, an active compound as described herein, when administered in one or more doses as monotherapy or in combination therapy to an individual having a complement-mediated disease or disorder, improves cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the cognitive function in the individual before treatment with the active compound.

In some embodiments, administering an active compound or its salt or composition as described herein reduces the rate of decline in cognitive function in the individual. For example, in some embodiments, an active compound or its salt, when administered in one or more doses as monotherapy or in combination therapy to an individual having a complement-mediated disease or disorder, reduces the rate of decline of cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the rate of decline in cognitive function in the individual before treatment with the active compound or its salt.

In some embodiments, administering an active compound or its salt or composition as described herein to an individual reduces neuron loss in the individual. For example, in some embodiments, an active compound or its salt, when administered in one or more doses as monotherapy or in combination therapy to an individual having a complement-mediated disease or disorder, reduces neuron loss in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to neuron loss in the individual before treatment with the active compound.

In some embodiments, administering an active compound or its salt or composition as described herein to an individual reduces phospho-Tau levels in the individual. For example, in some embodiments, an active compound or its salt, when administered in one or more doses as monotherapy or in combination therapy to an individual having a complement-mediated disease or disorder, reduces phospho-Tau in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the phospho-Tau level in the individual before treatment with the active compound or its salt.

In some embodiments, administering an active compound or its salt or composition as described herein to an individual reduces glial cell activation in the individual. For example, in some embodiments, an active compound or its salt, when administered in one or more doses as monotherapy or in combination therapy to an individual having a complement-mediated disease or disorder, reduces glial activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to glial cell activation in the individual before treatment with the active compound or its salt. In some embodiments, the glial cells are astrocytes or microglia.

In some embodiments, administering an active compound or its salt or composition as described herein to an individual reduces lymphocyte infiltration in the individual. For example, in some embodiments, an active compound or its salt, when administered in one or more doses as monotherapy or in combination therapy to an individual having a complement-mediated disease or disorder, reduces lymphocyte infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to lymphocyte infiltration in the individual before treatment with the active compound or its salt.

In some embodiments, administering an active compound or its salt or composition as described herein to an individual reduces macrophage infiltration in the individual. For example, in some embodiments, an active compound or its salt, when administered in one or more doses as monotherapy or in combination therapy to an individual having a complement-mediated disease or disorder, reduces macrophage infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to macrophage infiltration in the individual before treatment with the active compound or its salt.

In some embodiments, administering an active compound or its salt or composition as described herein to an individual reduces antibody deposition in the individual. For example, in some embodiments, an active compound or its salt, when administered in one or more doses as monotherapy or in combination therapy to an individual having a complement-mediated disease or disorder, reduces antibody deposition in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to antibody deposition in the individual before treatment with the active compound or its salt.

In some embodiments, administering an active compound or its salt or composition as described herein to an individual reduces anaphylatoxin (e.g., C3a, C4a, C5a) production in an individual. For example, in some embodiments, an active compound or its salt, when administered in one or more doses as monotherapy or in combination therapy to an individual having a complement-mediated disease or disorder, reduces anaphylatoxin production in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the level of anaphylatoxin production in the individual before treatment with the active compound or its salt.

The present disclosure provides for use of an active compound or its salt of the present disclosure or a pharmaceutical composition comprising an active compound or its salt of the present disclosure and a pharmaceutically acceptable excipient to treat an individual having a complement-mediated disease or disorder. In some embodiments, the present disclosure provides for use of an active compound or its salt of the present disclosure to treat an individual having a complement-mediated disease or disorder. In some embodiments, the present disclosure provides for use of a pharmaceutical composition comprising an active compound or its salt of the present disclosure and a pharmaceutically acceptable excipient to treat an individual having a complement-mediated disease or disorder.

### COMBINATION THERAPY

In some embodiments, an active compound or its salt or composition as described herein may be provided in combination or alternation with, or preceded by, concomitant with or followed by, an effective amount of at least one additional therapeutic agent, for example, for treatment of a disorder listed herein. Non-limiting examples of second active agents for such combination therapy are provided below.

In some embodiments, an active compound or its salt or composition as described herein may be provided in combination or alternation with at least one additional inhibitor of the complement system or a second active compound with a different biological mechanism of action. In the description below and herein generally, whenever any of the terms referring to an active compound or its salt or composition as described herein are used, it should be understood that pharmaceutically acceptable salts or compositions are considered included, unless otherwise stated or inconsistent with the text.

In non-limiting embodiments, an active compound or its salt or composition as described herein may be provided together with a protease inhibitor, a soluble complement regulator, a therapeutic antibody (monoclonal or polyclonal), complement component inhibitor, receptor agonist, or siRNA.

In other embodiments, an active compound described herein is administered in combination or alternation with an antibody against tumor necrosis factor (TNF), including but not limited to infliximab (Remicade), adalimumab, certolizumab, golimumab, or a receptor fusion protein such as etanercept (Embrel).

In one embodiment, an active compound as described herein can be administered in combination or alternation with an anti-CD20 antibody, including but not limited to rituximab (Rituxan), adalimumab (Humira), ofatumumab (Arzerra), tositumomab (Bexxar), obinutuzumab (Gazyva), or ibritumomab (Zevalin).

In one embodiment, an active compound as described herein can be administered in combination or alternation with an anti-IL6 antibody, including but not limited to tocilizumab (Actemra) and siltuximab (Sylvant).

In one embodiment, an active compound as described herein can be administered in combination or alternation with an IL 17 inhibitor, including but not limited to secukibumab (Cosentyx).

In one embodiment, an active compound as described herein can be administered in combination or alternation with a p40 (IL 12/IL23) inhibitor, including but not limited to ustekinumab (Stelara).

In one embodiment, an active compound as described herein can be administered in combination or alteration with an IL23 inhibitor, including but not limited to risankizumab.

In one embodiment, an active compound as described herein can be administered in combination or alteration with an anti-interferon α antibody, for example but not limited to sifalimumab.

In one embodiment, an active compound as described herein can be administered in combination or alteration with a kinase inhibitor, for example but not limited to a JAK1/JAK3 inhibitor, for example but not limited to tofacitinib (Xelianz). In an alternative embodiment, an active compound as described herein can be administered in combination or alteration with a JAK1/JAK2 inhibitor, for example but not limited to baracitibib.

In one embodiment, an active compound as described herein can be administered in combination or alteration with an anti-VEGF agent, for example but not limited to: aflibercept (Eylea^{®}; Regeneron Pharmaceuticals); ranibizumab (Lucentis^{®}: Genentech and Novartis); pegaptanib (Macugen^{®}; OSI Pharmaceuticals and Pfizer); bevacizumab (Avastin; Genentech/Roche); lapatinib (Tykerb); sunitinib (Sutent); axitinib (Inlyta); pazopanib; sorafenib (Nexavar); ponatinib (Inclusig); regorafenib (Stivarga); cabozantinib (Abometyx; Cometriq); vendetanib (Caprelsa); ramucirumab (Cyramza); lenvatinib (Lenvima); ziv-aflibercept (Zaltrap); cediranib (Recentin); anecortane acetate, squalamine lactate, and corticosteroids.

In another embodiment, an active compound as described herein can be administered in combination or alternation with an immune checkpoint inhibitor. Non-limiting examples of checkpoint inhibitors include anti-PD-1 or anti-PDL1 antibodies, for example, nivolumab (Opdivo), pembrolizumab (Keytruda), pidilizumab, AMP-224 (AstraZeneca and MedImmune), sasanlimab (PF-06801591) (Pfizer), MEDI0680 (AstraZeneca), PDR001 (Novartis), cemiplimab (REGN2810) (Regeneron), SHR-12-1 (Jiangsu Hengrui Medicine Company and Incyte Corporation), TSR-042 (Tesaro), sintilimab, tislelizumab, toripalimab, CK-301 (Checkpoint Therapeutics), atezolizumab, durvalumab, avelumab, and KN035. and the PD-L1/VISTA inhibitor CA-170 (Curis Inc.), or anti-CTLA4 antibodies, for example Ipilimumab, Tremelimumab, AGEN1884 and AGEN2041 (Agenus).

Non-limiting examples of active agents that can be used in combination with active compounds described herein are:
Protease inhibitors: plasma-derived C1-INH concentrates, for example Cetor^{®} (Sanquin), Berinert-P^{®} (CSL Behring, Lev Pharma), and Cinryze^{®}; recombinant human C1-inhibitors, for example Rhucin^{®}; ritonavir (Norvir^{®}, Abbvie, Inc.);
Complement regulators: Soluble complement receptor 1 (TP10) (Avant Immunotherapeutics); sCR1-sLe^{X}/TP-20 (Avant Immunotherapeutics); MLN-2222 /CAB-2 (Millenium Pharmaceuticals); Mirococept (Inflazyme Pharmaceuticals); Therapeutic antibodies: Eculizumab/Soliris and Ravulizumab/Ultomiris (Alexion Pharmaceuticals); Pexelizumab (Alexion Pharmaceuticals); BCD-148 (Biocad); ABP-959 (Amgen); SB-12 (Samsung Bioepis Co., Ltd.); Ofatumumab (Genmab A/S); TNX-234 (Tanox); TNX-558 (Tanox); TA106 (Taligen Therapeutics); Neutrazumab (G2 Therapies); Anti-properdin (Novelmed Therapeutics); HuMax-CD38 (Genmab A/S);
Complement component inhibitors: Compstatin/POT-4 (Potentia Pharmaceuticals); ARC1905 (Archemix); 4(1MEW)APL-1,APL-2 (Appelis); CP40/AMY-101,PEG-Cp40 (Amyndas);
PDGF inhibitors: Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; Anti-factor H or anti-factor B agents: Anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas);
Complement C3 or CAP C3 Convertase targeting molecules: TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH; Anti-CR3, anti-MASP2, anti C1s, and anti-C1n molecules: Cynryze (ViroPharma/Baxter); TNT003 (True North); OMS721 (Omeros); OMS906 (Omeros); and Imprime PGG (Biothera);
Receptor agonists: PMX-53 (Peptech Ltd.); JPE-137 (Jerini); JSM-7717 (Jerini); and
Others: Recombinant human MBL (rhMBL; Enzon Pharmaceuticals); Imides and glutarimide derivatives such as thalidomide, lenalidomide, pomalidomide. Additional non-limiting examples that can be used in combination or alternation with an active compound or its salt or composition as described herein include the following:

| **Non-limiting examples for combination therapy** | | | |
|---|---|---|---|
| **Name** | **Target** | **Company** | **Class of Molecule** |
| LFG316 | C5 | Novartis/Morphosys | Monoclonal antibody |
| SB12 | C5 | Samsung Bioepsis | |
| IONIS-FB-LRx | CFB | Ionis | Antisense Inhibitor |
| 4(1MEW)APL-1,APL-2 | C3/C3b | Apellis | Compstatin Family |
| 4(1MeW)POT-4 | C3/C3b | Potentia | Compstatin Family |
| Anti-C5 siRNA | C5 | Alnylam | Si-RNA |
| Anti-FB siRNA | CFB | Alnylam | SiRNA |
| ARC1005 | C5 | Novo Nordisk | Aptamers |
| ATA | C5 | N.A. | Chemical |
| CCX168 (avocopan) | C5a Receptor | ChemoCentryx | Ligand |
| Coversin (nomacopan) | C5 | Akari Therapeutcs | recombinant small protein |
| CP40/AMY-101,PEG-Cp40 | C3/C3b | Amyndas | Compstatin Family |
| CRIg/CFH | CAP C3 convertase | NA | CFH-based protein |
| Cynryze | C1n/C1s | ViroPharma/Baxter | Human purified protein |
| FCFD4514S | CFD | Genentech/Roche | Monoclonal antibody |
| H17 | C3 (C3b/iC3b) | EluSys Therapeutics | Monoclonal antibody |
| IFX-1 (CaCp29) | C5a | InflaRx | Monoclonal antibody |
| Mini-CFH | CAP C3 convertase | Amyndas | CFH-based protein |
| Mirococept (APT070) | CAP and CCP C3 | NA | CR1-based protein |
| Zilucoplan (RA101348) | C5 | Ra Pharma | macrocylic peptide |
| sCR1 (CDX-1135) | CAP and CP C3 | Celldex | CR1-based protein |
| SOBI002 | C5 | Swedish Orphan Biovitrum | Affibody |
| SOMAmers | C5 | SomaLogic | Aptamers |
| SOMAmers | CFB and CFD | SomaLogic | Aptamers (SELEX) |
| TA106 | CFB | Alexion Pharmaceuticals | Monoclonal antibody |
| TNT003 | C1s | True North | Monoclonal antibody |
| TT30 (CR2/CFH) | CAP C3 convertase | Alexion | CFH-based protein |
| TT32 (CR2/CR1) | CAP and CCP C3 | Alexion Pharmaceuticals | CR1-based protein |
| Nafamostat (FUT-175, Futhan) | C1s, CFD, other proteases | Torri Pharmaceuticals | Small molecule |
| OMS721 | MASP-2 | Omeros | Monoclonal antibody |
| OMS906 | MASP-2 | Omeros | Monoclonal antibody |
| Bikaciomab, NM9308 | CFB | Novelmed | Monoclonal antibody |
| NM9401 | Properdin | Novelmed | Monoclonal antibody |
| CVF, HC-1496 | C3 | InCode | Recombinant peptide |
| ALXN1102/ALXN1103 (TT30) | C3-conv, C3b | Alexion Pharmaceuticals | Regulator |
| rFH | C3-conv, C3b | Optherion | Regulator |
| 5C6, AMY-301 | CFH | Amyndas | Regulator |
| Mubodina | C5 | Adienne Pharma | Monoclonal antibody |
| ARC1905 | C5 | Opthotech | Monoclonal Antibody |
| MEDI7814 | C5/C5a | MedImmune | Monoclonal Antibody |
| NOX-D19 | C5a | Noxxon | Aptamer (Spiegelmer) |
| IFX-1, CaCP29 | C5a | InflaRx | Monoclonal Antibody |
| PMX53, PMX205 | C5aR | Cephalon, Teva | Peptidomimetic |
| CCX168 | C5aR | ChemoCentryx | Small molecule |
| ADC-1004 | C5aR | Alligator Bioscience | Small molecule |
| Anti-C5aR-151, NN8209; Anti-C5aR-215, NN8210 | C5aR | Novo Nordisk | Monoclonal Antibody |
| Imprime PGG | CR3 | Biothera | Soluble beta-glucan |
| ANX005; ANX007 | C1q | Annexon | Monoclonal Antibody |
| LNP023 | FB | Novartis | Small molecule |
| Lampalizumab | fD | Roche | Monoclonal Antibody |
| avacincaptad pegol | C5 | Opthotech | Aptamer |
| regenemab | C6 | Regenesance | Monoclonal Antibody |
| BIVV020 | C1s | Bioverativ | Monoclonal Antibody |
| PRO-02 | C2 | Broteio/Argen-x | Monoclonal Antibody |
| 5C6, compsorbin | fH | Amyndas | Peptide |
| SOBI005 | C5 | Sobi | Protein |
| ISU305 | C5 | ISU ABXIS | Monoclonal Antibody |
| IFX-2, IFX-3 | C5a | InflaRx | Monoclonal Antibody |
| ALS-205 | C5aR1 | Alsonex | Peptide |
| DF2593A | C5aR1 | Dompé | Small Molecule |
| IPH5401 | C5aR1 | Innate Pharma | Monoclonal Antibody |
| C6-LNA | C6 | Regenesance | Oligonucleotide |
| Pozelimab (REGN3918) | C5 | Regeneron | Monoclonal Antibody |
| Aptamers to Factor D | fD | Vitrisa Therapeutics | Aptamer |
| CLG561 | Properdin | Novartis | Monoclonal Antibody |
| Tesidolumab; LFG316 | C5 | Novartis and MorphoSys | Monoclonal Antibody |
| Cemdisiran (ALN-CC5) | C5 | Alnylam | RNAi |
| Crovalimab (SKY59) | C5 | Roche/Chugai | Monoclonal antibody |

In one embodiment, the agent for combination therapy is a biosimilar of any agent named above.

In some embodiments, an active compound or its salt or composition as described herein may be provided together with a compound that inhibits an enzyme that metabolizes an administered protease inhibitor. In some embodiments, a compound or salt may be provided together with ritonavir.

In some embodiments, an active compound or its salt or composition as described herein may be provided in combination with a terminal complement inhibitor, for example a complement C5 inhibitor or C5 convertase inhibitor. C5 and C5 convertase inhibitors include, but are not limited to, eculizumab (Soliris^{®}; Alexion), ravulizumab (Ultomiris^{®}; Alexion), cemdisiran (Alnylan); prozelimab (Regeneron); BCD-148 (Biocad); ABP-959 (Amgen); SB-12 (Samsung Bioepis Co., Ltd.); LFG316 (Novartis); coversin (nomacopan; Akari)); zilucoplan (Ra Pharma); crovalimab (SKY59; Roche/Chugai); and mubodina (Adienne Pharma). In certain complement disorders, for example paroxysmal nocturnal hemoglobinuria (PNH), it has been discovered that targeting the terminal complement component C5 alone-which is the current standard of care treatment-may result in an inadequate inhibition of the complement system For example, in PNH patients, it has been discovered that treatment with an anti-C5 inhibitor, for example, a anti-C5 inhibitor, results in a heterogenous response, with a significant occurrence of intravascular hemolysis due to pharmacokinetic and pharmacodynamic breakthrough, and extravascular hemolysis due to C3-mediated extravascular hemolysis. Such inadequate responses can be clinically significant and life threatening. By administering a therapeutic regimen that includes a anti-C5 inhibitor and an active compound or its salt or composition as described herein, both the proximal and terminal components of complement mediated disorders can be targeted, thereby reducing breakthrough or tick-over activation and drastically improving therapeutic response.

In some embodiments, an active compound or its salt or composition as described herein may be provided in combination with eculizumab, a monoclonal antibody directed to the complement factor C5 and manufactured and marketed by Alexion Pharmaceuticals under the tradename Soliris, or a biosimilar thereof. Eculizumab has been approved by the U.S. FDA for the treatment of PNH, aHUS, neuromyelitis optica spectrum disorder in adults who are anti-aquaporin-4 (AQP4) antibody positive, and individuals with generalized myasthenia gravis who are anti-acetylcholine receptor (AchR) antibody positive. Eculizumab is generally administered as a loading dose of between 600 and 900 mg weekly for 4 weeks, followed by a fifth dose of between 900 and 1200 mg 1 week later, followed by a dose of between 900 and 12200 mg every 2 weeks thereafter, wherein the dose is dependent on the indication being treated. In certain embodiments herein, administration of the complement factor D inhibitors described herein in combination with a eculizumab treatment regimen allows for a reduction in the eculizumab dose administered to the subject.

In another embodiment, an active compound or its salt or composition as described herein may be provided in combination with ravulizumab, a monoclonal antibody directed to the complement factor C5 and manufactured and marketed by Alexion Pharmaceuticals under the tradename Ultomiris^{®}, or a biosimilar thereof. Ravulizumab was designed to exploit targeted reengineering of eculizumab: two histidine switches were included in the complementary determining regions (CDRs) to promote more efficient pH-dependent dissociation of the mAb:C5 complex, and two additional amino acides changes were included in the Fc region to increase the affinity for the FcRn. Ravulizumab has exhibited a reduced target-dependent drug disposition (TDDM) and a longer half-life as compared to its parental molecule eculizumab. Ravulizumab-cwvz ("ravulizumab") has been approved by the U.S. FDA for the treatment of PNH and aHUS to inhibit complement-mediated thrombotic microangiopathy (TMA). Ravulizumab is generally administered as a loading dose of between 600 and 3000 mg, followed by a maintenance dose between 300 mg and 3600 mg once every eight weeks, dependent on body weight and indication. In certain embodiments herein, administration of the complement factor D inhibitors described herein in combination with a ravulizumab treatment regimen allows for a reduction in the ravulizumab dose administered to the subject.

In some embodiments, an active compound or its salt or composition as described herein is administered in combination with an anti-inflammatory drug, antimicrobial agent, anti-angiogenesis agent, immunosuppressant, antibody, steroid, ocular antihypertensive drug or combinations thereof. Examples of such agents include amikacin, anecortane acetate, anthracenedione, anthracycline, an azole, amphotericin B, bevacizumab, camptothecin, cefuroxime, chloramphenicol, chlorhexidine, chlorhexidine digluconate, clortrimazole, a clotrimazole cephalosporin, corticosteroids, dexamethasone, desamethazone, econazole, eftazidime, epipodophyllotoxin, fluconazole, flucytosine, fluoropyrimidines, fluoroquinolines, gatifloxacin, glycopeptides, imidazoles, itraconazole, ivermectin, ketoconazole, levofloxacin, macrolides, miconazole, miconazole nitrate, moxifloxacin, natamycin, neomycin, nystatin, ofloxacin, polyhexamethylene biguanide, prednisolone, prednisolone acetate, pegaptanib, platinum analogs, polymicin B, propamidine isethionate, pyrimidine nucleoside, ranibizumab, squalamine lactate, sulfonamides, triamcinolone, triamcinolone acetonide, triazoles, vancomycin, anti-vascular endothelial growth factor (VEGF) agents, VEGF antibodies, VEGF antibody fragments, vinca alkaloid, timolol, betaxolol, travoprost, latanoprost, bimatoprost, brimonidine, dorzolamide, acetazolamide, pilocarpine, ciprofloxacin, azithromycin, gentamycin, tobramycin, cefazolin, voriconazole, gancyclovir, cidofovir, foscarnet, diclofenac, nepafenac, ketorolac, ibuprofen, indomethacin, fluoromethalone, rimexolone, anecortave, cyclosporine, methotrexate, tacrolimus, anti-PDGFR molecule, and combinations thereof.

In some embodiments of the present disclosure, an active compound or its salt or composition as described herein can be administered in combination or alternation with at least one immunosuppressive agent. The immunosuppressive agent as non-limiting examples, may be a calcineurin inhibitor, e.g. a cyclosporin or an ascomycin, e.g. Cyclosporin A (NEORAL^{®}), FK506 (tacrolimus), pimecrolimus, a mTOR inhibitor, e.g. rapamycin or a derivative thereof, e.g. Sirolimus (RAPAMUNE^{®}), Everolimus (Certican^{®}), temsirolimus, zotarolimus, biolimus-7, biolimus-9, a rapalog, e.g.ridaforolimus, azathioprine, campath 1H, a S1P receptor modulator, e.g. fingolimod or an analog thereof, an anti IL-8 antibody, mycophenolic acid or a salt thereof, e.g. sodium salt, or a prodrug thereof, e.g., Mycophenolate Mofetil (CELLCEPT^{®}), OKT3 (ORTHOCLONE OKT3^{®}), Prednisone, ATGAM^{®}, THYMOGLOBULIN^{®}, Brequinar Sodium, OKT4, T10B9.A-3A, 33B3.1, 15-deoxyspergualin, tresperimus, Leflunomide ARAVA^{®}, CTLAI-Ig, anti-CD25, anti-IL2R, Basiliximab (SIMULECT^{®}), Daclizumab (ZENAPAX^{®}), mizorbine, methotrexate, dexamethasone, ISAtx-247, SDZ ASM 981 (pimecrolimus, Elidel^{®}), CTLA4lg (Abatacept), belatacept, LFA3lg, etanercept (sold as Enbrel^{®} by Immunex), adalimumab (Humira^{®}), infliximab (Remicade^{®}), an anti-LFA-1 antibody, natalizumab (Antegren^{®}), Enlimomab, gavilimomab, antithymocyte immunoglobulin, siplizumab, Alefacept efalizumab, pentasa, mesalazine, asacol, codeine phosphate, benorylate, fenbufen, naprosyn, diclofenac, etodolac and indomethacin, tocilizumab (Actemra), siltuximab (Sylvant), secukibumab (Cosentyx), ustekinumab (Stelara), risankizumab, sifalimumab, aspirin, and ibuprofen.

Examples of anti-inflammatory agents include methotrexate, dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine, aspirin, ibuprofen, suprofen, piroxicam, meloxicam, flubiprofen, naproxan, ketoprofen, tenoxicam, diclofenac sodium, ketotifen fumarate, diclofenac sodium, nepafenac, bromfenac, flurbiprofen sodium, suprofen, celecoxib, naproxen, rofecoxib, glucocorticoids, diclofenac, and any combination thereof. In some embodiments, an active compound or its salt or composition as described herein is combined with one or more non-steroidal anti-inflammatory drugs (NSAIDs) selected from naproxen sodium (Anaprox^{®}), celecoxib (Celebrex^{®}), sulindac (Clinoril^{®}), oxaprozin (Daypro^{®}), salsalate (Disalcid^{®}), diflunisal (Dolobid^{®}), piroxicam (Feldene^{®}), indomethacin (Indocin^{®}), etodolac (Lodine^{®}), meloxicam (Mobic^{®}), naproxen (Naprosyn^{®}), nabumetone (Relafen^{®}), ketorolac tromethamine (Toradol^{®}), naproxen/esomeprazole (Vimovo^{®}), and diclofenac (Voltaren^{®}), and combinations thereof.

In some embodiments, an active compound or its salt or composition as described herein is administered in combination or alteration with an omega-3 fatty acid or a peroxisome proliferator-activated receptor (PPARs) agonist. Omega-3 fatty acids are known to reduce serum triglycerides by inhibiting DGAT and by stimulating peroxisomal and mitochondrial beta oxidation. Two omega-3 fatty acids, eicosapentaenoic acid (EPA) and docosahexaenoic acid (DHA), have been found to have high affinity for both PPAR-alpha and PPAR-gamma. Marine oils, e.g., fish oils, are a good source of EPA and DHA, which have been found to regulate lipid metabolism. Omega-3 fatty acids have been found to have beneficial effects on the risk factors for cardiovascular diseases, especially mild hypertension, hypertriglyceridemia and on the coagulation factor VII phospholipid complex activity. Omega-3 fatty acids lower serum triglycerides, increase serum HDL- cholesterol, lower systolic and diastolic blood pressure and the pulse rate, and lower the activity of the blood coagulation factor VII-phospholipid complex. Further, omega-3 fatty acids seem to be well tolerated, without giving rise to any severe side effects. One such form of omega-3 fatty acid is a concentrate of omega-3, long chain, polyunsaturated fatty acids from fish oil containing DHA and EPA and is sold under the trademark Omacor^{®}. Such a form of omega-3 fatty acid is described, for example, in U.S. Patent Nos. 5,502,077, 5,656,667, and 5,698,594.

Peroxisome proliferator-activated receptors (PPARs) are members of the nuclear hormone receptor superfamily ligand-activated transcription factors that are related to retinoid, steroid and thyroid hormone receptors. There are three distinct PPAR subtypes that are the products of different genes and are commonly designated PPAR-alpha, PPAR-beta/delta (or merely, delta) and PPAR-gamma. General classes of pharmacological agents that stimulate peroxisomal activity are known as PPAR agonists, e.g., PPAR-alpha agonists, PPAR-gamma agonists and PPAR-delta agonists. Some pharmacological agents are combinations of PPAR agonists, such as alpha/gamma agonists, etc., and some other pharmacological agents have dual agonist/antagonist activity. Fibrates such as fenofibrate, bezafibrate, clofibrate and gemfibrozil, are PPAR-alpha agonists and are used in patients to decrease lipoproteins rich in triglycerides, to increase HDL and to decrease atherogenic-dense LDL. Fibrates are typically orally administered to such patients. Fenofibrate or 2-[4-(4-chlorobenzoyl)phenoxy]-2-methyl-propanoic acid, 1-methylethyl ester, has been known for many years as a medicinally active principle because of its efficacy in lowering blood triglyceride and cholesterol levels.

In some embodiments, the present disclosure provides an active compound or its salt or composition as described herein for use in a method of treating or preventing age-related macular degeneration (AMD), the method comprising administering to a subject in need thereof an effective amount of the active compound or its salt or composition as described herein in combination with an anti-VEGF agent. Non-limiting examples of anti-VEGF agents include, but are not limited to, aflibercept (Eylea^{®}; Regeneron Pharmaceuticals); ranibizumab (Lucentis^{®}: Genentech and Novartis); pegaptanib (Macugen^{®}; OSI Pharmaceuticals and Pfizer); bevacizumab (Avastin^{®}; Genentech/Roche); lapatinib (Tykerb^{®}); sunitinib (Sutent^{®}); axitinib (Inlyta^{®}); pazopanib; sorafenib (Nexavar^{®}); ponatinib (Inclusig^{®}); regorafenib (Stivarga^{®}); Cabozantinib (Abometyx^{®}; Cometriq^{®}); vendetanib (Caprelsa^{®}); ramucirumab (Cyramza^{®}); lenvatinib (Lenvima^{®}); ziv-aflibercept (Zaltrap^{®}); cediranib (Recentin^{®}); anecortane acetate, squalamine lactate, and corticosteroids, including, but not limited to, triamcinolone acetonide.

In some embodiments, the present disclosure provides an active compound or its salt or composition as described herein for use in a method of treating or preventing age-related macular degeneration (AMD), the method comprising administering to a subject in need thereof an effective amount of the active compound or its salt or composition as described herein in combination with a complement C5 inhibitor, for example, a complement C5 inhibitor described herein and in the table above titled Non-limiting examples of potential therapeutics for combination therapy, including, but not limited to, eculizumab; ravulizumab; LFG316 (Novartis/Morphosys); cemdisiran (Alnylan); ARC1005 (Novo Nordisk); Mubodine (Adienne Pharma); RA101348 (Ra Pharma); SOBI002 (Swedish Orphan Biovitrum); SOMAmers (SomaLogic); Erdigna (Adienne Pharma); ARC1905 (Opthotech); MEDI7814 (MedImmune); NOX-D19 (Noxxon); IFX-1, CaCP29 (InflaRx); PMX53, PMX205 (Cephalon, Teva); CCX168 (ChemoCentryx); ADC-1004 (Alligator Bioscience); and Anti-C5aR-151, NN8209; Anti-C5aR-215, NN8210 (Novo Nordisk); prozelimab (Regeneron); BCD-148 (Biocad); ABP-959 (Amgen); SB-12 (Samsung Bioepis Co., Ltd.); coversin (nomacopan; Akari)); zilucoplan (Ra Pharma); and crovalimab (SKY59; Roche/Chugai).

In some embodiments, the present disclosure provides an active compound or its salt or composition as described herein for use in a method of treating or preventing age-related macular degeneration (AMD), the method comprisingadministering to a subject in need thereof an effective amount of the active compound or its salt or composition as described herein in combination with anti-properidin agent, for example, an anti-properidin agent as described above, including but not limited to NM9401 (Novelmed).

In some embodiments, the present disclosure provides an active compound or its salt or composition as described herein for use in a method of treating or preventing age-related macular degeneration (AMD), the method comprising administering to a subject in need thereof an effective amount of the active compound or its salt or composition as described herein in combination with a complement C3 inhibitor for example, a complement C3 inhibitor described above, including, but not limited to, a compstatin or compstatin analog, for example Compstatin/POT-4 (Potentia Pharmaceuticals); ARC1905 (Archemix); 4(1MEW)APL-1, APL-2 (Appelis); CP40/AMY-101, PEG-Cp40 (Amyndas) Complement C3 or CAP C3 Convertase targeting molecules: TT30 (CR2/FH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) rFH (Optherion); H17 C3 (C3b/iC3b) (EluSys Therapeutics); Mini-CFH (Amyndas) Mirococept (APT070); sCR1 (CDX-1135) (Celldex); and CRIg/CFH.

In some embodiments, the present disclosure provides an active compound or its salt or composition as described herein for use in a method of treating or preventing age-related macular degeneration (AMD), the method comprising administering to a subject in need thereof an effective amount of the active compound or its salt or composition as described herein in combination with an anti-factor H or anti-factor B agent selected from Anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas).

In some embodiments, the present disclosure provides an active compound or its salt or composition as described herein for use in a method of treating or preventing age-related macular degeneration (AMD), the method comprising administering to a subject in need thereof an effective amount of the active compound or its salt or composition as described herein in combination with an anti-MASP2, anti-C1s or anti-CR3 molecules, for example, but not limited to: Cynryze (ViroPharma/Baxter); TNT003 (True North); OMS721 (Omeros); OMS906 (Omeros); and Imprime PGG (Biothera).

In some embodiments, the present disclosure provides an active compound or its salt or composition as described herein for use in a method of treating or preventing age-related macular degeneration (AMD), the method comprising administering to a subject in need thereof the effective amount of an active compound or its salt or composition as described herein in combination with an PDGF inhibitor, for example as described herein including but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil.

In some embodiments, the present disclosure provides an active compound or its salt or composition as described herein for use in a method of treating or preventing paroxysmal nocturnal hemoglobinuria (PNH), the method comprising administering to a subject in need thereof an effective amount of the active compound or its salt or composition as described herein with an additional inhibitor of the complement system or another active compound with a different biological mechanism of action. In another embodiment, the present disclosure provides an active compound or its salt or composition as described herein for use in a method of treating or preventing paroxysmal nocturnal hemoglobinuria (PNH), the method comprising administering to a subject in need thereof an effective amount of the active compound or its salt or composition as described herein in combination or alternation with eculizumab or ravulizumab. In another embodiment, the present disclosure provides an active compound or its salt or composition as described herein for use in a method of treating or preventing paroxysmal nocturnal hemoglobinuria (PNH), the method comprising administering to a subject in need thereof an effective amount of the active compound or its salt or composition as described herein in combination or alternation with CP40. In some embodiments, the additional agent is PEGylated-CP40. CP40 is a peptide inhibitor that shows a strong binding affinity for C3b and inhibits hemolysis of paroxysmal nocturnal hemoglobinuria (PNH) erythrocytes. In some embodiments, the additional agent is a complement component inhibitor, for example but not limited to Compstatin/POT-4 (Potentia Pharmaceuticals); ARC1905 (Archemix); 4(1MEW)APL-1,APL-2 (Appelis); CP40/AMY-101,PEG-Cp40 (Amyndas); a PDGF inhibitor, for example, but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; an anti-factor H or anti-factor B agent, for example anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas); a complement C3 or CAP C3 convertase targeting molecule, for example but not limited to TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH, an anti-CR3, anti-MASP2, anti C1s, or anti-C1n molecule, for example but not limited to Cynryze (ViroPharma/Baxter); TNT003 (True North); OMS721 (Omeros); OMS906 (Omeros); and Imprime PGG

### (Biothera)

In some embodiments, the present disclosure provides a composition for use in a method of treating or preventing rheumatoid arthritis, the method comprising administering to a subject in need thereof an effective amount of a composition, wherein the composition comprises an active compound or its salt or composition as described herein in combination or alternation with an additional inhibitor of the complement system, or an active agent that functions through a different mechanism of action. In another embodiment, the present disclosure provides an active compound or its salt or composition as described herein for use in a method of treating or preventing rheumatoid arthritis, the method comprising administering to a subject in need thereof an effective amount of the active compound or its salt or composition as described herein in combination or alternation with methotrexate. In certain embodiments, an active compound or its salt or composition as described herein is administered in combination or alternation with at least one additional therapeutic agent selected from: salicylates including aspirin (Anacin, Ascriptin, Bayer Aspirin, Ecotrin) and salsalate (Mono-Gesic, Salgesic); nonsteroidal anti-inflammatory drugs (NSAIDs); nonselective inhibitors of the cyclo-oxygenase (COX-1 and COX-2) enzymes, including diclofenac (Cataflam, Voltaren), ibuprofen (Advil, Motrin), ketoprofen (Orudis), naproxen (Aleve, Naprosyn), piroxicam (Feldene), etodolac (Lodine), indomethacin, oxaprozin (Daypro), nabumetone (Relafen), and meloxicam (Mobic); selective cyclo-oxygenase-2 (COX-2) inhibitors including Celecoxib (Celebrex); disease-modifying antirheumatic drugs (DMARDs), including azathioprine (Imuran), cyclosporine (Sandimmune, Neoral), gold salts (Ridaura, Solganal, Aurolate, Myochrysine), hydroxychloroquine (Plaquenil), leflunomide (Arava), methotrexate (Rheumatrex), penicillamine (Cuprimine), and sulfasalazine (Azulfidine); biologic drugs including abatacept (Orencia), etanercept (Enbrel), infliximab (Remicade), adalimumab (Humira), and anakinra (Kineret); corticosteroids including betamethasone (Celestone Soluspan), cortisone (Cortone), dexamethasone (Decadron), methylprednisolone (SoluMedrol, DepoMedrol), prednisolone (Delta-Cortef), prednisone (Deltasone, Orasone), and triamcinolone (Aristocort); gold salts, including Auranofin (Ridaura); Aurothioglucose (Solganal); Aurolate; Myochrysine; or any combination thereof.

In some embodiments, the present disclosure provides an active compound or its salt or composition as described herein a method of treating or preventing multiple sclerosis, the method comprising administering to a subject in need thereof an effective amount of the active compound or its salt or composition as described herein in combination or alternation with an additional inhibitor of the complement system, or an active agent that functions through a different mechanism of action. In another embodiment, the present disclosure provides an active compound or its salt or composition as described herein a method of treating or preventing multiple sclerosis, the method comprising administering to a subject in need thereof an effective amount of the active compound or its salt or composition as described herein in combination or alternation with a corticosteroid. Examples of corticosteroids include, but are not limited to, prednisone, dexamethasone, solumedrol, and methylprednisolone. In some embodiments, an active compound or its salt or composition as described herein is combined with at least one anti-multiple sclerosis drug, for example, selected from: Aubagio (teriflunomide), Avonex (interferon beta-1a), Betaseron (interferon beta-1b), Copaxone (glatiramer acetate), Extavia (interferon beta-1b), Gilenya (fingolimod), Lemtrada (alemtuzumab), Novantrone (mitoxantrone), Plegridy (peginterferon beta-1a), Rebif (interferon beta-1a), Tecfidera (dimethyl fumarate), Tysabri (natalizumab), Solu-Medrol (methylprednisolone), High-dose oral Deltasone (prednisone), H.P. Acthar Gel (ACTH), and a combination thereof.

In some embodiments, an active compound or its salt or composition as described herein is useful in a combination with another pharmaceutical agent to ameliorate or reduce a side effect of the agent. For example, in some embodiments, an active compound or its salt or composition as described herein may be used in combination with adoptive cell transfer therapies to reduce an associated inflammatory response associated with such therapies, for example, a cytokine mediated response such as cytokine release syndrome. In some embodiments, the adoptive cell transfer therapy includes the use of a chimeric antigen receptor T-Cell (CAR T). In some embodiments, the adoptive cell transfer therapy includes the use of a chimeric antigen receptor T-Cell (CAR T) or a dendritic cell to treat a hematologic or solid tumor, for example, a B-cell related hematologic cancer. In some embodiments, the hematologic or solid tumor is acute lymphoblastic leukemia (ALL), acute myeloid leukemia (AML), non-Hodgkin's lymphoma, chronic lymphocytic leukemia (CLL), pancreatic cancer, glioblastoma, or a cancer that expresses CD19.

In an additional alternative embodiment, an active compound or its salt or composition as described herein may be provided in combination with eculizumab or ravulizumab for the treatment of PNH, aHUSs, STEC-HUS, ANCA-vasculitis, AMD, CAD, C3 glomerulopathy, for example DDD or C3GN, chronic hemolysis, neuromyelitis optica, neuromyelitis optica spectrum disorder in adults who are anti-aquaporin-4 (AQP4) antibody positive, myasthenia gravis, generalized myasthenia gravis or transplantation rejection. In some embodiments, an active compound or its salt or composition as described herein may be provided in combination with compstatin or a compstatin derivative for the treatment of PNH, aHUSs, STEC-HUS, ANCA-vasculitis, AMD, CAD, C3 glomerulopathy, for example DDD or C3GN, chronic hemolysis, neuromyelitis optica, neuromyelitis optica spectrum disorder in adults who are anti-aquaporin-4 (AQP4) antibody positive, myasthenia gravis, generalized myasthenia gravis, or transplantation rejection. In some embodiments, the additional agent is a complement component inhibitor, for example but not limited to Compstatin/POT-4 (PotentiaPharmaceuticals); ARC1905 (Archemix); 4(1MEW)APL-1,APL-2 (Appelis); CP40/AMY-101,PEG-Cp40 (Amyndas); a PDGF inhibitor, for example, but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; an anti-factor H or anti-factor B agent, for example anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas); a complement C3 or CAP C3 convertase targeting molecule, for example but not limited to TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH, an anti-CR3, anti-MASP2, anti C1s, or anti-C1n molecule, for example but not limited to Cymyze (ViroPharma/Baxter); TNT003 (True North); OMS721 (Omeros); OMS906 (Omeros); and Imprime PGG (Biothera).

In some embodiments, an active compound or its salt or composition as described herein may be provided in combination with rituxan for the treatment of a complement mediated disorder. In some embodiments, the complement mediated disorder is, for example, rheumatoid arthritis, Granulomatosis with Polyangiitis (GPA) (Wegener's Granulomatosis), and Microscopic Polyangiitis (MPA). In some embodiments, the disorder is Lupus.

In some embodiments, an active compound or its salt or composition as described herein may be provided in combination with cyclophosphamide for the treatment of a complement mediated disorder. In some embodiments, the disorder is an autoimmune disease. In some embodiments, the complement mediated disorder is, for example, rheumatoid arthritis, Granulomatosis with Polyangiitis (GPA) (Wegener's Granulomatosis), and Microscopic Polyangiitis (MPA). In some embodiments, the disorder is Lupus.

In some embodiments, an active compound or its salt or composition as described herein is dosed in combination with a conventional DLE treatment for the treatment of lupus to a subject in need thereof.

Examples of conventional DLE treatments include topical corticosteroid ointments or creams, such as triamcinolone acetonide, fluocinolone, flurandrenolide, betamethasone valerate, and betamethasone dipropionate. Resistant plaques can be injected with an intradermal corticosteroid. Other potential DLE treatments include calcineurin inhibitors such as pimecrolimus cream or tacrolimus ointment. Particularly resistant cases can be treated with systemic antimalarial drugs, such as hydroxychloroquine (PLAQUENIL^{®}).

In some embodiments, an active compound or its salt or composition as described herein may be provided in combination with methotrexate for the treatment of Lupus.

In some embodiments, an active compound or its salt or composition as described herein may be provided in combination with azathioprine for the treatment of Lupus.

In some embodiments, an active compound or its salt or composition as described herein may be provided in combination with a non-steroidal anti-inflammatory drug for the treatment of Lupus.

In some embodiments, an active compound or its salt or composition as described herein may be provided in combination with a corticosteroid for the treatment of Lupus.

In some embodiments, an active compound or its salt or composition as described herein may be provided in combination with a belimumab (Benlysta) for the treatment of Lupus.

In some embodiments, an active compound or its salt or composition as described herein may be provided in combination with hydroxychloroquine (Plaquenil) for the treatment of Lupus.

In some embodiments, an active compound or its salt or composition as described herein may be provided in combination with sifalimumab for the treatment of Lupus.

In some embodiments, an active compound or its salt or composition as described herein may be provided in combination with OMS721 (Omeros) for the treatment of a complement mediated disorder. In some embodiments, an active compound or its salt or composition as described herein may be provided in combination with OMS906 (Omeros) for the treatment of a complement mediated disorder. In some embodiments, the complement mediated disorder is, for example, thrombotic thrombocytopenic purpura (TTP) or aHUS.

In some embodiments, an active compound or its salt or composition as described herein may be provided in combination with an anti-inflammatory agent, immunosuppressive agent, or anti-cytokine agent for the treatment or prevention of cytokine or inflammatory reactions in response to the administration of pharmaceuticals or biotherapeutics (e.g., adoptive T-cell therapy (ACT) such as CAR T-cell therapy, or monoclonal antibody therapy). In some embodiments, an active compound or its salt or composition as described herein may be provided in combination with a corticosteroid, for example prednisone, dexamethasone, solumedrol, and methylprednisolone, and/or anti-cytokine compounds targeting, e.g., IL-4, IL-10, IL-11, IL-13 and TGFβ. In some embodiments, an active compound or its salt or composition as described herein may be provided in combination with an anti-cytokine inhibitor including, but are not limited to, adalimumab, infliximab, etanercept, protopic, efalizumab, alefacept, anakinra, siltuximab, secukibumab, ustekinumab, golimumab, and tocilizumab, or a combination thereof. Additional anti-inflammatory agents that can be used in combination with an active compound or its salt or composition as described herein include, but are not limited to, non-steroidal anti-inflammatory drug(s) (NSAIDs); cytokine suppressive anti-inflammatory drug(s) (CSAIDs); CDP-571/BAY-10-3356 (humanized anti-TNFα antibody; Celltech/Bayer); cA2/infliximab (chimeric anti-TNFα antibody; Centocor); 75 kdTNFR-IgG/etanercept (75 kD TNF receptor-IgG fusion protein; Immunex); 55 kdTNF-IgG (55 kD TNF receptor-IgG fusion protein; Hoffmann-LaRoche); IDEC-CE9.1/SB 210396 (non-depleting primatized anti-CD4 antibody; IDEC/SmithKline); DAB 486-IL-2 and/or DAB 389-IL-2 (IL-2 fusion proteins; Seragen); Anti-Tac (humanized anti-IL-2Rα; Protein Design Labs/Roche); IL-4 (anti-inflammatory cytokine; DNAX/Schering); IL-10 (SCH 52000; recombinant IL-10, anti-inflammatory cytokine; DNAX/Schering); IL-4; IL-10 and/or IL-4 agonists (e.g., agonist antibodies); IL-1RA (IL-1 receptor antagonist; Synergen/Amgen); anakinra (Kineret^{®}/Amgen); TNF-bp/s-TNF (soluble TNF binding protein); R973401 (phosphodiesterase Type IV inhibitor); MK-966 (COX-2 Inhibitor); Iloprost, leflunomide (anti-inflammatory and cytokine inhibiton); tranexamic acid (inhibitor of plasminogen activation); T-614 (cytokine inhibitor); prostaglandin E1; Tenidap (non-steroidal anti-inflammatory drug); Naproxen (non-steroidal anti-inflammatory drug); Meloxicam (non-steroidal anti-inflammatory drug); Ibuprofen (non-steroidal anti-inflammatory drug); Piroxicam (non-steroidal anti-inflammatory drug); Diclofenac (non-steroidal anti-inflammatory drug); Indomethacin (non-steroidal anti-inflammatory drug); Sulfasalazine; Azathioprine; ICE inhibitor (inhibitor of the enzyme interleukin-1β converting enzyme); zap-70 and/or lck inhibitor (inhibitor of the tyrosine kinase zap-70 or lck); TNF-convertase inhibitors; anti-IL-12 antibodies; anti-IL-18 antibodies; interleukin-11; interleukin-13; interleukin-17 inhibitors; gold; penicillamine; chloroquine; chlorambucil; hydroxychloroquine; cyclosporine; cyclophosphamide; anti-thymocyte globulin; anti-CD4 antibodies; CD5-toxins; orally-administered peptides and collagen; lobenzarit disodium; Cytokine Regulating Agents (CRAB) HP228 and HP466 (Houghten Pharmaceuticals, Inc.); ICAM-1 antisense phosphorothioate oligo-deoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline; anti-IL2R antibodies; marine and botanical lipids (fish and plant seed fatty acids); auranofin; phenylbutazone; meclofenamic acid; flufenamic acid; intravenous immune globulin; zileuton; azaribine; mycophenolic acid (RS-61443); tacrolimus (FK-506); sirolimus (rapamycin); amiprilose (therafectin); and cladribine (2-chlorodeoxyadenosine).

In one embodiment, an active compound or its salt or composition as described herein may be provided in combination with a corticosteroid for the treatment or prevention of cytokine or inflammatory reactions in response to the administration of pharmaceuticals or biotherapeutics. In one embodiment, an active compound or its salt or composition as described herein may be provided in combination with etarnercept for the treatment or prevention of cytokine or inflammatory reactions in response to the administration of pharmaceuticals or biotherapeutics. In one embodiment, an active compound or its salt or composition as described herein may be provided in combination with tocilizumab for the treatment or prevention of cytokine or inflammatory reactions in response to the administration of pharmaceuticals or biotherapeutics. In one embodiment, an active compound or its salt or composition as described herein may be provided in combination with etarnercept and tocilizumab for the treatment or prevention of cytokine or inflammatory reactions in response to the administration of pharmaceuticals or biotherapeutics. In one embodiment, an active compound or its salt or composition as described herein may be provided in combination with infliximab for the treatment or prevention of cytokine or inflammatory reactions in response to the administration of pharmaceuticals or biotherapeutics. In one embodiment, an active compound or its salt or composition as described herein may be provided in combination with golimumab for the treatment or prevention of cytokine or inflammatory reactions in response to the administration of pharmaceuticals or biotherapeutics.

### C5 Inhibitor Combinations

Provided herein is a compound disclosed herein for use in a method for treating PNH in a subject, the method comprising administering to the subject an effective amount of a C5 inhibitor in combination or alternation with an effective amount of the compound described herein.

C5 inhibitors are known in the art. In some embodiments, the C5 inhibitor is a monoclonal antibody targeting C5. In some embodiments, the C5 inhibitor is eculizumab (Soliris^{®} Alexion Pharmaceuticals, see, e.g., U.S. Patent No. 9,352,035), or a biosimilar molecule thereof. In some embodiments, the C5 inhibitor is ravulizumab (Ultomiris^{®} Alexion Pharmaceuticals, see, e.g., U.S. Patent No. 9,371,377; 9,079,949 and 9,663,574), or a biosimilar thereof.

In some embodiments, the C5 inhibitor may be, but is not limited to: a recombinant human minibody, for example Mubodina^{®} (monoclonal antibody, Adienne Pharma and Biotech, Bergamo, Italy; see U.S. Patent No. 7,999,081); coversin (nomacopan, Akari Therapeutics; see e.g. Penabad et al. Lupus, 2012, 23(12):1324-6); LFG316 (monoclonal antibody, Novartis, Basel, Switzerland, and Morphosys, Planegg, Germany; see U.S. Patent Nos. 8,241,628 and 8,883,158); ARC-1905 (pegylated RNA aptamer, Ophthotech, Princeton, NJ and New York, NY; see Keefe et al., Nature Reviews Drug Discovery, 9, 537-550); RA101348 and zilucoplan (macrocyclic peptides, Ra Pharmaceuticals, Cambridge, MA); SOBI002 (affibody, Swedish Orphan Biovitrum, Stockholm, Sweden); cemdisiran (Alnylam Pharmaceuticals, Cambridge, MA); ARC1005 (aptamers, Novo Nordisk, Bagsvaerd, Denmark); SOMAmers (aptamers, SomaLogic, Boulder, Co); SSL7 (bacterial protein toxin, see, e.g. Laursen et al. Proc. Natl. Acad. Sci. U.S.A., 107(8):3681-6); MEDI7814 (monoclonal antibody, MedImmune, Gaithersburg, MD); aurin tricarboxylic acid; aurin tricarboxylic acid derivatives (Aurin Biotech, Vancouver, BC, see U.S. 2013/003592); crovalimab (RG6107/SKY59; anti-C5 recycling antibody, Roche Pharmaceuticals, Basel, Switzerland); ALXN5500 (monoclonal antibodies, Alexion Pharmaceuticals); TT30 (fusion protein, Alexion Pharmaceuticals); prozelimab (REGN3918; monoclonal antibody, Regeneron, Tarrytown, NY); ABP959 (eculizumab biosimilar, Amgen, Thousand Oaks, CA); BCD-148 (Biocad); and SB-12 (Samsung Bioepis Co., Ltd.); and combinations thereof.

In some embodiments, the C5 inhibitor is a recombinant human minibody, for example Mubodina^{®}. Mubodina^{®} is a fully human recombinant antibody C5 developed by Adienne Pharma and Biotech. Mubodina^{®} is described in U.S. Patent No. 7,999,081.

In some embodiments, the C5 inhibitor is coversin. Coversin is a recombinant protein derived from a protein discovered in the saliva of the *Ornithodoros moubata* tick currently developed as a recombinant protein by Akari Therapeutics (also known as nomacopan). Coversin is described in Penabad et al. Lupus 2012, 23(12):1324-6.

In some embodiments, the C5 inhibitor is Tesidolumab/LFG316. Tesidolumab is a monoclonal antibody developed by Novartis and Morphosys. Tesidolumab is described in U.S. Patent Nos. 8,241,628 and 8,883,158.

In some embodiments, the C5 inhibitor is ARC-1905. ARC-1905 is a pegylated RNA aptamer developed by Ophthotech. ARC-1905 is described in Keefe et al. Nature Reviews Drug Discovery, 9:537-550.

In some embodiments, the C5 inhibitor is RA101348. RA101348 is a macrocyclic peptide developed by Ra Pharmaceuticals.

In some embodiments, the C5 inhibitor is RA101495. RA101495, also known as zilucoplan, is a macrocyclic peptide developed by Ra Pharmaceuticals.

In some embodiments, the C5 inhibitor is SOBI002. SOBI002 is an affibody developed by the Swedish Orphan Biovitrum.

In some embodiments, the C5 inhibitor is ARC1005. ARC1005 is an aptamer developed by Novo Nordisk.

In some embodiments, the C5 inhibitor is SOMAmers for C5. SOMAmers are aptamers developed by SomaLogic.

In some embodiments, the C5 inhibitor is SSL7. SSL7 is a bacterial protein toxin described in Laursen et al. Proc. Natl. Acad. Sci. U.S.A., 107(8):3681-6.

In some embodiments, the C5 inhibitor is MEDI7814. MEDI7814 is a monoclonal antibody developed by MedImmune.

In some embodiments, the C5 inhibitor is aurin tricarboxylic acid. In another embodiment, the C5 inhibitor is an aurin tricarboxylic acid derivative. These aurin derivatives were developed by Aurin Biotech and are further described in U.S. 2013/003592).

In some embodiments, the C5 inhibitor is RG6107/SKY59, also known as crovalimab. RG6107/SKY59 is an anti-C5 recycling antibody developed by Roche Pharmaceuticals.

In another embodiment, the C5 inhibitor is ALXN5500. ALXN5500 are monoclonal antibodies developed by Alexion Pharmaceuticals.

In some embodiments, the C5 inhibitor is TT30. TT30 is a fusion protein developed by Alexion Pharmaceuticals.

In some embodiments, the C5 inhibitor is ABP959. ABP959 is an eculizamab biosimilar monoclonal antibody developed by Amgen.

In some embodiments, the C5 inhibtor is Anti-C5 siRNA cemdisiran. Anti-C5 siRNA was developed by Alnylam Pharmaceuticals.

In some embodiments, the C5 inhibitor is Erdigna^{®}. Erdigna^{®} is an antibody developed by Adienne Pharma.

In some embodiments, the C5 inhibitor is avacincaptad pegol/Zimura^{®}. Avacincaptad pegol is in aptamer developed by Opthotech.

In some embodiments, the C5 inhibitor is SOBI005. SOBI005 is a protein in developed by the Swedish Orphan Biovitrum.

In some embodiments, the C5 inhibitor is ISU305. ISU305 is a monoclonal antibody developed by ISU ABXIS.

In some embodiments, the C5 inhibitor is REGN3918. REGN3918 is a monoclonal antibody developed by Regeneron.

In some embodiments, the C5 inhibitor is BCD-148. BCD is an eculizumab biosimilar being developed by Biocad.

In some embodiments, the C5 inhibitor is SB-12. SB-12 is an eculizumab biosimilar being developed by Samsung Bioepis Co., Ltd.).

### C3 Inhibitors

Provided herein is a compound disclosed herein for use in amethod for treating PNH in a subject, the method comprising administering to the subject an effective amount of a C3 inhibitor in combination or alternation with an effective amount of the compound described herein.

C3 inhibitors are known in the art. In some embodiments, a compound of the present disclosure is administered in combination or alternation with compstatin and/or a compstatin analog. Compstatin and compastin analogs are known and are found to be useful inhibitors of C3, see U.S. Patent Nos. 9,056,076; 8,168,584; 9,421,240; 9,291,622; 8,580,735; 9371365; 9,169,307; 8,946,145; 7,989,589; 7,888,323; 6,319,897; and US Pat. No. Patent Appl. Pub. Nos. 2016/0060297; 2016/0015810; 2016/0215022; 2016/0215020; 2016/0194359; 2014/0371133; 2014/0323407; 2014/0050739; 2013/0324482; and 2015/0158915. In some embodiments, the compstatin analog having the amino acid sequence ICVVQDWGHHCRT (SEQ. ID. NO. 1). In another embodiment, the C3 inhibitor is a compstatin analog. In some embodiments, the compstatin analog is 4(1MeW)/APL-1 of the sequence Ac-ICV(1-mW)QDWGAHRCT(SEQ. ID. NO. 2), wherein Ac is acetyl and 1-mW is 1-methyltryptophan. In another embodiment, the compstatin analog is Cp40/AMY-101, which has an amino acid sequence yICV(1mW)QDW-Sar-AHRC-mI (SEQ. ID. NO. 3), wherein y is D-tyrosine, 1mW is 1-methyltryptophan, Sar is sarcosine, and mI is N-methylisoleucine. In yet another embodiment, the compstatin analog is PEG-Cp40, having the amino acid sequence PEG-yICV(1mW)QDW-Sar-AHRC-mI (SEQ. ID. NO. 4), wherein PEG is polyethyleneglycol (40 kDa), y is D-tyrosine, 1mW is 1-methyltryptophan, Sar is sarcosine, and mI is N-methylisoleucine. In yet another embodiment, the compstatin analog is 4(1MeW)POT-4. 4(1MeW)POT-4 was developed by Potentia. In yet another embodiment, the compstatin analog is AMY-201. AMY-201 was developed by Amyndas Pharmaceuticals.

In some embodiments, a compound of the present disclosure can be combined with C3 inhibitors that include, but are not limited to: H17 (monoclonal antibody, EluSys Therapeutics, Pine Brook, NJ); mirococept (CR1-based protein); sCR1 (CR1-based protein, Celldex, Hampton, NJ); TT32 (CR-1 based protein, Alexion Pharmaceuticals, New Haven, CT); HC-1496 (recombinant peptide); CB 2782 (enzyme, Catalyst Biosciences, South San Francisco, CA); APL-2 (pegylated synthetic cyclic peptide, Apellis Pharmaceuticals, Crestwood, KY); or combinations thereof.

In some embodiments, the C3 inhibitor is H17. H17 is a humanized monoclonal antibody in development by EluSys Therapeutics. H17 is described in Paixao-Cavalcante et al. J. Immunol. 2014, 192(10):4844-4851.

In some embodiments, the C3 inhibitor is mirococept. Mirococept is a CR1-based protein developed by Inflazyme Pharmaceuticals.

In some embodiments, the C3 inhibitor is sCR1. sCR1 is a soluble form of the CR1 protein developed by Celldex.

In some embodiments, the C3 inhibitor is TT32. TT32 is a CR-1 based protein developed by Alexion Pharmaceuticals.

In some embodiments, the C3 inhibitor is HC-1496. HC-1496 is a recombinant peptide developed by InCode.

In some embodiments, the C3 inhibitor is CB 2782. CB 2782 is novel protease derived from human membrane type serine protease 1 (MTSP-1) that was developed by Catalyst Biosciences.

In some embodiments, the C3 inhibitor is APL-2. APL-2 is a pegylated version of APL-1 developed by Apellis Pharmaceuticals.

### Complement Factor B (CFB) Inhibitors

Provided herein is a compound of the present disclosure for use in a method for treating PNH, the method comprising administering a CFB inhibitor in combination or alternation with a compound of the present disclosure. CFB inhibitors are known in the art. In some embodiments, a compound of the present disclosure can be combined with CFB inhibitors that include, but are not limited to: anti-FB SiRNA (Alnylam Pharmaceuticals, Cambridge, MA); TA106 (monoclonal antibody, Alexion Pharmaceuticals, New Haven, CT); LNP023 (small molecule, Novartis, Basel, Switzerland); SOMAmers (aptamers, SomaLogic, Boulder, CO); bikaciomab (Novelmed Therapeutics, Cleveland, OH); complin (see, Kadam et al., J. Immunol. 2010, DOI:10.409/jimmunol.10000200); Ionis-FB-L_{Rx} (ligand conjugated antisense drug, Ionis Pharmaceuticals, Carlsbad, CA); or a combination thereof. In another embodiment, CFB inhibitors that can be combined with a compound of the present disclosure include those disclosed in PCT/US17/39587. In another embodiment, CFB inhibitors that can be combined with a compound of the present disclosure as described herein include those disclosed in PCT/US17/014458. In another embodiment, CFB inhibitors that can be combined with a compound of the present disclosure as described herein include those disclosed in U.S. Patent Appl. Pub. No. 2016/0024079; . PCT Int. Appl. WO 2013/192345; PCT Int. Appl. WO 2013/164802; PCT Int. Appl. WO 2015/066241; PCT Int. Appl. WO 2015/009616 (assigned to Novartis AG).

In some embodiments, the CFB inhibitor is

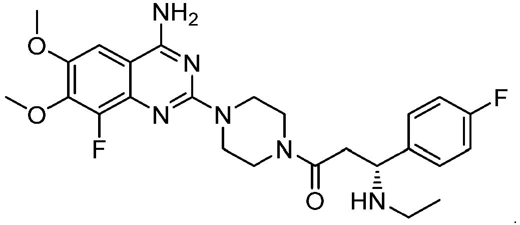

In another embodiment, the CFB inhibitor is

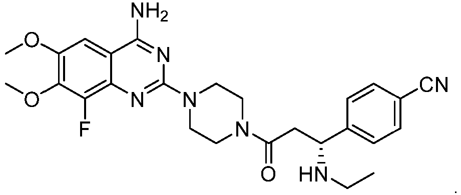

In another embodiment, the CFB inhibitor is

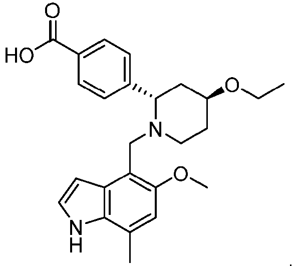

In some embodiments, the CFB inhibitor is anti-FB siRNA. Anti-FB siRNA have been developed by Alnylam Pharmaceuticals.

In some embodiments, the CFB inhibitor is TA106. TA106 is a monoclonal antibody developed by Alexion Pharmaceuticals.

In some embodiments, the CFB inhibitor is LNP023. LNP023 is a small molecule inhibitor of CFB developed by Novartis.

In some embodiments, the CFB inhibitor is complin. Complin is a peptide inhibitor that is described in Kadam et al. J. Immunol. 2010 184(12):7116-24.

In some embodiments, the CFB inhibitor is Ionis-FB-L_{Rx}. Ionis-FB-L_{Rx} is a ligand conjugated antisense drug developed by Ionis Pharmaceuticals.

### Pan-inhibitors of Complement Components

Provided herein is a compound of the present disclosure for use in a method for treating PNH, the method comprising administering a pan-inhibitor of complement components in combination or alternation with a compound of the present disclosure. Pan-inhibitors of complement components are known in the art. In some embodiments, the inhibitor is FUT-175.

### COMBINATIONS FOR PROPHYLACTIC OR CONCOMMITANT ANTI-BACTERIAL THERAPY

In one aspect of the present disclosure, an active compound or its salt or composition is provided for use in a method for treating a host in need thereof, the method comprising administering an effective amount of a prophylactic anti-bacterial vaccine prior to administration of the active compound or its salt or composition for any of the disorders described herein. In another aspect of the present disclosure, an active compound or its salt or composition is provided for use in a method for treating a host in need thereof, the method comprising administering an effective amount of a prophylactic anti-bacterial drug, such as a pharmaceutical drug, prior to administration of the active compound or its salt or composition for any of the disorders described herein. In one aspect of the present disclosure, an active compound or its salt or composition is provided for use in a method for treating a host in need thereof, the method comprising administering an effective amount of an anti-bacterial vaccine after administration of the active compound or its salt or composition for any of the disorders described herein. In another aspect of the present disclosure, an active compound or its salt or composition is provided for use in a method for treating a host in need thereof, the method comprising administering an effective amount of an anti-bacterial drug, such as a pharmaceutical drug, after administration of the active compound or its salt or composition for any of the disorders described herein. In one embodiment, the disorder is PNH, C3G, or aHUS. In one embodiment, the host has received an organ or other tissue or biological fluid transplant. In one embodiment, the host is also administered eculizumab.

In one aspect of the present disclosure, an active compound or its salt or composition as described herein is administered to a host concomitantly to a subject following the prophylactic administration of a vaccine against a bacterial infection. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present disclosure, an active compound or its salt or composition as described herein is administered to a subject concomitantly with the prophylactic administration of a vaccine against a bacterial infection. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present disclosure, an active compound or its salt or composition as described herein is administered to a subject and, during the administration period of the compound or salt, a vaccine against a bacterial infection is administered to the subject. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present disclosure, the subject is administered an active compound or its salt or composition as described herein in combination with an antibiotic compound for the duration of Factor D inhibitor administration. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present disclosure, an active compound or its salt or composition as described herein is administered to a subject following the prophylactic administration of a vaccine against a bacterial infection, and in combination with an antibiotic compound for the duration of Factor D inhibitor administration. In one embodiment, the complement mediated disorder is PNH or aHUS. In one embodiment, the subject has received an organ or other tissue or biological fluid transplant. In one embodiment, the subject is also administered eculizumab. In one embodiment, the subject, prior to receiving an active compound or its salt or composition as described herein, is vaccinated against a bacterial infection caused by the bacterium Neisseria meningitidis. In one embodiment, the subject is vaccinated against a bacterial infection caused by the bacterium Haemophilus influenzae. In one embodiment, the Haemophilus influenzae is Haemophilus influenzae serotype B (Hib). In one embodiment, the subject is vaccinated against a bacterial infection caused by Streptococcus pneumoniae. In one embodiment, the subject is vaccinated against a bacterial infection caused by the bacterium Nisseria meningitidis, Haemophilus influenzae, or Streptococcus pneumoniae, or a combination of one or more of Nisseria meningitidis, Haemophilus influenzae, or Streptococcus pneumoniae. In one embodiment, the subject is vaccinated against a bacterial infection caused by the bacterium Nisseria meningitidis, Haemophilus influenzae, and Streptococcus pneumoniae.

In other embodiments, the subject is vaccinated against a bacterial infection caused by a bacterium selected from a Gram-negative bacterium. In one embodiment, the subject is vaccinated against a bacterial infection caused by a bacterium selected from a Gram-positive bacterium. In one embodiment, the subject is vaccinated against a bacterial infection caused by the bacterium Nisseria meningitidis, Haemophilus influenzae, or Streptococcus pneunemoniae, or a combination of one or more of Nisseria meningitidis, Haemophilus influenzae, or Streptococcus pneumoniae, and one or more of, but not limited to, Bacillus anthracis, Bordetella pertussis, Clostridium tetani, Corynebacterium diphtheria, Coxiella burnetii, Mycobacterium tuberculosis, Salmonella typhi, Vibrio cholerae, Anaplasma phagocytophilum, Ehrlichia ewingii, Ehrlichia chaffeensis, Ehrlichia canis, Neorickettsia sennetsu, Mycobacterium leprae, Borrelia burgdorferi, Borrelia mayonii, Borrelia afzelii, Borrelia garinii, Mycobacterium bovis, Staphylococcus aureus, Streptococcus pyogenes, Treponema pallidum, Francisella tularensis, or Yersinia pestis.

In one embodiment, the subject is vaccinated with one or more vaccines selected from, but not limited to, typhoid vaccine, live (Vivotif Berna Vaccine, PaxVax), typhoid Vi polysaccharide vaccine (Typhim Vi, Sanofi), pneumococcal 23-polyvalent vaccine, PCV13 (Pneumovax 23, Merck), pneumococcal 7-valent vaccine, PCV7 (Prevnar, Pfizer), pneumococcal 13-valent vaccine, PCV13 (Prevnar 13, Pfizer), haemophilus b conjugate (prp-t) vaccine (ActHIB, Sanofi; Hibrix, GSK), haemophilus b conjugate (hboc) vaccine (HibTITER, Neuron Biotech), haemophilus b conjugate (prp-omp) vaccine (PedvaxHIB, Merck), haemophilus b conjugate (prp-t) vaccine/meningococcal conjugate vaccine (MenHibrix, GSK), haemophilus b conjugate (prp-t) vaccine/meningococcal conjugate vaccine/Hepatitis B vaccine (Comvax, Merck), meningococcal polysaccharide vaccine (Menomune A / C / Y / W-135, Sanofi), meningococcal conjugate vaccine/diphtheria CRM197 conjugate (Menveo, GSK; Menactra, Sanofi), meningococcal group B vaccine (Bexsero, GSK; Trumenba, Pfizer), anthrax vaccine adsorbed (Biothrax, Emergent Biosolutions), tetanus toxoid (Te Anatoxal Berna, Hendricks Regional Health), Bacillus Calmette and Guérin, live, intravesical (TheraCys, Sanofi; Tice BCG, Organon), cholera vaccine, live, oral (Vachora, Sanofi; Dukoral, SBL Vaccines; ShanChol, Shantha Biotec; Micromedex, Truven Health), tetanus toxoids and diphtheria absorbed (Tdap; Decavac, Sanofi; Tenivac, Sanofi; td, Massachusetts Biological Labs), diphtheria and tetanus toxois and pertussis (DTap; Daptacel, Sanofi; Infanrix, GSK; Tripedia, Sanofi), diphtheria and tetanus toxois and pertussis/polio (Kinrix, GSK; Quadracel, Sanofi), diphtheria and tetanus toxois and pertussis tetanus/hepatitis B/polio (Pediarix, GSK), diphtheria and tetanus toxois and pertussis/ polio, haemophilus influenza tybe b (Pentacel, Sanofi), and/or diphtheria, and pertussis (Tdap; Boostrix, GSK; Adacel, Sanofi), or a combination thereof.

As described herein, a subject receiving a compound of the present disclosure to treat a disorder is prophylactically administered an antibiotic compound in addition to a Factor D inhibitor described herein. In one embodiment, the subject is administered an antibiotic compound for the duration of administration of the active compound to reduce the development of a bacterial infection. Antibiotic compounds for concomitant administration with a Factor D inhibitor described herein can be any antibiotic useful in preventing or reducing the effect of a bacterial infection. Antibiotics are well known in the art and include, but are not limited to, amikacin (Amikin), gentamicin (Garamycin), kanamycin (Kantrex), neomycin (Neo-Fradin), netilmicin (Netromycin), tobramycin (Nebcin), paromomycin (Humatin), streptomycin, spectinomycin (Trobicin), geldanamycin, herbimycin, rifaximin (Xifaxan), loracarbef (Lorabid), ertapenem (Invanz), doripenem (Doribax), imipenem/cilastatin (Primaxin), meropenem (Merrem), cefadroxil (Duricef), cefazolin (Ancef), cefalotin/cefalothin (Keflin), cephalexin (Keflex), cefaclor (Distaclor), cefamandole (Mandol), cefoxitin (Mefoxin), cefprozil (Cefzil), cefuroxime (Ceftin, Zinnat), cefixime (Cefspan), cefdinir (Omnicef, Cefdiel), cefditoren (Spectracef, Meiact), cefoperazone (Cefobid), cefotaxime (Claforan), cefpodoxime (Vantin) ceftazidime (Fortaz), ceftibuten (Cedax), ceftizoxime (Cefizox), ceftriaxone (Rocephin), cefepime (Maxipime), ceftaroline fosamil (Teflaro), ceftobiprole (Zeftera), teicoplanin (Targocid), vancomycin (Vancocin), telavancin (Vibativ), dalbavancin (Dalvance), oritavancin (Orbactiv), clindamycin (Cleocin), lincomycin (Lincocin), daptomycin (Cubicin), azithromycin (Zithromax, Sumamed, Xithrone), clarithromycin (Biaxin), dirithromycin (Dynabac), erythromycin (Erythocin, Erythroped), roxithromycin, troleandomycin (Tao), telithromycin (Ketek), spiramycin (Rovamycine), aztreonam (Azactam), furazolidone (Furoxone), nitrofurantoin (Macrodantin, Macrobid), linezolid (Zyvox), posizolid, radezolid, torezolid, amoxicillin (Novamox, Amoxil), ampicillin (Principen),azlocillin, carbenicillin (Geocillin), cloxacillin (Tegopen), dicloxacillin (Dynapen), flucloxacillin (Floxapen), mezlocillin (Mezlin), methicillin (Staphcillin), nafcillin (Unipen),oxacillin (Prostaphlin), penicillin G (Pentids),penicillin V (Veetids (Pen-Vee-K), piperacillin (Pipracil), penicillin G (Pfizerpen), temocillin (Negaban),ticarcillin (Ticar), amoxicillin/clavulanate (Augmentin), ampicillin/sulbactam (Unasyn), piperacillin/tazobactam (Zosyn), ticarcillin/clavulanate (Timentin),bacitracin, colistin (Coly-Mycin-S), polymyxin B, ciprofloxacin (Cipro, Ciproxin, Ciprobay), enoxacin (Penetrex), gatifloxacin (Tequin), gemifloxacin (Factive), levofloxacin (Levaquin), lomefloxacin (Maxaquin), moxifloxacin (Avelox), nalidixic acid (NegGram), norfloxacin (Noroxin), ofloxacin (Floxin, Ocuflox), trovafloxacin (Trovan), grepafloxacin (Raxar), sparfloxacin (Zagam), temafloxacin (Omniflox), mafenide (Sulfamylon), sulfacetamide (Sulamyd, Bleph-10), sulfadiazine (Micro-Sulfon), silver sulfadiazine (Silvadene), sulfadimethoxine (Di-Methox, Albon), sulfamethizole (Thiosulfil Forte), sulfamethoxazole (Gantanol), sulfanilamide, sulfasalazine (Azulfidine), sulfisoxazole (Gantrisin), trimethoprim-sulfamethoxazole (Co-trimoxazole) (TMP-SMX) (Bactrim, Septra), sulfonamidochrysoidine (Prontosil), demeclocycline (Declomycin), doxycycline (Vibramycin), minocycline (Minocin), oxytetracycline (Terramycin), tetracycline (Sumycin, Achromycin V, Steclin), clofazimine (Lamprene), dapsone (Avlosulfon), capreomycin (Capastat), cycloserine (Seromycin), ethambutol (Myambutol), ethionamide (Trecator), isoniazid (I.N.H.), pyrazinamide (Aldinamide), rifampicin (Rifadin, Rimactane), rifabutin (Mycobutin), rifapentine (Priftin), streptomycin, arsphenamine (Salvarsan), chloramphenicol (Chloromycetin), fosfomycin (Monurol, Monuril), fusidic acid (Fucidin), metronidazole (Flagyl), mupirocin (Bactroban), platensimycin, quinupristin/dalfopristin (Synercid), thiamphenicol, tigecycline (Tigacyl), tinidazole (Tindamax Fasigyn), trimethoprim (Proloprim, Trimpex), and/or teixobactin, or a combination thereof.

In one embodiment, the subject is administered a prophylactic antibiotic selected from cephalosporin, for example, ceftriaxone or cefotaxime, ampicillin-sulbactam, Penicillin G, ampicillin, chloramphenicol, fluoroquinolone, aztreonam, levofloxacin, moxifloxacin, gemifloxacin, vancomycin, clindamycin, cefazolin, azithromycin, meropenem, ceftaroline, tigecycline, clarithromycin, moxifloxacin, trimethoprim/sulfamethoxazole, cefuroxime, axetil, ciprofloxacin, rifampin, minocycline, spiramycin, and cefixime, or a combination of two or more thereof.

### PROCESS OF PREPARATION OF COMPOUNDS OF OF THE PRESENT DISCLOSURE ABBREVIATIONS

- AcCl: Acetyl chloride
- AcOEt, EtOAc: ethyl acetate
- AcOH: Acetic acid
- AcOK: Potassium acetate
- AlCl₃: Aluminum chloride
- BINAP: 2,2'-bis(diphenylphosphino)-1,1'-binaphthyl)
- Boc: tert-butoxycarbonyl
- BH₃-THF: Borane tetrahydrofuran complex
- Br₂PPH₃: Triphenylphosphine dibromide
- CHCl₃: Trichloromethane; chloroform
- CH₃OH, MeOH: Methanol
- Cs₂CO₃: Cesium carbonate
- CuI: Cuprous iodide
- DAST: Diethylaminosulfur trifluoride
- DCM, CH₂Cl₂: Dichloromethane
- DIBAl-H: Diisobutylaluminum hydride
- DIEA, DIPEA: N,N-diisopropylethylamine
- DMBNH₂: 2,4-Dimethoxybenzylamine
- DME: Dimethyl ether
- DMF: N,N-dimethylformamide
- DMSO: Dimethylsulfoxide
- Et₂AlCl: Diethylaluminum chloride
- EtOAc: Ethylacetate
- EtOH: Ethanol
- HATU: 1-[Bis(dimethylamino)methylene]-1H-1,2,3-triazolo[4,5-b]pyridinium3-oxide hexafluorophosphate
- HBTU: Hexafluorophosphate Benzotriazole Tetramethyl Uronium
- H₂O₂: Hydrogen peroxide
- H₂SO₄: Sulfuric acid
- HCl: Hydrochloric acid
- HMPA: Hexamethylphosphoramide
- HNO₃: Nitric acid
- K₂CO₃: Potassium carbonate
- K₂PO₄: Potassium hydrogen phosphate
- LiAlH₄: Lithium aluminum hydride
- LDA: Lithium diisopropylamide
- LiOH: Lithium hydroxide
- LiOH.H₂O: Lithium hydroxide monohydrate
- m-CPBA: meta-Chloroperoxybenzoic acid
- MeI: Methyl iodide
- MeCN: acetonitrile
- MeMgBr: Methylmagnesium bromide
- MTBE: Methyl *^{t}*butylether
- Na₂SO₄: Sodium sulfate
- NaNO₂: Sodium nitrite
- NaH: Sodium hydride
- NaI: Sodium iodide
- NaOAc: Sodium acetate
- NaOH: Sodium hydroxide
- NaHCO₃: Sodium bicarbonate
- NH₃.H₂O: Ammonium hydroxide
- NH₄Cl: Ammonium chloride
- Pd (OAc) ₂: Palladium acetate
- Pd(ally)Cl₂: Allylpalladium(II) chloride dimer
- Pd(dppf)Cl₂: [1,1'-Bis(diphenylphosphino) ferrocene]dichloropalladium(II)
- Pd(PPh₃)₄: Tetrakis(triphenylphosphine)palladium(0)
- Pd/C: Palladium on carbon
- PoCl₃: Phosphoryl chloride
- Py, py: Pyridine
- RT: Room temperature
- TBAF: Tetra-n-butylammonium fluoride
- tBuBrettphos: 2-(Di-tert-butylphosphino)-2',4',6'- triisopropyl-3,6-dimethoxy-1,1'-piphenyl,
- tBuONa: Sodium *tert*-butoxide
- tBuOK: Potassium *tert*-butoxide
- TBSOTf: Trimethylsilyl trifluoromethanesulfonate
- TEA: Trimethylamine
- Tf₂O: Trifluoromethanesulfonic anhydride
- TFA: Trifluoroacetic acid
- TFAA: Trifluoroacetic anhydride
- THF: Tetrahydrofuran
- Ti(OEt)₄: Titanium ethoxide
- Zn: Zinc
- Zn (CN)₂: Zinc cyanide

### GENERAL METHODS

All nonaqueous reactions were performed under an atmosphere of dry argon or nitrogen gas using anhydrous solvents. The progress of reactions and the purity of target compounds were determined using one of the two liquid chromatography (LC) methods listed below. The structure of starting materials, intermediates, and final products was confirmed by standard analytical techniques, including NMR spectroscopy and mass spectrometry.

### LC Method A

Instrument: Waters Acquity Ultra Performance LC
Column: ACQUITY UPLC BEH C18 2.1 × 50 mm, 1.7 µm
Column Temperature: 40 °C
Mobile Phase: Solvent A: H₂O + 0.05% FA; Solvent B: CH₃CN + 0.05% FA
Flow Rate: 0.8 mL/min
Gradient: 0.24 min @ 15% B, 3.26 min gradient (15-85% B), then 0.5 min @ 85% B.
Detection: UV (PDA), ELS, and MS (SQ in EI mode)

### LC Method B

Instrument: Shimadzu LC-2010A HT
Column: Athena, C18-WP, 50 × 4.6 mm, 5 µm
Column Temperature: 40 °C
Mobile Phase: Solvent A: H₂O/CH₃OH/FA = 90/10/0.1; Solvent B: H₂O/CH₃OH/FA = 10/90/0.1
Flow Rate: 3 mL/min
Gradient: 0.4 min @ 30% B, 3.4 min gradient (30-100% B), then 0.8 min @ 100% B
Detection: UV (220/254 nm)

### EXAMPLE 1. NON-LIMITING SYNTHETIC EXAMPLES OF COMPOUNDS OF THE PRESENT DISCLOSURE

The below schemes are non-limiting examples of methods to make compounds of the present disclosure. The skilled artisan will recognize that there are various modifications that can be performed to make analogs or prepare compounds in other ways. For example, wherever a Suzuki coupling is used, there are other methods known in the art to conduct the coupling, such as those described in the literature, for example, Molander, G. A., Trice, S. L. J., & Kennedy, S. M. (2012), "Scope of the two-step, one-pot palladium-catalyzed borylation/Suzuki cross-coupling reaction utilizing bis-boronic acid." Journal of Organic Chemistry, 77(19), 8678-8688.)

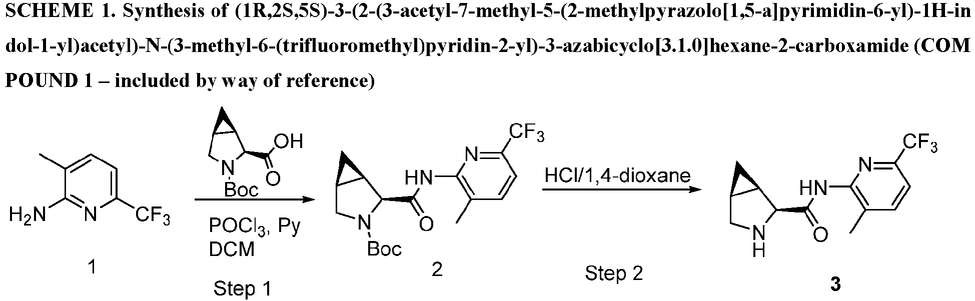

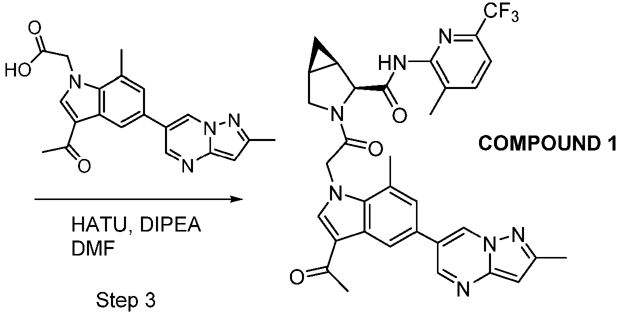

**Step 1:** To a solution of intermediate **1** (50 mg, 0.284 mmol) in DCM (5 mL) was added (1R,2S,5S)-3-(tert-butoxycarbonyl)-3-azabicyclo[3.1.0]hexane-2-carboxylic acid (64.5 mg, 0.284 mmol), pyridine (112.32 mg, 1.420 mmol) followed by POCl₃ (48 mg, 0.312 mmol) at 0 °C. The mixture was stirred at room temperature for 2 hours before diluted with DCM, dried over anhydrous Na₂SO₄, filtered and concentrated to dryness under reduced pressure. The residue was purified by chromatography on silica gel (PE: EtOAc = 10: 1) to give intermediate **2** (35 mg, yield 32.1%) as colorless oil. LC/MS (ESI) (m/z): 386 (M+H)⁻.

**Step 2:** A solution of intermediate **2** (35 mg, 0.09 mmol) in HCl/1,4-dioxane (1 mL, 4M) was stirred at 0 °C and then warmed to 25 °C within 2 hours. The mixture was concentrated to dryness under reduced pressure and the residue was washed with diethyl ether and dried under vacuum to give intermediate **3** (23 mg, yield 77.2%) as a brown solid, which was directly used in the next step without further purification. LC/MS (ESI) (m/z): 286 (M+H)⁺.

**Step 3:** To a mixture of intermediate **3** (23 mg, 0.070 mmol) and 2-(3-acetyl-7-methyl-5-(2-methylpyrazolo[1,5-a]pyrimidin-6-yl)-1H-indol-1-yl)acetic acid (25 mg, 0.070 mmol) in DMF (3 mL) was added DIPEA (36 mg, 0.28 mmol) and HATU (53 mg, 0.140 mmol) at 0 °C. The mixture was stirred at room temperature under N₂ atmosphere for 12 hours before diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND 1** (2.3 mg, yield 5.2%) as a white solid. ¹H-NMR (400 MHz, CDCl₃) δ 8.82 (s, 1H), 8.68 (d, J = 2.9 Hz, 3H), 8.45 (s, 1H), 7.64 (s, 2H), 7.32 (d, J = 7.8 Hz, 1H), 7.09 (s, 1H), 6.42 (s, 1H), 5.15 (d, J = 17.0 Hz, 1H), 4.99 (d, J = 17.0 Hz, 1H), 4.22 (d, J = 13.1 Hz, 1H), 3.89 (s, 1H), 3.76 (d, J = 9.6 Hz, 1H), 3.24 (s, 3H), 2.64 (s, 3H), 2.45 (d, J = 14.0 Hz, 9H), 1.18 (s, 4H), 0.87 - 0.70 (m, 2H), LC/MS (ESI) m/z: 631(M+H)⁺.

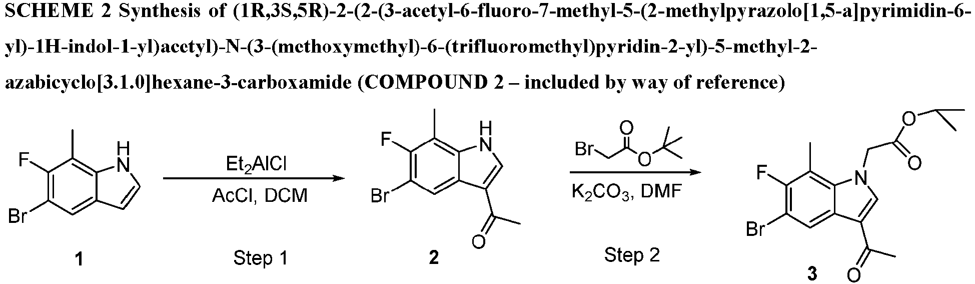

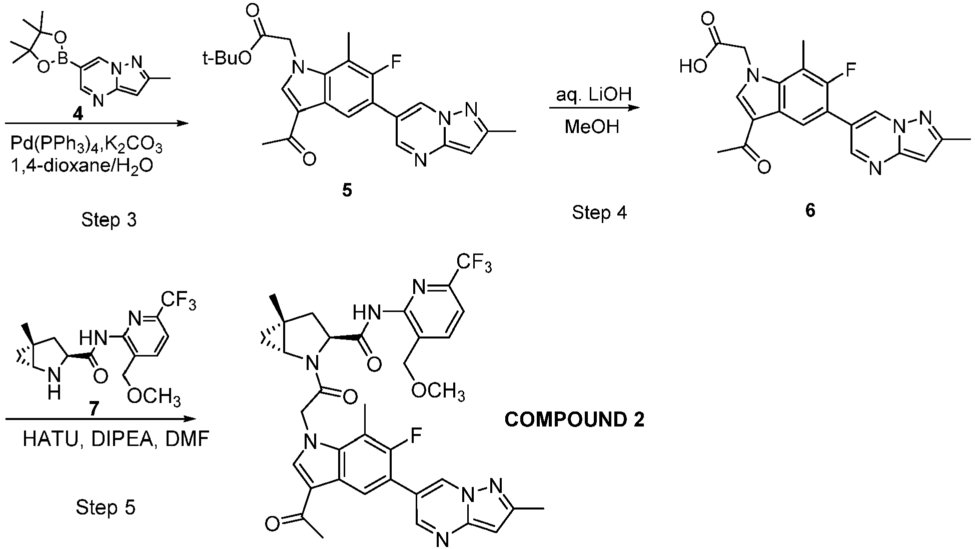

**Step 1:** To a solution of intermediate **1** (278 mg, 1.22 mmol) in DCM (5mL) was added Et₂AlCl (0.9 mL, 1.84 mmol) at 0 °C under N₂ atmosphere. The mixture was stirred at 0 °C under N₂ atmosphere for 0.5 hours before AcCl (0.13 mL, 1.84 mmol) was added drop-wisely at 0 °C. The mixture was stirred at 25 °C under N₂ atmosphere for 12 hours before quenched with ice-water and diluted with EtOAc, washed with water and brine, dried over Na₂SO₄ and concentrated to dryness under reduced pressure to give crude product, which was purified by flash chromatography on silica gel (PE: EtOAc = 3: 2) to give intermediate **2** (213 mg, yield 64.5%) as yellow solid. LC/MS (ESI) (m/z): 270/272 (M+H)⁺.

**Step 2:** To a solution of intermediate **2** (213 mg, 0.79 mmol) in DMF (5 mL) was added potassium carbonate (0.33 g, 2.4 mmol) and tert-butyl 2-bromoacetate (0.2 mL, 1.2 mmol). The mixture was stirred at room temperature for 2 hours before diluted with EtOAc and washed with saturated aq.NH₄Cl solution and brine, dried over anhydrous Na₂SO₄, filtered and concentrated to dryness under reduced pressure. The residue was purified by chromatography on silica gel (PE: EtOAc = 3: 1) to give intermediate **3** (265 mg, yield 88.3%) as a white solid. LC/MS (ESI) (m/z): 384/386 (M+H)⁺.

**Step 3:** To a mixture of intermediate **3** (0.11 g, 0.29 mmol) and compound 4 (90 mg, 0.35 mmol) in 1,4-dioxane (4 mL) and water (0.5 mL) was added K₂CO₃ (99 mg, 0.73 mmol), Pd(PPh₃)₄ (33 mg, 0.029 mmol) under N₂ atmosphere. The mixture was degassed under N₂ atmosphere for three times and stirred at 90 °C under N₂ atmosphere for 2 hours before diluted with EtOAc and washed with water and brine, dried over anhydrous Na₂SO₄ and concentrated to dryness under reduced pressure to give crude product, which was purified by flash chromatography on silica gel (PE: EtOAc = 1: 1) to give intermediate **5** (103 mg, yield 82%) as a white solid. LC/MS (ESI) (m/z): 437 (M+H)⁺.

**Step 4:** To a solution of intermediate **5** (103 mg, 0.24 mmol) in MeOH (5 mL) and water (1 mL) was added a solution of lithium hydroxide monohydrate (96 mg, 2.4 mmol) at 0 °C. The mixture was stirred at room temperature for 1 hour before concentrated to 1/5 volume, diluted with water and washed with MTBE twice. The aqueous layer was acidified with 1N aq.HCl to pH~3, extracted with DCM twice. The combined organic layers were washed with brine, dried over anhydrous Na₂SO₄, and concentrated to dryness under reduced pressure to give intermediate 6 (98 mg, yield 100%) as a white solid. LC/MS (ESI) (m/z): 381 (M+H)⁺.

**Step 5:** To a mixture of intermediate **6** (90 mg, 0.24 mmol) and compound **7** (101 mg, 0.31 mmol) in DMF (5 mL) was added DIPEA (0.2 mL, 1.2 mmol) HATU (180 mg, 0.48 mmol) at 0 °C. The mixture was stirred at room temperature for 2 hours under N₂ atmosphere before diluted with EtOAc and washed with saturated aq.NH₄Cl solution and brine, dried over anhydrous Na₂SO₄, filtered and concentrated to dryness under reduced pressure. The residue was purified by chromatography on silica gel (PE: EtOAc = 4: 1) to give crude product which was further purified by prep-HPLC to give **COMPOUND 2** (26.6 mg, yield 16.6%) as a white solid. ¹H-NMR (400 MHz, DMSO-*d*₆) δ 10.69 (s, 1H), 9.13 - 9.11 (m, 1H), 8.60 (t, *J=* 2.1 Hz, 1H), 8.36 (s, 1H), 8.21 (d, *J=* 7.7 Hz, 1H), 8.11 (d, *J=* 7.9 Hz, 1H), 7.80 (d, *J=* 8.0 Hz, 1H), 6.59 (s, 1H), 5.80 (d, *J =* 18.1 Hz, 1H), 5.42 (d, *J =* 18.0 Hz, 1H), 4.46 - 4.37 (m, 1H), 4.28 (d, *J* = 7.3 Hz, 2H), 3.58 (d, *J=* 5.4 Hz, 1H), 3.12 (s, 3H), 2.53 (d, *J =* 1.8 Hz, 4H), 2.45 (d, *J =* 6.7 Hz, 7H), 2.05 (dd, *J* = 13.3, 6.0 Hz, 1H), 1.34 (s, 3H), 1.01 (t, *J=* 5.5 Hz, 1H), 0.91 (d, *J =* 4.8 Hz, 1H); LC/MS (ESI) m/z: 692 (M+H)⁺.

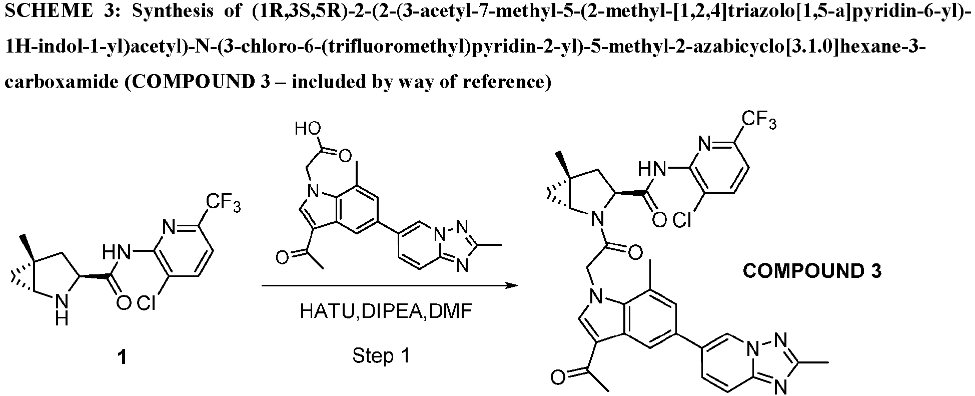

**Step 1:** To a mixture of intermediate 1 (120 mg, 0.38 mmol) and 2-(3-acetyl-7-methyl-5-(2-methyl-[1,2,4]triazolo[1,5-a]pyridin-6-yl)-1H-indol-1-yl)acetic acid (136 mg, 0.38 mmol) in DMF (6 mL) was added DIPEA (245 mg, 1.90 mmol) followed by HATU (217 mg, 0.57 mmol) at 0 °C and the mixture was stirred at room temperature for 2 hours. The mixture was diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND** 3 (11.1 mg, yield 4.4%) as a white solid. ¹H-NMR (400 MHz, CDCl₃) δ 8.85 (s, 1H), 8.70 (d, J = 2.0 Hz, 1H), 8.55 (d, J = 1.6 Hz, 1H), 7.85 - 7.80 (m, 2H), 7.71 - 7.65 (m, 2H), 7.39 (d, J = 8.0 Hz, 1H), 7.19 (s, 1H), 5.45 (d, J = 17.6 Hz, 1H), 5.33 (d, J = 17.6 Hz, 1H), 5.20 - 5.11 (m, 1H), 3.20 - 3.15 (m, 1H), 2.70 (s, 3H), 2.62 (s, 3H), 2.60 - 2.54 (m, 1H), 2.53 (s, 3H), 2.52 - 2.46 (m, 1H), 1.42 (s, 3H), 1.16 (t, J = 5.4 Hz, 1H), 0.90 (dd, J = 5.2, 5.2 Hz, 1H);LC/MS (ESI) m/z: 664 (M+H)⁺.

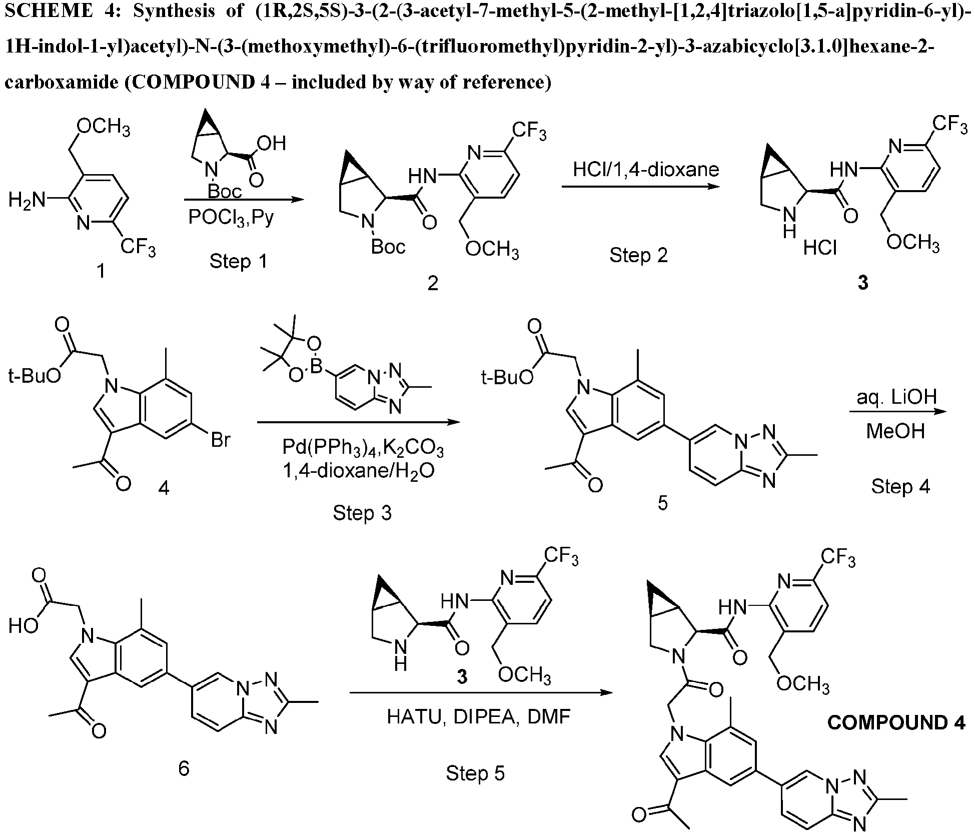

**Step 1:** To a solution of intermediate **1** (50 mg, 0.242 mmol) in DCM (5 mL) was added (1R,2S,5S)-3-(tert-butoxycarbonyl)-3-azabicyclo[3.1.0]hexane-2-carboxylic acid (55 mg, 0.24 mmol), pyridine (95 mg, 1.21 mmol) followed by POCl₃ (40 mg, 0.266 mmol) at 0 °C. The mixture was stirred at room temperature for 2 hours before diluted with DCM, dried over anhydrous Na₂SO₄, filtered and concentrated under reduced pressure to dryness. The residue was purified by chromatography on silica gel (PE: EtOAc = 10: 1) to give intermediate **2** (33 mg, yield 33.2%) as colorless oil. LC/MS (ESI) (m/z): 416 (M+H)⁺.

**Step 2:** A solution of intermediate **2** (33 mg, 0.07 mmol) in HCl/1,4-dioxane (1 mL, 4M) was stirred at 0 °C and then warmed to 25 °C within 2 hours. The mixture was concentrated to dryness under reduced pressure and the residue was washed with diethyl ether and dried under vacuum to give intermediate **3** (17 mg, yield 59.2%) as a brown solid, which was directly used in the next step without further purification. LC/MS (ESI) (m/z):316 (M+H)⁺.

**Step 3:** To a mixture of intermediate **4** (440 mg, 1.206 mmol) and 2-methyl-6-(4,4,5,5-tetramethyl-1,3,2-dioxaborolan-2-yl)-[1,2,4]triazolo[1,5-a]pyridine (375 mg, 1.447 mmol) in 1,4-dioxane (4 mL) and water (0.5 mL) was added K₂CO₃ (500 mg, 3.618 mmol), Pd(PPh₃)₄ (139.2 mg, 0.120 mmol) under N₂ atmosphere. The mixture was degassed under N₂ atmosphere for three times and stirred at 90 °C under N₂ atmosphere for 2 hours. The mixture was diluted with EtOAc and washed with water and brine, dried over anhydrous Na₂SO₄ and concentrated to dryness under reduced pressure to give crude product, which was purified by flash chromatography on silica gel (PE: EtOAc = 9: 1) to give intermediate **5** (190 mg, yield 37.6%) as a white solid. LC/MS (ESI) (m/z): 419 (M+H)⁺.

**Step 4:** To a solution of intermediate **5** (190 mg, 0.45 mmol) in MeOH (5 mL) and water (1 mL) was added a solution of lithium hydroxide monohydrate (37.8 mg, 0.90 mmol) at 0 °C. The mixture was stirred at room temperature for 1 hour before concentrated to 1/5 volume, diluted with water and washed with MTBE twice. The aqueous layer was acidified with 1N aq.HCl to pH~3 and extracted with DCM twice. The combined organic layers were washed with brine, dried over anhydrous Na₂SO₄, and concentrated to dryness under reduced pressure to give intermediate **6** (150 mg, yield 92.4%) as a white solid. LC/MS (ESI) (m/z): 363 (M+H)⁺.

**Step 5:** To a mixture of intermediate **6** (17 mg, 0.047 mmol) and compound **3** (17 mg, 0.047 mmol) in DMF (3 mL) was added DIPEA (24 mg, 0.18 mmol) followed by HATU (35 mg, 0.094 mmol) at 0 °C. The mixture was stirred at room temperature for 2 hours before extracted with ethyl acetate twice, dried over anhydrous Na₂SO₄, filtered and concentrated to dryness under reduced pressure. The residue was purified by chromatography on silica gel (PE: EtOAc = 1: 2) and further purified by prep-HPLC to give **COMPOUND** 4 (2.0 mg, yield 6.44 %) as a white solid. ¹H-NMR (400 MHz, CDCl₃) δ 8.72 (d, J = 27.8 Hz, 2H), 8.52 (s, 1H), 7.81 (d, J = 9.1 Hz, 1H), 7.75 - 7.57 (m, 3H), 7.38 (d, J = 7.6 Hz, 1H), 7.26 (s, 4H), 7.18 (s, 1H), 5.22 (d, J = 17.0 Hz, 1H), 5.04 (d, J = 17.2 Hz, 1H), 4.58 (d, J = 13.1 Hz, 1H), 4.27 (d, J = 12.7 Hz, 1H), 4.03 - 3.92 (m, 1H), 3.82 (d, J = 9.2 Hz, 1H), 3.31 (s, 2H), 2.71 (s, 3H), 2.62 (s, 3H), 2.50 (s, 4H), 1.89 (s, 1H), 1.04 (d, J = 4.9 Hz, 1H), 0.84 (d, J = 6.0 Hz, 1H); LC/MS (ESI) m/z: 660 (M+H)⁺.

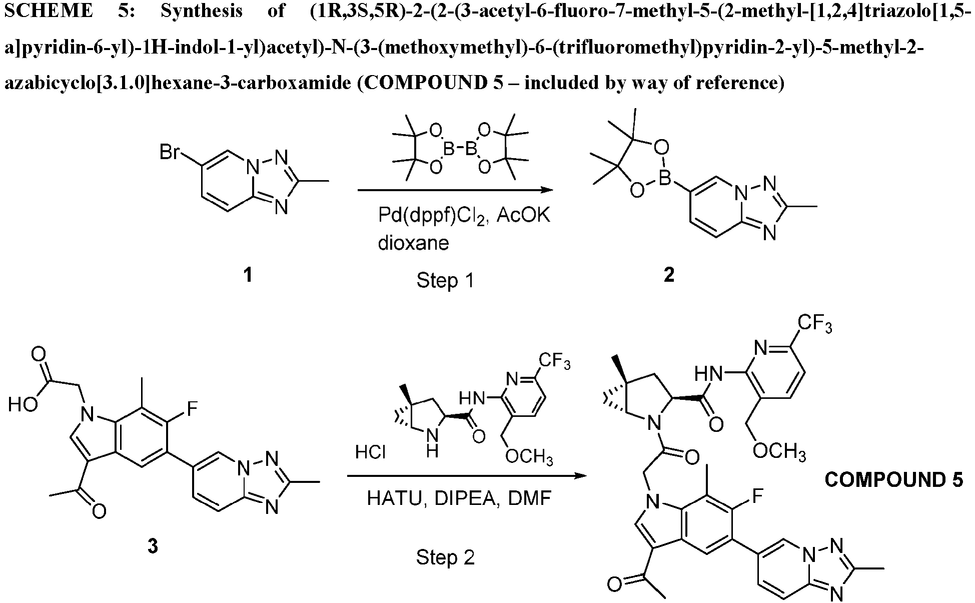

**Step 1:** To a mixture of intermediate 1 (0.5 g, 2.4 mmol) and 4,4,4',4',5,5,5',5'-octamethyl-2,2'-bi(1,3,2-dioxaborolane) (722 mg, 2.8 mmol) in 1,4-dioxane (4 mL) was added AcOK (700 mg, 7.1 mmol), Pd(dppf)Cl₂ (88 mg, 0.12 mmol) under N₂ atmosphere. The mixture was degassed under N₂ atmosphere for three times and stirred at 85 °C under N₂ atmosphere for 2 hours before diluted with EtOAc and washed with water and brine, dried over anhydrous Na₂SO₄ and concentrated to dryness under reduced pressure to give crude product, which was further purified by flash chromatography on silica gel (PE: EtOAc = 10: 1) to give intermediate **2** (0.49 g, yield 80%) as brown oil. LC/MS (ESI) (m/z): 260 (M+H)⁺.

**Step 2:** To a mixture of intermediate **3** (17 mg, 0.04 mmol) and (1R,3S,5R)-N-(3-(methoxymethyl)-6-(trifluoromethyl)pyridin-2-yl)-5-methyl-2-azabicyclo[3.1.0]hexane-3-carboxamide hydrochloride (13 mg, 0.06 mmol) in DMF (3 mL) was added DIPEA (0.04 mL, 0.2 mmol) and HATU (34 mg, 0.08 mmol) at 0 °C. The mixture was stirred at room temperature for 2 hours under N₂ atmosphere before diluted with EtOAc and washed with saturated aq.NH₄Cl solution and brine, dried over anhydrous Na₂SO₄, filtered and concentrated to dryness under reduced pressure. The residue was purified by chromatography on silica gel (PE: EA = 4: 1) to give crude product which was further purified by prep-HPLC to give **COMPOUND** 5 (1.8 mg, yield 5.8%) as a white solid. ¹H-NMR (400 MHz, CD₃OD) δ 8.70 (d, *J* = 1.4 Hz, 1H), 8.20 (d, *J =* 7.7 Hz, 1H), 8.06 (s, 1H), 7.99 (d, *J =* 7.9 Hz, 1H), 7.75 (dt, *J =* 9.3, 1.7 Hz, 1H), 7.64 - 7.55 (m, 2H), 5.59 (d, *J =* 18.1 Hz, 1H), 5.44 - 5.36 (m, 1H), 4.44 (s, 2H), 4.27 (d, *J =* 7.2 Hz, 2H), 3.42 (dd, *J =* 5.6, 2.4 Hz, 1H), 3.25 (s, 3H), 3.07 (s, 3H), 2.55 (dd, *J =* 13.5, 9.2 Hz, 1H), 2.47 (s, 3H), 2.40 (s, 3H), 2.20 - 2.11 (m, 1H), 1.30 (s, 3H), 0.99 (t, *J =* 5.5 Hz, 1H), 0.86 (dd, *J =* 5.6, 2.4 Hz, 1H). LC/MS (ESI) m/z: 692 (M+H)⁺.

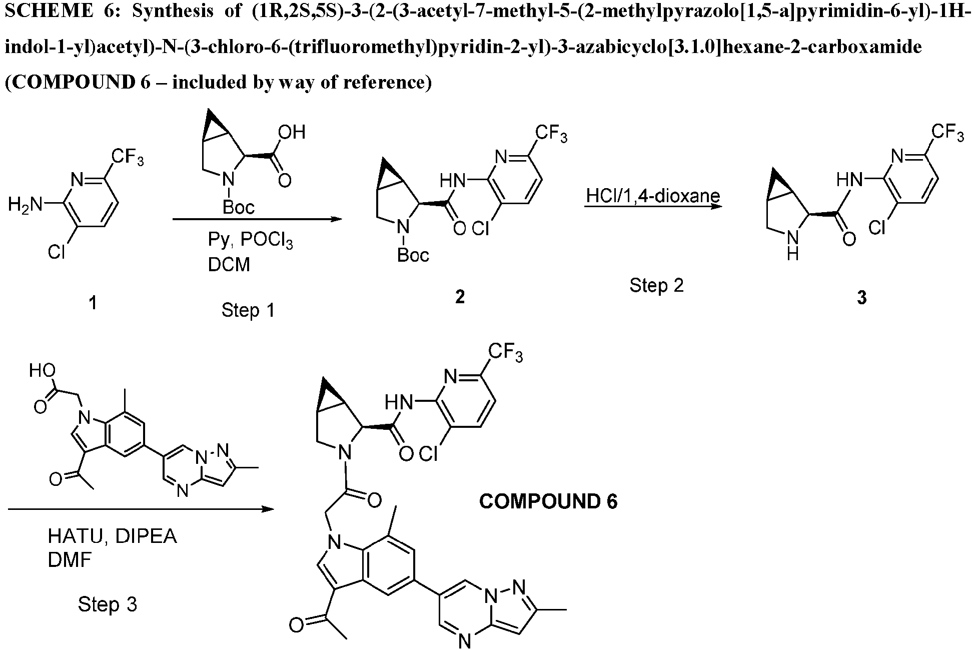

**Step 1:** To a solution of intermediate **1** (50 mg, 0.26 mmol) and (1R,2S,5S)-3-(tert-butoxycarbonyl)-3-azabicyclo[3.1.0]hexane-2-carboxylic acid (59 mg, 0.26 mmol) in DCM (3 mL) was added pyridine (0.2 mL, 1.3 mmol) followed by drop-wise addition of POCl₃ (0.05 mg, 0.29 mmol) at 0 °C. The mixture was stirred at room temperature for 3 hours before quenched with ice water and extracted with DCM twice. The mixture was concentrated to dryness under reduced pressure and purified by chromatography on silica gel (PE: EtOAc= 20:1 to 5: 1) to give intermediate 2 (22 mg, yield 21.0 %) as light yellow oil. LC/MS (ESI) (m/z): 406 (M+H)⁺.

**Step 2:** A solution of intermediate **2** (22 mg, 0.05 mmol) in HCl/1,4-dioxane (2 mL, 4M 1,4-dioxane solution) was stirred at room temperature for 2 hours. The mixture was concentrated to dryness under reduced pressure to give intermediate **3** (20 mg, yield 100.0%) as yellow solid. which was directly used in the next step without further purification. LC/MS (ESI) m/z: 306 (M+H)⁺.

**Step 3:** To a mixture of intermediate **3** (20 mg, 0.05 mmol) and 2-(3-acetyl-7-methyl-5-(2-methylpyrazolo[1,5-a]pyrimidin-6-yl)-1H-indol-1-yl)acetic acid (22 mg, 0.06 mmol) in DMF (2 mL) was added DIPEA (0.05 mL, 0.3 mmol) followed by HATU (34 mg, 0.09 mmol) at 0 °C. The mixture was stirred at room temperature overnight before diluted with EtOAc and washed with saturated aq.NH₄Cl solution and brine, dried over anhydrous Na₂SO₄, filtered and concentrated to dryness under reduced pressure. The residue was purified by prep-HPLC (DCM : MeOH=20:1) to give **COMPOUND** 6 (2 mg, yield 5.1%) as a white solid. ¹H-NMR (400 MHz, CDCl₃) δ 8.88 (s, 1H), 8.73 (t, J = 2.6 Hz, 2H), 8.51 (s, 1H), 7.70 (s, 2H), 7.38 (d, J = 7.6 Hz, 1H), 7.15 (s, 1H), 6.48 (s, 1H), 5.21 (d, J = 17.2 Hz, 1H), 5.05 (d, J = 17.2 Hz, 1H), 4.55 (d, J = 12.8 Hz, 1H), 4.28 (d, J = 12.8 Hz, 1H), 3.82 (d, J = 9.6 Hz, 1H), 2.71 (s, 3H), 2.53 (s, 4H), 2.49 (s, 4H), 1.90 (s, 1H), 1.04 (d, J = 5.2 Hz, 1H), 0.84 (s, 2H). LC/MS (ESI) m/z: 650 (M+H)⁺.

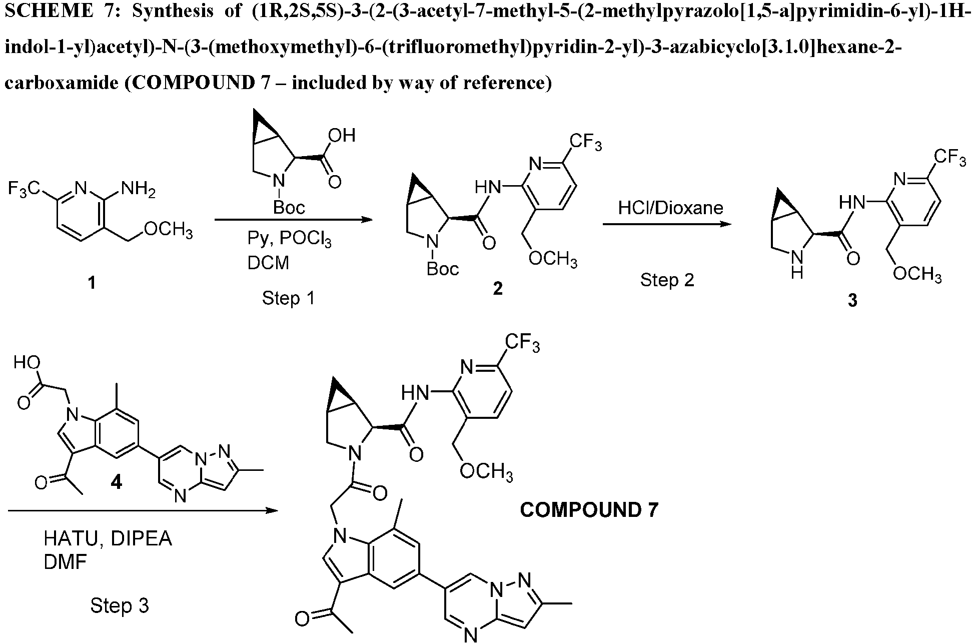

**Step 1:** To a solution of intermediate **1** (50 mg, 0.24 mmol) and (1R,2S,5S)-3-(tert-butoxycarbonyl)-3-azabicyclo[3.1.0]hexane-2-carboxylic acid (54 mg, 0.24 mmol) in DCM (2 mL) was added pyridine (0.2 mL, 1.2 mmol) followed by addition of POCl₃ (0.05 mg, 0.26 mmol) at 0 °C. The mixture was stirred at room temperature for 3 hours before quenched with ice water and extracted with DCM twice. The mixture was concentrated to dryness under reduced pressure and purified by prep-HPLC (PE: EtOAc =5: 1) to give intermediate **2** (65 mg, yield 65.0%) as light yellow oil. LC/MS (ESI) (m/z): 416 (M+H)⁺.

**Step 2:** A solution of intermediate **2** (65 mg, 0.16 mmol) in HCl/1,4-dioxane (2.5 mL, 4M 1,4-dioxane solution) was stirred at room temperature for two hours before concentrated to dryness under reduced pressure to give intermediate **3** (55 mg, yield 100.0%) as yellow solid, which was directly used in the next step without further purification. LC/MS (ESI) m/z: 316 (M+H)⁺.

**Step 3:** To a mixture of intermediate **3** (55 mg, 0.16 mmol) and compound 4 (58 mg, 0.16 mmol) in DMF (3 mL) was added DIPEA (0.15 mL, 0.8 mmol) followed by HATU (91 mg, 0.24 mmol) at 0 °C. The mixture was stirred at room temperature overnight before diluted with EtOAc and washed with saturated aq.NH₄Cl solution and brine, dried over anhydrous Na₂SO₄, filtered and concentrated to dryness under reduced pressure. The residue was purified by prep-HPLC (DCM :MeOH =20:1) to give **COMPOUND 7** (2.7 mg, yield 2.6%) as a white solid. ¹H-NMR (400 MHz, CD₃OD) δ 8.84 (d, J = 2.4 Hz, 1H), 8.68 (d, J = 2.4 Hz, 1H), 8.32 (d, J = 2.0 Hz, 1H), 8.01 - 7.94 (m, 2H), 7.52 (d, J = 8.4 Hz, 1H), 7.17 (s, 1H), 6.39 (s, 1H), 5.26 (d, J = 7.6 Hz, 2H), 4.48 (s, 5H), 3.93 (dd, J = 10,9.6 Hz, 1H), 3.79 (d, J = 10 Hz, 1H), 2.59 (s, 3H), 2.40 (s, 7H), 2.13 (s, 1H), 1.89 (s, 1H), 0.90 - 0.77 (m, 3H); LC/MS (ESI) m/z: 660 (M+H)⁺.

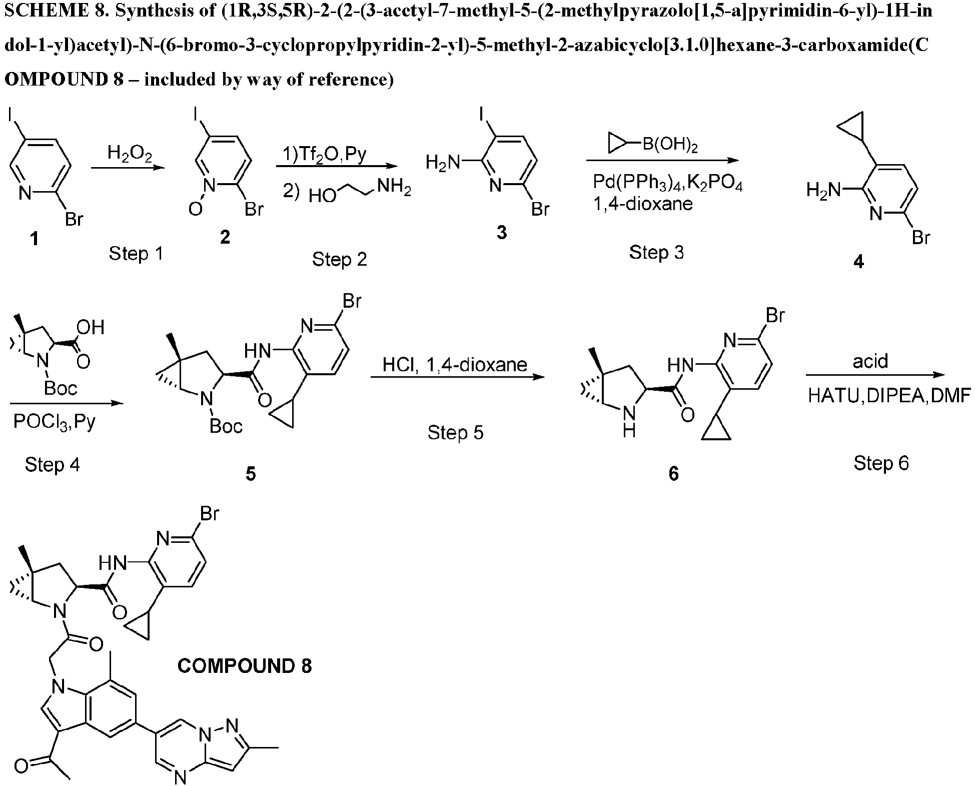

**Step 1:** A solution of 2-bromo-5-iodopyridine (1 g, 3.53 mmol) in TFA (6mL) was cooled to 0 °C, followed by dropwise addition of 30% H₂O₂ (10 mL, 18 eq). The solution was then allowed to warm to room temperature before heated to reflux for 45 minutes. After completion of the reaction, the solvent was removed under reduced pressure and intermediate **2** was purified by chromatography on silica (PE:EA = 1:1) as brown semi-solid (700 mg, yield 67%), LC/MS (ESI) (m/z):300 (M+H)⁺.

**Step 2:** To a stirred solution of intermediate **2** (700 mg, 2.34 mmol) and pyridine (740.376 mg, 9.36 mmol) in acetonitrile (15 mL) at 0 °C was added a solution of trifluoromethanesulfonic anhydride (738.504 mg, 3.51mmol) in acetonitrile (4mL) over 20 minutes. The reaction mixture was stirred at room temperature for an additional 3 hours. The reaction mixture was allowed to cool down to 10 °C and ethanolamine (214.3mg, 3.51mmol) was added to it. The reaction mixture was stirred at room temperature for an additional 3 hours before diluted with EtOAc and washed with saturated aq.NH₄Cl solution and brine, dried over anhydrous Na₂SO₄, filtered and concentrated to dryness under reduced pressure. The residue was purified by chromatography on silica gel (PE: EtOAc = 7:3) to give intermediate **3** (180mg, yield 26%) as colorless oil. LC/MS (ESI) (m/z): 299 (M+H)⁺.

**Step 3:** To a mixture of intermediate **3** (180 mg, 0.60 mmol) and cyclopropylboronic acid (39 mg, 0.45 mmol) in 1,4-dioxane (2.4 mL) and water (0.3 mL) was added Pd(PPh₃)₄ (40.8 mg, 0.028 mmol), potassium phosphate (0.15 g, 0.70 mmol) and under N₂ atmosphere. The mixture was degassed under N₂ atmosphere for three times and stirred at 95 °C under N₂ atmosphere for 12 hours. The mixture was diluted with EtOAc, washed with water and brine, dried over Na₂SO₄ and concentrated to dryness under reduced pressure to give crude product, which was purified by flash chromatography on silica gel (PE: EtOAc = 50: 1 to 3: 2) to give intermediate **4** (50 mg, yield 39%) as a white solid. LC/MS (ESI) (m/z): 212 (M+H)⁺.

**Step 4:** To a solution of (1R,3S,5R)-2-(tert-butoxycarbonyl)-5-methyl-2-azabicyclo[3.1.0]hexane-3-carboxylic acid (50 mg, 0.23 mmol) in DCM (5 mL) was added intermediate **4** (49 mg, 0.23 mmol), pyridine (73 mg, 0.92 mmol) and POCl₃ (39 mg, 0.253 mmol) at 0°C. The mixture was stirred at room temperature for 2 hours before diluted with DCM, dried over anhydrous Na₂SO₄, filtered and concentrated to dryness under reduced pressure. The residue was purified by chromatography on silica gel (PE: EtOAc = 10: 1) to give intermediate **5** (50 mg, yield 50%) as colorless oil. LC/MS (ESI) (m/z): 436 (M+H)⁺.

**Step 5:** A solution of intermediate **5** (50 mg, 0.11 mmol) in 1,4-dioxane/HCl (5 mL, 26.13 mmol) was stirred at 0 °C and the mixture was warmed up to 25 °C for 2 hours. The mixture was concentrated to dryness and the residue was washed with diethyl ether twice and dried under reduced pressure to give intermediate **6** (40 mg, yield 94%) as a brown solid, which was directly used in the next step without further purification. LC/MS (ESI) (m/z): 336 (M+H)⁺.

**Step 6:** To a mixture of intermediate **6** (20 mg, 0.05 mmol) and 1-(3-acetyl-7-methyl-5-(2-methylpyrimidin-5-yl)-1H-indol-1-yl)propan-2-one (12 mg, 0.05 mmol) in DMF (3 mL) was added DIPEA (25.8 mg, 0.2 mmol), HATU (38mg, 0.10 mmol) at 0 °C. The mixture was stirred at room temperature under N₂ atmosphere for 1 hour before diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND 8** (3.1 mg, yield 7.6%) as a white solid. ¹H NMR (400 MHz, DMSO-*d*₆) δ 10.42 (s, 1H), 9.18 (s, 1H), 8.79 (d, J = 2.1 Hz, 1H), 8.37 (s, 1H), 8.30 (s, 1H), 7.63 (q, J = 8.1 Hz, 2H), 7.37 (s, 1H), 6.55 (s, 1H), 4.54 - 4.43 (m, 1H), 3.55 (d, J = 3.3 Hz, 1H), 2.64 (s, 3H), 2.08 (dd, J = 13.0, 5.5 Hz, 1H), 1.93 (s, 1H), 1.32 (s, 3H), 1.01 (s, 1H), 0.93 - 0.80 (m, 3H), 0.72 - 0.57 (m, 2H), LC/MS (ESI) m/z: 681(M+H)⁻.

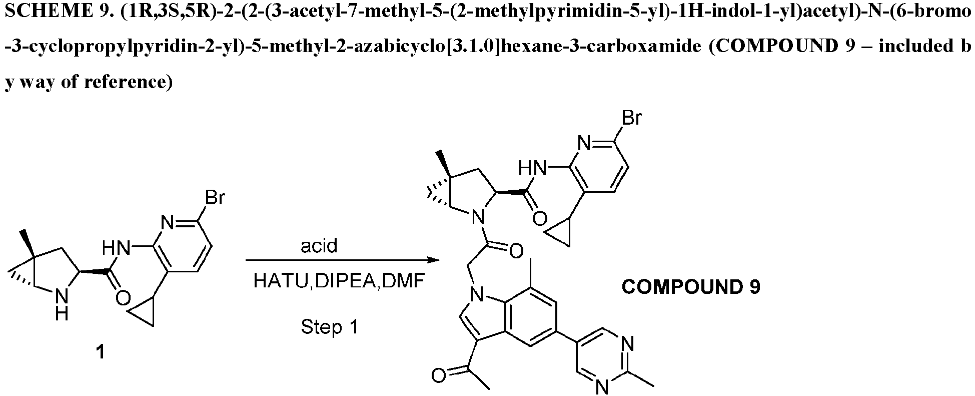

**Step 1:** To a mixture of intermediate 1 (20 mg, 0.05 mmol) and 1-(3-acetyl-7-methyl-5-(2-methylpyrimidin-5-yl)-1H-indol-1-yl)propan-2-one (12 mg, 0.05 mmol) in DMF (3 mL) was added DIPEA (25.8 mg, 0.2 mmol), HATU (38mg, 0.10 mmol) at 0 °C. The mixture was stirred at room temperature under N₂ atmosphere for 1 hour before diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND 9** (2.6 mg, yield 6.4%) as a white solid. ¹H NMR (400 MHz, CD₃OD) δ 8.88 (d, J = 9.7 Hz, 2H), 8.37 (d, J = 1.6 Hz, 1H), 8.07 (s, 1H), 7.32 - 7.10 (m, 3H), 5.61 - 5.36 (m, 2H), 3.39 (dd, J = 5.8, 2.2 Hz, 2H), 2.64 (s, 3H), 2.61 (s, 3H), 2.43 (s, 3H), 2.26 (dd, J = 13.3, 5.1 Hz, 1H), 1.31 (s, 3H), 1.19 (s, 1H), 1.02 (t, J = 5.6 Hz, 1H), 0.86 (dd, J = 5.5, 2.3 Hz, 1H), 0.77 - 0.63 (m, 2H), 0.50 - 0.38 (m, 2H), LC/MS (ESI) m/z:641(M+H)⁺.

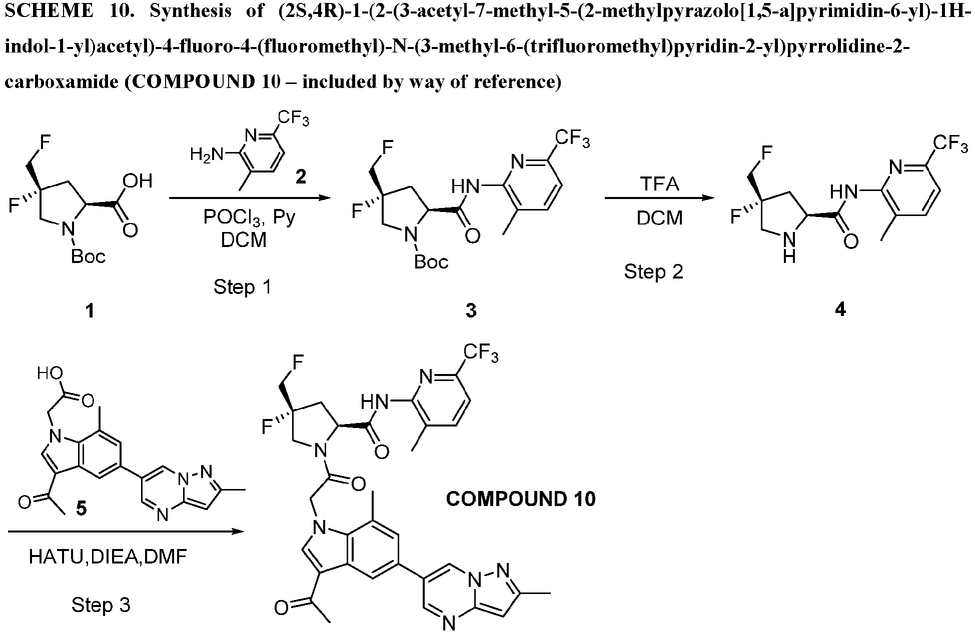

**Step 1:** To a mixture of intermediate 1 (330 mg, 1.24 mmol) and compound 2 (218 mg, 1.24 mmol) in anhydrous DCM (4 mL) was added pyridine (490 mg, 6.20 mmol), followed by addition of POCl₃ (208 mg, 1.36 mmol) at 0 °C. The reaction was stirred at room temperature for 2 hours. The reaction mixture was poured into ice water and extracted with DCM twice. The organic layer was washed with brine, dried over Na₂SO₄ and concentrated to dryness. The residue was purified by column chromatography on silica gel (PE: EtOAc = 20: 1 to 1: 1) to give intermediate **3** (180 mg, yield 34.4%) as light oil. LC/MS (ESI) m/z : 424 (M+H)⁺.

**Step 2:** To a solution of intermediate **3** (180 mg,0.43 mmol) in DCM (2 mL) was added TFA (1 mL) at 0 °C and the reaction was stirred at room temperature for 2 hours. The reaction was concentrated to dryness, washed with ether and dried under vacuum to give intermediate **4** (130 mg, yield 94.9%) as yellow solid, which was used directly in the next step. LC/MS (ESI) m/z : 324 (M+H)⁺.

**Step 3:** To a mixture of intermediate **4** (30 mg, 0.093 mmol) and compound **5** (34 mg, 0.093 mmol) in DMF (3 mL) was added DIPEA (60 mg, 0.47 mmol), followed by addition of HATU (53 mg, 0.14 mmol) at 0 °C. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by prep-HPLC to give **COMPOUND 10** (3.0 mg, yield 4.8%) as a white solid. ¹H NMR (400 MHz, δ 10.72 (s, 1H), 9.19 (d, *J =* 2.0 Hz, 1H), 8.79 (d, *J =* 2.4 Hz, 1H), 8.37 (d, *J =* 2.0 Hz, 1H), 8.25 (s, 1H), 7.93 (d, *J =* 8.0 Hz, 1H), 7.69 (d, *J =* 8.0 Hz, 1H), 7.35 (d, *J =* 1.6 Hz, 1H), 6.55 (s, 1H), 5.58 - 5.45 (m, 2H), 5.00 - 4.77 (m, 3H), 4.24 - 3.99 (m, 2H), 2.75 - 2.66 (m, 1H), 2.62 (s, 3H), 2.45 (d, *J* = 1.2 Hz, 6H), 2.37 - 2.28 (m, 1H), 2.16 (s, 3H). LC/MS (ESI) m/z : 668 (M+H)⁺.

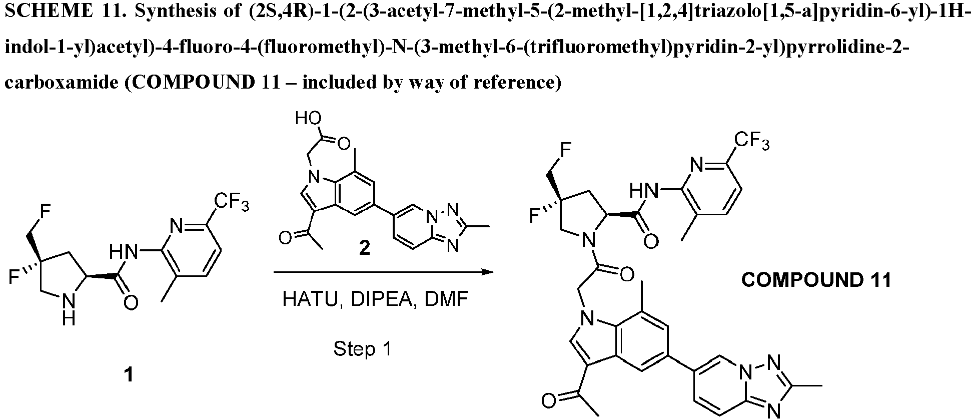

**Step 1:** To a mixture of intermediate **1** (40.0 mg TFA salt, 0.12 mmol), compound **2** (43.4 mg, 0.12 mmol) and DIPEA (0.12 mL, 0.48 mmol) in DMF (2 mL) was added HATU (91.2 mg, 0.24 mmol) at 0 °C under N₂. The reaction was stirred at 25 °C for 2 hours before water (10 mL) was added. The reaction was extracted with EtOAc and the organic layer was washed with brine, dried over Na₂SO₄, filtered and concentrated to dryness. The residue was purified by column chromatography on silica gel (DCM : MeOH = 20: 1) and further purified by prep-HPLC to give **COMPOUND 11** (6.4 mg, yield 7.9%) as a white solid. ¹H NMR (400 MHz, CD₃OD) δ 8.90 (s, 1H), 8.52 (s, 1H), 8.47 (d, *J* = 1.7 Hz, 1H), 8.13 (s, 1H), 8.01 (dd, *J =* 9.3, 1.8 Hz, 1H), 7.85 (d, *J =* 7.9 Hz, 1H), 7.72 (d, *J =* 8.5 Hz, 1H), 7.58 (d, *J =* 7.6 Hz, 1H), 7.33 (s, 1H), 5.49 (q, *J =* 17.9 Hz, 2H), 5.34 (t, *J =* 4.5 Hz, 1H), 4.77 - 4.66 (m, 2H), 4.30 - 4.18 (m, 1H), 4.15 - 4.04 (m, 1H), 2.71 (s, 3H), 2.56 (s, 3H), 2.53 (s, 3H), 2.23 (s, 3H), 2.18 (d, *J =* 7.8 Hz, 1H), 2.03 (d, *J =* 5.7 Hz, 1H). LC/MS (ESI) m/z : 668 (M+H)⁺.

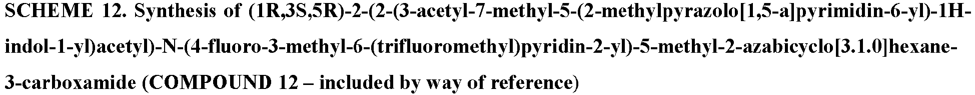

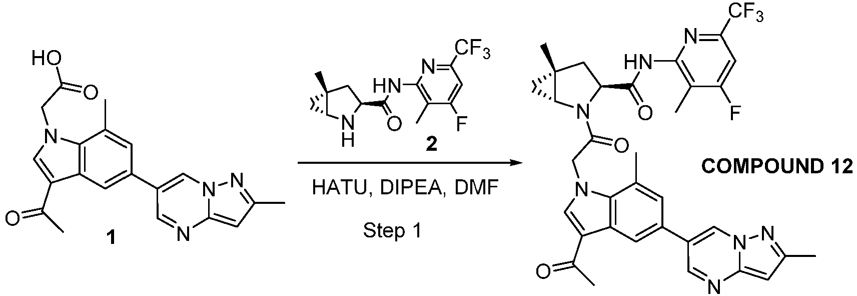

**Step 1:** To a mixture of compound **2** (22 mg, 0.069 mmol) and intermediate **1** (25 mg, 0.069 mmol) in DMF (3 mL) were added DIPEA (0.06 mL, 0.345 mmol) and HATU (52 mg, 0.14 mmol) at 0 °C under N₂ atmosphere and the mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM:MeOH = 96: 4) to give **COMPOUND 12** (16 mg, crude) as a brown solid. The residue was further purified by prep-HPLC to give **COMPOUND 12** (1.4 mg, yield 3.0%) as a white solid. ¹H NMR (400 MHz, CD₃OD) δ 8.85 (dd, *J=* 5.9, 1.9 Hz, 1H), 8.69 (t, *J =* 3.1 Hz, 1H), 8.35 (t, *J =* 4.9 Hz, 1H), 8.03 (s, 1H), 7.42 (d, *J =* 8.3 Hz, 1H), 7.20 (s, 1H), 6.40 (s, 1H), 5.53 (d, *J =* 18.0 Hz, 1H), 5.43 (d, *J =* 18.0 Hz, 1H), 4.48 (s, 1H), 3.40 (dd, *J* = 5.5, 2.3 Hz, 1H), 2.60 (s, 3H), 2.58 - 2.50 (m, 1H), 2.41 (d, *J =* 3.7 Hz, 7H), 2.21 - 2.13 (m, 2H), 2.03 (s, 3H), 1.30 (s, 3H), 1.01 (t, *J =* 5.3 Hz, 1H), 0.86 (dd, *J =* 5.4, 2.3 Hz, 1H). LC/MS (ESI) m/z : 662 (M+H)⁺.

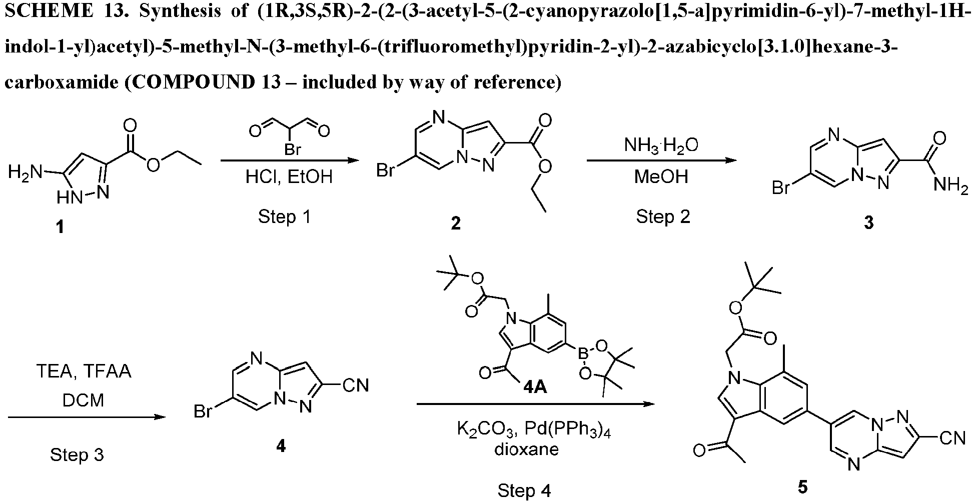

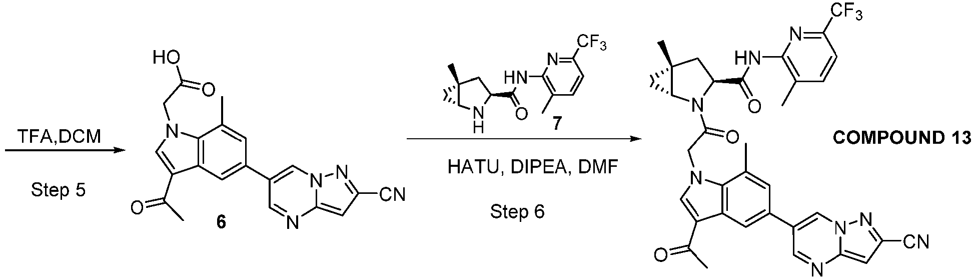

**Step 1:** To a mixture of intermediate **1** (500 mg, 3.2 mmol) and 2-bromopropanedial (480 mg, 3.2 mmol) in EtOH (7 mL) was added HCl (0.33 mL, 3.9 mmol) at 0 °C. The mixture was stirred at 25 °C for 1 hour. The mixture was diluted with EtOAc and washed with brine, dried over anhydrous Na₂SO₄, filtered and concentrated to dryness. The residue was purified by chromatography on silica gel (DCM : MeOH = 100: 1 to 20: 1) to give intermediate **2** (600 mg, yield 68.9%) as a white solid. LC/MS (ESI) m/z : 270 (M+H)⁺.

**Step 2:** To a solution of intermediate **2** (450 mg, 1.7 mmol) in MeOH (6 mL) was added NH₃·H₂O (6 mL) and the mixture was stirred at 60 °C for 9 hours. The mixture was diluted with H₂O and extracted with DCM twice. The combined organic layers were washed with brine, dried over anhydrous Na₂SO₄, filtered and concentrated to dryness. The residue was purified by chromatography on silica gel (PE : EtOAc = 20: 1 to 5: 1) to give intermediate **3.** (229 mg, yield 57.0%) as a white solid. LC/MS (ESI) m/z : 241 (M+H)⁺.

**Step 3:** To a solution of intermediate **3** (201 mg, 0.8 mmol) in DCM (8 mL) were added TEA (269 mg, 1.8 mmol) and TFAA (189 mg, 0.9 mmol) at 0°C under N₂ atmosphere. The mixture was stirred at room temperature overnight. The mixture was diluted with DCM and water. The organic layer was washed with brine, dried over anhydrous Na₂SO₄, filtered and concentrated to dryness. The residue was purified by chromatography on silica gel (PE : EtOAc = 20: 1 to 5: 1) to give intermediate **4** (140 mg, yield 75.3%) as a white solid. LC/MS (ESI) m/z : 223 (M+H)⁺.

**Step 4:** To a mixture of intermediate **4** (40 mg,0.18 mmol) and compound **4A** (74 mg,0.18 mmol) in dioxane was added K₂CO₃ (62 mg, 0.45 mmol), followed by addition of Pd(PPh₃)₄ (23 mg, 0.02 mmol) at 0 °C under N₂ atmosphere. The mixture was stirred at 90 °C overnight. The mixture was cooled to 20 °C and poured into iced-water and extracted with EtOAc twice. The combined organic layer was washed with brine, dried over anhydrous Na₂SO₄, filtered and concentrated to dryness. The residue was purified by prep-TLC (DCM : MeOH = 20: 1) to give intermediate 5 (45 mg, yield 58.4%) as yellow solid. LC/MS (ESI) m/z : 430 (M+H)⁺.

**Step 5:** To a solution of intermediate **5** (45 mg, 0.11 mmol.) in DCM (2mL) was added TFA (1mL) at 0 °C and the mixture was stirred at room temperature for 4 hours. The reaction was concentrated to dryness, washed with DCM and dried over anhydrous Na₂SO₄, concentrated under vacuum to give intermediate **6** (30 mg, yield 76.7%) as yellow solid, which was used directly in the next step. LC/MS (ESI) m/z : 374 (M+H)⁺.

**Step 6:** To a mixture of intermediate **6** (30 mg, 0.08 mmol) and compound **7** (24 mg, 0.08 mmol) in DMF (3 mL) was added DIPEA (52 mg, 0.4 mmol), followed by addition of HATU (46 mg, 0.12 mmol) at 0 °C. The mixture was stirred at 25 °C for 2 hours. The mixture was diluted with EtOAc and washed with saturated NH₄Cl solution and brine, dried over anhydrous Na₂SO₄, filtered and concentrated to dryness. The residue was purified by prep-TLC (DCM:MeOH = 20: 1) to give **COMPOUND 13** (4 mg, yield 7.6%) as a white solid. ¹H NMR (400 MHz, DMSO-*d*₆) δ 10.49 (s, 1H), 9.50 (d, *J =* 2.0 Hz, 1H), 9.11 (d, *J =* 2.0 Hz, 1H), 8.45 (d, *J =* 2.0 Hz, 1H), 8.33 (s, 1H), 7.94 (d, *J =* 8.0 Hz, 1H), 7.69 (d, *J =* 7.6 Hz, 1H), 7.59 (s, 1H), 7.44 (s, 1H), 5.76 (d, *J =* 18.0 Hz, 1H), 5.42 (d, *J =* 18.0 Hz, 1H), 4.47 (dd, *J=* 9.2, 9.2 Hz, 1H), 3.56 (dd, *J =* 5.6, 5.6 Hz, 1H), 2.65 (s, 3H), 2.59 - 2.55 (m, 1H), 2.47 (s, 3H), 2.19 (s, 3H), 2.05 (dd, *J =* 12.4, 12.8 Hz, 1H), 1.33 (s, 3H), 1.02 (t, *J =* 5.4 Hz, 1H), 0.90 (dd, *J =* 4.4, 4.8 Hz, 1H). LC/MS (ESI) m/z : 655 (M+H)⁺.

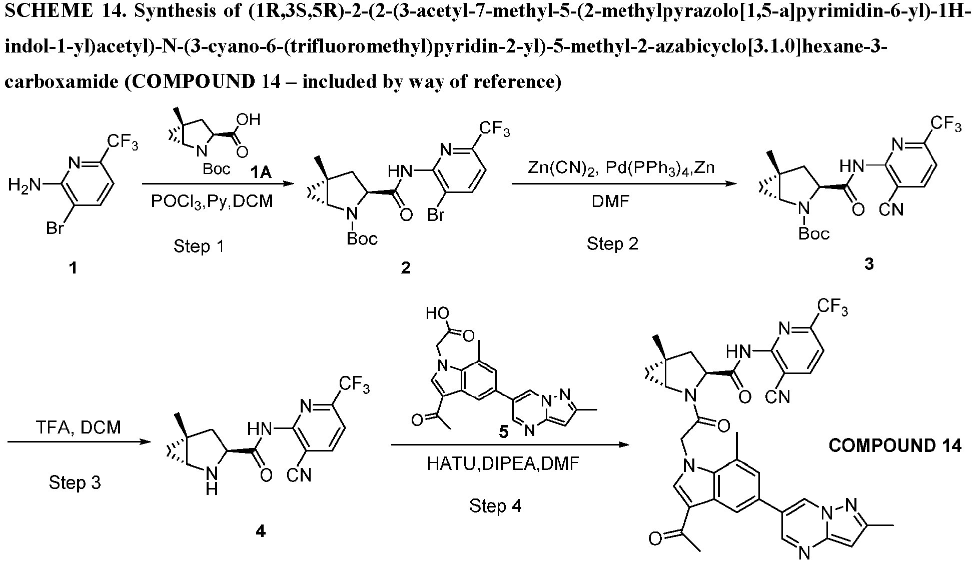

**Step 1:** To a mixture of intermediate **1** (100 mg, 0.42 mmol) and compound **1A** (100 mg, 0.42 mmol) in DCM (8 mL) was added pyridine (164 mg, 2.07 mmol), followed by addition of POCl₃ (70 mg, 0.45 mmol) at 0 °C under N₂ atmosphere. The mixture was stirred at 35 °C for 4 hours. The mixture was cooled to 20 °C and poured into iced-water and extracted with DCM twice. The combined organic layer was washed with brine, dried over anhydrous Na₂SO₄, filtered and concentrated to dryness. The residue was purified by chromatography on silica gel (PE : EtOAc = 100: 1 to 5: 1) to give intermediate **2** (63 mg, yield 32.7%) as a white solid. LC/MS (ESI) m/z : 464 (M+H)⁺.

**Step 2:** To a solution of intermediate **2** (63 mg, 0.14 mmol) in DMF (5 mL) was added Zn(CN)₂ (49 mg, 0.42 mmol), followed by addition of Pd(PPh₃)₄ (35 mg, 0.03 mmol) and Zn (0.7 mg, 0.01 mmol) at room temperature under N₂ atmosphere. The mixture was stirred at 120 °C in a microwave for 0.5 hour. The mixture was cooled to room temperature and poured into iced-water and extracted with EtOAc twice. The combined organic layers were washed with brine, dried over anhydrous Na₂SO₄, filtered and concentrated to dryness. The residue was purified by chromatography on silica gel (PE : EtOAc = 100: 1 to 5: 1) to give intermediate **3** (32 mg, yield 57.5%) as a white solid. LC/MS (ESI) m/z : 411 (M+H)⁺.

**Step 3:** To a solution of intermediate **3** (32 mg, 0.08 mmol) in DCM (2 mL) was added TFA (1 mL) at 0 °C and the mixture was stirred at room temperature for 2 hours. The reaction was concentrated to dryness, washed with DCM and dried over anhydrous Na₂SO₄, filtered and concentrated under vacuum to give intermediate **4** (24 mg, yield 99.2%) as red solid, which was used directly in the next step. LC/MS (ESI) m/z : 311 (M+H)⁺.

**Step 4:** To a mixture of intermediate **4** (30 mg, 0.1 mmol) and compound **5** (36 mg, 0.1 mmol) in DMF (3 mL) was added DIPEA (65 mg, 0.5 mmol), followed by addition of HATU (57 mg, 0.15 mmol) at 0 °C. The mixture was stirred at 25 °C for 2 hours. The mixture was diluted with EtOAc and washed with saturated NH₄Cl solution and brine, dried over anhydrous Na₂SO₄, filtered and concentrated to dryness. The residue was purified by prep-TLC (DCM : MeOH = 20: 1) to give **COMPOUND 14** (2 mg, yield 3.1%) as a white solid. ¹H NMR (400 MHz, DMSO-*d*₆) δ 11.40 (s, 1H), 9.18 (d, *J =* 2.4 Hz, 1H), 8.78 (d, *J =* 2.0 Hz, 1H), 8.68 (d, *J =* 80 Hz, 1H), 8.36 (d, *J =* 2.0 Hz, 1H), 8.30 (s, 1H), 7.94 (d, *J =* 80 Hz, 1H), 7.36 (s, 1H), 6.55 (s, 1H), 5.78 (d, *J =* 18.0 Hz, 1H), 5.41 (d, *J =* 18.0 Hz, 1H), 4.56 (dd, *J =* 9.2, 9.6 Hz, 1H), 3.60 (dd, *J =* 5.6, 5.6 Hz, 1H), 2.65 (s, 3H), 2.58 - 2.54 (m, 1H), 2.45 (s, 6H), 2.13 (dd, *J =* 13.2, 13.2 Hz, 1H), 1.34 (s, 3H), 1.04 (t, *J =* 5.4 Hz, 1H), 0.93 (dd, *J =* 5.2,5.2 Hz, 1H). LC/MS (ESI) m/z : 655 (M+H)⁺.

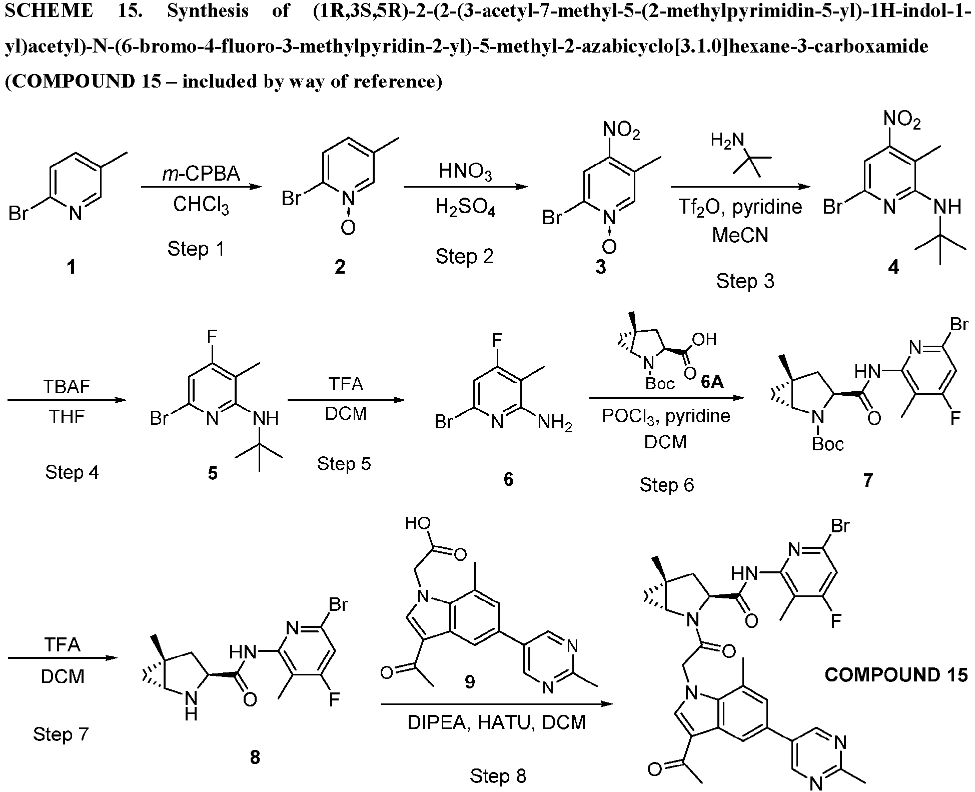

**Step 1:** To a solution of intermediate **1** (5 g, 29.2 mmol) in CHCl₃ (10 mL) was added *m*-CPBA (10 g, 58.4 mmol) at 0 °C and the mixture was stirred at 60 °C for 16 hours. The reaction was quenched with NaHCO₃ (20 mL) at 0 °C and the mixture was filtered. The filtrate was extracted with DCM and the organic layers were combined, dried over Na₂SO₄, filtered, concentrated to dryness to give intermediate **2** (5.1 g, yield 94.4%) as yellow solid. LC/MS (ESI) (m/z) : 188 (M+H)⁺.

**Step2:** To a solution of intermediate **2** (5.1 g, 27.2 mmol) in H₂SO₄ (15 mL) was added HNO₃ (24 mL) dropwise. The mixture was stirred at 100 °C for 4 hours. The reaction was cooled to 0 °C and poured into water (300 mL). The pH of the mixture was adjusted to 9 with 2N NaOH. The mixture was extracted with EtOAc and the organic layers were combined, dried over Na₂SO₄, filtered, concentrated to dryness to give intermediate **3** (4.6 g, yield 73.0%) as yellow solid. LC/MS (ESI) (m/z) : 232 (M+H)⁺.

**Step 3:** To a mixture of intermediate **3** (1 g, 4.31 mmol) and pyridine (682 mg, 8.62 mmol) in MeCN (15 mL) was added a solution of Tf₂O (1.2 g, 4.31 mmol) in MeCN (4 mL) slowly at 0 °C. The reaction was stirred at room temperature for 3 hours. The reaction was cooled to 10 °C and 2-methylpropan-2-amine (3.1 g, 43.1 mmol) was added. The reaction was stirred at room temperature for 16 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 2: 1) to give intermediate **4** (310 mg, yield 25%) as colorless oil. LC/MS (ESI) (m/z) : 288 (M+H)⁺.

**Step 4:** To a solution of intermediate **4** (310 mg, 0.92 mmol) in THF (5 mL) was added TBAF (440 mg, 1.39 mmol) at 0 °C under N₂. The reaction was stirred at 0 °C for 20 minutes. The reaction was quenched with water (10 mL) at 0 °C and extracted with DCM. The organic layer was combined, dried over Na₂SO₄, filtered and concentrated to dryness. The residue was purified by chromatography on silica gel (PE : EtOAc = 5: 1) to give intermediate **5** (200 mg, yield 83.3%) as yellow solid. LC/MS (ESI) (m/z) : 261 (M+H)⁺.

**Step 5:** To a solution of intermediate **5** (200 mg, 0.76 mmol) in DCM (5 mL) was added TFA (2.5 mL) at 0 °C under N₂. The reaction was stirred at 25 °C for 2 hours. The mixture was concentrated under reduced pressure to give intermediate **6** (170 mg, crude) as a white solid. LC/MS (ESI) m/z : 205 (M+H)⁺.

**Step 6:** To a mixture of intermediate **6** (170 mg, 0.83 mmol), compound **6A** (200 mg, 0.83 mmol) and pyridine (0.32 mL, 4.15 mmol) in DCM (3 mL) was added POCl₃ (0.1 mL, 0.91 mmol) dropwise at 0 °C under N₂. The reaction was stirred at 25 °C for 2 hours before quenched with ice-water (10 mL). The reaction mixture was extracted with DCM and the combined organic layer was washed with 1N HCl and brine, dried over anhydrous Na₂SO₄, filtered and concentrated to dryness. The residue was purified by column chromatography on silica gel (PE : EtOAc = 20: 1 to 5: 1) to give intermediate **7** (150 mg, yield 42.3%) as a white solid. LC/MS (ESI) m/z : 428 (M+H)⁺.

**Step 7:** To a solution of intermediate **7** (150 mg, 0.35 mmol) in DCM (3 mL) was added TFA (1 mL) at 0 °C under N₂. The reaction was stirred at 25 °C for 1.5 hours and concentrated under reduced pressure to give intermediate **8** (143 mg, crude TFA salt), which was used directly in the next step. LC/MS (ESI) m/z : 328 (M+H)⁺.

**Step 8:** To a mixture of intermediate **8** (30.0 mg TFA salt, 0.09 mmol), compound **9** (39.0 mg, 0.11 mmol) and DIPEA (0.06 mL, 0.35 mmol) in DMF (2 mL) was added HATU (67.6 mg, 0.178 mmol) at 0 °C under N₂. The reaction was stirred at 25 °C for 2 hours before water (10 mL) was added. The reaction mixture was extracted with EtOAc and the organic layer was washed with brine, dried over Na₂SO₄, filtered and concentrated to dryness. The residue was purified by column chromatography on silica gel (DCM : MeOH = 20: 1) and further purified by prep-HPLC to give **COMPOUND 15** (2.3 mg, yield 3.5%) as a white solid. ¹H NMR (400 MHz, CD₃OD) δ 8.97 (s, 2H), 8.47 (d, *J =* 1.9 Hz, 1H), 8.16 (s, 1H), 7.56 (d, *J =* 8.1 Hz, 1H), 7.32 (s, 1H), 5.61 (q, *J =* 18.0 Hz, 2H), 4.51 (dd, *J =* 9.2, 5.4 Hz, 1H), 3.70 (dd, *J =* 8.7, 4.3 Hz, 1H), 3.53 - 3.44 (m, 2H), 2.72 (d, *J =* 7.1 Hz, 6H), 2.68 - 2.59 (m, 1H), 2.53 (s, 3H), 2.33 - 2.24 (m, 1H), 2.16 (d, *J =* 6.8 Hz, 3H), 2.03 (d, *J =* 5.6 Hz, 1H), 1.11 (t, *J=* 5.5 Hz, 1H), 0.96 (dd, *J =* 5.4, 2.4 Hz, 1H). LC/MS (ESI) m/z : 633 (M+H)⁺.

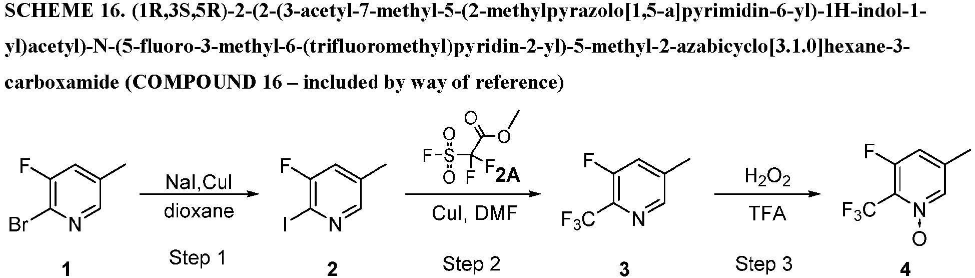

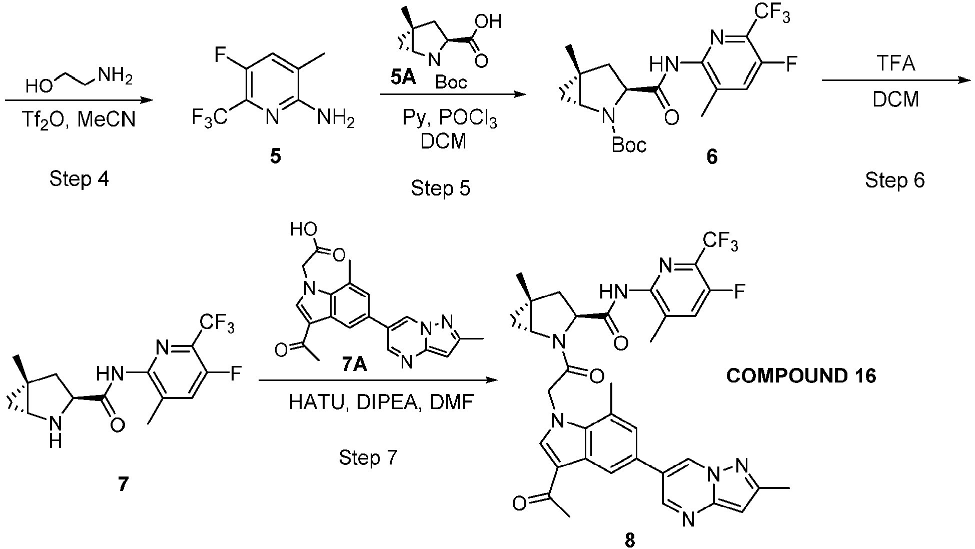

**Step 1:** To a solution of intermediate **1** (5 g, 26.4 mmol) in dioxane (10 mL) were added NaI (3.9 g, 26.4 mmol) and CuI (503 mg, 2.64 mmol). The mixture was stirred at 110 °C for 16 hours. The reaction was quenched with water (20 mL) at 0 °C and the mixture was filtered. The filtrate was extracted with DCM and the organic layers were combined, dried over Na₂SO₄, filtered, concentrated to dryness to give intermediate **2** (5.1 g, yield 73.6%) as yellow solid. LC/MS (ESI) (m/z) : 238 (M+H)⁺.

**Step 2:** To a solution of intermediate **2** (2.0 g, 8.43 mmol) in DMF (5 mL) were added compound **2A** (1.94 g, 10.1 mmol, 1.3 mL) and CuI (642 mg, 3.37 mmol) at room temperature under N₂. The mixture was stirred at 100 °C for 16 hours. The reaction was quenched with NH₄Cl saturated solution (20 mL) at 0 °C. The mixture was extracted with EtOAc and the organic layers were combined, dried over Na₂SO₄, filtered, and concentrated to dryness. The reaction was purified by chromatography on silica gel (PE : EtOAc = 5: 1) to give intermediate **3** (1.1 g, yield 73.0%) as yellow solid. LC/MS (ESI) (m/z) : 180 (M+H)⁺.

**Step 3:** To a solution of intermediate **3** (1.1 g, 6.1 mmol) in TFA (5 mL) was added H₂O₂ (1 mL) at 0 °C under N₂. The reaction was stirred at 80 °C for 1 hour and the mixture was extracted with DCM. The organic layers were combined, dried over Na₂SO₄, filtered and concentrated to dryness to give intermediate **4** (1.04 g, yield 88.1%) as a white solid. LC/MS (ESI) (m/z) : 196 (M+H)⁺.

**Step 4:** To a mixture of intermediate **4** (1 g, 5.33 mmol) and pyridine (1.68 g, 21.3 mmol) in MeCN (15 mL) was added a solution of Tf₂O (2.25 g, 7.9 mmol) in MeCN (4 mL) slowly at 0 °C. The reaction was stirred at room temperature for 3 hours. The reaction mixture was cooled to 0 °C and ethanolamine (3.25 g, 53.3 mmol) was added. The reaction was stirred at room temperature for 16 hours. The mixture was diluted with EtOAc and washed with saturated NH₄Cl solution, brine, dried over anhydrous Na₂SO₄, filtered and concentrated to dryness. The residue was purified by chromatography on silica gel (PE : EtOAc = 3: 1) to give intermediate 5 (286 mg, yield 27.8%) as yellow solid. LC/MS (ESI) (m/z) : 195 (M+H)⁺.

**Step 5:** To a mixture of intermediate **5** (270 mg, 1.39 mmol), compound **5A** (400 mg, 1.67 mmol) and pyridine (0.60 mL, 6.95 mmol) in DCM (3 mL) was added POCl₃ (0.15 mL, 1.53 mmol) dropwise at 0 °C under N₂. The reaction was stirred at 25 °C for 2 hours before quenched with ice-water (10 mL). The reaction mixture was extracted with DCM (20 mL x 2) and the combined organic layers were washed with 1N HCl and brine, dried over Na₂SO₄, filtered and concentrated to dryness. The residue was purified by column chromatography on silica gel (PE : EtOAc =10: 1 to 5: 1) to give intermediate **6** (246 mg, yield 42.4%) as a white solid. LC/MS (ESI) m/z: 418 (M+H)⁺.

**Step 6:** To a solution of intermediate **6** (246 mg, 0.58 mmol) in DCM (5 mL) was added TFA (2 mL) at 0 °C under N₂. The reaction was stirred at 25 °C for 1.5 hours and concentrated under reduced pressure to give intermediate **7** (180 mg, crude TFA salt), which was used directly in the next step. LC/MS (ESI) m/z : 318 (M+H)⁺.

**Step 7:** To a mixture of intermediate **7** (40.0 mg TFA salt, 0.13 mmol), compound **7A** (45.5 mg, 0.13 mmol) and DIPEA (0.12 mL, 0.52 mmol) in DMF (2 mL) was added HATU (98.8 mg, 0.26 mmol) at 0 °C under N₂. The reaction was stirred at 25 °C for 2 hours before water (10 mL) was added. The reaction mixture was extracted with EtOAc and the organic layer was washed with brine, dried over Na₂SO₄, filtered and concentrated to dryness. The residue was purified by column chromatography on silica gel (DCM : MeOH = 20: 1) and further purified by prep-HPLC to give **COMPOUND 16** (3.5 mg, yield 4.1%) as a white solid. ¹ H NMR (400 MHz, CD₃OD) δ 8.99 (d, *J =* 2.2 Hz, 1H), 8.82 (d, *J =* 2.2 Hz, 1H), 8.48 (d, *J =* 1.9 Hz, 1H), 8.16 (s, 1H), 7.74 (d, *J =* 10.3 Hz, 1H), 7.34 (s, 1H), 6.52 (s, 1H), 5.61 (d, *J =* 21.1 Hz, 2H), 4.55 - 451(m, 1H), 3.55 - 3.41 (m, 1H), 2.72 (s, 3H), 2.67 - 3.62 (m, 1H), 2.53 (s, 3H), 2.51 (s, 3H), 2.26 (s, 3H), 2.21 - 2.17 (m, 1H), 1.40 (s, 3H), 1.11 (t, *J =* 5.4 Hz, 1H), 0.97 (d, *J =* 5.9 Hz, 1H). LC/MS (ESI) m/z : 662 (M+H)⁺.

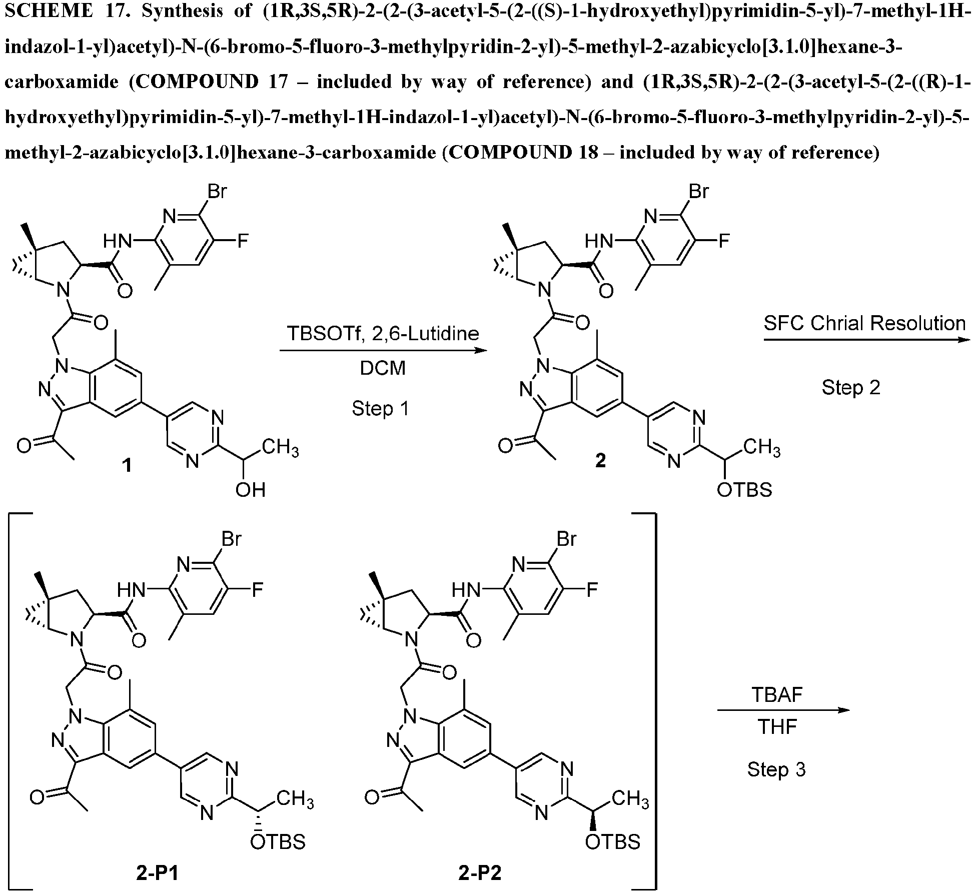

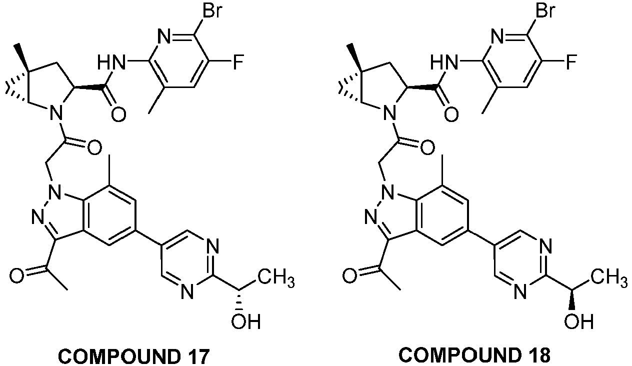

**Step 1:** To a solution of intermediate **1** (190 mg, 0.29 mmol) in DCM (8 mL) was added 2, 6-Lutidine (92 mg, 0.86 mmol), followed by addition of TBSOTf (91 mg, 0.34 mmol) at 0 °C. The mixture was stirred at room temperature overnight. The mixture was diluted with DCM and washed with brine, dried over anhydrous Na₂SO₄, filtered and concentrated to dryness. The residue was purified by chromatography on silica gel (DCM : MeOH = 100: 1 to 80: 1) to give intermediate **2** (200 mg, yield 90.1%) as a white solid, which was further purified by SFC chiral resolution to give intermediate **2-P1** (67 mg, 29.7% yield) and intermediate **2-P2** (76 mg, 33.7% yield). LC/MS (ESI) m/z : 778/780 (M+H)⁺.

**Step 2:** Mixture of intermediate **2-P1** (67 mg, 0.086 mmol) and TBAF (3 mL, 1M in THF) was stirred at 60 °C for 1 hour. The reaction mixture was washed with NH₄Cl solution and extracted with EtOAc. The organic layer was washed with brine, dried over Na₂SO₄ and concentrated to dryness. The residue was purified by prep-HPLC to give **COMPOUND 17** (23.8 mg, yield 41.6%) as a white solid. ¹H NMR (400 MHz, δ 10.37 (s, 1H), 9.15 (s, 2H), 8.38 (d, *J =* 1.2 Hz, 1H), 7.86 (d, *J =* 8.4 Hz, 1H), 7.67 (s, 1H), 6.07 (d, *J* = 17.6 Hz, 1H), 5.72 (d, *J =* 17.6 Hz, 1H), 5.34 (d, *J =* 5.2 Hz, 1H), 4.93 - 4.86 (m, 1H), 4.43 (dd, *J =* 9.2, 9.2 Hz, 1H), 3.63 (dd, *J =* 5.6, 5.6 Hz, 1H), 2.72 (s, 3H), 2.69 (s, 3H), 2.63-2.57 (m, 1H), 2.11 (s, 3H), 2.09 - 2.03 (m, 1H), 1.51 (d, *J =* 6.8 Hz, 3H), 1.36 (s, 3H), 1.06 (t, *J =* 5.4 Hz, 1H), 0.98 (dd, *J =* 5.2, 5.2 Hz, 1H). LC/MS (ESI) m/z : 664/666 (M+H)⁺.

**Step 3:** Mixture of intermediate **2-P2** (76 mg, 0.098 mmol) and TBAF (3 mL, 1M in THF) was stirred at 60 °C for 1 hour. The reaction mixture was washed with NH₄Cl solution and extracted with EtOAc. The organic layer was washed with brine, dried over Na₂SO₄ and concentrated to dryness. The residue was purified by prep-HPLC to give **COMPOUND 18** (24.3 mg, yield 37.5%) as a white solid. ¹H NMR (400 MHz, δ 10.33 (s, 1H), 9.11 (s, 2H), 8.34 (d, *J =* 1.2 Hz, 1H), 7.82 (d, *J =* 8.4 Hz, 1H), 7.64 (s, 1H), 6.04 (d, *J =* 18.0 Hz, 1H), 5.68 (d, *J =* 17.6 Hz, 1H), 5.29 (d, *J =* 5.6 Hz, 1H), 4.90 - 4.80 (m, 1H), 4.39 (dd, *J =* 9.2, 9.2 Hz, 1H), 3.59 (dd, *J=* 5.2, 5.2 Hz, 1H), 2.68 (s, 3H), 2.65 (s, 3H), 2.60 - 2.53 (m, 1H), 2.07 (s, 3H), 2.06 - 2.00 (m, 1H), 1.47 (d, *J =* 6.4 Hz, 3H), 1.32 (s, 3H), 1.02 (st, *J =* 5.2 Hz, 1H), 0.94 (dd, *J =* 5.2, 5.2 Hz, 1H). LC/MS (ESI) m/z : 664/666 (M+H)⁺.

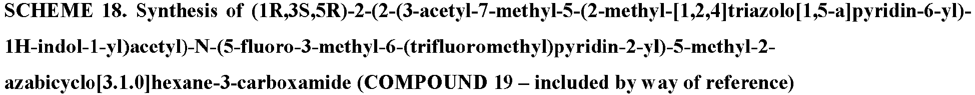

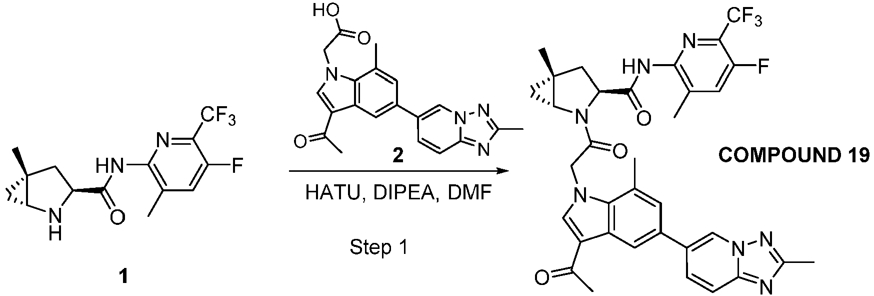

**Step 1:** To a mixture of intermediate **1** (100 mg TFA salt, 0.31 mmol), compound **2** (113 mg, 0.31mmol) and DIPEA (0.16 ml, 1.24 mmol) in DMF (2 mL) was added HATU (235 mg, 0.62 mmol) at 0 °C under N₂. The reaction was stirred at 25 °C for 2 hours before water (10 mL) was added. The reaction was extracted with EtOAc and the organic layer was washed with brine, dried over Na₂SO₄, filtered and concentrated to dryness. The residue was purified by column chromatography on silica gel (DCM : MeOH = 20: 1) and further purified by prep-HPLC to give **COMPOUND 19** (25.2 mg, yield 12.2%) as a white solid. ¹H NMR (400 MHz, CD₃OD) δ 8.99 (d, *J =* 1.5 Hz, 1H), 8.82 (d, *J =* 2.2 Hz, 1H), 8.48 (d, *J =* 1.5 Hz, 1H), 8.16 (s, 1H), 7.74 (d, *J =* 10.2 Hz, 1H), 7.33 (s, 1H), 6.52 (s, 1H), 5.61 (dd, *J =* 39.1, 18.1 Hz, 2H), 4.56 - 4.47 (m, 1H), 3.49 (dd, *J =* 7.2, 4.8 Hz, 2H), 2.72 (s, 3H), 2.67 - 2.60 (m, 1H), 2.53 (s, 3H), 2.51 (s, 3H), 2.26 (s, 3H), 2.22 - 2.16 (m, 1H), 1.40 (s, 3H), 1.12 (d, *J =* 5.0 Hz, 1H), 0.97 (d, *J =* 5.3 Hz, 1H). LC/MS (ESI) m/z : 662 (M+H)⁺.

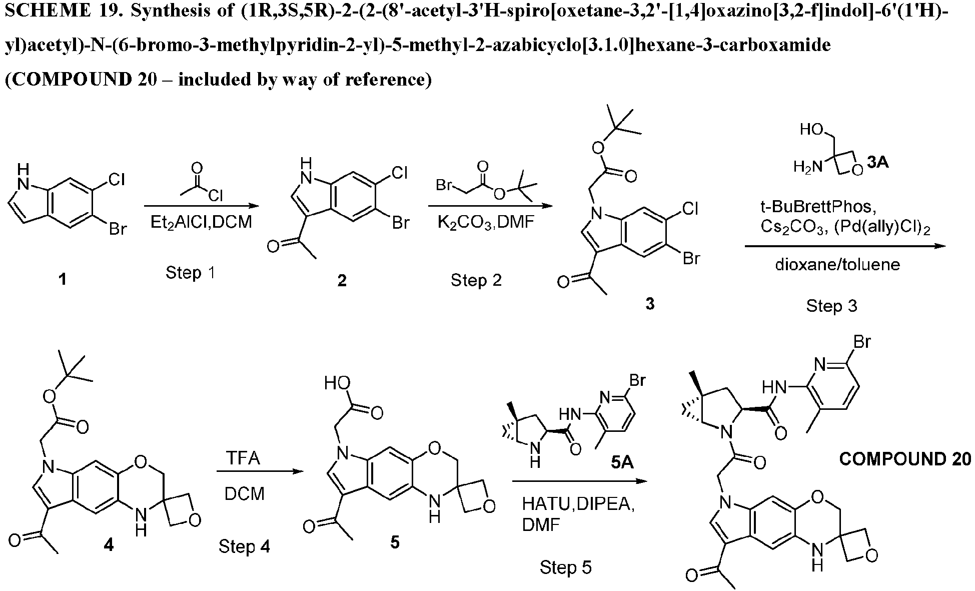

**Step 1:** To a solution of intermediate **1** (1.0 g, 4.35 mmol) in DCM (10 mL) was added Et₂AlCl (3.26 mL, 6.53 mmol, 2M) at 0°C under N₂ atmosphere and the mixture was stirred at 0 °C for 30 minutes. Acetyl chloride (512 mg, 6.53 mmol) was dropwise added to the reaction and the mixture was stirred at 0 °C for another 2 hours. The mixture was quenched with ice-water and extracted with DCM twice. The combined organic layers were washed with brine, dried over anhydrous Na₂SO₄, filtered and concentrated under reduced pressure to give intermediate **2** (1.18 g, yield 99.7%) as yellow solid, which was used directly in the next step. LC/MS(ESI) m/z : 272/274 (M+H)⁺.

**Step 2:** To a solution of intermediate **2** (500 mg, 1.85 mmol) in DMF (6 mL) was added K₂CO₃ (891 mg, 6.45 mmol) and tert-butyl 2-bromoacetate (540 mg, 2.77 mmol) at 0 °C and the mixture was stirred at 35 °C overnight. The mixture was quenched with ice-water and extracted with EtOAc twice. The combined organic layer was washed with brine, dried over anhydrous Na₂SO₄, filtered and concentrated to dryness. The residue was purified by chromatography on silica gel (PE : EtOAc= 50: 1 to 2: 1) to give intermediate **3** (560 mg, yield 78.9%) as a white solid. LC/MS(ESI) m/z : 386/388 (M+H)⁺.

**Step 3:** To a solution of intermediate **3** (75 mg, 0.19 mmol) in 1,4-dioxane (1 mL) and toluene (4 mL) were added Cs₂CO₃ (146 mg, 0.45 mmol), *t*-BuBrettPhos (44 mg, 0.092 mmol) and allylpalladium chloride dimer (21 mg, 0.058 mmol) at 0 °C under N₂ atmosphere, followed by addition of compound **3A** (50 mg, 0.49 mmol). The reaction was stirred at 90 °C overnight. The mixture was cooled to room temperature and was poured into iced-water and extracted with EtOAC twice. The combined organic layers were washed with brine, dried over anhydrous Na₂SO₄, filtered and concentrated to dryness. The residue was purified by prep-TLC (PE : EtOAc= 1: 1) to give intermediate **4** (40 mg, yield 55.56%) as yellow solid. LC/MS (ESI) m/z : 373 (M+H)⁺.

**Step 4:** To a solution of intermediate **4** (25 mg, 0.067 mmol) in DCM (1.6 mL) was added TFA (1.4 mL) at 0 °C and the reaction was stirred at room temperature overnight. The reaction mixture was concentrated to dryness, washed with ether and dried under vacuum to give intermediate **5** (21 mg, yield 98.9%) as yellow solid, which was used directly in the next step. LC/MS (ESI) m/z : 317 (M+H)⁺.

**Step 5:** To a mixture of intermediate **5** (21 mg, 0.066 mmol) and compound **5A** (21 mg, 0.066 mmol) in DMF (3 mL) was added DIPEA (43 mg, 0.33 mmol), followed by addition of HATU (38 mg, 0.10 mmol) at 0 °C. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by prep-HPLC to give **COMPOUND 20** (3.9 mg, yield 9.8%) as light yellow solid. ¹H NMR (400 MHz, δ 10.25 (s, 1H), 8.02 (s, 1H), 7.63 (d, *J =* 8.0 Hz, 1H), 7.45 (d, *J =* 2.8 Hz, 1H), 6.79 (s, 1H), 6.67 (s, 1H), 5.27 (d, *J =* 17.8 Hz, 1H), 5.07 (d, *J =* 17.8 Hz, 1H), 4.51 (d, *J =* 6.4 Hz, 2H), 4.46-4.41 (m, 2H), 4.39 - 4.34 (m, 1H), 4.23 (s, 2H), 3.55 - 3.52 (m, 1H), 2.55 - 2.52 (m, 1H), 2.35 (s, 3H), 2.03 (s, 3H), 2.03 - 1.97 (m, 1H), 1.30 (s, 3H), 0.98 (t, *J =* 5.2 Hz, 1H), 0.90 (dd, *J=* 5.2, 5.2 Hz, 1H). LC/MS (ESI) m/z : 608 (M+H)⁺.

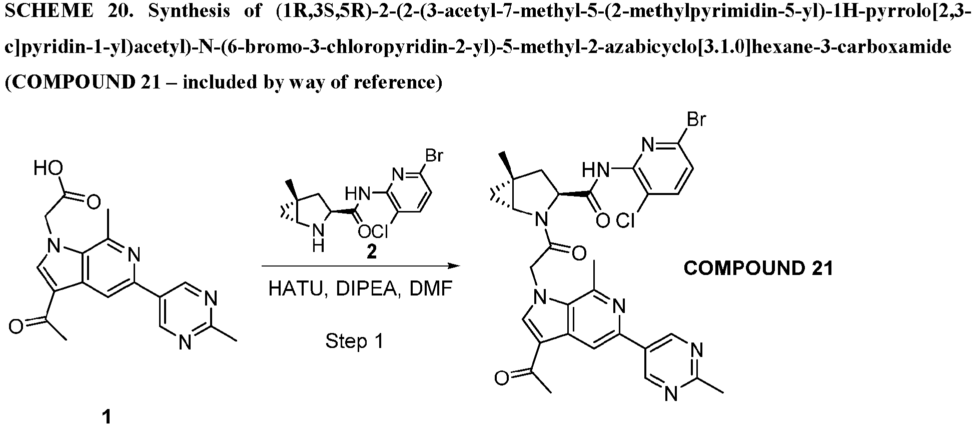

**Step 1:** To a mixture of intermediate **1** (36 mg, 0.11 mmol) and compound **2** (28 mg, 0.085 mmol) in DMF (5 mL) were added DIPEA (0.07 mL, 0.43 mmol) and HATU (65 mg, 0.17 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 30: 1) to give **COMPOUND 21** (28 mg, crude) as a brown solid, which was further purified by prep-HPLC to give **COMPOUND 21** (8.8 mg, yield 16.3%) as a white solid. ¹H NMR (400 MHz, CD₃OD) δ 9.25 (s, 2H), 8.55 (s, 1H), 8.30 (s, 1H), 8.07 (d, *J =* 8.6 Hz, 1H), 7.81 (d, *J =* 8.6 Hz, 1H), 5.71 (d, *J =* 17.9 Hz, 1H), 5.58 (d, *J =* 18.0 Hz, 1H), 4.48 (dd, *J =* 8.8, 5.7 Hz, 1H), 3.52 (dd, *J* = 5.4, 2.2 Hz, 1H), 2.89 (s, 3H), 2.74 (s, 3H), 2.60 - 2.54 (m, 1H), 2.52 (s, 3H), 2.15 (dd, *J* = 13.1, 5.5 Hz, 1H), 1.37 (s, 3H), 1.09 (t, *J =* 5.4 Hz, 1H), 0.95 (dd, *J =* 5.5, 2.3 Hz, 1H). LC/MS (ESI) m/z : 636/638 (M+H)⁺.

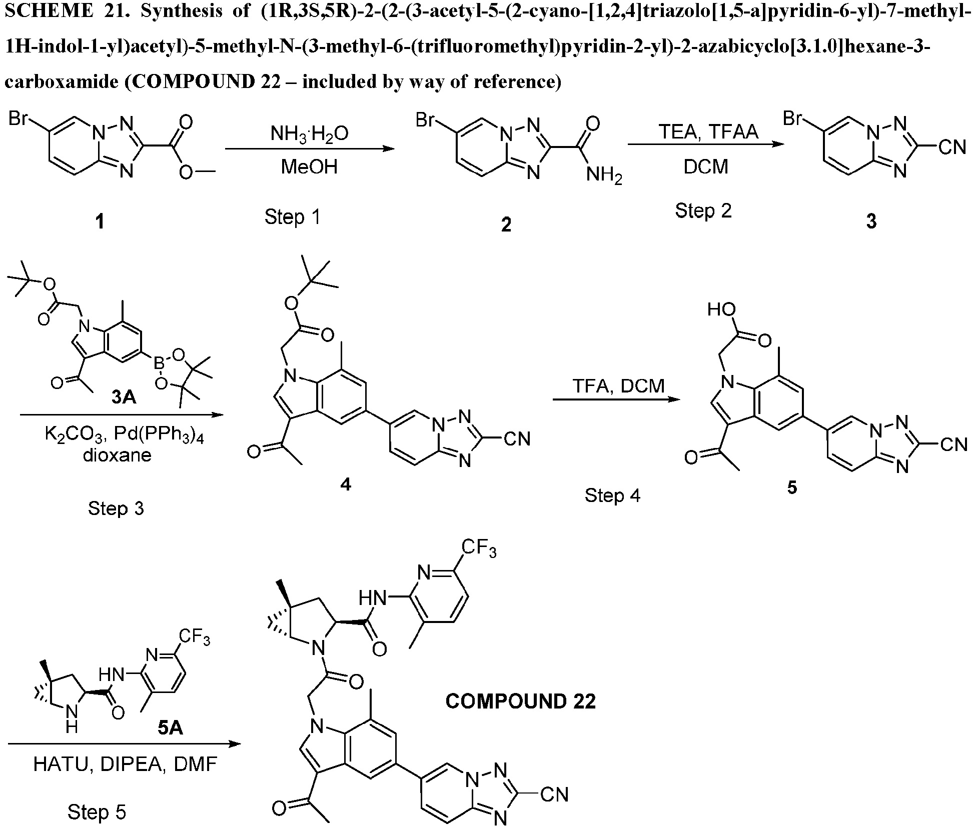

**Step 1:** To a solution of intermediate **1** (300 mg, 1.2mmol) in MeOH (5 mL) was added NH₃·H₂O (10 mL) and the mixture was stirred at 60 °C overnight. The mixture was diluted with water and extracted with DCM twice. The combined organic layer was washed with brine, dried over anhydrous Na₂SO₄, filtered and concentrated to dryness to give intermediate **2** (214 mg, yield 74.3%) as a white solid, which was used directly in the next step. LC/MS (ESI) m/z : 241 (M+H)⁺.

**Step 2:** To a solution of intermediate **2** (214 mg, 0.89 mmol) in DCM (10 mL) were added TEA (194 mg, 1.92mmol) and TFAA (202 mg, 0.96 mmol) at 0 °C under N₂ atmosphere. The mixture was stirred at room temperature overnight. The mixture was diluted with DCM and water and the organic layer was washed with brine, dried over anhydrous Na₂SO₄, filtered and concentrated to give intermediate **3** (190 mg, yield 96.0%) as a white solid, which was used directly in the next step. LC/MS (ESI) m/z : 223 (M+H)⁺.

**Step 3:** To a mixture of intermediate **3** (27 mg, 0.12 mmol) and compound **3A** (50 mg, 0.12 mmol) was added K₂CO₃ (41 mg, 0.30 mmol), followed by addition of Pd(PPh₃)₄ (12 mg, 0.01mmol) at 0 °C under N₂ atmosphere. The mixture was stirred at 90 °C overnight. The mixture was cooled to 20 °C and poured into iced-water and extracted with EtOAc twice. The combined organic layer was washed with brine, dried over anhydrous Na₂SO₄, filtered and concentrated to dryness. The residue was purified by prep-TLC (DCM : MeOH= 20: 1) to give intermediate **4** (35 mg, yield 68.6%) as yellow solid. LC/MS (ESI) m/z : 430 (M+H)⁺.

**Step 4:** To a solution of intermediate **4** (35 mg, 0.08 mmol.) in DCM (2 mL) was added TFA (1 mL) at 0 °C and the mixture was stirred at room temperature for 4 hours. The reaction mixture was concentrated to dryness, washed with DCM and dried under vacuum to give intermediate **5** (30 mg, yield 100%) as yellow solid, which was used directly in the next step. LC/MS (ESI) m/z : 374 (M+H)⁻.

**Step 5:** To a mixture of intermediate **5** (30 mg, 0.08 mmol) and compound **5A** (24 mg, 0.08 mmol) in DMF (5mL) were added DIPEA (52 mg, 0.4 mmol) and HATU (46 mg, 0.12 mmol) at 0 °C under N₂. The mixture was stirred at 25 °C for 4 hours. The mixture was diluted with EtOAc and washed with saturated NH₄Cl solution and brine, dried over anhydrous Na₂SO₄, filtered and concentrated to dryness. The residue was purified by prep-TLC (DCM : MeOH = 20: 1) to give **COMPOUND 22** (6.4 mg, yield 12.3%) as a white solid. ¹H NMR (400 MHz, CDCl₃) δ 8.79 (s, 1H), 8.58 (d, *J =* 1.5 Hz, 1H), 8.02 (dd, *J =* 12.0, 8.0 Hz, 1H), 7.85 (d, *J* = 120 Hz, 1H), 7.72 (s, 1H), 7.66 (d, *J =* 8.0 Hz, 1H), 7.43 (d, *J =* 8.0 Hz, 1H), 7.22 (s, 1H), 5.41 (dd, *J =* 68.0, 52.0 Hz, 2H), 4.96 (s, 1H), 3.16 (s, 1H), 2.74 (s, 3H), 2.64 (d, *J* =12.0 Hz, 1H), 2.53 (s, 3H), 2.42 (s, 1H), 2.24 (s, 3H), 1.43 (s, 3H), 1.18 (s, 1H), 0.90 (d, *J =* 4.0 Hz, 1H). LC/MS (ESI) m/z : 655 (M+H)⁺.

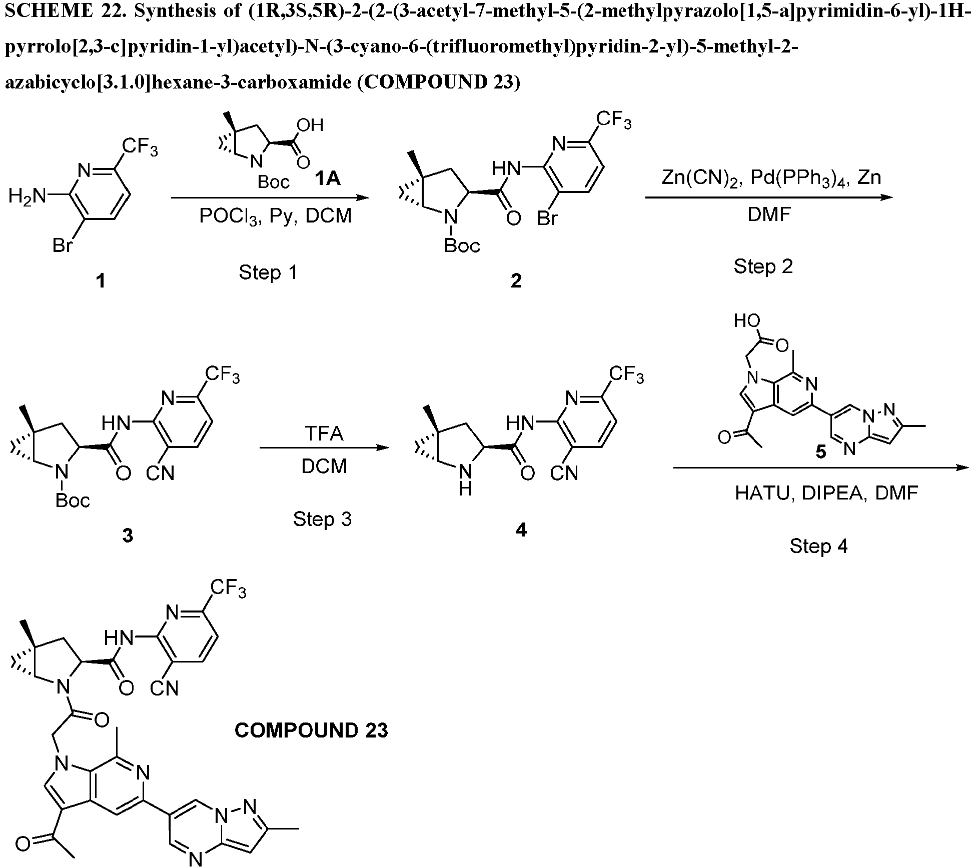

**Step 1:** To a mixture of intermediate **1** (100 mg, 0.41 mmol) and compound **1A** (100 mg, 0.41 mmol) in DCM (5mL) was added pyridine (162 mg, 2.05 mmol), followed by addition of POCl₃ (69 mg, 0.45 mmol) at 0 °C under N₂ atmosphere. The mixture was stirred at room temperature for 3 hours. The mixture was poured into iced-water and extracted with DCM twice. The combined organic layer was washed with brine, dried over anhydrous Na₂SO₄, filtered and concentrated to dryness. The residue was purified by chromatography on silica gel (PE : EtOAc = 100: 1 to 5: 1) to give intermediate **2** (89 mg, yield 46.8%) as a white solid. LC/MS (ESI) m/z : 464 (M+H)⁺.

**Step 2:** To a solution of intermediate **2** (89 mg, 0.19 mmol) in DMF (5mL) were added Zn(CN)₂ (67 mg, 0.57 mmol), Pd(PPh₃)₄ (46 mg, 0.04 mmol) and Zn (1 mg, 0.02 mmol) at room temperature under N₂ atmosphere. The mixture was stirred at 120 °C by microwave for 0.5 hour. The mixture was cooled to room temperature, poured into ice-water and extracted with EtOAc twice. The combined organic layer was washed with brine, dried over anhydrous Na₂SO₄, filtered and concentrated to dryness. The residue was purified by prep-TLC (PE : EtOA = 5: 1) to give intermediate **3.** (25 mg, yield 32.1%) as a white solid. LC/MS (ESI) m/z : 411 (M+H)⁺.

**Step 3:** To a solution of intermediate **3** (25 mg, 0.06 mmol) in DCM (2 mL) was added TFA (1 mL) at 0°C and the mixture was stirred at room temperature for 2 hours. The reaction mixture was concentrated to dryness, washed with DCM and dried under vacuum to give intermediate **4** (19 mg, yield 99.2%), as red solid, which was used directly in the next step.LC/MS (ESI) m/z : 311 (M+H)⁺.

**Step 4:** To a mixture of intermediate **4** (19 mg, 0.06 mmol) and compound 5 (43 mg, 0.12 mmol) in DMF (3 mL) were added DIPEA (39 mg, 0.3 mmol) and HATU (34 mg, 0.09 mmol) at 0 °C. The mixture was stirred at 25 °C for 2 hours. The mixture was diluted with EtOAc and washed with saturated NH₄Cl solution and brine, dried over anhydrous Na₂SO₄, filtered and concentrated to dryness. The residue was purified by prep-TLC (DCM : MeOH = 20:1) to give **COMPOUND 23** (6.4 mg, yield 16.4%) as a white solid. ¹H NMR (400 MHz, δ 11.40 (s, 1H), 9.42 (dd, *J =* 2.4, 2.0 Hz, 1H), 9.12 (d, *J =* 2.0 Hz, 1H), 8.67 (d, *J =* 7.6 Hz, 1H), 8.50 (s, 1H), 8.47 (s, 1H), 7.93 (d, *J =* 8.0 Hz, 1H), 6.56 (s, 1H), 5.84 (d, *J =* 17.6 Hz, 1H), 5.49 (d, *J =* 21.6 Hz, 1H), 4.56 (dd, *J =* 9.2, 9.6 Hz, 1H), 3.64 - 3.61 (m, 1H), 2.84 (s, 3H), 2.59 - 2.54 (m, 1H), 2.51 (s, 3H), 2.45 (s, 3H), 2.13 (dd, *J =* 13.2, 13.6 Hz, 1H), 1.34 (s, 3H), 1.05 (t, *J* = 5.4 Hz, 1H), 0.95 (dd, *J =* 4.8, 5.2 Hz, 1H). LC/MS (ESI) m/z : 655 (M+H)⁺.

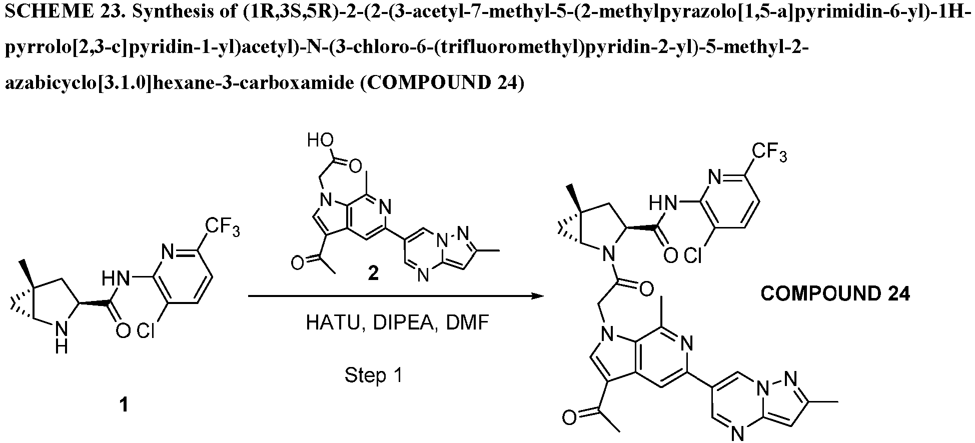

**Step 1:** To a mixture of intermediate 1 (63 mg, 0.2 mmol) and compound 2 (144 mg, 0.22 mmol) in DMF (3 mL) were added DIPEA (0.2 mL, 1.0 mmol) and HATU (114 mg, 0.3 mmol) at 0 °C and the mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM: MeOH = 20: 1) to give **COMPOUND 24** (8.3 mg, yield 6.2%) as a white solid. ¹H NMR (400 MHz, δ 10.70 (s, 1H), 9.42 (d, *J =* 1.6 Hz, 1H), 9.13 (d, *J =* 2.0 Hz, 1H), 8.50 (s, 1H), 8.46 (s, 1H), 8.29 (d, *J =* 8.0 Hz, 1H), 7.82 (d, *J =* 8.0 Hz, 1H), 6.56 (s, 1H), 5.79 (d, *J =* 18.0 Hz, 1H), 5.47 (d, *J =* 18.0 Hz, 1H), 4.53 (dd, *J =* 9.6, 8.8 Hz, 1H), 3.56 (dd, *J =* 5.2,1.2 Hz, 1H), 2.83 (s, 3H), 2.61 - 2.53 (m, 1H), 2.51 (s, 3H), 2.46 (s, 3H), 2.13 (dd, *J =* 13.2, 13.2 Hz, 1H), 1.33 (s, 3H), 1.04 (t, *J =* 3.4 Hz, 1H), 0.93 (dd, *J =* 4.8, 4.8 Hz, 1H). LC/MS (ESI) m/z : 665 (M+H)⁺.

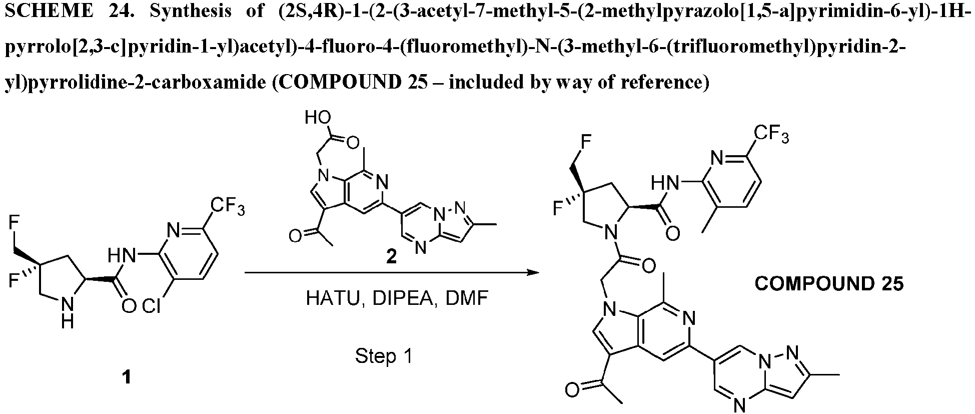

**Step 1:** To a mixture of intermediate **1** (35 mg, 0.11 mmol) and compound 2 (80 mg, 0.22 mmol) in DMF (3 mL) were added DIPEA (0.1 mL, 0.55 mmol) and HATU (65 mg, 0.17 mmol) at 0 °C and the mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 20: 1) to give **COMPOUND 25** (2.1 mg, yield 2.9%) as a white solid. ¹H NMR (400 MHz, δ 10.72 (s, 1H), 9.43 (d, *J* = 1.6 Hz, 1H), 9.13 (d, *J =* 2.4 Hz, 1H), 8.51 (s, 1H), 8.42 (s, 1H), 7.93 (d, *J =* 4.0 Hz, 1H), 7.68 (d, *J =* 7.6 Hz, 1H), 6.56 (s, 1H), 5.60 (dd, *J =* 38.4, 38.0 Hz, 2H), 5.01 - 4.77 (m, 3H), 4.15 (ddd, *J* =14.8, 12.8, 12.0 Hz, 2H), 2.83 (s, 3H), 2.70 - 2.67 (m, 1H), 2.46 (s, 3H), 2.15 (s, 3H), 1.24 (s, 1H). LC/MS (ESI) m/z : 669 (M+H)⁺.

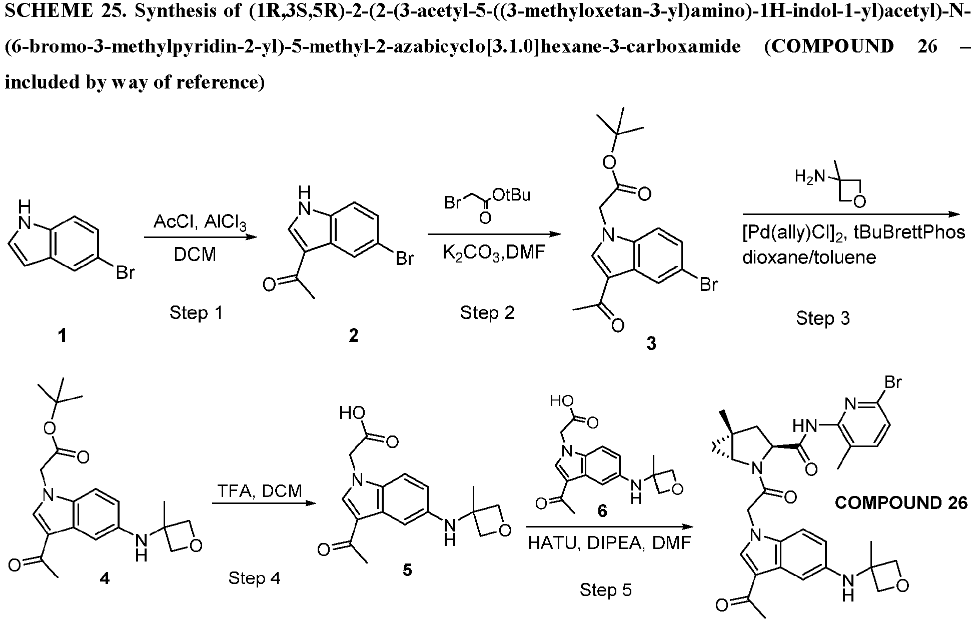

**Step 1:** To a solution of intermediate **1** (1 g, 5.1 mmol) in DCM (5 mL) was added AlCl₃ (922 mg, 7.6 mmol) at 0 °C under N₂. After stirring for 1 hour, acetyl chloride (600 mg, 7.6 mmol) was added. The resulting mixture was stirred at 25 °C for 2 hours. The mixture was poured into ice-water and extracted with DCM twice. The combined organic layer was washed with brine, dried over anhydrous Na₂SO₄, filtered and concentrated to dryness to give intermediate **2** (610 mg, yiled 50.8%) as a brown solid. LC/MS (ESI) (m/z) : 237 (M+H)⁺.

**Step 2:** To a solution of intermediate **2** (610 mg, 2.58 mmol) in DMF (3 mL) were added K₂CO₃ (891 mg, 6.46 mmol) and tert-butyl 2-bromoacetate (1 g, 5.16 mmol) at room temperature. The mixture was stirred at room temperature for 16 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 2: 1) to give intermediate **3** (500 mg , yield 55.2%) as yellow solid. LC/MS (ESI) (m/z) : 352 (M+H)⁺.

**Step 3:** To a mixture of intermediate **3** (500 mg, 1.42 mmol) and Cs₂CO₃ (1.38 g, 4.26 mmol) in dioxane (0.75 mL) and toluene (3 mL) were added allylpalladium chloride dimer (104 mg, 0.284 mmol), t-ButylBrettPhos (138 mg, 0.284 mmol) and 3-methyloxetan-3-amine (250 mg, 2.84 mmol) at room temperature under N₂. The mixture was stirred at 100 °C for 16 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 2 : 1) to give intermediate **4** (190 mg , yield 37.3%) as yellow solid . LC/MS (ESI) (m/z) : 359(M+H)⁺.

**Step 4:** To a solution of intermediate **4** (150 mg, 0.41 mmol) in DCM (5 mL) was added TFA (2.5 mL) at 0 °C under N₂. The reaction was stirred at 25 °C for 2 hours. The mixture was concentrated under reduced pressure to give intermediate **5** (80 mg, crude) as a white solid. LC/MS (ESI) m/z: 302(M+H)⁺.

**Step 5:** To a mixture of intermediate **5** (30.0 mg TFA salt, 0.09 mmol), compound **6** (30.8 mg, 0.09mmol) and DIPEA (0.06 mL, 0.18 mmol) in DMF (2 mL) was added HATU (67.6 mg, 0.178 mmol) at 0 °C under N₂. The reaction was stirred at 25 °C for 2 hours before water (10 mL) was added. The reaction mixture was extracted with EtOAc and the organic layer was washed with brine, dried over Na₂SO₄, filtered and concentrated to dryness. The residue was purified by column chromatography on silica gel (DCM : MeOH = 20: 1) and further purified by prep-HPLC to give **COMPOUND 26** (2.9 mg, yield 5.4%) as a white solid. ¹H NMR (400 MHz, CD₃OD) δ 8.28 (s, 1H), 8.04 (s, 1H), 7.55 (t, *J =* 8.2 Hz, 2H), 7.40 (d, *J =* 8.0 Hz, 1H), 7.07 (d, *J =* 9.3 Hz, 1H), 5.41 (dd, *J =* 47.0, 17.4 Hz, 3H), 4.96 (d, *J =* 7.1 Hz, 3H), 4.58 (d, *J =* 7.2 Hz, 2H), 4.52 (dd, *J* = 9.4, 5.2 Hz, 1H), 3.52 (dd, *J =* 5.5, 2.4 Hz, 1H), 2.62 (dd, *J =* 13.5, 9.4 Hz, 1H), 2.52 (s, 3H), 2.30 (dd, *J =* 13.3, 5.3 Hz, 1H), 2.12 (s, 3H), 1.62 (s, 4H), 1.40 (s, 3H), 1.10 (t, *J =* 5.2 Hz, 1H), 0.98 (dd, *J =* 5.6, 2.4 Hz, 1H). LC/MS (ESI) m/z : 594 (M+H)⁺.

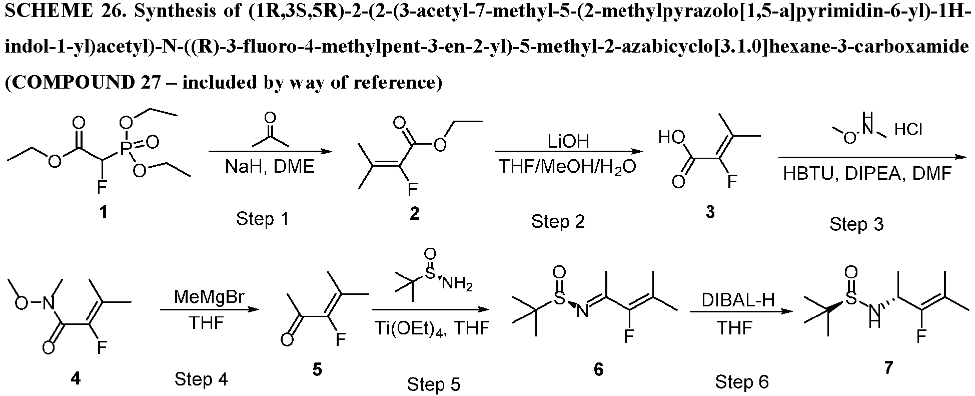

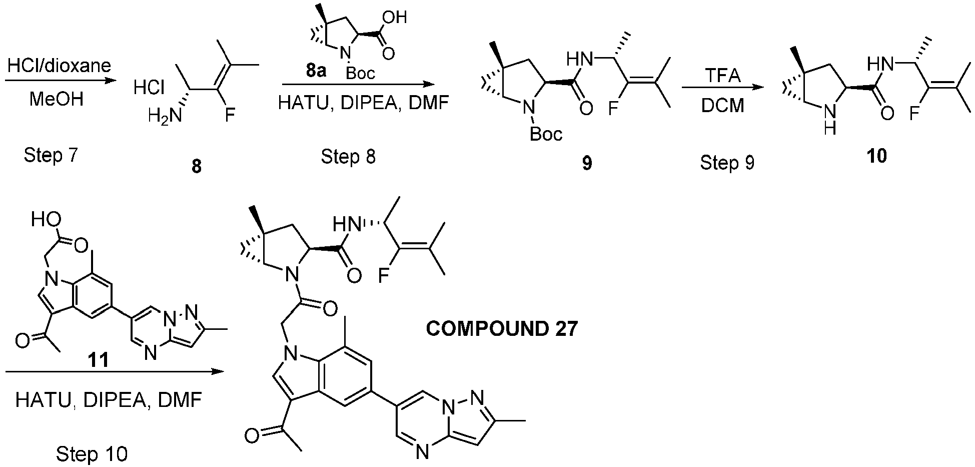

**Step 1:** To a solution of NaH (1.81 g, 45.4 mmol) in DME (20 mL) was added intermediate **1** (10.0 g, 41.3 mmol) dropwise at 0 °C under N₂. The mixture was stirred at 20 °C for 1 hour. Acetone (4.55 mL, 61.3 mmol) was then added and the mixture was stirred at 90 °C for 16 hours. The mixture was diluted with DCM, washed with brine, dried over anhydrous Na₂SO₄, filtered and concentrated. The crude residue was purified by chromatography on silica gel (PE : EtOAc = 9: 1) to give intermediate **2** (2.68 g, yield 44.7 %) as yellow oil. LC/MS (ESI) (m/z) : 147 (M+H)⁺.

**Step 2:** To a solution of intermediate **2** (2.68 g, 18.2 mmol) in MeOH (8 mL), THF (8 mL) and water (8 mL) was added LiOH·H₂O (3.8 g, 91.2 mmol) at 0 °C. The mixture was stirred at 50 °C for 12 hours. The mixture was concentrated to 1/5 volume, diluted with water and washed with MTBE twice. The aqueous layer was acidified with 1N aq.HCl to pH = 3, extracted with DCM twice. The combined organic layers were washed with brine, dried over anhydrous Na₂SO₄, and concentrated under reduced pressure to dryness to give intermediate **3** (2.16 g, yield 99.9%) as a white solid. LC/MS (ESI) (m/z) : 119 (M+H)⁺.

**Step 3:** To a mixture of intermediate **3** (1.57 g, 13.2 mmol) and N,O-dimethylhydroxylamine (1.93 g, 19.8 mmol) in DMF (15 mL) were added DIPEA (6.6 mL, 39.6 mmol) and HBTU (7.52 g, 19.8 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE: EtOAc = 14: 1) to give intermediate **4** (1.8 g, yield 84.1%) as colorless oil. LC/MS (ESI) (m/z): 162 (M+H)⁺.

**Step 4:** To a solution of intermediate **4** (3.7 g, 22.8 mmol) in THF (30 mL) was added dropwise MeMgBr (3M in Et₂O, 15 mL, 68.4 mmol) at 0 °C under N₂ atmosphere. The mixture was stirred at 25 °C for 1.5 hours. The reaction mixture was quenched with water at 0 °C and extracted with DCM, dried over anhydrous Na₂SO₄ and concentrated (by water pump at 0 °C) to dryness to give intermediate **5** (2.2 g, yield 82.4%) as yellow oil, which was used directly in next step without further purification.

**Step 5:** To a solution of (R)-2-methylpropane-2-sulfinamide (220 mg, 1.80 mmol) in THF (3 mL) was added a solution of intermediate **5** (190 mg, 1.64 mmol) in THF (2 mL), followed by addition of Ti(OEt)₄(1.05 g, 4.59 mmol) at 0 °C under N₂ atmosphere. The mixture was stirred at 70 °C for 16 hours. The mixture was poured into brine and filtered. The filter cake was washed with EtOAc twice. The organic layers were washed with brine, dried over anhydrous Na₂SO₄, and concentrated under reduced pressure to dryness. The residue was purified by chromatography on silica gel (PE : EtOAc = 5: 1) to give intermediate **6** (145 mg, yield 53.5%) as yellow oil. LC/MS (ESI) (m/z) : 220 (M+H)⁺.

**Step 6:** To a solution of intermediate **6** (145 mg, 0.66 mmol) in THF (5 mL) was added dropwise DIBAL-H (1M in Hexane, 1.3 mL, 1.98 mmol) at -78 °C under N₂ atmosphere. The mixture was stirred at -78 °C for 1 hour. To the mixture was added saturated NH₄Cl solution at -78 °C and the mixture was extracted with DCM twice, dried over anhydrous Na₂SO₄, filtered and concentrated to dryness. The residue was purified by chromatography on silica gel (PE: EtOAc = 1: 1) to give intermediate **7** (120 mg, yield 81.6%) as yellow oil. LC/MS (ESI) m/z : 222 (M+H)⁺.

**Step 7:** To a solution of intermediate **7** (120 mg, 0.54 mmol) in MeOH (1 mL) was added 4 M HCl/dioxane (0.8 mL, 3.24 mmol) at 0 °C and the mixture was stirred at room temperature for 5 hours. The mixture was concentrated to give intermediate **8** (63 mg, yield 99%) as brown oil. The crude product was used directly in next step without further purification. LC/MS (ESI) (m/z) : 118 (M+H)⁺.

**Step 8:** To a mixture of intermediate **8** (30 mg, 0.26 mmol) and compound **8a** (92 mg, 0.39 mmol) in DMF (3 mL) were added DIPEA (0.21 mL, 1.3 mmol) and HATU (194 mg, 0.52 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE: EtOAc = 5: 1) to give intermediate **9** (45 mg, yield 52%) as yellow semi-solid. LC/MS (ESI) (m/z) : 341 (M+H)⁺.

**Step 9:** To a solution of intermediate **9** (36 mg, 0.1 mmol) in DCM (3 mL) was added TFA (1 mL) at 0 °C and the mixture was stirred at room temperature for 1 hour. The mixture was concentrated to give intermediate **10** (25 mg, yield 99%) as brown oil. The crude product was used directly in next step without further purification. LC/MS (ESI) (m/z) : 241 (M+H)⁺.

**Step 10:** To a mixture of intermediate **10** (30 mg, 0.12 mmol) and compound **11** (45 mg, 0.12 mmol) in DMF (3 mL) were added DIPEA (0.1 mL, 0.6 mmol) and HATU (95 mg, 0.24 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM: MeOH = 97: 3) to give **COMPOUND** 27 (45 mg, crude) as a brown solid. The residue was purified by prep-HPLC to give **COMPOUND** 27 (14.4 mg, yield 20.0%) as a white solid. ¹H NMR (400 MHz, CD₃OD) δ 8.92 (d, *J =* 2.2 Hz, 1H), 8.77 (d, *J =* 2.2 Hz, 1H), 8.42 (d, *J =* 1.7 Hz, 1H), 8.08 (s, 1H), 7.28 (s, 1H), 6.49 (s, 1H), 5.57 - 5.45 (m, 2H), 5.00 - 4.87 (m, 1H), 4.32 (dd, *J =* 9.2, 5.2 Hz, 1H), 3.40 (dd, J = 5.5, 2.4 Hz, 1H), 2.65 (s, 3H), 2.50 (s, 6H), 2.48 - 2.43 (m, 1H), 2.04 (dd, *J =* 12.9, 4.7 Hz, 1H), 1.64 (d, *J =* 2.9 Hz, 3H), 1.54 (d, *J =* 3.3 Hz, 3H), 1.35 (s, 3H), 1.24 (d, *J =* 7.0 Hz, 3H), 1.07 (t, *J =* 5.2 Hz, 1H), 0.87 (dd, *J =* 5.4, 2.4 Hz, 1H); LC/MS (ESI) m/z : 585 (M+H)⁺.

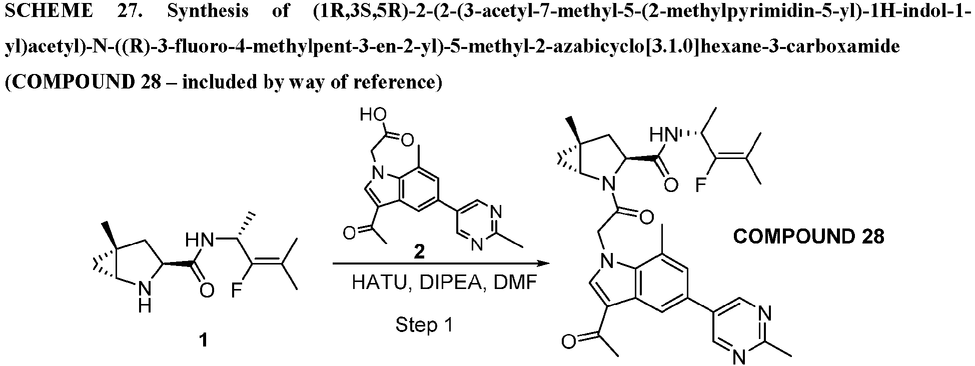

**Step 1:** To a mixture of intermediate **1** (27 mg, 0.11 mmol) and compound **2** (43 mg, 0.13 mmol) in DMF (5 mL) were added DIPEA (0.09 mL, 0.55 mmol) and HATU (85 mg, 0.22 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM: MeOH = 97: 3) to give **COMPOUND 28** (31 mg, crude) as a brown solid. The residue was purified by prep-HPLC to give **COMPOUND 28** (4.0 mg, yield 6.6%) as a white solid. ¹H NMR (400 MHz, CD₃OD) δ 8.96 (s, 2H), 8.46 (d, *J =* 1.5 Hz, 1H), 8.13 (s, 1H), 7.31 (s, 1H), 5.61 - 5.48 (m, 2H), 5.01 - 4.89 (m, 1H), 4.32 (dd, *J* = 9.2, 5.2 Hz, 1H), 3.41 (dd, *J =* 5.4, 2.3 Hz, 1H), 2.73 (s, 3H), 2.68 (s, 3H), 2.51 (d, *J =* 4.4 Hz, 3H), 2.49 - 2.43 (m, 1H), 2.04 (dd, *J =* 13.3, 5.2 Hz, 1H), 1.64 (d, *J =* 2.9 Hz, 3H), 1.54 (d, *J =* 3.3 Hz, 3H), 1.35 (s, 3H), 1.24 (d, *J =* 7.0 Hz, 3H), 1.07 (t, *J =* 5.4 Hz, 1H), 0.87 (dd, *J =* 5.4, 2.4 Hz, 1H); LC/MS (ESI) m/z : 546 (M+H)⁺.

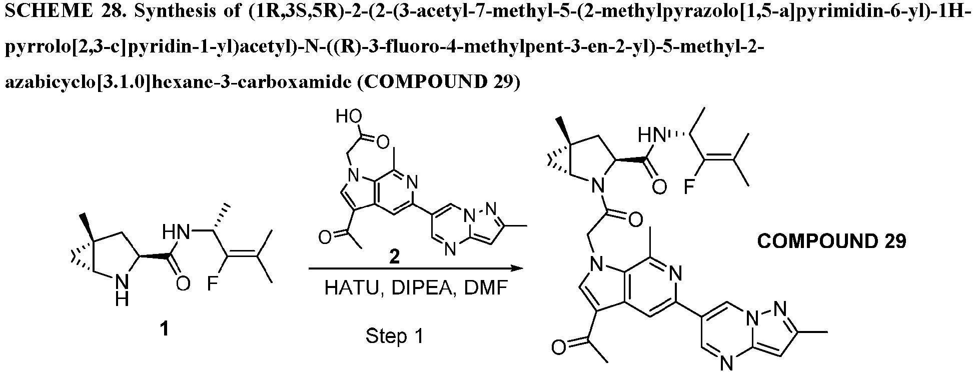

**Step 1:** To a mixture of intermediate **1** (27 mg, 0.11 mmol) and compound **2** (45 mg, 0.12 mmol) in DMF (5 mL) were added DIPEA (0.09 mL, 0.55 mmol) and HATU (85 mg, 0.22 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM: MeOH = 96: 4) to give **COMPOUND 29** (88 mg, crude) as a brown solid. The residue was purified by prep-HPLC to give **COMPOUND 29** (19.7 mg, yield 30.3%) as a white solid. ¹H NMR (400 MHz, CD₃OD) δ 9.26 (dd, *J* = 2.1, 0.7 Hz, 1H), 9.07 (d, *J* = 2.1 Hz, 1H), 8.48 (s, 1H), 8.19 (s, 1H), 6.50 (s, 1H), 5.52 (q, *J =* 17.9 Hz, 2H), 4.99 - 4.89 (m, 1H), 4.33 (dd, *J =* 9.1, 5.2 Hz, 1H), 3.40 (dd, *J =* 5.5, 2.3 Hz, 1H), 2.80 (s, 3H), 2.50 (s, 6H), 2.49 - 2.44 (m, 1H), 2.05 (dd, *J =* 12.6, 5.0 Hz, 1H), 1.63 (d, *J =* 2.9 Hz, 3H), 1.51 (d, *J =* 3.3 Hz, 3H), 1.36 (s, 3H), 1.24 (d, *J =* 7.0 Hz, 3H), 1.08 (t, *J =* 5.1 Hz, 1H), 0.89 (dd, *J =* 5.4, 2.4 Hz, 1H); LC/MS (ESI) m/z : 586 (M+H)⁺.

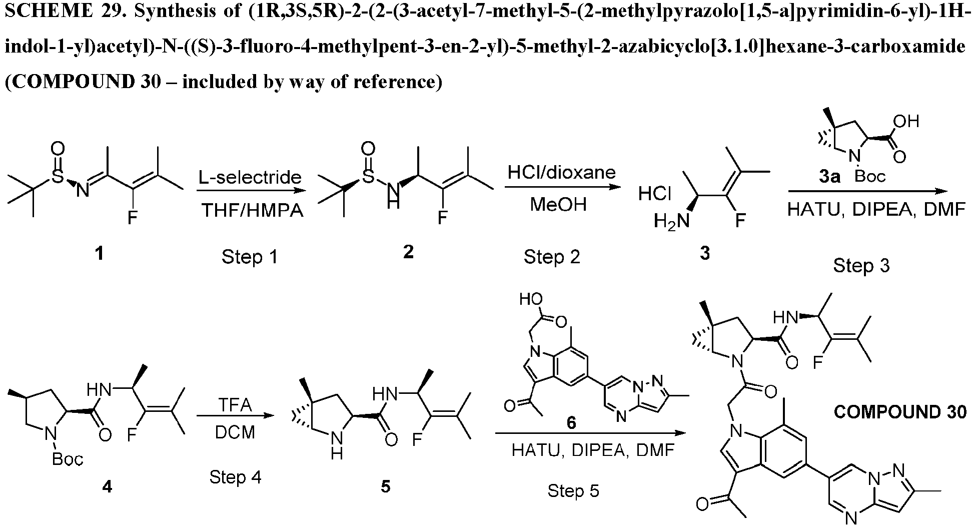

**Step 1:** To a mixture of intermediate 1 (280 mg, 1.28 mmol) and HMPA (0.6 mL) in THF (3 mL) was added dropwise L-selectride (1M in THF, 3.8 mL, 3.84 mmol) at -78 °C under N₂ atmosphere. The mixture was stirred at - 78 °C for 1 hour. The mixture was added saturated NH₄Cl solution at -78 °C and extracted with DCM twice, dried over anhydrous Na₂SO₄, filtered and concentrated to dryness. The residue was purified by chromatography on silica gel (PE : EtOAc = 1: 1) to give intermediate 2 (278 mg, yield 98.6%) as yellow oil. LC/MS (ESI) m/z : 222 (M+H)⁺.

**Step 2:** To a solution of intermediate 2 (278 mg, 1.26 mmol) in MeOH (8 mL) was added 4 M HCl/dioxane (1.89 mL, 7.56 mmol) at 0 °C and the mixture was stirred at room temperature for 5 hours. The mixture was concentrated to give intermediate **3** (140 mg, yield 95.2%) as brown oil. The crude product was used directly in next step without further purification. LC/MS (ESI) (m/z) : 118 (M+H)⁺.

**Step 3:** To a mixture of intermediate **3** (140 mg, 1.20 mmol) and compound **3a** (154 mg, 1.32 mmol) in DMF (10 mL) were added DIPEA (0.48 mL, 6.0 mmol) and HATU (441 mg, 2.4 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 5: 1) to give intermediate **4** (327 mg, yield 80.1%) as yellow oil. LC/MS (ESI) (m/z) : 341 (M+H)⁺.

**Step 4:** To a solution of intermediate **4** (327 mg, 0.96 mmol) in DCM (6 mL) was added TFA (2 mL) at 0 °C and the mixture was stirred at room temperature for 1 hour. The mixture was concentrated to give intermediate **5** (213 mg, yield 92.2%) as yellow oil. The crude product was used directly in next step without further purification. LC/MS (ESI) (m/z) : 241 (M+H)⁺.

**Step 5:** To a mixture of intermediate **5** (30 mg, 0.12 mmol) and compound **6** (45 mg, 0.12 mmol) in DMF (3 mL) were added DIPEA (0.1 mL, 0.6 mmol) and HATU (95 mg, 0.24 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 30** (56 mg, crude) as a white solid. The residue was purified by prep-HPLC to give **COMPOUND 30** (8.0 mg, yield 10.9%) as a white solid. ¹H NMR (400 MHz, CD₃OD) δ 8.88 (d, *J=* 34.4 Hz, 1H), 8.74 (d, *J =* 21.0 Hz, 1H), 8.40 (d, *J =* 25.1 Hz, 1H), 8.07 (d, *J =* 26.5 Hz, 1H), 7.25 (d, *J =* 29.2 Hz, 1H), 6.47 (d, *J =* 12.9 Hz, 1H), 5.59 - 5.34 (m, 2H), 4.98 - 4.86 (m, 1H), 4.32 - 4.21 (m, 1H), 3.39 (d, *J =* 7.2 Hz, 1H), 2.72 - 2.59 (m, 3H), 2.54 - 2.47 (m, 6H), 2.44 (dd, *J =* 8.5, 4.8 Hz, 1H), 1.97 (dd, *J =* 13.4, 5.3 Hz, 1H), 1.69 (d, *J =* 2.6 Hz, 3H), 1.62 (t, *J =* 5.2 Hz, 3H), 1.34 (s, 3H), 1.20 (dd, *J =* 7.0, 3.8 Hz, 3H), 1.04 (d, *J =* 5.0 Hz, 1H), 0.85 (s, 1H), LC/MS (ESI) m/z: 585 (M+H)⁺.

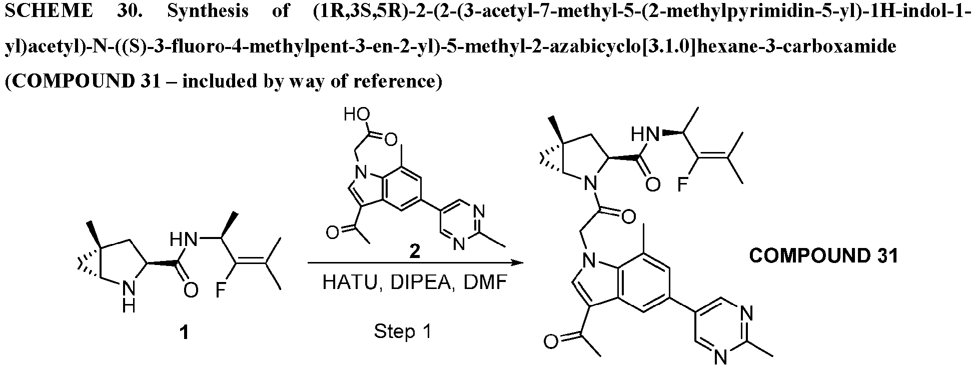

**Step 1:** To a mixture of intermediate **1** (31 mg, 0.13 mmol) and compound **2** (50 mg, 0.16 mmol) in DMF (4 mL) were added DIPEA (0.1 mL, 0.65 mmol) and HATU (98 mg, 0.26 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 31** (56 mg, crude) as a brown solid. The residue was purified by prep-HPLC to give **COMPOUND 31** (2.8 mg, yield 4.0%) as a white solid. ¹H NMR (400 MHz, CD₃OD) δ 8.96 (s, 2H), 8.45 (d, *J* = 1.5 Hz, 1H), 8.14 (s, 1H), 7.31 (s, 1H), 5.54 (q, *J =* 17.9 Hz, 2H), 4.97 - 4.87 (m, 1H), 4.27 (dd, *J =* 9.1, 5.7 Hz, 1H), 3.41 (dd, *J =* 5.5, 2.3 Hz, 1H), 2.72 (s, 3H), 2.69 (s, 3H), 2.52 (s, 3H), 2.45 (dd, *J =* 13.2, 9.1 Hz, 1H), 1.97 (dd, *J =* 13.1, 5.7 Hz, 1H), 1.69 (d, *J =* 2.8 Hz, 3H), 1.61 (d, *J =* 3.3 Hz, 3H), 1.34 (s, 3H), 1.21 (d, *J =* 7.0 Hz, 3H), 1.04 (t, *J =* 5.3 Hz, 1H), 0.86 (dd, *J* = 5.4, 2.3 Hz, 1H); LC/MS (ESI) m/z : 546 (M+H)⁺.

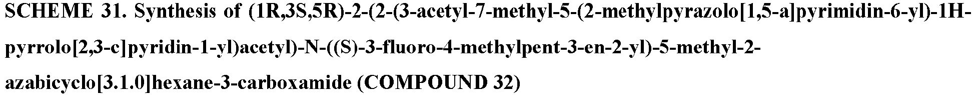

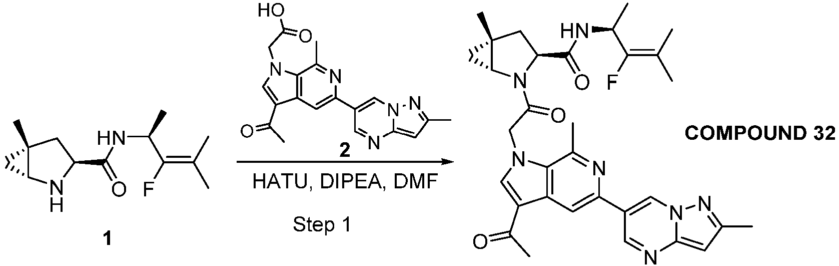

**Step 1:** To a mixture of intermediate 1 (20 mg, 0.08 mmol) and compound 2 (45 mg, 0.12 mmol) in DMF (5 mL) were added DIPEA (0.07 mL, 0.4 mmol) and HATU (63 mg, 0.16 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 96: 4) to give **COMPOUND 32** (36 mg, crude) as a brown solid. The residue was purified by prep-HPLC to give **COMPOUND 32** (5.9 mg, yield 12.3%) as a white solid. ¹H NMR (400 MHz, CD₃OD) δ 9.20 (d, *J =* 1.4 Hz, 1H), 9.03 (d, *J=* 2.0 Hz, 1H), 8.43 (s, 1H), 8.17 (s, 1H), 6.46 (s, 1H), 5.52 (d, *J =* 17.9 Hz, 1H), 5.41 (d, *J =* 17.9 Hz, 1H), 4.97 - 4.86 (m, 1H), 4.28 (dd, *J =* 9.1, 5.7 Hz, 1H), 3.38 (dd, *J =* 5.4, 2.3 Hz, 1H), 2.79 (d, *J =* 8.7 Hz, 3H), 2.49 (d, *J =* 12.0 Hz, 6H), 2.47 - 2.42 (m, 1H), 1.97 (dd, *J =* 13.2, 5.6 Hz, 1H), 1.69 (d, *J=* 2.8 Hz, 3H), 1.61 (d, *J=* 3.3 Hz, 3H), 1.34 (s, 3H), 1.19 (d, *J=* 7.0 Hz, 3H), 1.05 (t, *J =* 5.3 Hz, 1H), 0.87 (dd, *J =* 5.4, 2.3 Hz, 1H); LC/MS (ESI) m/z : 586 (M+H)⁺.

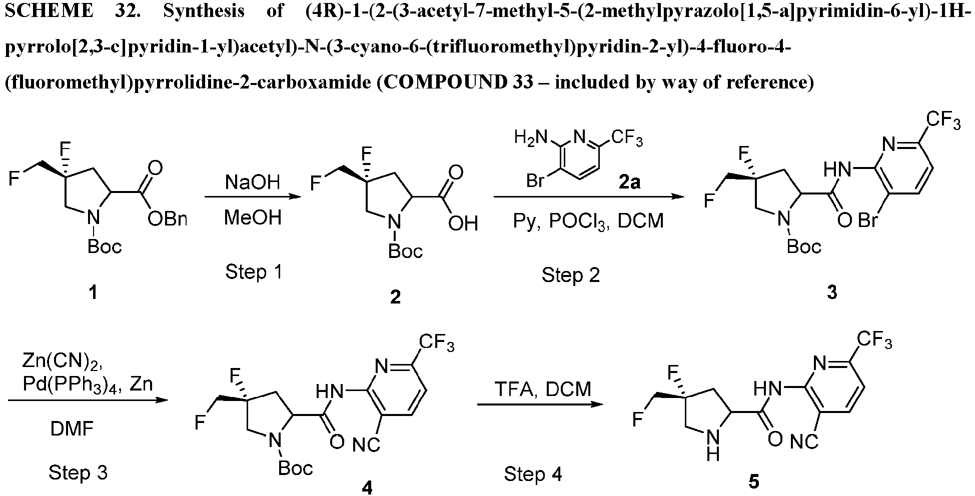

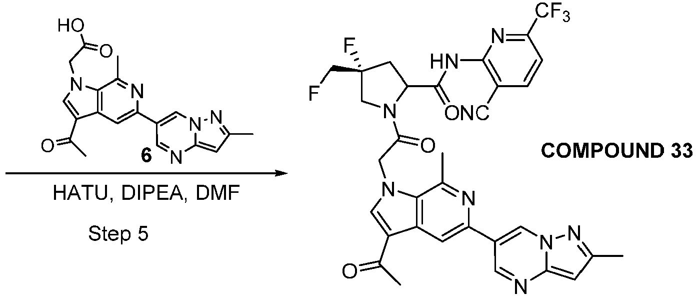

**Step 1:** To a solution of intermediate **1** (47 mg, 0.13 mmol) in MeOH (2 mL) was added NaOH solution (0.26 mL, 1M) at room temperature under N₂ atmosphere. The mixture was stirred at room temperature for 4 hours. The mixture was poured into iced-water and acidified with 2M HCI solution to pH= 3. The mixture was extracted with DCM twice. The combined organic layers were washed with brine, dried over anhydrous Na₂SO₄, filtered and concentrated to dryness to give intermediate **2** (35 mg, yield 57.5%) as a white solid. which was used directly in the next step without further purification. LC/MS (ESI) m/z : 266 (M+H)⁺.

**Step 2:** To a mixture of intermediate **2** (35 mg, 0.13 mmol) and compound **2a** (31 mg, 0.13 mmol) in DCM (3 mL) was added pyridine (51 mg, 0.65 mmol), followed by addition of POCl₃ (22 mg, 0.14 mmol) at 0 °C under N₂ atmosphere. The mixture was stirred at 25 °C for 2 hours. The mixture was poured into iced-water and extracted with DCM twice. The combined organic layer was washed with brine, dried over anhydrous Na₂SO₄, filtered and concentrated to dryness. The residue was purified by prep-TLC (PE : EtOAc = 5: 1) to give intermediate **3** (45 mg, yield 71.4%) as a white solid. LC/MS (ESI) m/z : 488 (M+H)⁺.

**Step 3:** To a solution of intermediate **3** (45 mg, 0.09 mmol) in DMF (2 mL) was added Zn(CN)₂ (32 mg, 0.27 mmol), followed by addition of Pd(PPh₃)₄ (23 mg, 0.02 mmol) and Zn (0.7 mg, 0.01 mmol) at room temperature under N₂ atmosphere. The mixture was stirred at 120 °C by microwave for 0.5 hour. The mixture was cooled to room temperature and poured into iced-water. The mixture was extracted with EtOAc twice. The combined organic layers were washed with brine, dried over anhydrous Na₂SO₄, filtered and concentrated to dryness. The residue was purified by prep-TLC (PE : EtOAc = 5: 1) to give intermediate **4** (36 mg, yield 89.9%) as a white solid. LC/MS (ESI) m/z : 435 (M+H)⁺.

**Step 4:** To a solution of intermediate **4** (36 mg, 0.08 mmol) in DCM (2 mL) was added TFA (1 mL) at 0 °C and the mixture was stirred at room temperature for 2 hours. The reaction was concentrated to dryness, washed with DCM and dried over anhydrous Na₂SO₄, filtered and concentrated under vacuum to give intermediate **5** (21 mg, yield 75.8%) as a white solid, which was used directly in the next step without further purification. LC/MS (ESI) m/z : 335 (M+H)⁺.

**Step 5:** To a mixture of intermediate **5** (21 mg, 0.06 mmol) and compound **6** (43 mg, 0.12 mmol) in DMF (3 mL) was added DIPEA (39 mg, 0.3 mmol), followed by addition of HATU (34 mg, 0.09 mmol) at 0 °C under N₂. The mixture was stirred at 25 °C for 2 hours. The mixture was diluted with EtOAc and washed with saturated NH₄Cl solution and brine, dried over anhydrous Na₂SO₄, filtered and concentrated to dryness. The residue was purified by prep-TLC (DCM : MeOH = 20: 1) to give **COMPOUND 33** (2.1 mg, yield 4.9%) as a white solid. ¹H NMR (400 MHz, CD₃OD) δ 9.25 (dd, *J =* 2.0, 2.0 Hz, 1H), 9.05 (d, *J =* 2.4 Hz, 1H), 8.49 (s, 1H), 8.41 (d, *J* = 8.0 Hz, 1H), 8.20 (s, 1H), 7.74 (d, *J* = 8.0 Hz, 1H), 6.49 (s, 1H), 5.55 - 5.43 (m, 2H), 4.99 - 4.90 (m, 3H), 4.78 - 4.57 (m, 1H), 4.34 - 4.09 (m, 3H), 2.85 (s, 3H), 2.82 - 2.71 (m, 1H), 2.50 (s, 3H), 2.49 (s, 3H), 2.48 - 2.37 (m, 1H).LC/MS (ESI) m/z : 680 (M+H)⁺.

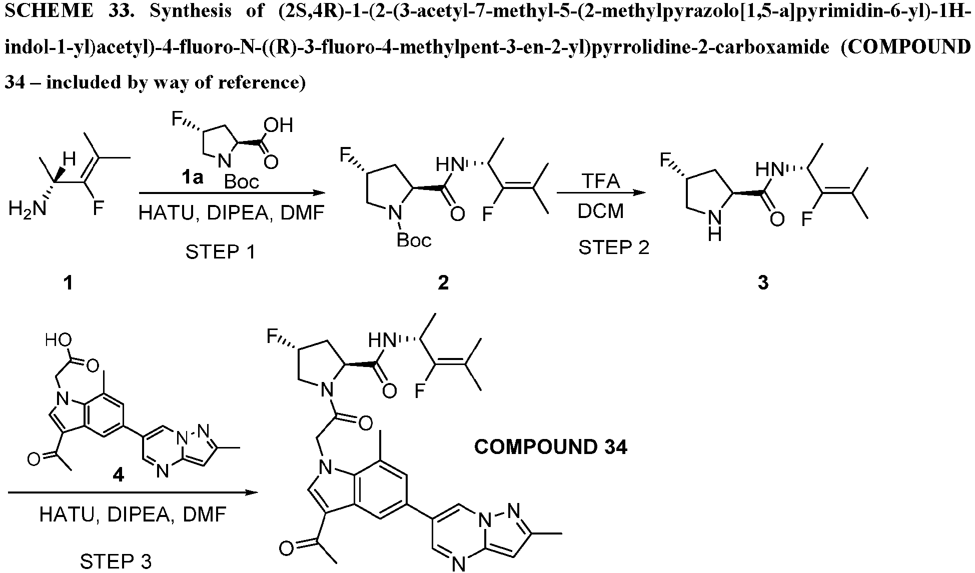

**Step 1:** To a mixture of intermediate **1** (60 mg, 0.51 mmol) and compound **1a** (137 mg, 0.62 mmol) in DMF (5 mL) were added DIPEA (0.42 mL, 2.55 mmol) and HATU (390 mg, 1.02 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 1: 1) to give intermediate **2** (142 mg, yield 83.5%) as yellow semi-solid. LC/MS (ESI) (m/z) : 333 (M+H)⁺.

**Step 2:** To a solution of intermediate **2** (142 mg, 0.43 mmol) in DCM (3 mL) was added TFA (1 mL) at 0 °C and the mixture was stirred at room temperature for 1 hour. The mixture was concentrated to give intermediate **3** (98 mg, yield 99%) as brown oil. The crude product was used directly in next step without further purification. LC/MS (ESI) (m/z): 233 (M+H)⁺.

**Step 3:** To a mixture of intermediate **3** (38 mg, 0.17 mmol) and compound **4** (50 mg, 0.14 mmol) in DMF (5 mL) were added DIPEA (0.11 mL, 0.7 mmol) and HATU (105 mg, 0.28 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 34** (65 mg, crude) as a brown solid. The residue was further purified by prep-HPLC to give **COMPOUND 34** (123 mg, yield 15.4%) as a white solid. ¹H NMR (400 MHz, CD₃OD) δ 8.94 (dd, *J* = 10.9, 1.8 Hz, 1H), 8.78 (dd, *J* = 7.4, 2.2 Hz, 1H), 8.43 (dd, *J* = 10.8, 1.6 Hz, 1H), 8.07 (s, 1H), 7.30 (d, *J =* 15.9 Hz, 1H), 6.50 (s, 1H), 5.52 - 5.41 (m, 1H), 5.40 - 5.25 (m, 2H), 4.95 (ddd, *J =* 21.2, 14.5, 7.7 Hz, 1H), 4.60 - 4.50 (m, 1H), 4.13 (dd, *J =* 20.5, 10.9 Hz, 1H), 3.92 (ddd, *J =* 36.1, 12.4, 2.9 Hz, 1H), 2.65 (s, 2H), 2.58 (s, 1H), 2.51 - 2.49 (m, 6H), 2.26 - 2.07 (m, 1H), 1.78 (d, *J =* 2.9 Hz, 1H), 1.68 (dd, *J* = 8.6, 3.0 Hz, 1H), 1.60 (dd, *J* = 7.7, 3.1 Hz, 3H), 1.50 (dd, *J =* 19.8, 3.3 Hz, 3H), 1.36 (d, *J =* 7.1 Hz, 1H), 1.29 - 1.21 (m, 3H); LC/MS (ESI) m/z : 577 (M+H)⁺.

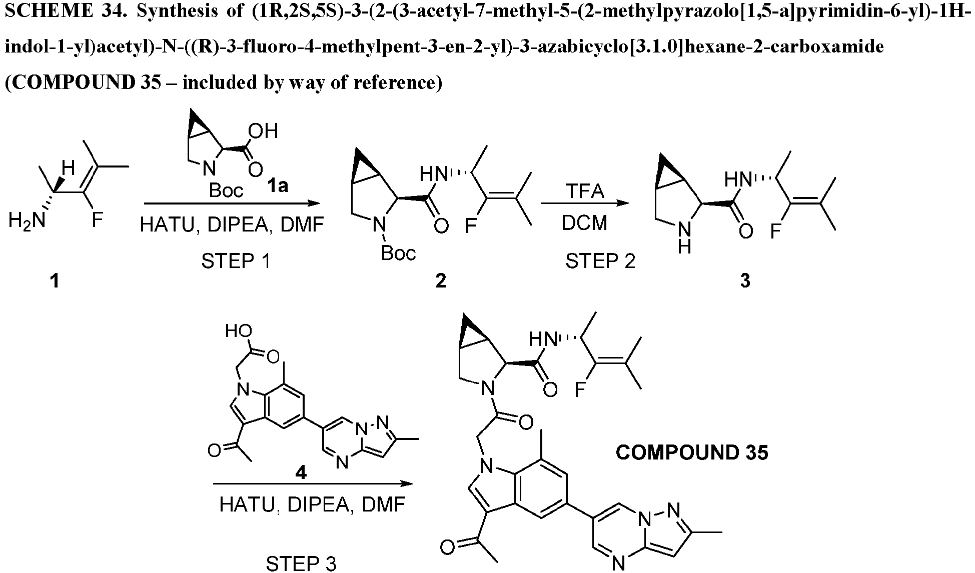

**Step 1:** To a mixture of intermediate **1** (50 mg, 0.43 mmol) and compound **1a** (126 mg, 0.56 mmol) in DMF (5 mL) were added DIPEA (0.35 mL, 2.15 mmol) and HATU (325 mg, 0.86 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 1: 1) to give intermediate **2** (135 mg, yield 97.1%) as yellow semi-solid. LC/MS (ESI) (m/z) : 327 (M+H)⁺.

**Step 2:** To a solution of intermediate **2** (135 mg, 0.41 mmol) in DCM (3 mL) was added TFA (1 mL) at 0 °C and the mixture was stirred at room temperature for 1 hour. The mixture was concentrated to give intermediate **3** (90 mg, yield 96.8%) as brown oil. The crude product was used directly in next step without further purification. LC/MS (ESI) (m/z) : 227 (M+H)⁺.

**Step 3:** To a mixture of intermediate **3** (47 mg, 0.21 mmol) and compound **4** (50 mg, 0.14 mmol) in DMF (5 mL) were added DIPEA (0.11 mL, 0.7 mmol) and HATU (105 mg, 0.28 mmol) at 0 °C under N₂ atmosphere and the mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 35** (85 mg, crude) as a brown solid. The residue was further purified by prep-HPLC to give **COMPOUND 35** (19.5 mg, yield 25.3%) as a white solid. ¹H NMR (400 MHz, DMSO-*d₆*) δ 9.20 (d, *J* = 4.4 Hz, 1H), 8.77 (d, *J* = 16.7 Hz, 1H), 8.36 (s, 1H), 8.20 (s, 1H), 8.03 (s, 1H), 7.38 (s, 1H), 6.55 (s, 1H), 5.33 (s, 2H), 4.78 (d, *J* = 29.1 Hz, 2H), 4.39 (s, 1H), 3.89 (s, 1H), 3.74 (d, *J* = 9.4 Hz, 1H), 2.61 (s, 3H), 2.46 (s, 3H), 1.98 - 1.67 (m, 3H), 1.66 - 1.39 (m, 6H), 1.28 (d, *J =* 6.9 Hz, 1H), 1.15 (d, *J* = 6.8 Hz, 2H), 0.69 (d, *J =* 12.4 Hz, 2H); LC/MS (ESI) m/z : 571 (M+H)⁺.

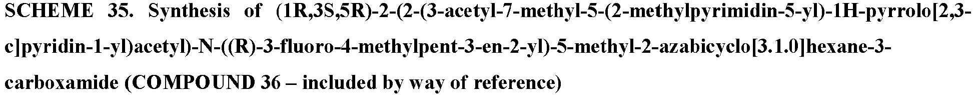

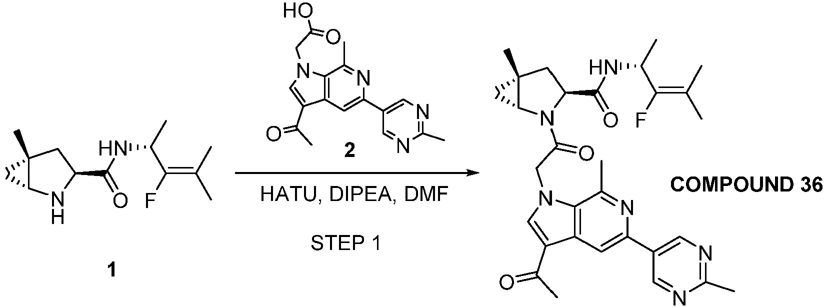

**Step 1:** To a mixture of intermediate **1** (21 mg, 0.087 mmol) and compound **2** (28 mg, 0.087 mmol) in DMF (3 mL) were added DIPEA (0.07 mL, 0.44 mmol) and HATU (66 mg, 0.17 mmol) at 0 °C under N₂ atmosphere. The mixture was stirred at room temperature for 1 hour. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 36** (35 mg, crude) as a brown solid. The residue was further purified by prep-HPLC to give **COMPOUND 36** (8.7 mg, yield 18.1%) as a white solid. ¹H NMR (400 MHz, DMSO-*d₆*) δ 9.27 (s, 2H), 8.50 (d, *J =* 8.3 Hz, 2H), 8.04 (d, *J* = 8.0 Hz, 1H), 5.74 (d, *J =* 17.9 Hz, 1H), 5.45 (d, *J =* 17.8 Hz, 1H), 4.90 - 4.72 (m, 1H), 4.24 (dd, *J =* 9.1, 4.8 Hz, 1H), 3.47 (dd, *J* = 5.4, 2.1 Hz, 1H), 2.80 (s, 3H), 2.68 (s, 3H), 2.49 (s, 3H), 2.37 (dd, *J* = 13.2, 9.1 Hz, 1H), 1.88 (dd, *J =* 13.2, 4.6 Hz, 1H), 1.61 (d, *J* = 2.8 Hz, 3H), 1.51 (d, *J* = 3.1 Hz, 3H), 1.28 (s, 3H), 1.15 (d, *J* = 7.0 Hz, 3H), 1.01 (t, *J =* 5.2 Hz, 1H), 0.83 (dd, *J =* 5.0, 2.2 Hz, 1H); LC/MS (ESI) m/z : 547 (M+H)⁺.

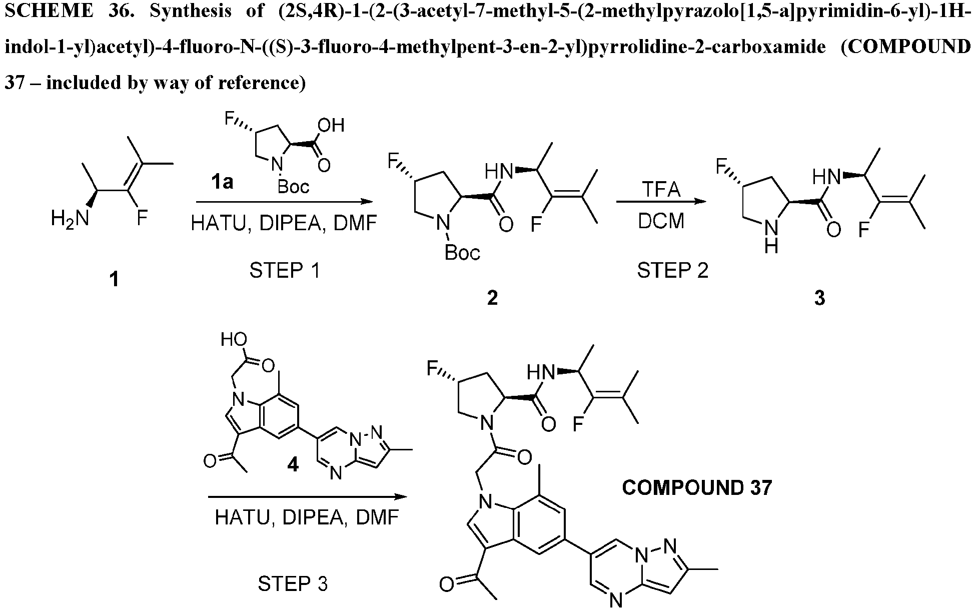

**Step 1:** To a mixture of intermediate **1** (50 mg, 0.43 mmol) and compound **1a** (124 mg, 0.56 mmol) in DMF (5 mL) were added DIPEA (0.35 mL, 2.15 mmol) and HATU (325 mg, 0.86 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 1: 1) to give intermediate **2** (123 mg, yield 86.6%) as yellow semi-solid. LC/MS (ESI) (m/z) : 333 (M+H)⁺.

**Step 2:** To a solution of intermediate **2** (123 mg, 0.37 mmol) in DCM (3 mL) was added TFA (1 mL) at 0 °C and the mixture was stirred at room temperature for 1 hour. The mixture was concentrated to give intermediate **3** (86 mg, yield 100%) as brown oil. The crude product was used directly in next step without further purification. LC/MS (ESI) (m/z) : 233 (M+H)⁺.

**Step 3:** To a mixture of intermediate **3** (48 mg, 0.21 mmol) and compound **4** (50 mg, 0.14 mmol) in DMF (5 mL) were added DIPEA (0.11 mL, 0.7 mmol) and HATU (105 mg, 0.28 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give crude **COMPOUND 37** (33 mg) as a brown solid. The residue was purified by prep-HPLC to give **COMPOUND 37** (16.6 mg, yield 21%) as a white solid. ¹H NMR (400 MHz, DMSO-*d₆*) δ 9.21 (d, *J* = 1.4 Hz, 1H), 8.81 (d, *J* = 2.2 Hz, 1H), 8.40 - 8.37 (m, 1H), 8.33 - 8.24 (m, 2H), 7.40 (s, 1H), 6.56 (s, 1H), 5.46 (dt, *J =* 30.2, 12.7 Hz, 3H), 4.83 - 4.68 (m, 1H), 4.45 (t, *J* = 8.4 Hz, 1H), 4.09 (dd, *J =* 21.5, 12.4 Hz, 1H), 3.83 (dd, *J =* 380, 9.8 Hz, 1H), 2.63 (s, 3H), 2.46 (s, 6H), 1.69 (d, *J* = 2.8 Hz, 1H), 1.61 (d, *J =* 2.8 Hz, 3H), 1.56 (d, *J* = 3.1 Hz, 3H), 1.31 (d, *J =* 7.0 Hz, 1H), 1.12 (d, *J* = 7.0 Hz, 3H); LC/MS (ESI) m/z : 577 (M+H)⁺.

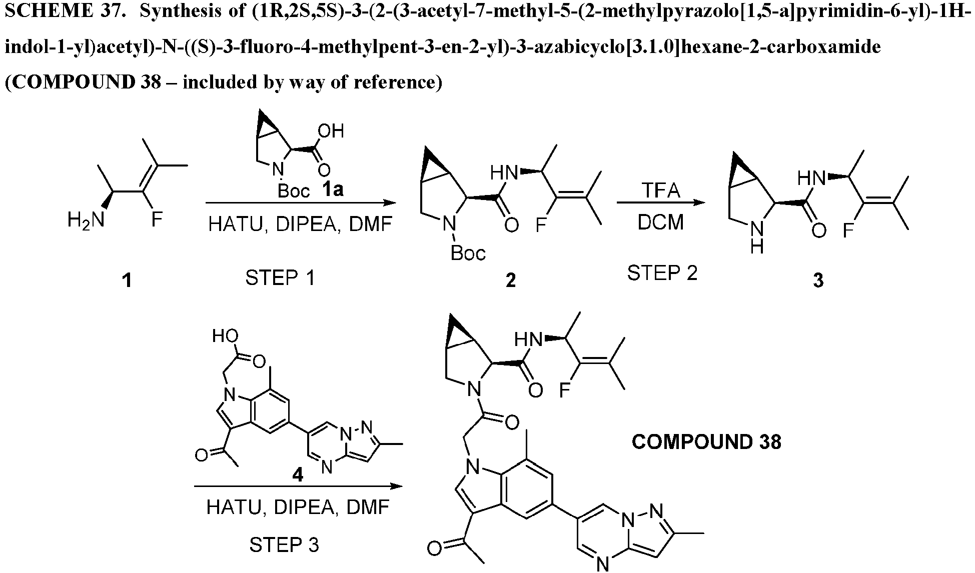

**Step** 1: To a mixture of intermediate **1** (50 mg, 0.43 mmol) and compound **1a** (126 mg, 0.56 mmol) in DMF (5 mL) were added DIPEA (0.35 mL, 2.15 mmol) and HATU (325 mg, 0.86 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (PE : EtOAc = 1: 1) to give intermediate **2** (128 mg, yield 91.4%) as yellow semi-solid. LC/MS (ESI) (m/z) : 327 (M+H)⁺.

**Step 2:** To a solution of intermediate **2** (128 mg, 0.39 mmol) in DCM (3 mL) was added TFA (1 mL) at 0 °C and the mixture was stirred at room temperature for 1 hour. The mixture was concentrated to give intermediate **3** (88 mg, yield 99%) as brown oil. The crude product was used directly in next step without further purification. LC/MS (ESI) (m/z) : 227 (M+H)⁺.

**Step 3:** To a mixture of intermediate **3** (47 mg, 0.21 mmol) and compound **4** (50 mg, 0.14 mmol) in DMF (5 mL) were added DIPEA (0.11 mL, 0.7 mmol) and HATU (105 mg, 0.28 mmol) at 0 °C under N₂ atmosphere. The mixture was stirred at room temperature for 2 hours. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 38** (97 mg, crude) as a brown solid. The residue was further purified by prep-HPLC to give **COMPOUND 38** (22.1 mg, yield 28%) as a white solid. ¹H NMR (400 MHz, DMSO-*d₆*) δ 9.20 (d, *J =* 1.5 Hz, 1H), 8.80 (d, *J =* 2.2 Hz, 1H), 8.37 (d, *J =* 1.6 Hz, 1H), 8.20 (s, 1H), 8.10 (d, *J =* 8.1 Hz, 1H), 7.39 (s, 1H), 6.56 (s, 1H), 5.33 (d, *J =* 26.9 Hz, 2H), 4.79 (ddd, *J =* 29.2, 16.4, 9.0 Hz, 1H), 4.38 (d, *J* = 5.3 Hz, 1H), 3.86 (dd, *J =* 9.8, 5.0 Hz, 1H), 3.75 (d, *J =* 9.8 Hz, 1H), 2.63 (s, 3H), 2.45 (s, 4H), 1.83 (ddd, *J* = 17.4, 8.3, 4.4 Hz, 2H), 1.70 (d, *J=* 2.7 Hz, 1H), 1.62 (d, *J=* 2.7 Hz, 3H), 1.57 (d, *J* = 3.1 Hz, 3H), 1.29 (d, *J* = 7.0 Hz, 1H), 1.11 (d, *J* = 7.0 Hz, 3H), 0.75 (dd, *J* = 8.7, 4.4 Hz, 1H), 0.64 (d, *J =* 4.7 Hz, 1H); LC/MS (ESI) m/z : 571 (M+H)⁺.

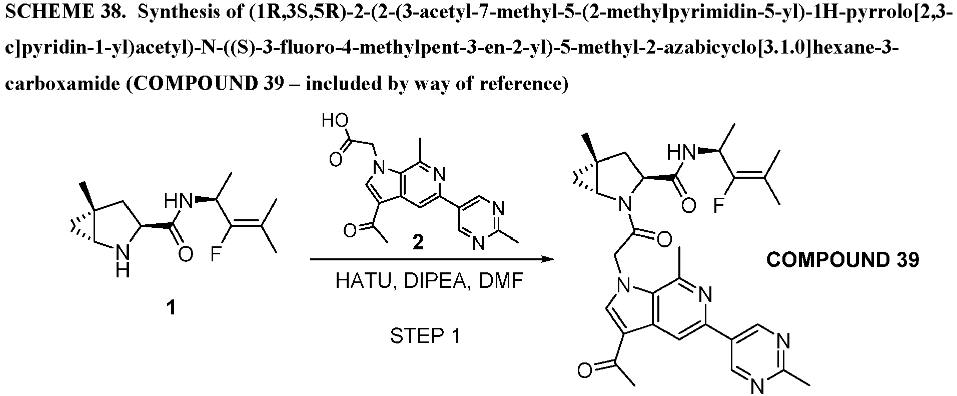

**Step 1:** To a mixture of intermediate **1** (30 mg, 0.12 mmol) and compound **2** (40 mg, 0.12 mmol) in DMF (3 mL) were added DIPEA (0.1 mL, 0.6 mmol) and HATU (95 mg, 0.24 mmol) at 0 °C under N₂ atmosphere. The mixture was stirred at room temperature for 1 hour. The mixture was diluted with EtOAc and washed with saturated NH₄Cl solution and brine, dried over anhydrous Na₂SO₄, filtered and concentrated to dryness. The residue was purified by chromatography on silica gel (DCM : MeOH = 97: 3) to give **COMPOUND 39** (65 mg, crude) as a brown solid. The residue was further purified by prep-HPLC to give **COMPOUND 39** (17.6 mg, yield 25.9%) as a white solid. ¹H NMR (400 MHz, CD₃OD) δ 9.24 (s, 2H), 8.52 (s, 1H), 8.26 (s, 1H), 5.55 (dd, *J=* 36.7, 17.9 Hz, 2H), 4.96 - 4.87 (m, 1H), 4.28 (dd, *J* = 9.1, 5.7 Hz, 1H), 3.42 (dd, *J* = 5.5, 2.3 Hz, 1H), 2.84 (s, 3H), 2.73 (s, 3H), 2.52 (d, *J* = 4.4 Hz, 3H), 2.46 (dd, *J* = 13.3, 9.1 Hz, 1H), 1.98 (dd, *J =* 12.9, 5.3 Hz, 1H), 1.69 (d, *J =* 2.9 Hz, 3H), 1.61 (d, *J =* 3.3 Hz, 3H), 1.35 (s, 3H), 1.20 (d, *J* = 7.0 Hz, 3H), 1.06 (t, *J* = 5.2 Hz, 1H), 0.88 (dd, *J* = 5.5, 2.4 Hz, 1H); LC/MS (ESI) m/z : 547 (M+H)⁺.

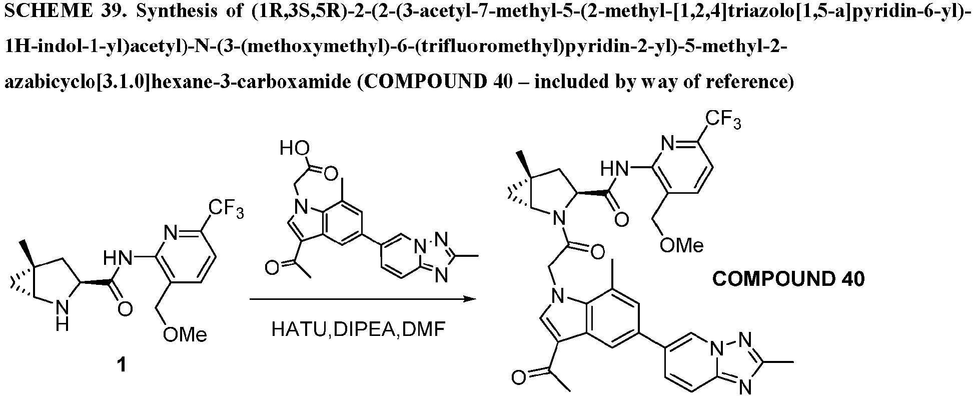

**Step 1:** To a mixture of intermediate **1** (100 mg, 0.30 mmol) and 2-(3-acetyl-7-methyl-5-(2-methyl-[1,2,4]triazolo[1,5-a]pyridin-6-yl)-1H-indol-1-yl)acetic acid (108 mg, 0.30 mmol) in DMF (5 mL) was added DIPEA (194 mg, 1.50 mmol) followed by HATU (171 mg, 0.45 mmol) at 0 °C. The mixture was stirred at room temperature for 2 hours before diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND 40** (27.0 mg, yield 13.2%) as a white solid. ¹H NMR (400 MHz, CDCl₃) δ 8.96 (s, 1H), 8.69 (t, J = 1.2 Hz, 1H), 8.52 (d, J = 2.0 Hz, 1H), 7.84 - 7.74 (m, 2H), 7.72 (s, 1H), 7.66 (d, J = 9.2 Hz, 1H), 7.42 (d, J = 8.0 Hz, 1H), 7.18 (s, 1H), 5.44 (d, J = 17.6 Hz, 1H), 5.32 (d, J = 17.6 Hz, 1H), 5.12 - 4.93 (m, 1H), 4.47 (d, J = 12.8 Hz, 1H), 4.32 (d, J = 12.8 Hz, 1H), 3.31 (s, 3H), 3.24 - 3.18 (m, 1H), 2.71 (s, 3H), 2.62 (s, 3H), 2.60 - 2.39 (m, 5H), 1.41 (s, 3H), 1.12 (t, J = 5.4 Hz, 1H), 0.88 (dd, J = 5.2, 5.2 Hz, 1H); LC/MS (ESI) m/z: 674 (M+H)⁺.

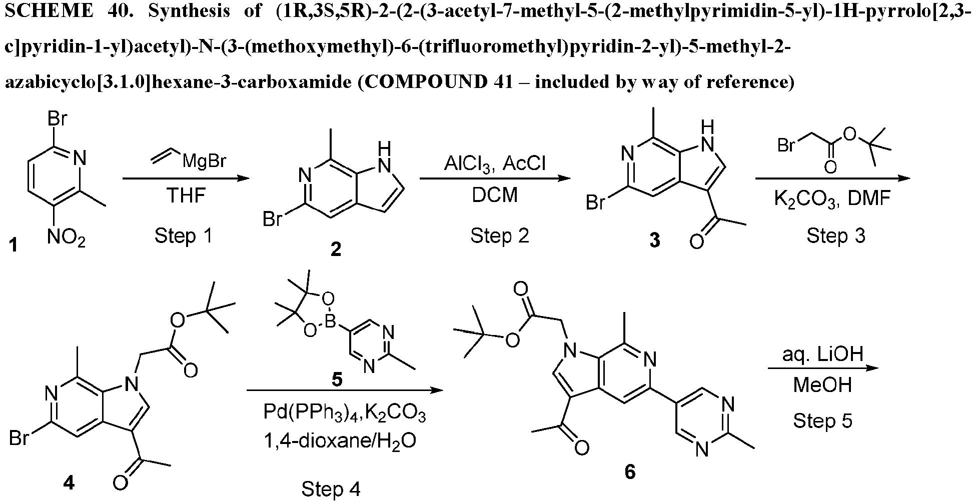

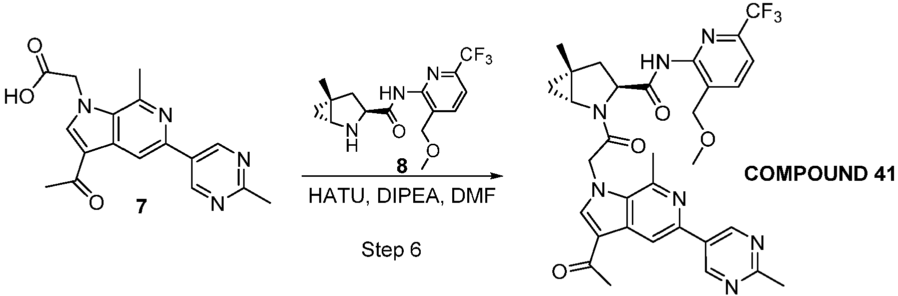

**Step 1:** To a solution of intermediate **1** (3.00 g, 13.8 mmol) in THF (120 mL) at -78 °C was added vinylmagnesium bromide (70 mL, 69.1 mmol) dropwise. The mixture was stirred at -40 °C for 2 hours. The mixture was poured into ice water and extracted with EtOAc. The organic layer was washed with brine, dried over anhydrous Na₂SO₄, filtered and concentrated and the residue was purified by chromatography on silica gel (DCM: MeOH = 98:2) to give intermediate **2** (1.60 g, yield 55.2 %) as brown semi-solid. LC/MS (ESI) (m/z): 211 /213(M+H)⁺.

**Step 2:** To a solution of intermediate **2** (500 mg, 2.40 mmol) in DCM (10mL) was added AlCl₃ at 0 °C under N₂ atmosphere and the mixture was stirred at 0 °C for 0.5 hour. Then AcCl was slowly added at 0 °C under N₂ atmosphere. The mixture was stirred at 25 °C for 1 hour and quenched with MeOH. The mixture was concentrated to dryness under reduced pressure to give crude product, which was purified by flash chromatography (DCM: MeOH = 97: 3) to give intermediate **3** (360 mg, yield 60.0%) as yellow solid. LC/MS (ESI) (m/z): 253/255 (M+H)⁺.

**Step 3:** To a solution of intermediate **3** (360 mg, 1.42 mmol) in DMF (5 mL) were added K₂CO₃ (590 mg, 4.27 mmol) and tert-butyl 2-bromoacetate (416 mg, 0.35 mL, 2.13 mmol) and the mixture was stirred at 50 °C for 24 hours. The mixture was diluted with EtOAc and washed with saturated aq.NH₄Cl solution and brine, dried over anhydrous Na₂SO₄, filtered and concentrated to dryness under reduced pressure. The residue was purified by chromatography on silica gel (PE: EtOAc = 3: 2) to give intermediate **4** (300 mg, yield 57.1%) as a white solid. LC/MS (ESI) (m/z): 367/369 (M+H)⁺.

**Step 4:** To a mixture of intermediate **4** (300 mg, 0.820 mmol) and compound **5** (234 mg, 1.06 mmol) in 1,4-dioxane (4 mL) and water (0.5 mL) was added K₂CO₃ (280 mg, 2.05 mmol), Pd(PPh₃)₄ (94.0 mg, 0.082 mmol) under N₂ atmosphere. The mixture was stirred at 95 °C for 2 hours. The mixture was diluted with EtOAc and washed with water and brine, dried over anhydrous Na₂SO₄ and concentrated to dryness under reduced pressure to give crude product, which was purified by flash chromatography (DCM: MeOH = 95: 5) to give intermediate **6** (280 mg, yield 90.3%) as a white solid. LC/MS (ESI) (m/z): 381 (M+H)⁺.

**Step 5:** To a solution of intermediate **6** (280 mg, 0.740 mmol) in MeOH (5 mL) and water (1 mL) was added a solution of LiOH·H₂O (150 mg, 3.68 mmol) at 0 °C and the mixture was stirred at room temperature for 1 hour. The mixture was concentrated to 1/5 volume, diluted with water and washed with MTBE twice. The aqueous layer was acidified with 1N aq. HCl to pH = 3 and extracted with DCM twice. The combined organic layers were washed with brine, dried over anhydrous Na₂SO₄, and concentrated under reduced pressure to dryness to give intermediate **7** (230 mg, yield 99.8%) as a white solid. LC/MS (ESI) (m/z): 325 (M+H)⁺.

**Step 6:** To mixture of intermediate **7** (70.0 mg, 0.220 mmol) and compound **8** (107 mg, 0.320 mmol) in DMF (5 mL) were added DIPEA (0.18 mL, 1.10 mmol) and HATU (164 mg, 0.440 mmol) at 0 °C and the mixture was stirred at room temperature for 2 hours under N₂ atmosphere. The mixture was diluted with EtOAc and washed with saturated aq.NH₄Cl solution and brine, dried over anhydrous Na₂SO₄, filtered and concentrated to dryness under reduced pressure. The residue was purified by chromatography on silica gel (DCM: MeOH = 96: 4) to give crude **COMPOUND 41,** which was further purified by prep-HPLC to give **COMPOUND 41** (29.1 mg, yield 21.2%) as a white solid. ¹H NMR (400 MHz, CD₃OD) δ 9.25 (s, 2H), 8.56 (s, 1H), 8.31 (s, 1H), 8.08 (d, *J* = 7.9 Hz, 1H), 7.67 (d, *J* = 8.0 Hz, 1H), 5.73 (d, *J* = 18.0 Hz, 1H), 5.56 (d, *J* = 17.9 Hz, 1H), 4.54 (s, 1H), 4.41 - 4.27 (m, 2H), 3.53 (dd, *J =* 5.6, 2.4 Hz, 1H), 3.12 (s, 3H), 2.90 (s, 3H), 2.74 (s, 3H), 2.65 (dd, *J* = 13.4, 9.2 Hz, 1H), 2.53 (s, 3H), 2.26 (ddd, *J =* 13.4, 5.9, 1.3 Hz, 1H), 1.41 (s, 3H), 1.13 - 1.07 (m, 1H), 0.98 (dd, *J* = 5.6, 2.5 Hz, 1H); LC/MS (ESI) m/z: 636(M+H)⁺.

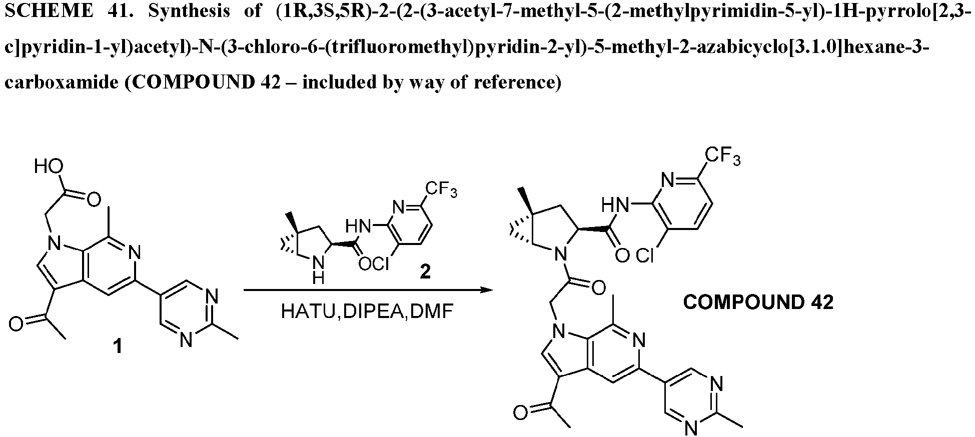

**Step 1:** To a mixture of intermediate **1** (50.0 mg, 0.150 mmol) and compound 2 (49.0 mg, 0.150 mmol) in DMF (3 mL) was added DIPEA (323 mg, 2.50 mmol), followed by addition of HATU (86.0 mg, 0.230 mmol) at 0 °C under N₂. The mixture was stirred at room temperature for 2 hours before diluted with EtOAc and washed with saturated NH₄Cl solution and brine, dried over anhydrous Na₂SO₄, filtered and concentrated to dryness under reduced pressure. The residue was purified by prep-HPLC to give **COMPOUND 42** (13.0 mg, yield 13.8%) as a white solid. ¹H NMR (400 MHz, CD₃OD) δ 9.26 (s, 2H), 8.56 (s, 1H), 8.30 (s, 1H), 8.11 (d, *J =* 8.0 Hz, 1H), 7.65 (d, *J =* 8.0 Hz, 1H), 5.71 (d, *J* = 18.0 Hz, 1H), 5.59 (d, *J =* 18.0 Hz, 1H), 4.74 (dd, *J =* 9.3, 4.8 Hz, 1H), 3.53 - 3.49 (m, 1H), 2.88 (s, 3H), 2.74 (s, 3H), 2.64 (dd, *J* = 9.2, 9.2 Hz, 1H), 2.52 (s, 3H), 2.37 - 2.30 (m, 1H), 1.41 (s, 3H), 1.15 (t, *J* = 5.4 Hz, 1H), 0.99 (dd, *J* = 5.6, 5.6 Hz, 1H); LC/MS (ESI) m/z: 626 (M+H)⁺.

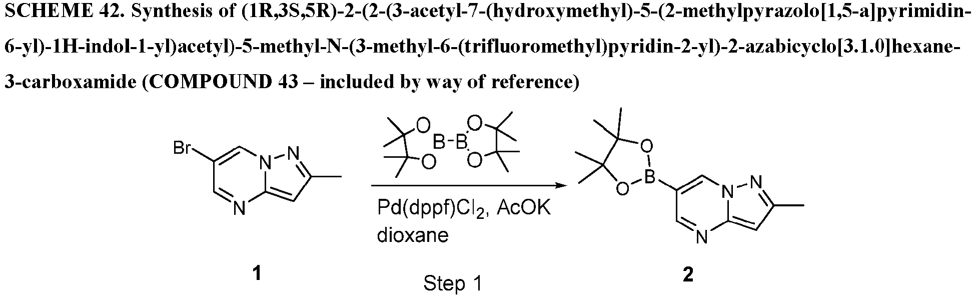

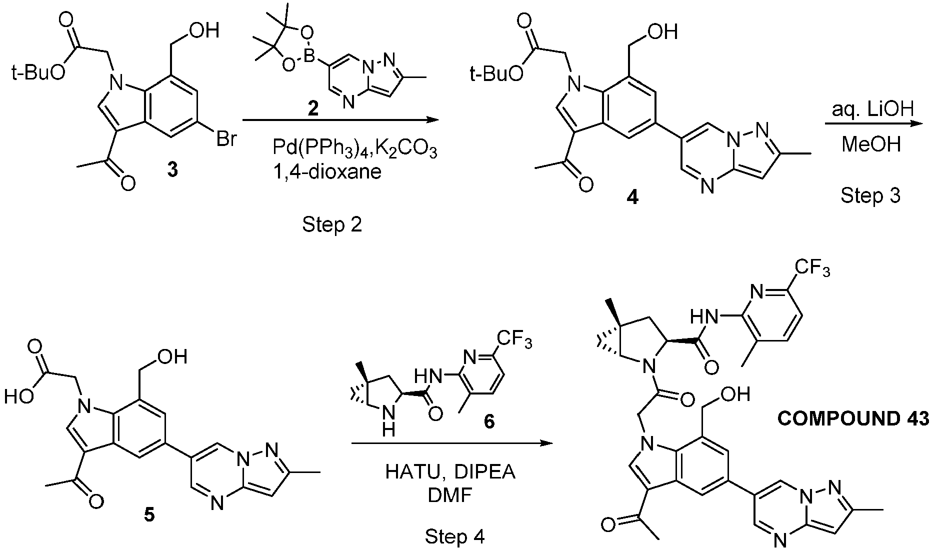

**Step 1:** To a mixture of intermediate **1** (0.297 g, 1.4 mmol) and 4,4,4',4',5,5,5',5'-octamethyl-2,2'-bi(1,3,2-dioxaborolane) (429 mg, 1.69 mmol) in 1,4-dioxane (4 mL) was added AcOK (414 mg, 6.16 mmol), Pd(dppf)Cl₂ (52 mg, 0.07 mmol) under N₂ atmosphere. The mixture was degassed under N₂ atmosphere for three times. The reaction was stirred at 85 °C under N₂ atmosphere for 2 hours before diluted with EtOAc and washed with water and brine, dried over Na₂SO₄ and concentrated to dryness under reduced pressure to give crude product, which was purified by flash chromatography on silica gel (PE: EtOAc = 10: 1) to give compound **2** (0.235 g, yield 65%) as a white solid. LC/MS (ESI) (m/z): 260 (M+H)⁺.

**Step 2:** To a mixture of intermediate **3** (0.1 g, 0.26 mmol) and compound **2** (82 mg, 0.31 mmol) in 1,4-dioxane (4 mL) and water (0.5 mL) was added K₂CO₃ (90 mg, 0.65 mmol), Pd(PPh₃)₄ (30 mg, 0.026 mmol) under N₂ atmosphere. The mixture was degassed under N₂ atmosphere for three times. The reaction was stirred at 90 °C under N₂ atmosphere for 2 hours before diluted with EtOAc and washed with water and brine, dried over Na₂SO₄ and concentrated to dryness under reduced pressure to give crude product, which was purified by flash chromatography on silica gel (PE: EtOAc = 9: 1) to give intermediate **4** (70 mg, yield 61.4%) as a white solid. LC/MS (ESI) (m/z): 435 (M+H)⁺.

**Step 3:** To a solution of intermediate **4** (70 mg, 0.16 mmol) in MeOH (5 mL) and water (1 mL) was added a solution of lithium hydroxide monohydrate (34 mg, 0.8 mmol) at 0 °C. The mixture was stirred at room temperature for 1 hour before concentrated to one-fifth volume, diluted with water and washed with MTBE twice. The aqueous layer was acidified with 1N aq.HCl to pH~3 and extracted with DCM twice. The combined organic layers were washed with brine, dried over anhydrous Na₂SO₄, and concentrated to dryness under reduced pressure to give intermediate **5** (40 mg, yield 66.7%) as a white solid. LC/MS (ESI) (m/z): 379 (M+H)⁺.

**Step 4:** To a mixture of intermediate **5** (40 mg, 0.12 mmol) and compound **6** (45 mg, 0.15 mmol) in DMF (3 mL) was added DIPEA (0.08 mL, 0.6 mmol) followed by HATU (90 mg, 0.24 mmol) at 0 °C. The mixture was stirred at room temperature for 2 hour before extracted with ethyl acetate twice, dried over anhydrous Na₂SO₄, filtered and concentrated to dryness under reduced pressure. The residue was purified by chromatography on silica gel (PE: EtOAc = 1: 2) and further purified by prep-HPLC to give **COMPOUND 43** (2.7 mg, yield 3.5%) as a white solid. ¹H-NMR (400 MHz, CD₃OD) δ 9.00 (d, *J* = 2.2 Hz, 1H), 8.82 (d, *J* = 2.2 Hz, 1H), 8.61 (d, *J =* 1.9 Hz, 1H), 8.20 (s, 1H), 7.85 (d, *J* = 7.8 Hz, 1H), 7.58 (d, *J* = 7.8 Hz, 1H), 7.49 (d, *J* = 2.0 Hz, 1H), 6.52 (s, 1H), 5.88 (d, *J* = 17.8 Hz, 1H), 5.71 (d, *J* = 17.8 Hz, 1H), 4.93 (d, *J =* 12.7 Hz, 1H), 4.79 (d, *J =* 12.6 Hz, 1H), 4.59 (d, *J =* 9.3 Hz, 1H), 3.54 - 3.47 (m, 1H), 2.64 (dd, *J* = 13.5, 9.3 Hz, 1H), 2.52 (d, *J =* 10.1 Hz, 6H), 2.35 - 2.27 (m, 1H), 2.24 (s, 3H), 1.41 (s, 3H), 1.28 (s, 1H), 1.13 (t, *J =* 5.5 Hz, 1H), 1.03 (dd, *J =* 5.5, 2.4 Hz, 1H). LC/MS (ESI) m/z: 660 (M+H)⁺.

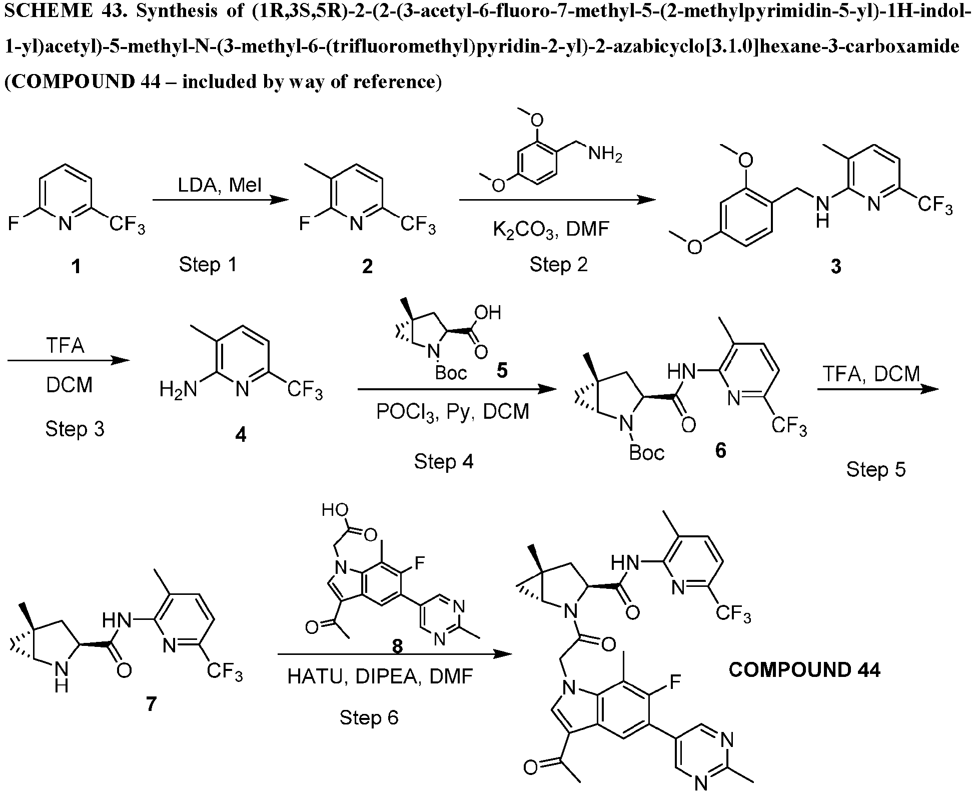

**Step 1:** To a solution of intermediate **1** (10.0 g, 66.6 mmol) in THF (100 mL) was added LDA (33.3 mL, 66.7 mmol, 2.0 M in THF) at -65 °C under N₂ over 30 minutes. After the reaction was stirred at -65 °C for 20 minutes, MeI (4.15 mL, 66.7 mmol) was added. The reaction was warmed to 25 °C within 1 hour and quenched with water (100 mL) at 0 °C. The mixture was extracted with MTBE (20 mL x 2) and the organic layers were combined, dried over Na₂SO₄, filtered. The reaction was purified by distillation under reduced pressure to give intermediate **2** (3.46 g, yield 31.8%) as colorless oil.

**Step 2:** To a solution of intermediate **2** (1.00 g, 5.58 mmol) in DMF (10 mL) was added K₂CO₃ (1.54 g, 11.2 mmol) and (2,4-dimethoxyphenyl)methanamine (1.26 mL, 8.37 mmol) at 25 °C under N₂. The reaction was heated to 110 °C for 2 hours before cooled to 25 °C and water (50 mL) was added to the mixture. The mixture was extracted with EtOAc (20 mL x 3) and the combined organic layers were washed with brine, dried over Na₂SO₄, filtered and concentrated under vacuum. The residue was purified by column chromatography on silica gel (PE: EtOAc = 20: 1) to give intermediate **3** (500 mg, 27.5% yield) as a white solid. LC/MS (ESI) m/z: 327 (M+H)⁺.

**Step 3:** To a solution of intermediate **3** (500 mg, 1.53 mmol) in DCM (5 mL) was added TFA (2.5 mL) at 0 °C under N₂. The reaction was stirred at 25 °C for 18 hours. The mixture was concentrated under reduced pressure to give crude product (700 mg). The residue was neutralized with saturated aq.NaHCO₃ solution and extracted with DCM twice. The combined organic layers were washed with brine, dried and concentrated to dryness to give intermediate **4** (200 mg, yield 74.2%) as a white solid. LC/MS (ESI) m/z: 177 (M+H)⁺.

**Step 4:** To a mixture of intermediate **4** (200 mg, 1.14 mmol), compound **5** (274 mg, 1.14 mmol) and pyridine (0.37 mL, 4.56 mmol) in DCM (2 mL) was added POCl₃ (0.12 mL, 1.25 mmol) drop-wisely at 0 °C under N₂. The reaction was stirred at 25 °C for 2 hours before quenched with ice-water (10 mL). The reaction mixture was extracted with DCM (10 mL x 2) and the combined organic layers were washed with 1N aq.HCl and brine, dried over Na₂SO₄, filtered and concentrated to dryness. The residue was purified by column chromatography on silica gel (PE: EtOAc = 20: 1 to 5: 1) to give intermediate **6** (120 mg, yield 26.3%) as a white solid. LC/MS (ESI) m/z: 400 (M+H)⁺.

**Step 5:** To a solution of intermediate **6** (120 mg, 0.30 mmol) in DCM (2 mL) was added TFA (0.70 mL) at 0 °C under N₂. The reaction was stirred at 25 °C for 1.5 hours and concentrated under reduced pressure to give intermediate **7** (190 mg, crude, TFA salt), which was directly used in the next step. LC/MS (ESI) m/z: 300 (M+H)⁺.

**Step 6:** To a solution of intermediate 7 (85.0 mg TFA salt, about 0.15 mmol), compound **8** (51.2 mg, 0.15 mmol) and DIPEA (0.24 mL, 1.50 mmol) in DMF (2 mL) was added HATU (171 mg, 0.45 mmol) at 0 °C under N₂. The reaction was stirred at 25 °C for 2 hours before water (10 mL) was added to the reaction. The reaction mixture was extracted with EtOAc (10 mL x 3) and the organic layers were washed with brine, dried over Na₂SO₄, filtered and concentrated to dryness. The residue was purified by column chromatography on silica gel (DCM: MeOH = 20: 1) and further purified by prep. HPLC to give **COMPOUND 44** (19.6 mg, yield 20.7%) as a white solid. ¹H-NMR (400 MHz, CDCl₃) δ 8.84 (s, 2H), 8.36 (d, *J =* 7.6 Hz, 1H), 7.73-7.60 (m, 2H), 7.41 (d, J = 7.7 Hz, 1H), 5.43 (d, *J =* 17.7 Hz, 1H), 5.31 (d, *J =* 17.8 Hz, 1H), 4.95 (s, 1H), 3.15 (s, 1H), 2.80 (s, 3H), 2.65-2.57 (m, 4H), 2.50 (s, 3H), 2.46-2.40 (m, 1H), 2.23 (s, 3H), 1.42 (s, 3H), 1.24 (s, 1H), 1.16 (t, *J =* 5.5 Hz, 1H), 0.88 (dd, *J =* 5.3, 2.1 Hz, 1H); LC/MS (ESI) m/z: 623 (M+H)⁺.

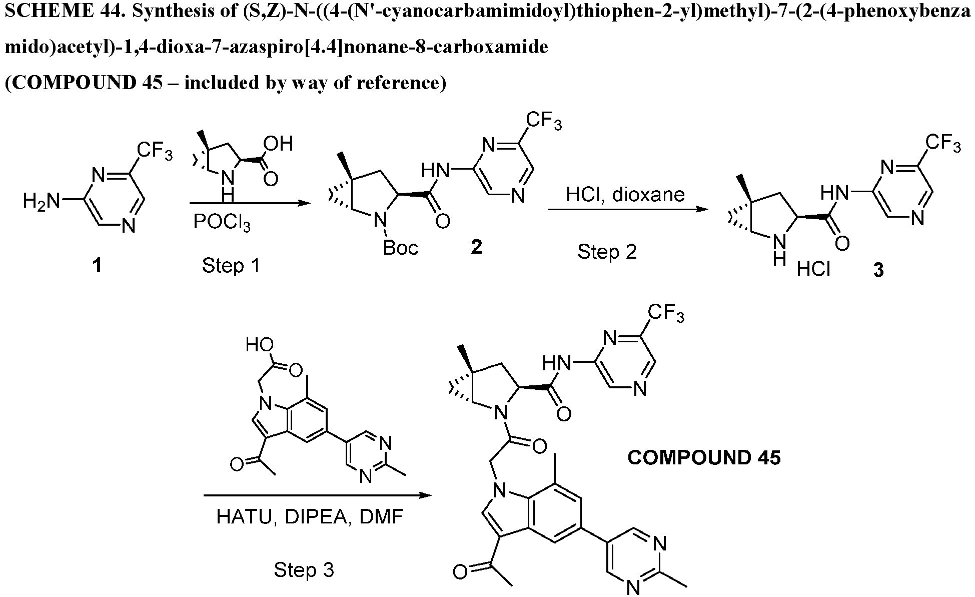

**Step 1:** To a solution of intermediate **1** (50 mg, 0.306 mmol) in DCM (5 mL) was added 4-(trifluoromethyl)pyrimidin-2-amine (73 mg, 0.306 mmol), pyridine (96.81 mg, 1.224 mmol) followed by POCl₃ (51 mg, 0.336 mmol) at 0 °C. The mixture was stirred at room temperature for 2 hours before diluted with DCM, dried over anhydrous Na₂SO₄, filtered and concentrated under reduced pressure to dryness. The residue was purified by chromatography on silica gel (PE: EtOAc = 10: 1) to give intermediate **2** (35 mg, yield 82.0%) as colorless oil. LC/MS (ESI) (m/z): 387 (M+H)⁺.

**Step 2:** A solution of intermediate **2** (35 mg, 0.16 mmol) in HCl/1,4-dioxane (1 mL, 4M) was stirred at 0 °C and then warmed to 25 °C within 2 hours. The mixture was concentrated to dryness under reduced pressure and the residue was washed with diethyl ether and dried under vacuum to give intermediate **3** (40 mg, yield 93%) as a brown solid, which was directly used in the next step without further purification. LC/MS (ESI) (m/z): 287 (M+H)⁺.

**Step 3:** To a mixture of intermediate **3** (22 mg, 0.069 mmol) and 1-(3-acetyl-7-methyl-5-(2-methylpyrimidin-5-yl)-1H-indol-1-yl)propan-2-one (19 mg, 0.058 mmol) in DMF (3 mL) was added DIPEA (30 mg, 0.232 mmol) and HATU (44 mg, 0.116 mmol) at 0 °C. The mixture was stirred at room temperature under N₂ atmosphere for 12 hours before diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND 45** (4 mg, yield 21.3%) as a white solid. ¹H-NMR (400 MHz, DMSO-d₆) δ 11.36 (s, 1H), 9.58 (s, 1H), 8.95 (s, 2H), 8.85 (s, 1H), 8.33 (d, J = 7.3 Hz, 2H), 7.28 (d, J = 49.7 Hz, 2H), 5.77 (d, J = 18.2 Hz, 1H), 5.51 - 5.25 (m, 1H), 4.49 (dd, J = 9.1, 6.4 Hz, 1H), 3.74 - 3.52 (m, 2H), 3.43 - 3.41 (m, 4H), 2.11 - 1.90 (m, 2H), 1.32 (s, 3H), 1.24 (s, 4H), 1.01 (t, J *=* 5.3 Hz, 1H), 0.91 - 0.84 (m, 2H). LC/MS (ESI) m/z: 592 (M+H)⁺.

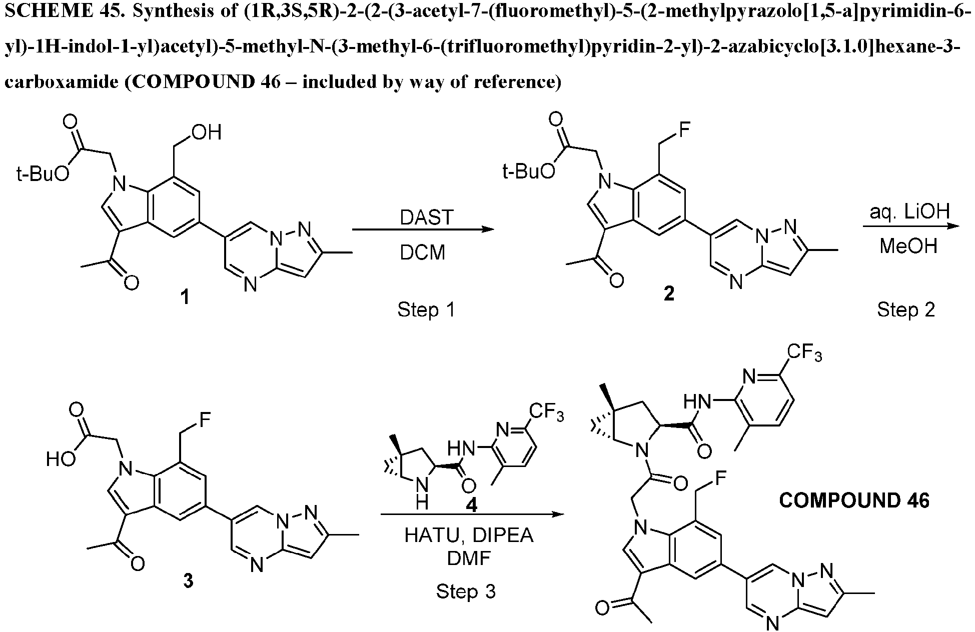

**Step 1:** To a solution of intermediate **1** (60 mg, 0.14 mmol) in DCM (3 mL) was added diethylaminosulfur trifluoride (67 mg, 0.42mmol) at -30 °C. The mixture was stirred at this temperature under N₂ atmosphere for 1 hour before mixture was poured into phosphate buffer (pH=7) and extracted with DCM twice. The combined organic layers were washed with brine, dried over anhydrous Na₂SO₄, filtered and concentrated to dryness. The residue was purified by chromatography on silica gel (PE: EtOAc= 3: 2) to give intermediate **2** (42 mg, yield 70%) as a white solid. LC/MS (ESI) (m/z): 437 (M+H)⁺.

**Step 2:** To a solution of intermediate **2** (42 mg, 0.096 mmol) in MeOH (5 mL) and water (1 mL) was added a solution of lithium hydroxide monohydrate (40 mg, 0.96 mmol) at 0 °C. The mixture was stirred at room temperature for 1 hour before concentrated to one-fifth volume, diluted with water and washed with MTBE twice. The aqueous layer was acidified with 1N aq.HCl to pH~3 and extracted with DCM twice. The combined organic layers were washed with brine, dried over anhydrous Na₂SO₄, and concentrated to dryness under reduced pressure to give compound **3** (40 mg, yield 100%) as a white solid. LC/MS (ESI) (m/z): 381 (M+H)⁺.

**Step 3:** To a mixture of intermediate **3** (20 mg, 0.053 mmol) and compound **4** (24 mg, 0.079 mmol) in DMF (3 mL) was added DIPEA (0.034 mL, 0.21 mmol) followed by HATU (40 mg, 0.11 mmol) at 0 °C. The mixture was stirred at room temperature for 2 hour before diluted with EtOAc and washed with water and brine, dried over anhydrous Na₂SO₄, filtered and concentrated to dryness. The residue was purified by chromatography on silica gel (PE: EtOAc = 2: 1) and further purified by prep-HPLC to give **COMPOUND 46** (8.4 mg, yield 24.8%) as a white solid. ¹H-NMR (400 MHz, CD₃OD) δ 9.01 (dd, *J* = 2.3, 0.9 Hz, 1H), 8.80 (d, *J* = 2.2 Hz, 1H), 8.69 (t, *J* = 2.1 Hz, 1H), 8.20 (s, 1H), 7.84 (d, *J =* 7.8 Hz, 1H), 7.59 - 7.54 (m, 2H), 6.51 (s, 1H), 5.65 (d, *J* = 11.3 Hz, 1H), 5.58 (d, *J =* 10.4 Hz, 2H), 5.55 - 5.50 (m, 1H), 4.59 (d, *J* = 9.4 Hz, 1H), 3.47 (dd, *J =* 5.6, 2.4 Hz, 1H), 2.66 (dd, *J =* 13.5, 9.2 Hz, 1H), 2.52 (d, *J* = 7.6 Hz, 6H), 2.34 - 2.27 (m, 1H), 2.24 (s, 3H), 1.41 (s, 3H), 1.13 (t, *J* = 5.5 Hz, 1H), 1.01 (dd, *J* = 5.7, 2.4 Hz, 1H). LC/MS (ESI) m/z: 662 (M+H)⁺.

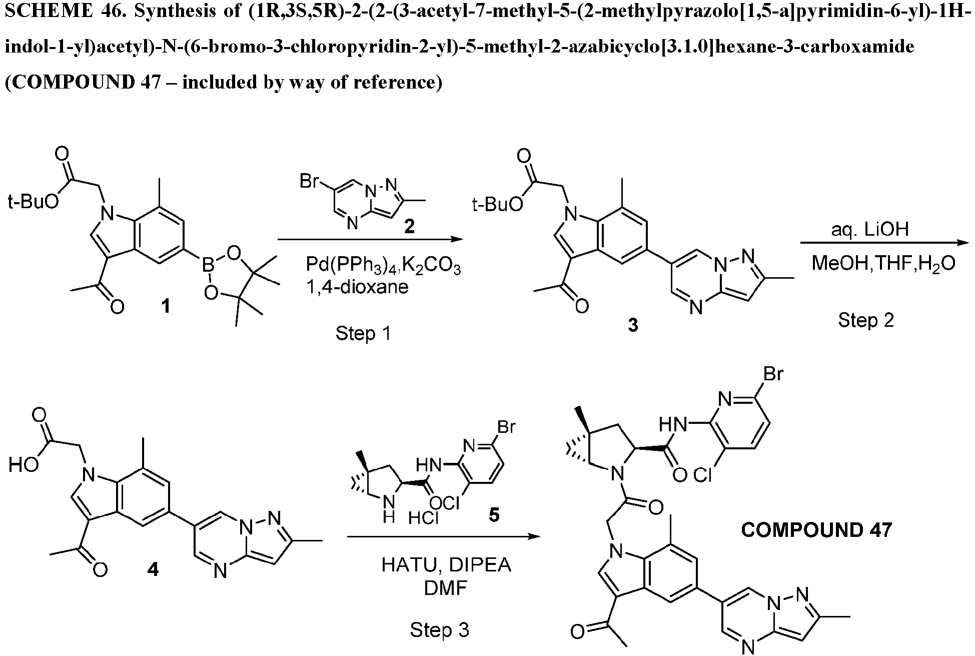

**Step 1:** To a mixture of intermediate **1** (300 mg, 0.73 mmol) and compound **2** (153 mg, 0.73 mmol) in 1,4-dioxane (7.0 mL) and water (1.0 mL) was added K₂CO₃ (253 mg, 1.3 mmol), followed by Pd(PPh₃)₄ (80.9 mg, 0.07 mmol) at 0 °C. The mixture was stirred at 90 °C overnight under N₂ atmosphere before poured into ice water and extracted with EtOAc twice. The combined organic layers were washed with brine, dried over anhydrous Na₂SO₄, filtered and concentrated to dryness under reduced pressure. The residue was purified by chromatography on silica gel (PE: EtOAc=10: 1 to 1: 1) to give intermediate **3** (270 mg, yield 89%) as yellow solid. LC/MS (ESI) (m/z): 419 (M+H)⁺.

**Step 2:** To a solution of intermediate **3** (270 mg, 0.65 mmol) in MeOH (4mL) and THF (2 mL) was added a solution of LiOH (137 mg 3.25 mmol) in water (2 mL) at 0 °C. The mixture was stirred at room temperature for 1 hour before washed with MTBE and the aqueous layer was acidified with 0.5M aq. HCl solution to pH~7 and extracted with EtOAc twice. The combined organic layers were washed with brine, dried over anhydrous Na₂SO₄, filtered and concentrated to dryness to give intermediate **4** (116 mg, yield 50%) as yellow solid. LC/MS (ESI) (m/z): 363 (M+H)⁺.

**Step 3:** To a mixture of intermediate **4** (51 mg, 0.14 mmol) and compound **5** (51 mg, 0.14 mmol) in DMF (2 mL) was added HATU (80 mg, 0.21 mmol), followed by DIPEA (90 mg, 0.7 mmol) at 0 °C. The mixture was stirred at room temperature for 1 hour before poured into aq.NH₄Cl solution and extracted with EtOAc twice. The organic layer was washed with brine, dried over anhydrous Na₂SO₄, filtered and concentrated to dryness. The residue was purified by prep-TLC (DCM: MeOH= 20: 1) to give **COMPOUND 47** (8 mg, yield 8.4%) as a white solid. ¹H-NMR (400 MHz, DMSO-*d*₆) δ 10.92 (s, 1H), 9.19 - 9.06 (m, 1H), 8.71 (d, *J* = 2.4 Hz, 1H), 8.29 (d, *J =* 1.6 Hz, 1H), 8.23 (s, 1H), 7.97 (q, *J* = 8.7 Hz, 2H), 7.31 (t, *J* = 1.2 Hz, 1H), 6.48 (s, 1H), 5.68 (d, *J* = 17.6 Hz, 1H), 5.33 (d, *J* = 18.0 Hz, 1H), 4.34 (dd, *J =* 8.8,9.2 Hz, 1H), 3.50 (dd, *J =* 5.6,5.6, Hz, 1H), 2.59 (s, 3H), 2.38 (d, *J* = 1.2 Hz, 6H), 1.91 (dd, *J =* 13.6,13.2 Hz, 1H), 0.92 (t, *J* = 5.4 Hz, 1H), 0.77 (dd, *J =* 5.6, 5.2 Hz, 1H). LC/MS (ESI) (m/z): 676 (M+H)⁺.

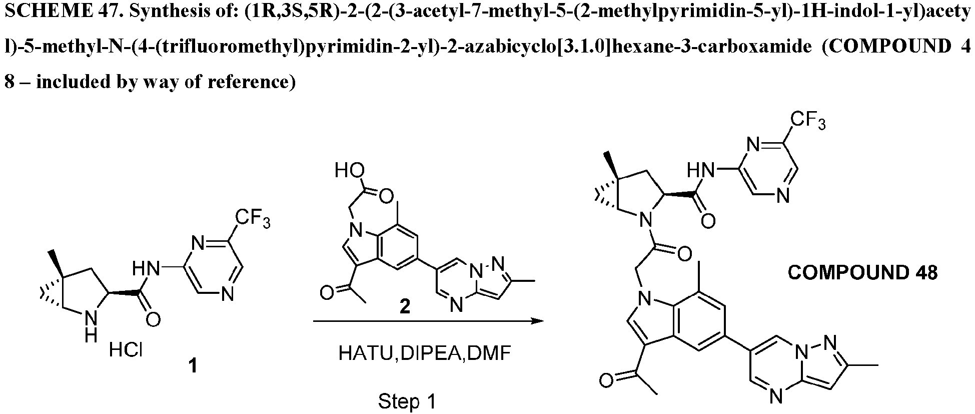

**Step 1:** To a mixture of intermediate **1** (22 mg, 0.069 mmol) and compound **2** (19 mg, 0.058 mmol) in DMF (3 mL) was added DIPEA (30 mg, 0.232 mmol) and HATU (44 mg, 0.116 mmol) at 0 °C. The mixture was stirred at room temperature under N₂ atmosphere for 12 hours before diluted with EtOAc and washed with saturated aq.NH₄Cl solution and brine, dried over anhydrous Na₂SO₄, filtered and concentrated to dryness under reduced pressure. The residue was purified by prep-HPLC to give **COMPOUND 48** (4 mg, yield 21.3%) as a white solid. ¹H-NMR (400 MHz, DMSO-d₆) δ 11.36 (s, 1H), 9.58 (s, 1H), 8.95 (s, 2H), 8.85 (s, 1H), 8.33 (d, J = 7.3 Hz, 2H), 7.28 (d, J = 49.7 Hz, 2H), 5.77 (d, J = 18.2 Hz, 1H), 5.51 - 5.25 (m, 1H), 4.49 (dd, J = 9.1, 6.4 Hz, 1H), 3.74 - 3.52 (m, 2H), 3.43 - 3.41 (m, 4H), 2.11 - 1.90 (m, 2H), 1.32 (s, 3H), 1.24 (s, 4H), 1.01 (t, J = 5.3 Hz, 1H), 0.91 - 0.84 (m, 2H). LC/MS (ESI) m/z: 631 (M+H)⁺.

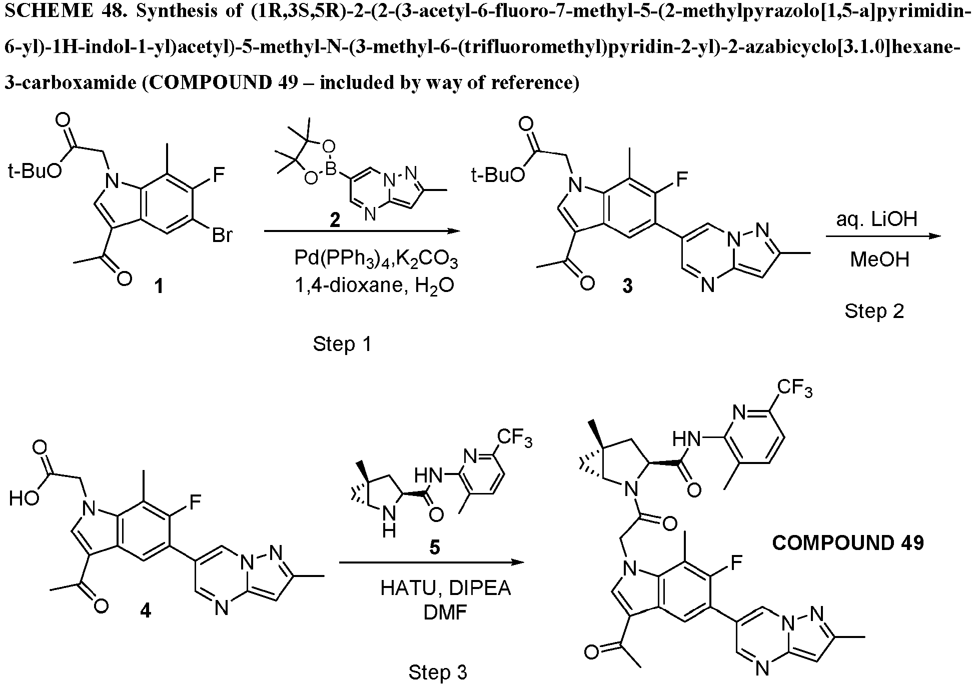

**Step 1:** To a mixture of intermediate **1** (99 mg, 0.26 mmol) and compound **2** (82 mg, 0.31 mmol) in 1,4-dioxane (4 mL) and water (0.5 mL) was added K₂CO₃ (90 mg, 0.65 mmol), Pd(PPh₃)₄ (30 mg, 0.026 mmol) under N₂ atmosphere. The mixture was degassed under N₂ atmosphere for three times. The reaction was stirred at 90 °C under N₂ atmosphere for 2 hours before diluted with EtOAc and washed with water and brine, dried over anhydrous Na₂SO₄ and concentrated to dryness under reduced pressure to give crude product, which was purified by flash chromatography on silica gel (PE: EtOAc = 9: 1) to give intermediate **3** (120 mg, yield 100%) as a white solid. LC/MS (ESI) (m/z): 437 (M+H)⁺.

**Step 2:** To a solution of intermediate **3** (120 mg, 0.27 mmol) in MeOH (5 mL) and water (1 mL) was added a solution of lithium hydroxide monohydrate (58 mg, 1.37 mmol) at 0 °C. The mixture was stirred at room temperature for 1 hour before concentrated to one-fifth volume, diluted with water and washed with MTBE twice. The aqueous layer was acidified with 1N aq.HCl to pH~3 and extracted with DCM twice. The combined organic layers were washed with brine, dried over anhydrous Na₂SO₄, and concentrated to dryness under reduced pressure to give compound **4** (105 mg, yield 100%) as a white solid. LC/MS (ESI) (m/z): 381 (M+H)⁺.

**Step 3:** To a mixture of intermediate **4** (60 mg, 0.16 mmol) and compound **5** (71 mg, 0.24 mmol) in DMF (3 mL) was added DIPEA (0.1 mL, 0.64 mmol) followed by HATU (120 mg, 0.32 mmol) at 0 °C and the mixture was stirred at room temperature for 2 hrs. The mixture was diluted with EtOAc and washed with water and brine, dried over anhydrous Na₂SO₄, filtered and concentrated to dryness under reduced pressure. The residue was purified by chromatography on silica gel (PE: EtOAc = 2: 1) and further purified by prep-HPLC to give **COMPOUND 49** (12.3 mg, yield 12.3%) as a white solid. ¹H-NMR (400 MHz, CD₃OD) δ 8.92 (dt, *J* = 2.2, 1.0 Hz, 1H), 8.65 (t, *J* = 2.0 Hz, 1H), 8.32 (d, *J =* 7.7 Hz, 1H), 8.15 (s, 1H), 7.86 (d, *J =* 7.8 Hz, 1H), 7.58 (d, *J =* 7.8 Hz, 1H), 6.53 (s, 1H), 5.65 (d, *J* = 18.1 Hz, 1H), 5.57 - 5.47 (m, 1H), 4.62 - 4.54 (m, 1H), 3.49 (dt, *J =* 6.3, 3.1 Hz, 1H), 2.65 (dd, *J =* 13.4, 9.2 Hz, 1H), 2.58 (d, *J* = 1.9 Hz, 3H), 2.50 (d, *J* = 5.9 Hz, 6H), 2.31 (dd, *J* = 5.6, 1.2 Hz, 1H), 2.26 (s, 3H), 1.41 (s, 3H), 1.13 - 1.07 (m, 1H), 0.96 (dd, *J* = 5.5, 2.4 Hz, 1H). LC/MS (ESI) m/z: 662 (M+H) ⁺.

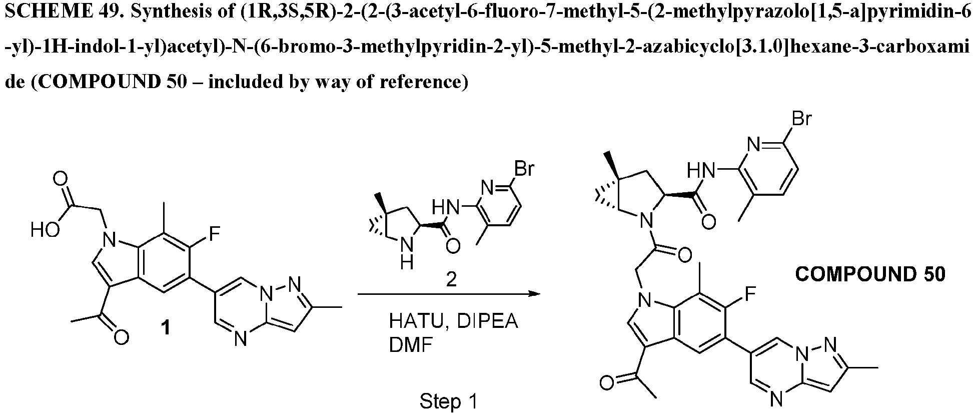

**Step 1:** To a mixture of intermediate **1** (40 mg, 0.10 mmol) and compound **2** (49 mg, 0.15 mmol) in DMF (3 mL) was added DIPEA (0.08 mL, 0.52 mmol), followed by HATU (80 mg, 0.20 mmol) at 0 °C and the mixture was stirred at room temperature for 2 hrs. The mixture was diluted with EtOAc and washed with water and brine, dried over anhydrous Na₂SO₄, filtered and concentrated to dryness under reduced pressure. The residue was purified by chromatography on silica gel (PE: EtOAc = 3: 1) and further purified by prep-HPLC to give **COMPOUND 50** (7.7 mg, yield 10.8%) as a white solid. ¹H-NMR (400 MHz, DMSO-*d*₆) δ 10.25 (s, 1H), 9.07 (dt, *J* = 2.1, 1.0 Hz, 1H), 8.55 (t, *J =* 2.1 Hz, 1H), 8.27 (s, 1H), 8.14 (d, *J =* 7.7 Hz, 1H), 7.58 - 7.52 (m, 1H), 7.36 (d, *J =* 7.9 Hz, 1H), 6.51 (s, 1H), 5.73 - 5.62 (m, 1H), 5.35 (d, *J =* 17.9 Hz, 1H), 4.33 (dd, *J =* 9.1, 5.7 Hz, 1H), 3.48 (dd, *J =* 5.6, 2.4 Hz, 1H), 3.38 - 3.33 (m, 3H), 2.52 - 2.47 (m, 1H), 2.38 (d, *J =* 4.4 Hz, 6H), 1.98 (s, 4H), 1.26 (s, 3H), 0.94 (t, *J =* 5.4 Hz, 1H), 0.83 (dd, *J=* 5.4, 2.4 Hz, 1H). LC/MS (ESI) m/z: 672/674 (M+H)⁺.

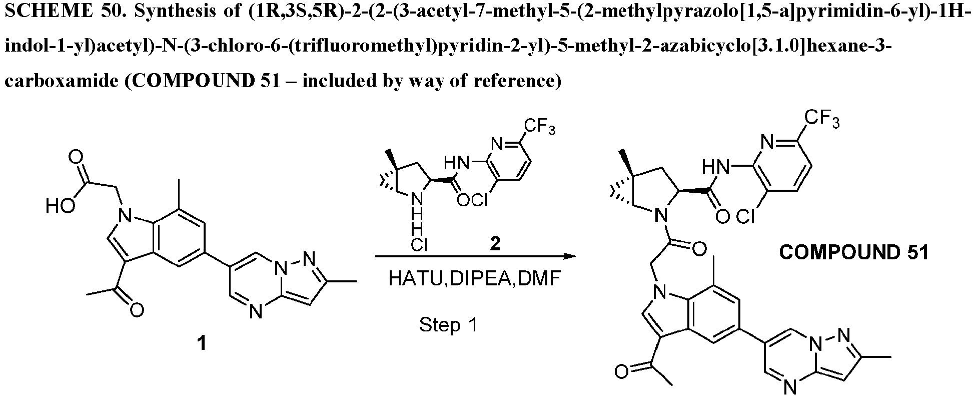

**Step 1:** To a mixture of intermediate **1** (100 mg, 0.28 mmol) and compound **2** (98 mg, 0.28 mmol) in DMF (5 mL) was added DIPEA (178 mg, 1.38 mmol), HATU (157 mg, 0.41 mmol) at 0 °C. The mixture was stirred at room temperature for 2 hours before diluted with EtOAc and washed with saturated aq.NH₄Cl solution and brine, dried over anhydrous Na₂SO₄, filtered and concentrated to dryness. The residue was purified by prep-HPLC to give **COMPOUND** 51 (72.0 mg, yield 39.3%) as a white solid. ¹H-NMR (400 MHz, CDCl₃) δ 8.80 - 8.72 (m, 2H), 8.53 (d, J = 1.6 Hz, 1H), 7.83 (dd, J = 8.0, 8.0 Hz, 1H), 7.69 (s, 1H), 7.38 (d, J = 8.0 Hz, 1H), 7.15 (dd, J = 2.0, 2.0 Hz, 1H), 6.50 (s, 1H), 5.44 (d, J = 17.6 Hz, 1H), 5.36 - 5.27 (m, 1H), 5.18-5.10 m, 1H), 3.21-3.15 (m, 1H), 2.69 (s, 3H), 2.62 - 2.55 (m, 1H), 2.55 - 2.53 (m, 3H), 2.52 - 2.43 (m, 4H), 1.42 (s, 3H), 1.15 (t, J = 5.4 Hz, 1H), 0.89 (dd, J = 5.2, 5.2 Hz, 1H)..LC/MS (ESI) (m/z): 664 (M+H)⁺.

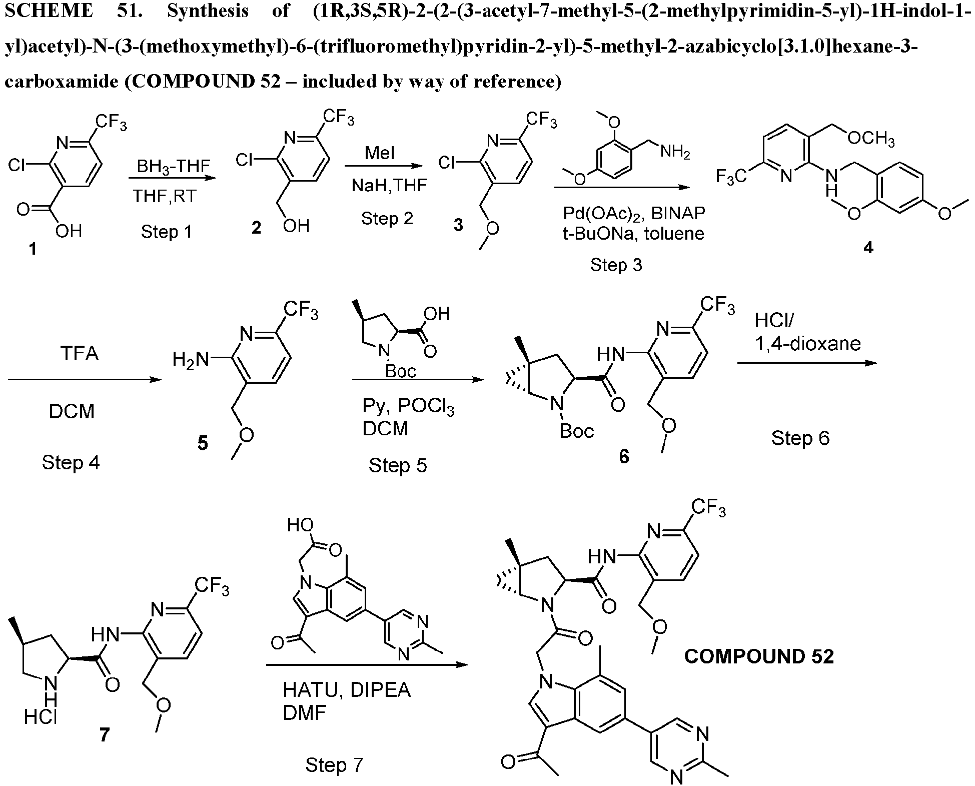

**Step 1:** To a solution of intermediate **1** (460 mg, 2.04 mmol) in THF (8 mL) was added BH₃-THF solution (6.12 mL, 6.12 mmol, 1M in THF) drop-wisely at 0 °C and the mixture was stirred at room temperature for 4 hrs. The mixture was quenched with MeOH at 0 °C and heated to 60 °C for 40 minutes. The mixture was concentrated to dryness and purified by chromatography on silica gel (PE: EtOAc= 20:1 to 5: 1) to give intermediate 2 (430 mg, yield 99.7%) as light yellow oil. LC/MS (ESI) (m/z): 212 (M+H)⁺.

**Step 2:** To a solution of intermediate **2** (210 mg, 1.0 mmol) in anhydrous THF (4 mL) was added NaH (80 mg, 2.0 mmol, 60% dispersion in mineral oil) at 0 °C under N₂ atmosphere. The mixture was stirred at 0 °C for 30 minutes before MeI (0.12 mL, 2.0 mmol) was added to the above mixture and the resulting mixture was stirred at room temperature for another 3 hrs. under N₂ atmosphere. The mixture was quenched with ice water and extracted with EtOAc. The organic layer was washed with brine, dried over Na₂SO₄, filtered and concentrated to dryness. The residue was purified by column chromatography on silica gel (eluted with PE: EtOAc = 50: 1 to 5: 1) to give intermediate **3** (200 mg, yield 88.9%) as light yellow oil. LC/MS (ESI) m/z: 226 (M+H)⁺.

**Step 3:** To a mixture of intermediate **3** (150 mg, 0.67 mmol), t-BuONa (129 mg, 1.34 mmol) in toluene (6 mL) was added BINAP (83 mg, 0.13 mmol), Pd(OAc)₂ (29 mg, 0.13 mmol) and (2,4-dimethoxyphenyl)methanamine (167 mg, 1.0 mmol) at 0 °C. The mixture was stirred at 80 °C for 4 hrs. under N₂ atmosphere before poured into ice water and extracted with EtOAc twice. The combined organic layers were washed with brine, dried over anhydrous Na₂SO₄, filtered and concentrated to dryness. The residue was purified by column chromatography on silica gel (eluted with PE: EtOAc = 40: 1 to 5: 1) give intermediate **4** (130 mg, yield 54.9%) as yellow oil. LC/MS (ESI) m/z: 357 (M+H)⁺.

**Step 4:** A solution of intermediate **4** (156 mg, 0.44 mmol) in DCM (3 mL) was added TFA (2 mL) and stirred at room temperature for 4 hours. The mixture was concentrated to dryness before poured into saturated aq.NaHCO₃ solution and extracted with DCM twice. The organic layers were washed with brine, dried over Na₂SO₄ and concentrated to dryness, purified by prep-TLC (PE: EtOAc = 5: 1) to give intermediate 5 (61 mg, yield 67.8%) as light oil. LC/MS (ESI) m/z: 207 (M+H)⁺.

**Step 5:** To a mixture of intermediate **5** (61 mg, 0.29 mmol) and (1R,3S,5R)-2-(tert-butoxycarbonyl)-5-methyl-2-azabicyclo[3.1.0]hexane-3-carboxylic acid (70 mg, 0.26 mmol) in anhydrous DCM (3 mL) was added pyridine (114 mg, 1.45 mmol) followed by drop-wise addition of POCl₃ (49 mg, 0.32 mmol) at 0 °C. The reaction was stirred at room temperature for 1 hr. before poured into ice water and extracted with DCM twice. The organic layers were washed with brine, dried over Na₂SO₄ and concentrated to dryness, purified by column chromatography on silica gel (PE: EtOAc = 20: 1 to 1: 1) to give intermediate **6** (65 mg, yield 51.2%) as a white solid. LC/MS (ESI) m/z: 430 (M+H)⁺.

**Step 6:** A solution of intermediate **6** (65 mg, 0.15 mmol) in HCl/1,4-dioxane (2.6 mL, 4M) was stirred at room temperature for 2 hrs. before concentrated to dryness to give compound **7** (50 mg, yield 100.0%) as yellow solid, which was directly used in the next step without further purification. LC/MS (ESI) m/z: 330 (M+H)⁺.

**Step 7:** To a mixture of intermediate 7 (28 mg, 0.076 mmol) and 2-(3-acetyl-7-methyl-5-(2-methylpyrimidin-5-yl)-1H-indol-1-yl)acetic acid (27 mg, 0.084 mmol) in DMF (3 mL) was added DIPEA (49 mg, 0.38 mmol) followed by HATU (43 mg, 0.11 mmol) at 0 °C and the mixture was stirred at room temperature for 1.5 hrs. The mixture was diluted with EtOAc and washed with saturated aq.NH₄Cl solution and brine, dried over anhydrous Na₂SO₄, filtered and concentrated to dryness. The residue was purified by prep-HPLC to give **COMPOUND 52** (5.1 mg, yield 10.6%) as a white solid. ¹H-NMR (400 MHz, CDCl₃) δ 8.95 (s, 1H), 8.88 (s, 2H), 8.53 (d, J = 2.0 Hz, 1H), 7.75 (s, 1H), 7.71 (s, 1H), 7.41 (d, J = 7.6 Hz, 1H), 7.16 (t, J = 1.4 Hz, 1H), 5.44 (d, J = 17.6 Hz, 1H), 5.31 (d, J = 17.6 Hz, 1H), 5.04 (s, 1H), 4.46 (d, J = 12.8 Hz, 1H), 4.31 (d, J = 12.8 Hz, 1H), 3.30 (s, 3H), 3.23 - 3.16 (m, 1H), 2.78 (s, 3H), 2.71 (s, 3H), 2.67 - 2.55 (m, 1H), 2.51 (s, 3H), 2.49 - 2.38 (m, 1H), 1.41 (s, 3H), 1.11 (t, J = 5.4 Hz, 1H), 0.87 (dd, J = 5.6, 5.6 Hz, 1H). LC/MS (ESI) m/z: 635 (M+H)⁺.

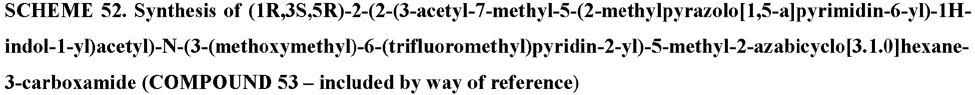

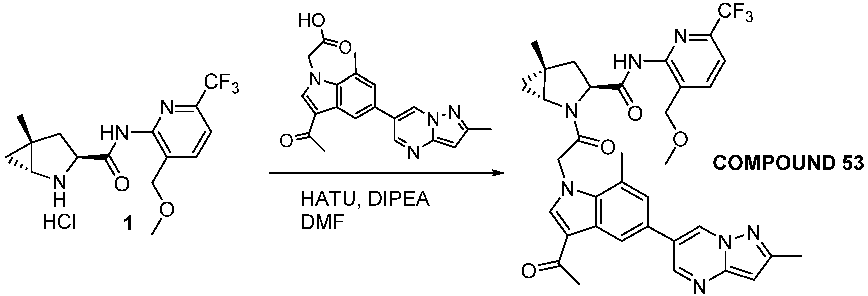

**Step 1:** To a mixture of intermediate **1** (28 mg, 0.076 mmol) and 2-(3-acetyl-7-methyl-5-(2-methylpyrazolo[1,5-a]pyrimidin-6-yl)-1H-indol-1-yl)acetic acid (30 mg, 0.084 mmol) in DMF (3 mL) was added DIPEA (49 mg, 0.38 mmol) followed by HATU (43 mg, 0.11 mmol) at 0 °C. The mixture was stirred at room temperature for 1 hour before diluted with EtOAc and washed with saturated aq.NH₄Cl solution and brine, dried over anhydrous Na₂SO₄, filtered and concentrated to dryness. The residue was purified by prep-HPLC to give **COMPOUND 53** (8.3 mg, yield 16.2%) as a white solid. ¹H-NMR (400 MHz, CDCl₃) δ 8.98 (s, 1H), 8.74 - 8.72 (m, 2H), 8.52 (d, J = 2.0 Hz, 1H), 7.76 (d, J = 6.0 Hz, 1H), 7.71 (s, 1H), 7.41 (d, J = 7.6 Hz, 1H), 7.14 (dd, J = 2.0, 2.0 Hz, 1H), 6.48 (s, 1H), 5.41 (d, J *=* 17.6 Hz, 1H), 5.28 (d, J = 17.6 Hz, 1H), 4.99 (s, 1H), 4.44 (d, J *=* 13.2 Hz, 1H), 4.29 (d, J = 13.2 Hz, 1H), 3.28 (s, 3H), 3.21 - 3.17 (m, 1H), 2.69 (s, 3H), 2.51 (d, J = 15.6 Hz, 7H), 2.47 - 2.41 (m, 1H), 1.39 (s, 3H), 1.09 (t, J = 5.4 Hz, 1H), 0.84 (dd, J = 5.6, 5.6 Hz, 1H). LC/MS (ESI) m/z: 674 (M+H)⁺.

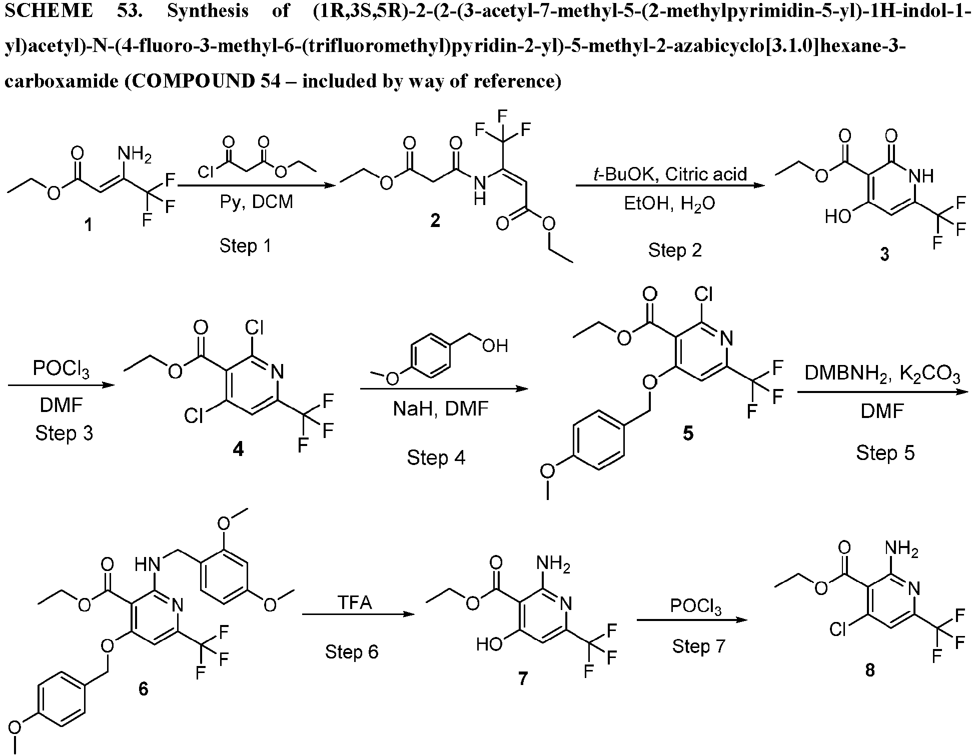

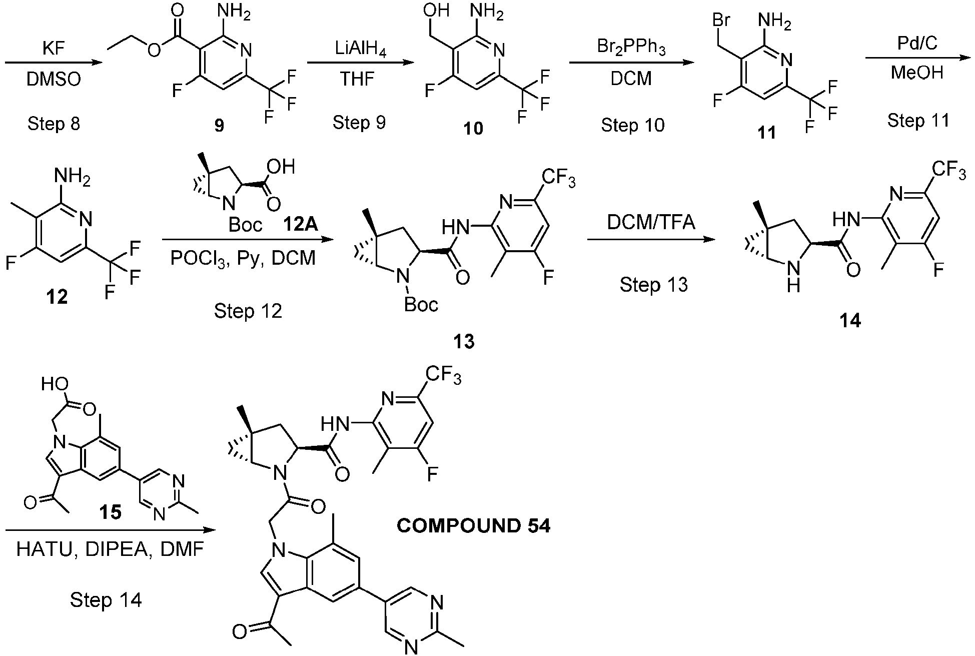

**Step 1:** To a solution of intermediate **1** (10.0 g, 54.6 mmol) in DCM (50 mL) and pyridine (5.30 mL, 65.5 mmol) was added ethyl 3-chloro-3-oxopropanoate (8.40 mL, 65.5 mmol) dropwise in a water bath. The mixture was stirred in the water bath for 2 hours and stirred at 25 °C overnight. The resulting solution was washed with 1N HCl, and NaHCO₃ (sat). The organic layer was washed with brine, dried over anhydrous Na₂SO₄, filtered and concentrated to give intermediate **2** (15.0 g, yield 94.0%) as brown oil. The crude product was used directly in the next step. LC/MS (ESI) (m/z): 298(M+H)⁺.

**Step 2:** To solution of intermediate **2** (18.5 g, 62.1 mmol) in EtOH (70 mL) was added *t*-BuOK (7.70 g, 68.5 mmol) in a water bath at ambient temperature in portions and the mixture was stirred at 70 °C for 2 hours. The reaction was cooled to room temperature and stirred overnight. The solvent was then removed in *vacuo* to give a solid. A saturated aqueous solution of citric acid was added to the solid and the resulting suspension was stirred for 30 minutes. The suspension was then filtered to give a gray solid. The solid was dried to give intermediate **3** (14.2 g, yield 91.0%) as gray solid. LC/MS (ESI) (m/z): 252 (M+H)⁺.

**Step 3:** To a solution of intermediate **3** (14.2 g, 56.4 mmol) in DMF (15 mL) were added POCl₃ (30 mL) at 0 °C and the mixture was stirred at 110 °C for 48 hours. The volatiles were removed *in vacuo.* The mixture was diluted with EtOAc and washed with saturated aq.NH₄Cl solution and brine, dried over anhydrous Na₂SO₄, filtered and concentrated to dryness. The residue was purified by chromatography on silica gel (PE: EtOAc = 9:1) to give intermediate **4** (7.20 g, yield 44.4%) as colorless oil. LC/MS (ESI) (m/z): 288 (M+H)⁺.

**Step 4:** To a mixture of intermediate **4** (5.80 g, 20.1 mmol) and (4-methoxyphenyl)methanol (2.50 mL, 20.1 mmol) in DMF (60 mL) was added NaH (772 mg, 20.1 mmol) at 0 °C and the mixture was stirred at 0 °C for 1.5 hours. Then ice-water was carefully added. The mixture was extracted with EtOAc, washed with water and brine, dried and concentrated to dryness to give crude product, which was purified by flash chromatography (PE: EtOAc = 4: 1) to give intermediate **5** (7.40 g, yield 94.3%) as a white solid. LC/MS (ESI) (m/z): 390 (M+H)⁺.

**Step 5:** To a solution of intermediate **5** (7.40 g, 19.0 mmol) in DMF (70 mL) were added K₂CO₃ (10.5 g, 76.1 mmol) and DMBNH₂ (6.40 g, 38.0 mmol) and the mixture was stirred at 110 °C for 2 hours. The mixture was extracted with EtOAc twice. The combined organic layers were washed with saturated aq.NH₄Cl solution and brine, dried over anhydrous Na₂SO₄, and concentrated under reduced pressure to dryness. The residue was purified by chromatography on silica gel (PE: EA = 10: 1) to give intermediate **6** (4.60 g, yield 46.5%) as a white solid. LC/MS (ESI) (m/z): 521 (M+H)⁺.

**Step 6:** A mixture of intermediate **6** (4.20 g, 8.06 mmol) and TFA (20 mL) was stirred at 60 °C for 2 hours. The volatiles were removed in *vacuo* and diluted with water. The pH of solution was adjusted to 7 by adding solid NaHCO₃. The resulting mixture was extracted with EtOAc twice, dried over anhydrous Na₂SO₄, filtered and concentrated to dryness. The residue was purified by re-crystallization from an EtOAc/Hexane mixture to give intermediate **7** (3.00 g, crude) as a white solid. LC/MS (ESI) m/z: 251(M+H)⁺.

**Step 7:** A mixture of intermediate **7** (3.00 g, 11.9 mmol) and POCl₃ (20 mL) was stirred at 110 °C for 2 hours. The volatiles were removed in *vacuo* and diluted with water. The pH of the solution was adjusted to 7 by adding sat.aq NaHCO₃. The mixture was diluted with EtOAc and washed with brine, dried over anhydrous Na₂SO₄, filtered and concentrated to dryness. The residue was purified by chromatography on silica gel (PE: EtOAc = 9: 1) to give intermediate **8** (980 mg, yield 30.8%) as a white solid. LC/MS (ESI) (m/z): 269 (M+H)⁺.

**Step 8:** To a solution of intermediate **8** (680 mg, 2.53 mmol) in DMSO (2.50 mL) was added KF (1.50 g, 25.3 mmol) and the mixture was stirred at 150 °C for 72 hours. The mixture was diluted with EtOAc and washed with water and brine, dried over anhydrous Na₂SO₄, filtered and concentrated to dryness. The residue was purified by chromatography on silica gel (PE: EtOAc = 94: 6) to give intermediate **9** (125 mg, yield 19.6%) as a white solid. LC/MS (ESI) (m/z): 253 (M+H)⁺.

**Step 9:** To a solution of intermediate **9** (80.0 mg, 0.320 mmol) in THF (5 mL) was added LiAlH₄ (30.0 mg, 0.790 mmol) at 0 °C and the mixture was stirred at 0 °C for 1 hour. The mixture was quenched with H₂O/aq NaOH (15%)/H₂O (1/1/3). The mixture was extracted with EtOAc and washed with brine, dried over anhydrous Na₂SO₄, filtered and concentrated to dryness to give intermediate **10** (76.0 mg, crude) as a white solid. The crude product was used directly in next step. LC/MS (ESI) (m/z): 211 (M+H)⁺.

**Step 10:** To a mixture of intermediate **10** (76.0 mg, 0.360 mmol) in DCM (6 mL) was added Br₂PPh₃ (760 mg, 1.80 mmol) at 0 °C and the mixture was stirred at 30 °C for 12 hours. The mixture was concentrated to dryness to give crude intermediate **11** (105 mg, crude) as a brown solid. The crude product was used directly in next step. LC/MS (ESI) (m/z): 273/275 (M+H)⁺.

**Step 11:** To a solution of intermediate **11** (105 mg, 0.380 mmol) in MeOH (5 mL) was added Pd/C (30.0 mg) and degassed under H₂ atmosphere for three times and the mixture was stirred at 25 °C for 1 hour. The mixture was filtered and filtrate was concentrated under reduced pressure to dryness to give intermediate **12** (70.0 mg, yield 93.3%) as a white solid. The crude product was used directly in next step. LC/MS (ESI) (m/z): 195 (M+H)⁺.

**Step 12:** To a solution of intermediate **12** (70.0 mg, 0.360 mmol) and compound **12A** (130 mg, 0.540 mmol) in DCM (5 mL) were added pyridine (0.14 mL, 1.80 mmol) and POCl₃ (0.033 mL, 0.360 mmol) at 0 °C. The mixture was stirred at 25 °C for 1 hour. The pH of the solution was adjusted to 7 by adding 1N HCl. The mixture was diluted with EtOAc and washed with brine, dried over anhydrous Na₂SO₄, filtered and concentrated to dryness. The residue was purified by chromatography on silica gel (PE: EtOAc = 4: 1) to give intermediate **13** (75.0 mg, yield 50.0%) as brown semi-solid. LC/MS (ESI) (m/z): 418 (M+H)⁺.

**Step 13:** To a solution of intermediate **13** (75.0 mg, 0.180 mmol) in DCM (2 mL) was added TFA (1 mL) at 0 °C and the mixture was stirred at room temperature for 1 hour. The mixture was concentrated to give intermediate **14** (86.0 mg, yield 100%) as brown oil. The crude product was used to next step directly. LC/MS (ESI) (m/z): 318 (M+H)⁺.

**Step 14:** To a mixture of intermediate **14** (20.0 mg, 0.063 mmol) and compound **15** (26.0 mg, 0.082 mmol) in DMF (3 mL) were added DIPEA (0.05 mL, 0.320 mmol) and HATU (48.0 mg, 0.130 mmol) at 0 °C. The mixture was stirred at room temperature for 2 hours under N₂ atmosphere. The mixture was diluted with EtOAc and washed with saturated aq.NH₄Cl solution and brine, dried over anhydrous Na₂SO₄, filtered and concentrated to dryness. The residue was purified by chromatography on silica gel (DCM: MeOH = 96: 4) to give **COMPOUND 54** (24.0 mg, crude) as a brown solid, which was further purified by prep-HPLC to give **COMPOUND 54** (1.2 mg, yield 3.0%) as a white solid. ¹H NMR (400 MHz, CD₃OD) δ 8.97 (s, 2H), 8.47 (d, *J* = 1.9 Hz, 1H), 8.16 (s, 1H), 7.52 (d, *J* = 8.3 Hz, 1H), 7.32 (t, *J =* 1.3 Hz, 1H), 5.66 (d, *J =* 18.0 Hz, 1H), 5.56 (d, *J =* 18.0 Hz, 1H), 4.57 (s, 1H), 3.50 (dd, *J* = 5.5, 2.4 Hz, 1H), 2.72 (d, *J* = 6.2 Hz, 7H), 2.65 (dd, *J =* 13.5, 9.2 Hz, 1H), 2.53 (s, 3H), 2.30 - 2.22 (m, 1H), 2.13 (d, *J* = 1.9 Hz, 3H), 1.40 (s, 3H), 1.13 - 1.08 (m, 1H), 0.96 (dd, *J =* 5.5, 2.4 Hz, 1H); LC/MS (ESI) m/z: 623(M+H)⁺.

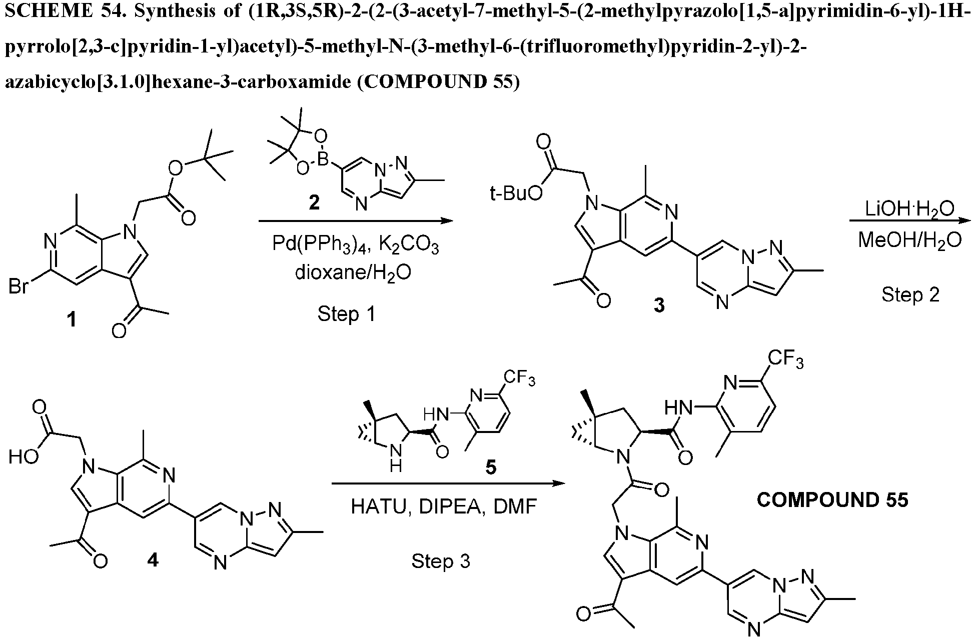

**Step 1:** To a mixture of intermediate **1** (50.0 mg, 0.140 mmol) and compound **2** (46.0 mg, 0.180 mmol) in 1,4-dioxane (4 mL) and water (0.5 mL) were added K₂CO₃ (47.0 mg, 0.350 mmol) and Pd(PPh₃)₄ (160 mg, 0.014 mmol) at 25 °C under N₂ atmosphere. The reaction was stirred at 95 °C for 2 hours. The mixture was diluted with EtOAc and washed with water and brine, dried and concentrated to dryness to give crude product, which was purified by flash chromatography (DCM: MeOH = 95: 5) to give intermediate **3** (55.0 mg, yield 96.5%) as yellow oil. LC/MS (ESI) (m/z): 420 (M+H)⁺.

**Step 2:** To a solution of intermediate **3** (55.0 mg, 0.13.0 mmol) in MeOH (5 mL) and water (1 mL) was added LiOH·H₂O (28.0 mg, 0.650 mmol) at 0 °C and the mixture was stirred at room temperature for 1 hour. The mixture was concentrated to 1/5 volume, diluted with water and washed with MTBE twice. The aqueous layer was acidified with 1N aq.HCl to pH = 3, extracted with DCM twice. The combined organic layers were washed with brine, dried over anhydrous Na₂SO₄, and concentrated under reduced pressure to dryness to give intermediate **4** (47.0 mg, yield 99.9%) as a white solid. LC/MS (ESI) (m/z): 364 (M+H)⁺.

**Step 3:** To a mixture of intermediate **4** (47.0 mg, 0.130 mmol) and compound **5** (46.0 mg, 0.160 mmol) in DMF (5 mL) were added DIPEA (0.11 mL, 0.650 mmol) and HATU (98.0 mg, 0.260 mmol) at 0 °C and the mixture was stirred at room temperature for 2 hours under N₂ atmosphere. The mixture was diluted with EtOAc and washed with saturated aq.NH₄Cl solution and brine, dried over anhydrous Na₂SO₄, filtered and concentrated to dryness. The residue was purified by chromatography on silica gel (DCM: MeOH = 97: 3) to give **COMPOUND 55** (34.0 mg, crude) as a brown solid. The residue was further purified by prep-HPLC to give **COMPOUND 55** (6.6 mg, yield 8.0%) as a white solid. ¹H NMR (400 MHz, CD₃OD) δ 9.28 (d, J = 6.4 Hz, 1H), 9.00 (s, 1H), 8.56 (d, J = 8.3 Hz, 1H), 8.38 (d, J = 6.6 Hz, 1H), 7.84 (d, J = 7.6 Hz, 1H), 7.57 (d, J = 7.9 Hz, 1H), 6.54 (d, J = 7.4 Hz, 1H), 5.79 - 5.66 (m, 1H), 5.59 (s, 1H), 4.59 (s, 1H), 3.51 (s, 1H), 3.21 (q, J = 7.3 Hz, 1H), 2.96 - 2.86 (m, 3H), 2.72 - 2.61 (m, 1H), 2.51 (q, J = 2.9, 1.8 Hz, 6H), 2.30 (dd, J = 13.6, 5.7 Hz, 1H), 2.25 (d, J = 2.3 Hz, 3H), 1.42 (s, 3H), 1.31 (t, J = 7.3 Hz, 1H), 1.13 (s, 1H), 0.99 (s, 1H); LC/MS (ESI) m/z: 645(M+H)⁺.

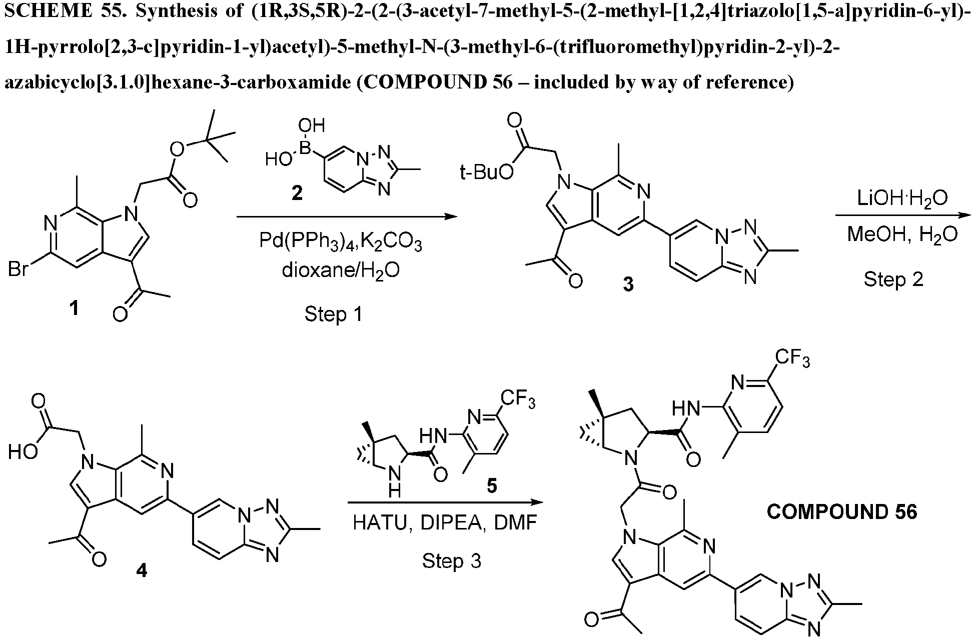

**Step 1:** To a mixture of intermediate **1** (50.0 mg, 0.140 mmol) and compound **2** (31.0 mg, 0.180 mmol) in 1,4-dioxane (4 mL) and water (0.5 mL) were added K₂CO₃ (47.0 mg, 0.350 mmol) and Pd(PPh₃)₄ (16.0 mg, 0.014 mmol) under N₂ atmosphere. The mixture was stirred at 95 °C for 2 hours. The mixture was diluted with EtOAc and washed with water and brine, dried and concentrated to dryness to give crude product, which was purified by flash chromatography (DCM: MeOH = 95: 5) to give intermediate **3** (38.0 mg, yield 66.7%) as yellow solid. LC/MS (ESI) (m/z): 420 (M+H)⁺.

**Step 2:** To a solution of intermediate **3** (35.0 mg, 0.084 mmol) in MeOH (5 mL) and water (1 mL) was added a solution of LiOH·H₂O (20.0 mg, 0.650 mmol) at 0 °C and the mixture was stirred at room temperature for 1 hour. The mixture was concentrated to 1/5 volume, diluted with water and washed with MTBE twice. The aqueous layer was acidified with 1N aq.HCl to pH = 3, extracted with DCM twice. The combined organic layers were washed with brine, dried over anhydrous Na₂SO₄, and concentrated under reduced pressure to dryness to give intermediate **4** (30.0 mg, yield 90.9%) as a white solid. LC/MS (ESI) (m/z): 364 (M+H)⁺.

**Step 3:** To a mixture of intermediate **4** (35.0 mg, 0.096 mmol) and compound **5** (37.0 mg, 0.120 mmol) in DMF (5 mL) were added DIPEA (0.08 mL, 0.480 mmol) and HATU (74.0 mg, 0.190 mmol) at 0 °C and the mixture was stirred at room temperature for 2 hours under N₂ atmosphere. The mixture was diluted with EtOAc and washed with saturated aq.NH₄Cl solution and brine, dried over anhydrous Na₂SO₄, filtered and concentrated to dryness. The residue was purified by chromatography on silica gel (DCM: MeOH = 96: 4) to give **COMPOUND 56** (70.0 mg, crude) as a brown solid. The residue was purified by prep-HPLC to give **COMPOUND 56** (10.6 mg, yield 17.1%) as a white solid. ¹H NMR (400 MHz, CD₃OD) δ 9.24 (d, *J* = 9.2 Hz, 1H), 8.57 - 8.50 (m, 1H), 8.28 (d, *J =* 5.8 Hz, 2H), 7.84 (d, *J* = 7.8 Hz, 1H); 7.71 (t, *J* = 8.1 Hz, 1H), 7.58 (dd, *J =* 7.8, 1.4 Hz, 1H), 5.68 (t, *J* = 13.7 Hz, 1H), 5.62 - 5.52 (m, 1H), 4.60 (d, *J =* 8.4 Hz, 1H), 3.50 (s, 1H), 2.90 - 2.85 (m, 3H), 2.66 (dd, *J =* 13.5, 9.2 Hz, 1H), 2.58 - 2.49 (m, 6H), 2.30 (dd, *J* = 13.5, 5.6 Hz, 1H), 2.24 (s, 3H), 1.41 (s, 3H), 1.12 (s, 1H), 0.98 (s, 1H); LC/MS (ESI) m/z: 645(M+H)⁺.

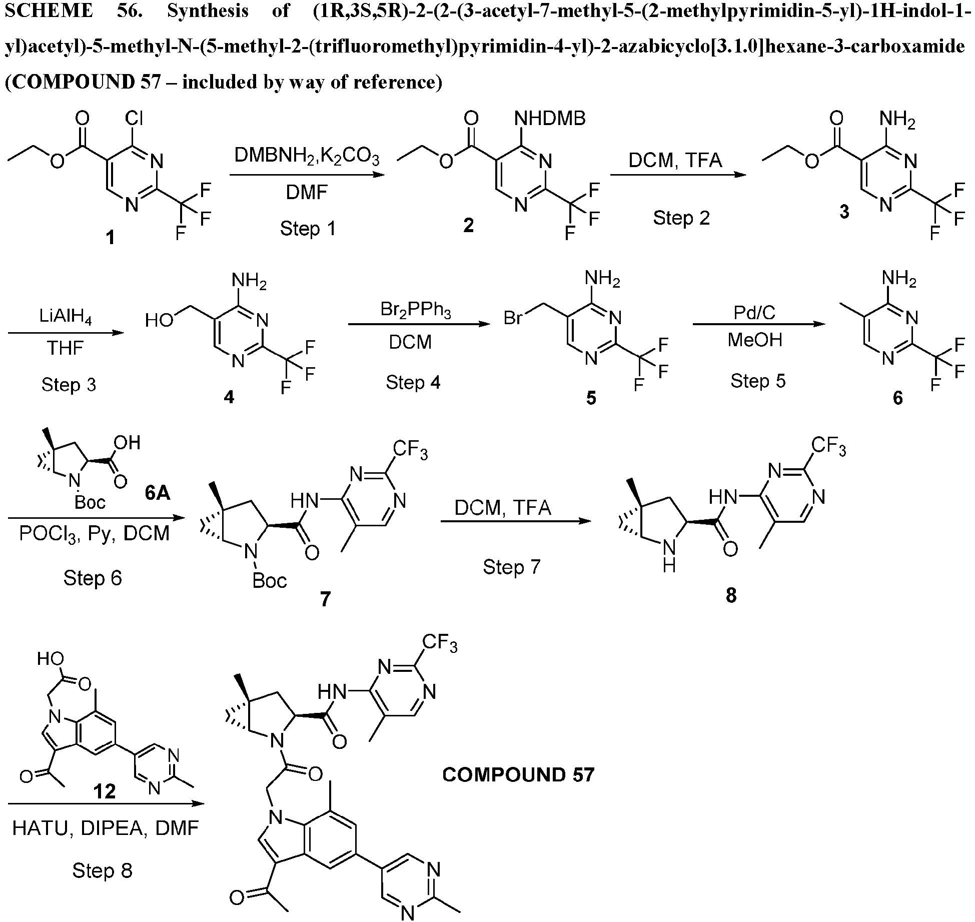

**Step 1:** To a solution of intermediate **1** (1.00 g, 3.94 mmol) in DMF (10 mL) were added K₂CO₃ (2.16 g, 15.8 mmol) and DMBNH₂ (1.30 g, 7.88 mmol) and the mixture was stirred at 110 °C for 2 hours. The mixture was extracted with EtOAc twice. The combined organic layers were washed with saturated aq.NH₄Cl solution and brine, dried over anhydrous Na₂SO₄, and concentrated under reduced pressure to dryness. The residue was purified by chromatography on silica gel (PE: EtOAc = 10: 1) to give intermediate **2** (1.47 g, yield 96.7%) as a white solid. LC/MS (ESI) (m/z): 386 (M+H)⁺.

**Step 2:** To a solution of intermediate **2** (300 mg, 0.780 mmol) in DCM (2 mL) was added TFA (2 mL) and the mixture was stirred at 40 °C for 12 hours. The volatiles were removed in *vacuo* to give intermediate **3** (345 mg, yield 100%) as pink solid. The crude product was used to next step directly. LC/MS (ESI) m/z: 236(M+H)⁺.

**Step 3:** To a solution of intermediate **3** (235 mg, 0.990 mmol) in THF (5 mL) were added LiAlH₄ (94.0 mg, 2.49 mmol) at 0 °C and the mixture was stirred at 0 °C for 1 hour. The mixture was quenched with H₂O/aq NaOH (15%)/H₂O (1/1/3). The mixture was extracted with EtOAc and washed with brine, dried over anhydrous Na₂SO₄, filtered and concentrated to dryness to give intermediate **4** (185 mg, yield 95.8%) as a white solid. The crude product was used to next step directly. LC/MS (ESI) (m/z): 194 (M+H)⁺.

**Step 4:** To a mixture of intermediate **4** (185 mg, 0.950 mmol) in DCM (6 mL) was added Br₂PPh₃ (2.01 g, 4.75 mmol) at 0 °C and the mixture was stirred at 30 °C for 12 hours. The mixture was concentrated to dryness to give crude intermediate **5** (200 mg, yield 82%) as a brown solid. The crude product was used to next step directly. LC/MS (ESI) (m/z): 256/258 (M+H)⁺.

**Step 5:** To a solution of intermediate **5** (200 mg, 0.78 mmol) in MeOH (5 mL) was added Pd/C (20.0 mg) and degassed under H₂ atmosphere for three times and the mixture was stirred at 25 °C for 1 hour. The mixture was filtered and filtrate was concentrated under reduced pressure to dryness to give intermediate **6** (90.0 mg, yield 64.3%) as a white solid. The crude product was used directly in next step. LC/MS (ESI) (m/z): 178 (M+H)⁺.

**Step 6:** To a solution of intermediate **6** (90.0 mg, 0.510 mmol) and compound **6A** (245 mg, 1.02 mmol) in DCM (5 mL) were added pyridine (0.40 mL, 5.10 mmol) and POCl₃ (0.09 mL, 1.02 mmol) at 0 °C. The mixture was stirred at 25 °C for 1 hour. The pH of the solution was adjusted to 7 by adding 1N HCl. The mixture was diluted with EtOAc and washed with brine, dried over anhydrous Na₂SO₄, filtered and concentrated to dryness. The residue was purified by chromatography on silica gel (PE: EtOAc = 4: 1) to give intermediate 7 (24.0 mg, yield 10.5%) as a white solid. LC/MS (ESI) (m/z): 401 (M+H)⁺.

**Step 7:** To a solution of intermediate **7** (24.0 mg, 0.06 mmol) in DCM (2 mL) was added TFA (1 mL) at 0 °C and the mixture was stirred at room temperature for 1 hour. The mixture was concentrated to dryness under reduced pressure to give intermediate **8** (20.0 mg, yield 100%) as brown oil. The crude product was used to next step directly. LC/MS (ESI) (m/z): 301 (M+H)⁺.

**Step 8:** To a mixture of intermediate **8** (20.0 mg, 0.066 mmol) and compound **12** (32.0 mg, 0.099 mmol) in DMF (3 mL) were added DIPEA (0.05 mL, 0.330 mmol) and HATU (48.0 mg, 0.130 mmol) at 0 °C and the mixture was stirred at room temperature for 2 hours under N₂ atmosphere. The mixture was diluted with EtOAc and washed with saturated aq.NH₄Cl solution and brine, dried over anhydrous Na₂SO₄, filtered and concentrated to dryness. The crude **COMPOUND 57** (24 mg) was purified by preparative TLC (DCM: MeOH = 20: 1) and was further purified by prep-HPLC to give **COMPOUND 57** (1.3 mg, yield 3.2%) as a white solid. ¹H NMR (400 MHz, CD₃OD) δ 8.96 (d, *J =* 1.8 Hz, 2H), 8.66 (s, 1H), 8.46 (s, 1H), 8.16 (d, *J* = 2.4 Hz, 1H), 7.31 (s, 1H), 5.68 (d, *J =* 18.6 Hz, 1H), 5.53 (d, *J =* 17.9 Hz, 1H), 4.72 (dd, *J =* 9.2, 5.4 Hz, 1H), 3.51 (d, *J =* 5.2 Hz, 1H), 2.72 (d, *J =* 8.2 Hz, 6H), 2.65 (dd, *J =* 13.5, 9.2 Hz, 1H), 2.52 (d, *J =* 1.8 Hz, 3H), 2.26 (dd, *J =* 13.5, 5.5 Hz, 1H), 2.21 (s, 3H), 1.40 (s, 3H), 1.11 (t, *J* = 5.5 Hz, 1H), 0.99 - 0.93 (m, 1H); LC/MS (ESI) m/z: 606(M+H)⁺.

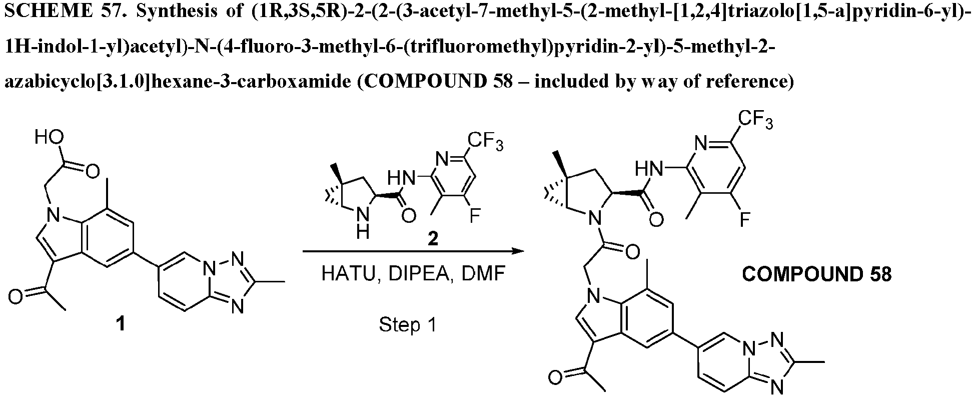

**Step 1:** To a mixture of intermediate **1** (65.0 mg, 0.200 mmol) and compound **2** (90.0 mg, 0.24 mmol) in DMF (3 mL) were added DIPEA (0.17 mL, 1.00 mmol) and HATU (155 mg, 0.400 mmol) at 0 °C and the mixture was stirred at room temperature for 2 hours under N₂ atmosphere. The mixture was diluted with EtOAc and washed with saturated aq. NH₄Cl solution and brine, dried over anhydrous Na₂SO₄, filtered and concentrated to dryness. The residue was purified by chromatography on silica gel (DCM: MeOH = 96: 4) to give **COMPOUND 58** (106 mg, crude) as a brown solid. The residue was further purified by prep-HPLC to give **COMPOUND 58** (20.2 mg, yield 15.5%) as a white solid. ¹H NMR (400 MHz, CD₃OD) δ 8.82 (t, *J =* 1.3 Hz, 1H), 8.41 (d, *J =* 1.9 Hz, 1H), 8.09 (s, 1H), 7.95 (dd, *J =* 9.2, 1.8 Hz, 1H), 7.69 - 7.64 (m, 1H), 7.50 (d, *J =* 8.3 Hz, 1H), 7.27 (d, *J =* 1.6 Hz, 1H), 5.59 (d, *J* = 18.1 Hz, 1H), 5.48 (d, *J =* 18.0 Hz, 1H), 4.55 (dd, *J =* 9.2, 5.7 Hz, 1H), 3.47 (dd, *J =* 5.5, 2.4 Hz, 1H), 2.67 (s, 3H), 2.65 - 2.59 (m, 1H), 2.55 (s, 3H), 2.49 (s, 3H), 2.25 (dd, *J* = 13.5, 5.6 Hz, 1H), 2.11 (d, *J =* 1.9 Hz, 3H), 1.39 (s, 3H), 1.09 (t, *J* = 5.4 Hz, 1H), 0.94 (dd, *J* = 5.6, 2.4 Hz, 1H); LC/MS (ESI) m/z: 662(M+H)⁺.

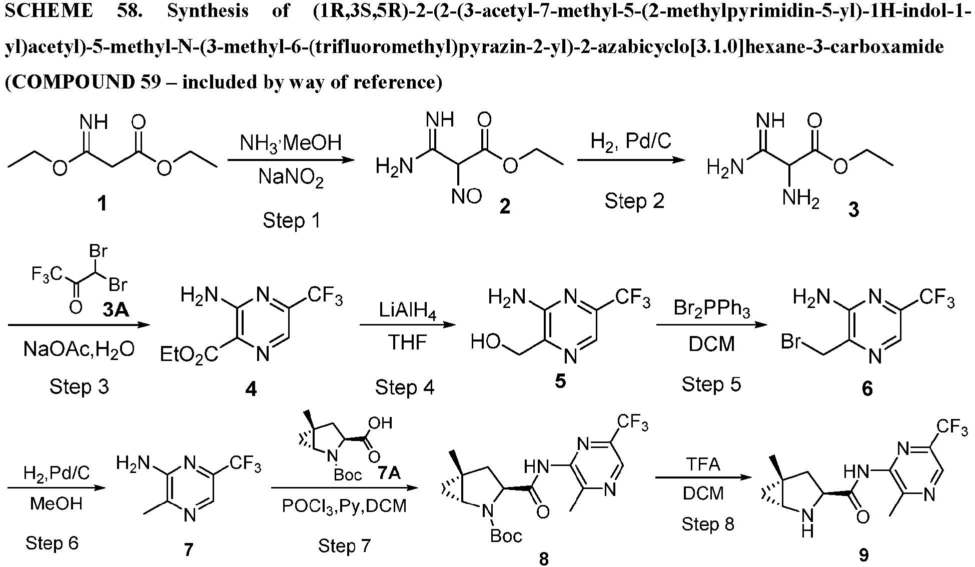

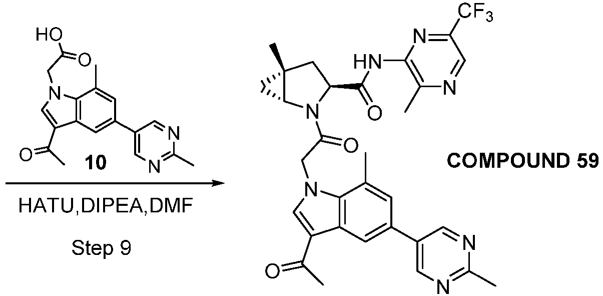

**Step 1:** A solution of NH₃-MeOH solution (22 mL, 153 mmol, 7 M) in MeOH (54 mL) was drop-wise added to intermediate **1** (12.5 g, 63.9 mmol) at 0 °C and the mixture was stirred at 0 °C for 3 hours, after which a solution of NaNO₂ (4.85 g, 70.3 mmol) in water (5 mL) was added in a single portion. The pH of the mixture was adjusted to pH 6 with the addition of 6N aq.HCl solution. The reaction mixture was stirred at room temperature overnight. The yellow precipitate formed was filtered under vacuum, washed with water and dried to give intermediate **2** (10.0 g, yield 98.1%) as yellow solid. LC/MS (ESI) m/z: 160 (M+H)⁺.

**Step 2:** To a solution of intermediate **2** (10.0 g, 62.9 mmol) in 6N HCl/MeOH (1: 1 ratio, 450 mL) was added 10% Pd/C (1.00 g) and the mixture was stirred at room temperature overnight under H₂. The mixture was filtered and the filtrate was concentrated to dryness to give intermediate **3** (9.00 g, yield 98.7 %) as a white solid. LC/MS (ESI) m/z: 146 (M+H)⁺.

**Step 3:** The mixture of compound **3A** (10.0 g, 37.1 mmol) and NaOAc (12.2 g, 148 mmol) in water (50 mL) was heated at 100 °C for 1 hour. The mixture was cooled to room temperature. A solution of intermediate **3** (2.69 g, 18.3 mmol) in water (13 mL) was added and the reaction was stirred at 30 °C overnight. The mixture was diluted with DCM twice, and the combined organic layers were washed with brine, dried over anhydrous Na₂SO₄, filtered and concentrated to dryness. The residue was purified by chromatography on silica gel (PE: EtOAc= 100: 1 to 1: 1) to give intermediate **4** (450 mg, yield 10.3%) as yellow solid. LC/MS (ESI) m/z: 236 (M+H)⁺.

**Step 4:** To a solution of intermediate **4** (300 mg, 1.28 mmol) in THF (3 mL) was added LiAlH₄ (121 mg, 3.19 mmol) at 0°C under N₂ atmosphere and the mixture was stirred at 0 °C for 2 hours. The mixture was quenched with water (0.2 mL), 15% aq. NaOH solution (0.2 mL) and water(0.6 mL) in sequence and stirred at room temperature for 30 minutes. The mixture was extracted with EtOAc twice. The combined organic layers were washed with brine, dried with anhydrous Na₂SO₄, filtered and concentrated and the residue was purified by chromatography on silica gel (PE: EtOAc=20: 1 to 2: 1) to give intermediate **5** (120 mg, yield 48.8%) as a white solid. LC/MS (ESI) m/z: 194 (M+H)⁺.

**Step 5:** To a solution of intermediate **5** (120 mg, 0.620 mmol) in DCM (5 mL) was added Br₂PPh₃ (1.57 g, 3.73 mmol) at 0 °C and the mixture was stirred at 25 °C for 4 hours. The mixture was concentrated under reduced pressure to give intermediate **6** (158 mg, yield 99.7%) as a white solid, which was used to next step directly without purification. LC/MS (ESI) m/z: 256/258 (M+H)⁺.

**Step 6:** To a solution of intermediate **6** (158 mg, 0.62 mmol) in MeOH (3 mL) was added 10% Pd/C (47.0 mg) and the mixture was stirred at 30 °C for 3 hours under H₂. The mixture was filtered and the filtrate was concentrated to dryness. The residue was purified by chromatography on silica gel (PE: EtOAc=50: 1 to 1: 1) to give intermediate **7** (45.0 mg, yield 41.2 %) as a white solid. LC/MS (ESI) m/z: 178 (M+H)⁺.

**Step 7:** To a mixture of intermediate **7** (30.0 mg, 0.170 mmol) and compound **7A** (61.0 mg, 0.260 mmol) in anhydrous DCM (3 mL) was added pyridine (134 mg, 1.70 mmol), followed by drop-wise addition of POCl₃ (52.0 mg, 0.34 mmol) at -15 °C. The reaction was stirred at -15 °C for 2 hours. The reaction mixture was poured into ice-water and extracted with DCM twice. The organic layers were washed with brine, dried over Na₂SO₄ and concentrated to dryness. The residue was purified by column chromatography on silica gel (PE: EtOAc = 20: 1 to 3: 1) to give intermediate **8** (28.0 mg, yield 41.2%) as a white solid. LC/MS (ESI) m/z: 401 (M+H)⁺.

**Step 8:** To a solution of intermediate **8** (28.0 mg, 0.070 mmol) in DCM (2 mL) was added TFA (1 mL) and the reaction was stirred at room temperature for 1 hour. The mixture was concentrated to dryness to give intermediate **9** (21.0 mg, yield 99.9%) as a yellow solid, which was used to next step directly without purification. LC/MS (ESI) m/z: 301 (M+H)⁺.

**Step 9:** To a mixture of intermediate **9** (10.0 mg, 0.030 mmol) and compound **10** (10.0 mg, 0.030 mmol) in DMF (2 mL) was added DIPEA (19.0 mg, 0.150 mmol), followed by addition of HATU (17.0 mg, 0.050 mmol) at 0 °C and the mixture was stirred at room temperature for 1 hour. The mixture was diluted with EtOAc and washed with saturated aq. NH₄Cl solution and brine, dried over anhydrous Na₂SO₄, filtered and concentrated to dryness. The residue was purified by pre-TLC (DCM: MeOH= 20: 1) to give **COMPOUND 59** (2.5 mg, yield 12.5%) as a white solid. ¹H NMR (400 MHz, CDCl₃) δ 9.39 (s, 1H), 8.90 (s, 2H), 8.57 (d, *J* = 2.0 Hz, 2H), 7.68 (s, 1H), 7.16 (s, 1H), 5.40 (d, *J =* 3.2 Hz, 2H), 4.90 (d, *J* = 6.4 Hz, 1H), 3.07 (d, *J =* 4.4 Hz, 1H), 2.88 (s, 1H), 2.79 (s, 3H), 2.64 (s, 3H), 2.53 (s, 3H), 2.39 (s, 3H), 2.29 - 2.23 (m, 1H), 1.46 (s, 3H), 1.23 (t, *J* = 4.4 Hz, 1H), 0.92 - 0.90 (m, 1H).LC/MS (ESI) m/z: 606 (M+H)⁺.

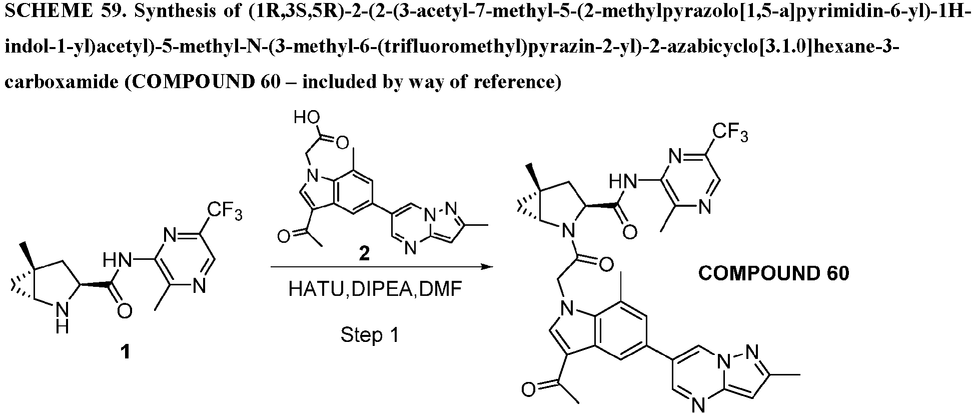

**Step 1:** To a mixture of intermediate **1** (10.0 mg, 0.030 mmol) and compound **2** (12.0 mg, 0.030 mmol) in DMF (2 mL) was added DIPEA (19.0 mg, 0.150 mmol), followed by addition of HATU (17.0 mg, 0.050 mmol) at 0 °C and the mixture was stirred at room temperature for 2 hours. The mixture was diluted with EtOAc and washed with saturated aq. NH₄Cl solution and brine, dried over anhydrous Na₂SO₄, filtered and concentrated to dryness. The residue was purified by prep-TLC to give **COMPOUND 60** (3.0 mg, yield 14.3%) as a white solid. ¹H NMR (400 MHz, CDCl₃) δ 9.42 (s, 1H), 8.77 (dd, *J* = 2.4, 2.4 Hz, 1H), 8.75 (d, *J* = 2.4 Hz, 1H), 8.56 (d, *J =* 2.0 Hz, 2H), 7.67 (s, 1H), 7.15 (d, 1H), 6.50 (s, 1H), 5.39 (s, 2H), 4.93 - 4.85 (m, 1H), 3.11 - 3.04 (m, 1H), 2.84 (d, *J =* 14.4 Hz, 1H), 2.63 (s, 3H), 2.54 (s, 3H), 2.52 (s, 3H), 2.40 (s, 3H), 2.32-2.21 (m, 1H), 1.46 (s, 3H), 1.22 (t, *J =* 5.4 Hz 1H), 0.94 - 0.87 (m, 1H). LC/MS (ESI) m/z: 645 (M+H)⁺.

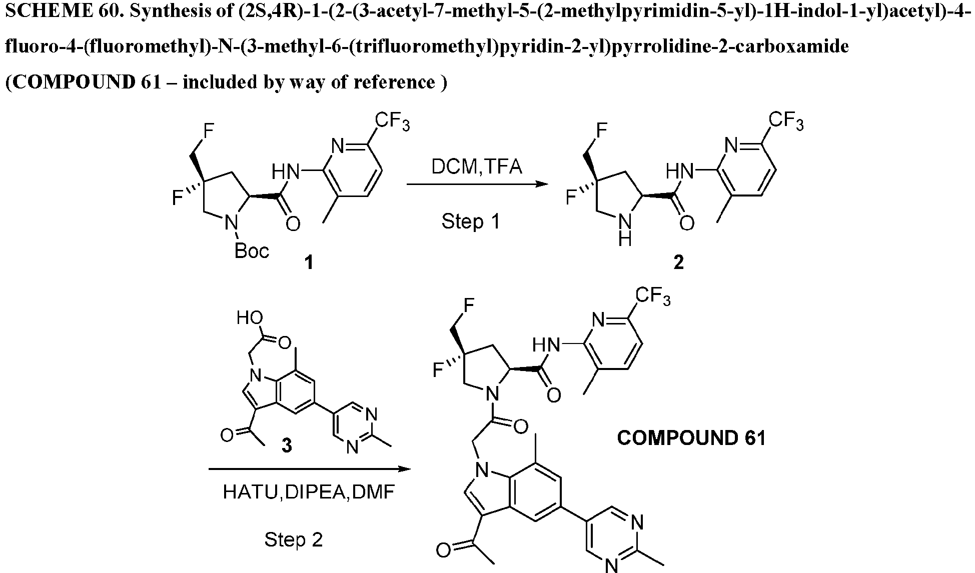

**Step 1:** To a solution of intermediate **1** (50.0 mg, 0.120 mmol) in DCM (4 mL) was added TFA (2 mL) at 0 °C and the reaction was stirred at room temperature for 2 hours. The mixture was concentrated to dryness to give intermediate **2** (38.0 mg, yield 100.0%) as a red solid. which was used to next step directly without purification. LC/MS (ESI) m/z: 324 (M+H)⁺.

**Step 2:** To a mixture of intermediate **2** (20.0 mg, 0.060 mmol) and compound **3** (20.0 mg, 0.060 mmol) in DMF (2 mL) was added DIPEA (0.05 mL, 0.300 mmol), followed by addition of HATU (34.0 mg, 0.090 mmol) at 0 °C. The mixture was stirred at room temperature overnight. The mixture was diluted with EtOAc and washed with saturated aq. NH₄Cl solution and brine, dried over anhydrous Na₂SO₄, filtered and concentrated to dryness. The residue was purified by prep-HPLC (DCM:MeOH=20:1) to give **COMPOUND 61** (3.5 mg, yield 9.2%) as a white solid. ¹H NMR (400 MHz, DMSO-*d*₆) δ 10.72 (s, 1H), 8.94 (s, 2H), 8.34 (d, *J =* 1.6 Hz, 1H), 8.26 (s, 1H), 7.93 (d, *J=* 8.0 Hz, 1H), 7.69 (d, *J =*7.6 Hz, 1H), 7.32 (d, *J =* 1.6 Hz, 1H), 5.59 - 5.44 (m, 2H), 5.01 - 4.72 (m, 3H), 4.25 - 3.94 (m, 2H), 2.69 - 2.64 (m, 4H), 2.62 (s, 3H), 2.45 (s, 3H), 2.37 - 2.29 (m, 1H), 2.15 (s, 3H).. LC/MS (ESI) m/z: 629 (M+H)⁺.

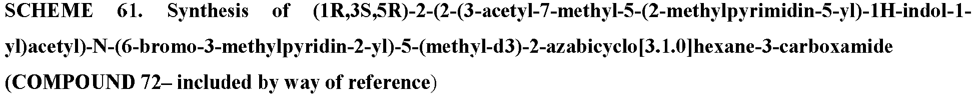

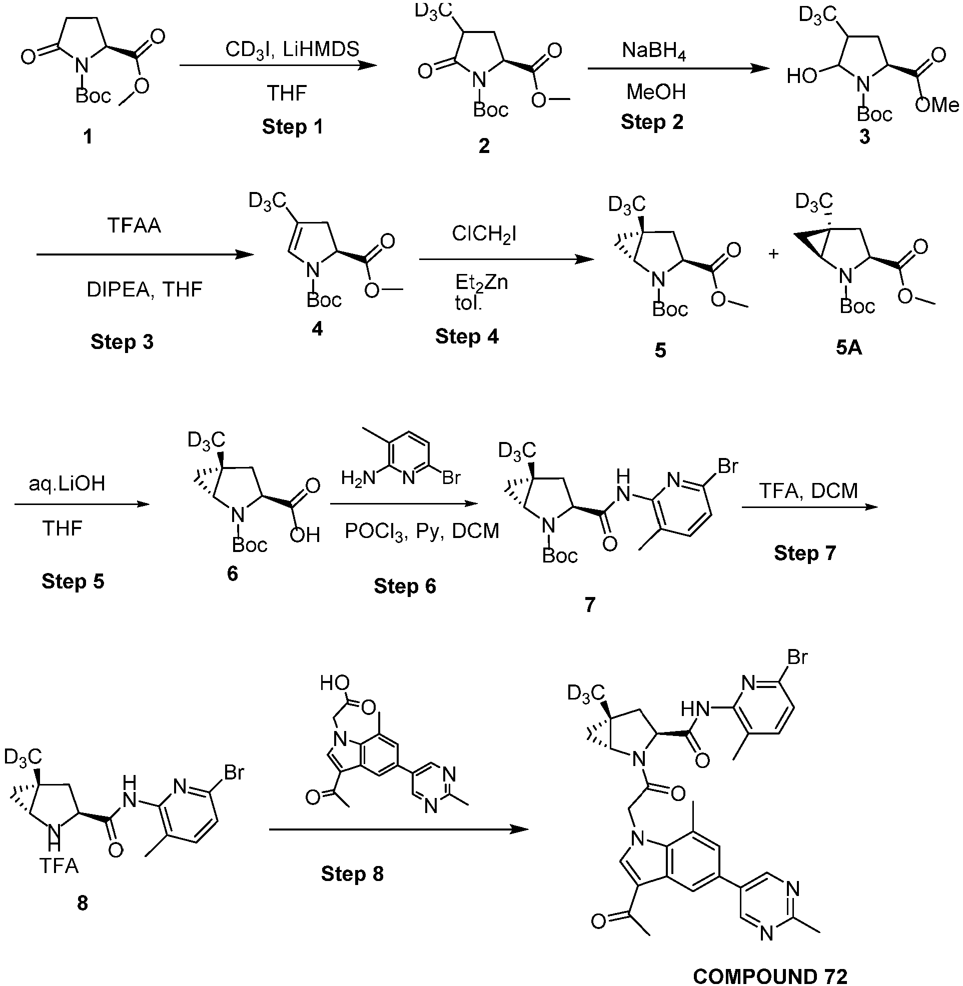

**Step 1:** To a solution of N-Boc-L-pyroglutamic acid methyl ester (86 g, 0.354 mol) in THF (500 mL) is added LiHMDS (354 mL, 1.0 M in THF) at -70 °C dropwise for 1 hr. The reaction is stirred at -70 °C for 45 min and iodomethane-d3 (100.5g, 0.708 mol) is added dropwise. The reaction is stirred at -70 °C for 2 hours and then at room temperature overnight. The reaction is quenched with acetic acid (50 mL) and water (500mL). The volatile is removed under reduced pressure and the mixture is extracted with EtOAc twice. The combined organic layers are washed with water and brine, dried over anhydrous Na₂SO₄, filtered and concentrated to dryness. The residue is purified by column chromatography on silica gel (eluted with petroleum ether: ethyl acetate = 5: 1 to 2: 1) to give intermediate **2.**

**Step 2:** To a solution of intermediate **2** (53g, 0.206 mol) in MeOH (500 mL) at -10 °C to -15 °C is added NaBH₄ (10.9 g, 0.29 mol) in small portions. The reaction is stirred at -15 °C for 6 hrs. and quenched by dropwise addition of water (300 mL). The volatile is removed under reduced pressure and the mixture is extracted with EtOAc twice. The combined organic phases are dried over anhydrous Na₂SO₄, filtered and concentrated to dryness to give intermediate **3** which is directly used in the next step without purification.

**Step 3:** To a solution of intermediate **3** (43 g, 166mmol) in THF (500 mL) is added DIPEA (118 g, 913 mmol) and the mixture is cooled to -65 °C. Trifluoroacetic acid anhydride (56 g,216 mmol) is added dropwise to the above mixture at -65 °C over 30 min. The reaction is stirred at -65 °C for 1 hrs. and then room temperature for 2 hrs. The reaction is quenched with water (400 mL). The volatile is removed under reduced pressure and the mixture is extracted with EtOAc twice. The combined organic phases are dried over anhydrous Na₂SO₄ and concentrated to dryness. The residue is purified by silica gel column chromatography (PE/ EtOAc = 15/1) to give intermediate **4.**

**Step 4:** Diethylzinc (1 M in toluene, 572mL, 572 mmol) is added dropwise over 20 min to a cooled (-23 °C) toluene (115 mL) solution of intermediate **4** (23 g, 95 mmol) and the reaction is stirred at -20 °C for 30 min. Chloroiodomethane (35g, 286 mmol) is added dropwise and the reaction mixture is stirred at -21 °C for 30 hrs. After addition of saturated aq. NaHCO₃ (100 mL) at -20 °C, the reaction mixture is stirred at room temperature for 10 min. The resulting mixture is filtered and the filter cake is washed with toluene. The filtrate is extracted with EtOAc and the organic layer is washed with brine, dried over anhydrous Na₂SO₄, filtered and concentrated to dryness to give crude product, which is purified by column chromatography on silica gel (eluted with petroleum ether: ethyl acetate = 60: 1 to 10: 1) to give intermediates **5** and **5A**

**Step 5:** To a solution of 2-(tert-butyl) 3-methyl (1R,3S,5R)-5-(methyl-d3)-2-azabicyclo [3.1.0] hexane-2,3-dicarboxylate (0.32 g, 1.19 mmol) in THF (5 mL) is added aq. LiOH solution (3 mL, 1M) and the reaction is stirred at room temperature for 3 hrs. The volatile is removed under reduced pressure and the mixture is washed with diethyl ether twice. The aqueous layer is acidified with citric acid and extracted with EtOAc twice. The combined organic layers are washed with water and brine, dried over anhydrous Na₂SO₄ and concentrated to give intermediate **6,** which is directly used in the next step.

**Step 6:** To a solution of (1R,3S,5R)-2-(tert-butoxycarbonyl)-5-(methyl-d3)-2-azabicyclo[3.1.0]hexane-3-carboxylic acid (0.15 g, 0.62 mmol) in DCM is added POCl₃ (1.1 equiv ), Pyridine (5 equiv, 1.87 mmol) and 6-bromopyridin-2-amine (0.11 g, 0.62 mmol) and the reaction is stirred at 0 °C under N₂ atmosphere 6 hrs. The mixture is diluted with EtOAc and washed with water and brine, dried over anhydrous Na₂SO₄ and concentrated. The residue is purified by column chromatography on silica gel eluted with PE/acetone (PE/EtOAc=20:1 to 3:1) to give intermediate **7.**

**Step 7:** To a solution of tert-butyl (1R,3S,5R)-3-((6-bromo-3-methylpyridin-2-yl) carbamoyl)-5-(methyl-d3)-2-azabicyclo[3.1.0]hexane-2-carboxylate (160 mg, 0.4 mmol) in DCM (6 mL) is added TFA (3 mL), then the reaction is stirred at room temperature for 1 h. The mixture is concentrated, and the residue is washed with Et₂O to give **8,** which is directly used to the reaction.

**Step 8:** To a solution of 2-(3-acetyl-7-methyl-5-(2-methylpyrimidin-5-yl)-1H-indol-1-yl)acetic acid (1 equiv) in DMF (10 vol) at 0 °C under nitrogen atmosphere is added (1R,3S,5R)-N-(6-bromo-3-methylpyridin-2-yl)-5-(methyl-d3)-2-azabicyclo[3.1.0]hexane-3-carboxamide TFA salt (8, 1 equiv), HATU (2.1 equiv), and DIPEA (5 equiv). The reaction mixture is stirred at room temperature for 3 h and then quenched with water (30 vol). The resulting mixture is extracted with DCM. The organic layer is washed with brine, dried over anhydrous Na2SO4, filtered, and then concentrated. The residue is purified by column chromatography on silica gel (MeOH/DCM) to give (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-(methyl-d3)-2-azabicyclo[3.1.0]hexane-3-carboxamide.

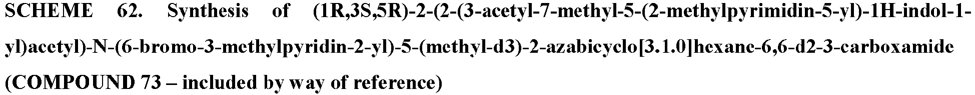

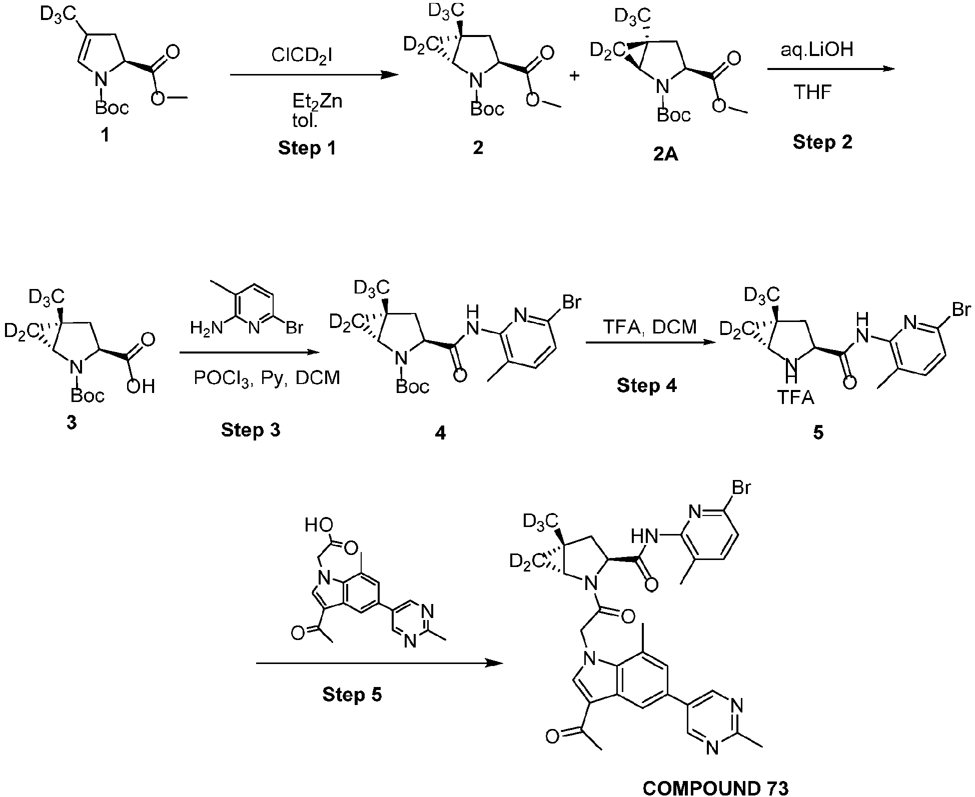

**Step 1:** Diethylzinc (1 M in toluene, 572mL, 572 mmol) is added dropwise over 20 min to a cooled (-23 °C) toluene (115 mL) solution of intermediate **1** (23 g, 95 mmol) and the reaction is stirred at -20 °C for 30 min. chloroiodomethane-d2 (35g, 286 mmol) is added dropwise and the reaction mixture is stirred at -21 °C for 30 hrs. After addition of saturated aq. NaHCO₃ (100 mL) at -20 °C, the reaction mixture is stirred at room temperature for 10 min. The resulting mixture is filtered and the filter cake is washed with toluene. The filtrate is extracted with EtOAc and the organic layer is washed with brine, dried over anhydrous Na₂SO₄, filtered and concentrated to dryness to give crude product, which is purified by column chromatography on silica gel (eluted with petroleum ether: ethyl acetate = 60: 1 to 10: 1) to give intermediates **2** and **2A**

**Step 2:** To a solution of 2-(tert-butyl) 3-methyl (1R,3S,5R)-5-(methyl-d3)-2-azabicyclo[3.1.0]hexane-2,3-dicarboxylate-6,6-d2 (0.32 g, 1.19 mmol) in THF (5 mL) is added aq. LiOH solution (3 mL, 1M) and the reaction is stirred at room temperature for 3 hrs. The volatile is removed under reduced pressure and the mixture is washed with diethyl ether twice. The aqueous layer is acidified with citric acid and extracted with EtOAc twice. The combined organic layers are washed with water and brine, dried over anhydrous Na₂SO₄ and concentrated to give intermediate **3,** which is directly used in the next step.

**Step 3:** To a solution of (1R,3S,5R)-2-(tert-butoxycarbonyl)-5-(methyl-d3)-2-azabicyclo[3.1.0]hexane-3-carboxylic-6,6-d2 acid (0.15 g, 0.62 mmol) in DCM is added POCl₃ (1.1 equiv ), Pyridine (5 equiv, 1.87 mmol) and 6-bromopyridin-2-amine (0.11 g, 0.62 mmol) and the reaction is stirred at 0 °C under N₂ atmosphere 6 hrs. The mixture is diluted with EtOAc and washed with water and brine, dried over anhydrous Na₂SO₄, and concentrated. The residue is purified by column chromatography on silica gel eluted with PE/acetone (PE/EtOAc=20:1 to 3:1) to give intermediate **4.**

**Step 4:** To a solution of tert-butyl (1R,3S,5R)-3-((6-bromo-3-methylpyridin-2-yl) carbamoyl)-5-(methyl-d3)-2-azabicyclo[3.1.0]hexane-2-carboxylate-6,6-d2 (160 mg, 0.4 mmol) in DCM (6 mL) is added TFA (3 mL), then the reaction is stirred at room temperature for 1 h. The mixture is concentrated, and the residue is washed with Et₂O to give **5,** which is directly used in the reaction.

**Step 5:** To a solution of 2-(3-acetyl-7-methyl-5-(2-methylpyrimidin-5-yl)-1H-indol-1-yl)acetic acid (1 equiv) in DMF (10 vol) at 0 °C under an atmosphere nitrogen atmosphere is added (1R,3S,5R)-N-(6-bromo-3-methylpyridin-2-yl)-5-(methyl-d3)-2-azabicyclo[3.1.0]hexane-6,6-d2-3-carboxamide TFA salt (5, 1 equiv), HATU (2.1 equiv), and DIPEA (5 equiv). The reaction mixture is stirred at room temperature for 3 h and then quenched with water (30 vol). The resulting mixture is extracted with DCM. The organic layer is washed with brine, dried over anhydrous Na2SO4, filtered, and then concentrated. The residue is purified by column chromatography on silica gel (MeOH/DCM) to give (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-(methyl-d3)-2-azabicyclo[3.1.0]hexane-6,6-d2-3-carboxamide.

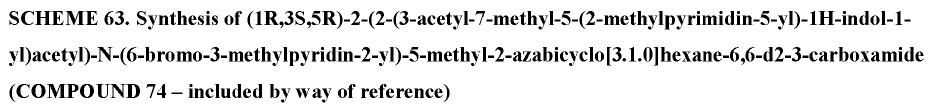

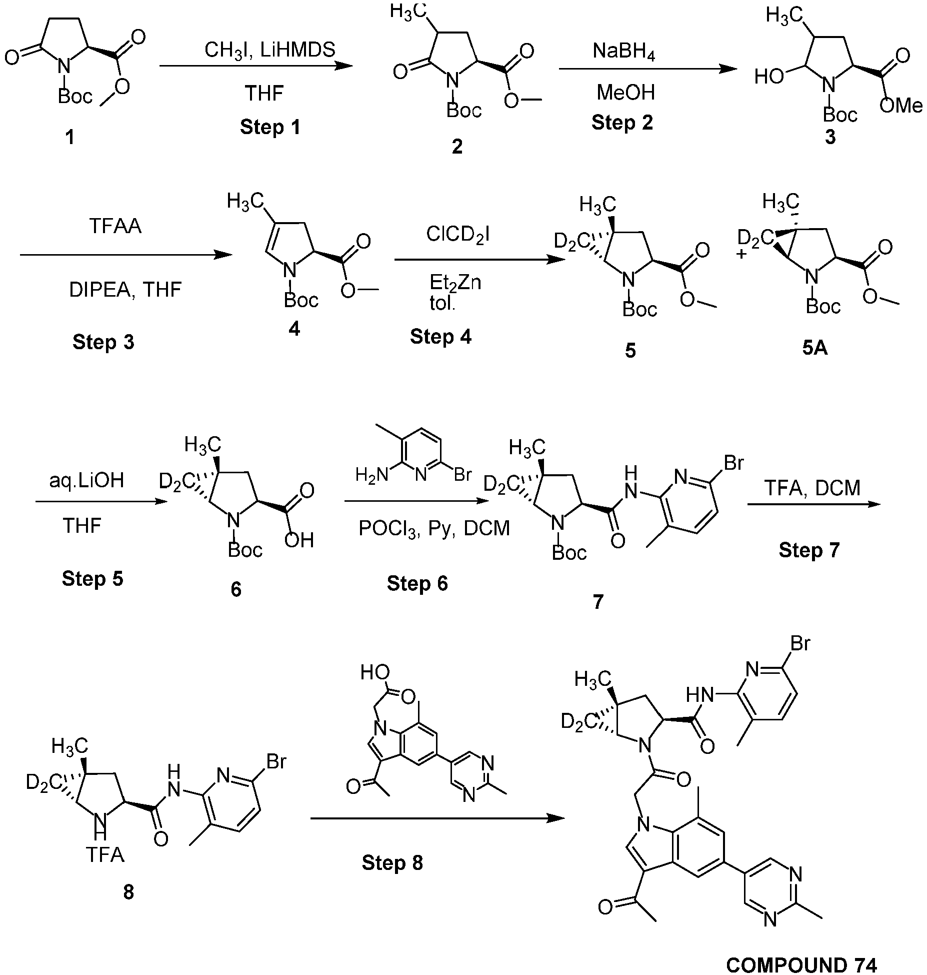

**Step 1:** To a solution of N-Boc-L-pyroglutamic acid methyl ester (86 g, 0.354 mol) in THF (500 mL) is added LiHMDS (354 mL, 1.0 M in THF) at -70 °C dropwise for 1 hr. The reaction is stirred at -70 °C for 45 min and iodomethane-d3 (100.5g, 0.708 mol) is added dropwise. The reaction is stirred at -70 °C for 2 hours and then at room temperature overnight. The reaction is quenched with acetic acid (50 mL) and water (500mL). The volatiles are removed under reduced pressure and the mixture is extracted with EtOAc twice. The combined organic layers are washed with water and brine, dried over anhydrous Na₂SO₄, filtered and concentrated to dryness. The residue is purified by column chromatography on silica gel (eluted with petroleum ether: ethyl acetate = 5: 1 to 2: 1) to give intermediate **2.**

**Step 2:** To a solution of intermediate **2** (53g, 0.206 mol) in MeOH (500 mL) at -10 °C to -15 °C is added NaBH₄ (10.9 g, 0.29 mol) in small portions. The reaction is stirred at -15 °C for 6 hrs. and quenched by dropwise addition of water (300 mL). The volatile is removed under reduced pressure and the mixture is extracted with EtOAc twice. The combined organic phases are dried over anhydrous Na₂SO₄, filtered and concentrated to dryness to give the intermediate **3,** which is directly used in the next step without purification.

**Step 3:** To a solution of intermediate **3** (43 g, 166mmol) in THF (500 mL) is added DIPEA (118 g, 913 mmol) and the mixture is cooled to -65 °C. Trifluoroacetic acid anhydride (56 g,216 mmol) is added dropwise to the above mixture at -65 °C over 30 min. The reaction is stirred at -65 °C for 1 hrs. and then room temperature for 2 hrs. The reaction is quenched with water (400 mL). The volatile is removed under reduced pressure and the mixture is extracted with EtOAc twice. The combined organic phases are dried over anhydrous Na₂SO₄ and concentrated to dryness. The residue is purified by silica gel column chromatography (PE/ EtOAc = 15/1) to give the intermediate **4.**

**Step 4:** Diethylzinc (1 M in toluene, 572mL, 572 mmol) is added dropwise over 20 min to a cooled (-23 °C) toluene (115 mL) solution of intermediate **4** (23 g, 95 mmol) and the reaction is stirred at -20 °C for 30 min. chloroiodomethane-d2 (35g, 286 mmol) is added dropwise and the reaction mixture is stirred at -21 °C for 30 hrs. After addition of saturated aq. NaHCO₃ (100 mL) at -20 °C, the reaction mixture is stirred at room temperature for 10 min. The resulting mixture is filtered and the filter cake is washed with toluene. The filtrate is extracted with EtOAc and the organic layer is washed with brine, dried over anhydrous Na₂SO₄, filtered and concentrated to dryness to give crude product, which is purified by column chromatography on silica gel (eluted with petroleum ether: ethyl acetate = 60: 1 to 10: 1) to give intermediate **5** and **5A**

**Step 5:** To a solution of 2-(tert-butyl) 3-methyl (1R,3S,5R)-5-methyl-2-azabicyclo[3.1.0]hexane-2,3-dicarboxylate-6,6-d2 (0.32 g, 1.19 mmol) in THF (5 mL) is added aq. LiOH solution (3 mL, 1M) and the reaction is stirred at room temperature for 3 hrs. The volatile is removed under reduced pressure and the mixture is washed with diethyl ether twice. The aqueous layer is acidified with citric acid and extracted with EtOAc twice. The combined organic layers are washed with water and brine, dried over anhydrous Na₂SO₄ and concentrated to give intermediate **6,** which is directly used in the next step.

**Step 6:** To a solution of ((1R,3S,5R)-2-(tert-butoxycarbonyl)-5-methyl-2-azabicyclo[3.1.0]hexane-3-carboxylic-6,6-d2 acid (0.15 g, 0.62 mmol) in DCM is added POCl₃ (1.1 equiv ), Pyridine (5 equiv, 1.87 mmol) and 6-bromopyridin-2-amine (0.11 g, 0.62 mmol) and the reaction is stirred at 0 °C under N₂ atmosphere 6 hrs. The mixture is diluted with EtOAc and washed with water and brine, dried over anhydrous Na₂SO₄ and concentrated. The residue is purified by column chromatography on silica gel eluted with PE/acetone (PE/EtOAc=20:1 to 3:1) to give intermediate 7.

**Step 7:** To a solution of tert-butyl (1R,3S,5R)-3-((6-bromo-3-methylpyridin-2-yl)carbamoyl)-5-methyl-2-azabicyclo[3.1.0]hexane-2-carboxylate-6,6-d2 (160 mg, 0.4 mmol) in DCM (6 mL) is added TFA (3 mL), then the reaction is stirred at room temperature for 1 h. The mixture is concentrated, and the residue is washed with Et₂O to give **8,** which is directly used to the reaction.

**Step 8:** To a solution of 2-(3-acetyl-7-methyl-5-(2-methylpyrimidin-5-yl)-1H-indol-1-yl)acetic acid (1 equiv) in DMF (10 vol) at 0 °C under an atmosphere nitrogen atmosphere is added (1R,3S,5R)-N-(6-bromo-3-methylpyridin-2-yl)-5-methyl-2-azabicyclo [3.1.0]hexane-6,6-d2-3-carboxamide TFA salt (8, 1 equiv), HATU (2.1 equiv), and DIPEA (5 equiv). The reaction mixture is stirred at room temperature for 3 h and then quenched with water (30 vol). The resulting mixture is extracted with DCM. The organic layer is washed with brine, dried over anhydrous Na2SO4, filtered, and then concentrated. The residue is purified by column chromatography on silica gel (MeOH/DCM) to give (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-methyl-2-azabicyclo[3.1.0]hexane-6,6-d2-3-carboxamide.

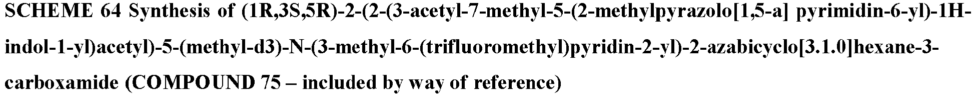

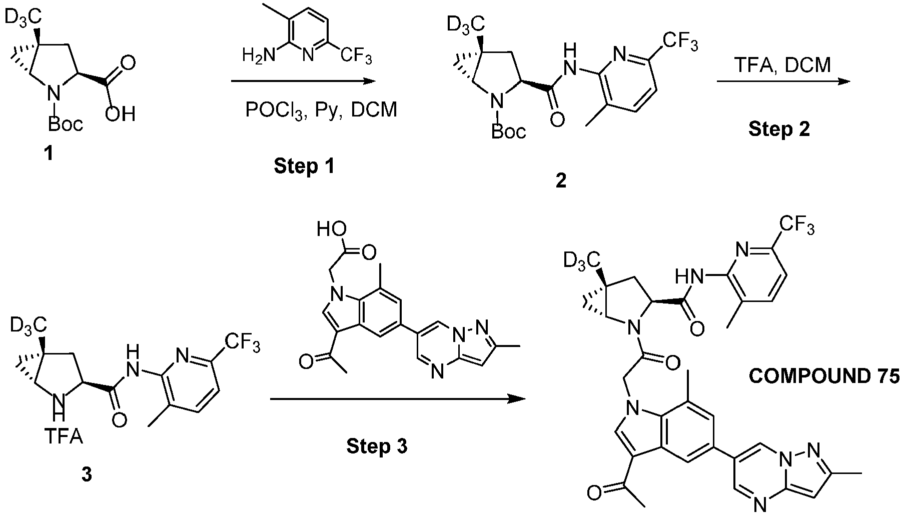

**Step 1:** To a solution of (1R,3S,5R)-2-(tert-butoxycarbonyl)-5-(methyl-d3)-2-azabicyclo[3.1.0]hexane-3-carboxylic acid (0.15 g, 0.62 mmol) in DCM is added POCl₃ (1.1 equiv ), Pyridine (5 equiv, 1.87 mmol) and 3-methyl-6-(trifluoromethyl)pyridin-2-amine (0.11 g, 0.62 mmol) and the reaction is stirred at 0 °C under N₂ atmosphere 6 hrs. The mixture is diluted with EtOAc and washed with water and brine, dried over anhydrous Na₂SO₄ and concentrated. The residue is purified by column chromatography on silica gel eluted with PE/acetone (PE/EtOAc=20:1 to 3:1) to give intermediate **2.**

**Step 2:** To a solution of tert-butyl (1R,3S,5R)-5-(methyl-d3)-3-((3-methyl-6-(trifluoromethyl)pyridin-2-yl)carbamoyl)-2-azabicyclo[3.1.0]hexane-2-carboxylate (160 mg, 0.4 mmol) in DCM (6 mL) is added TFA (3 mL), then the reaction is stirred at room temperature for 1 h. The mixture is concentrated, and the residue is washed with Et₂O to give **3,** which is directly used to the reaction.

**Step 3:** To a solution of 2-(3-acetyl-7-methyl-5-(2-methylpyrazolo[1,5-a]pyrimidin-6-yl)-1H-indol-1-yl)acetic acid (1 equiv) in DMF (10 vol) at 0 °C under an atmosphere nitrogen atmosphere is added (1R,3S,5R)-5-(methyl-d3)-N-(3-methyl-6-(trifluoromethyl)pyridin-2-yl)-2-azabicyclo[3.1.0]hexane-3-carboxamide TFA salt (3, 1 equiv), HATU (2.1 equiv), and DIPEA (5 equiv). The reaction mixture is stirred at room temperature for 3 h and then quenched with water (30 vol). The resulting mixture is extracted with DCM. The organic layer is washed with brine, dried over anhydrous Na2SO4, filtered, and then concentrated. The residue is purified by column chromatography on silica gel (MeOH/DCM) to give (1R,3S,5R)-2-(2-(3-acetyl-7-methyl-5-(2-methylpyrazolo[1,5-a]pyrimidin-6-yl)-1H-indol-1-yl)acetyl)-5-(methyl-d3)-N-(3-methyl-6-(trifluoromethyl)pyridin-2-yl)-2-azabicyclo[3.1.0]hexane-3-carboxamide.

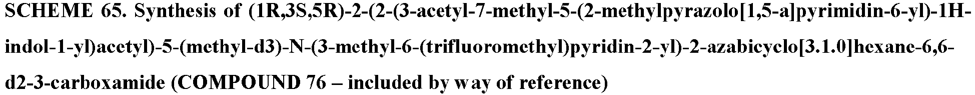

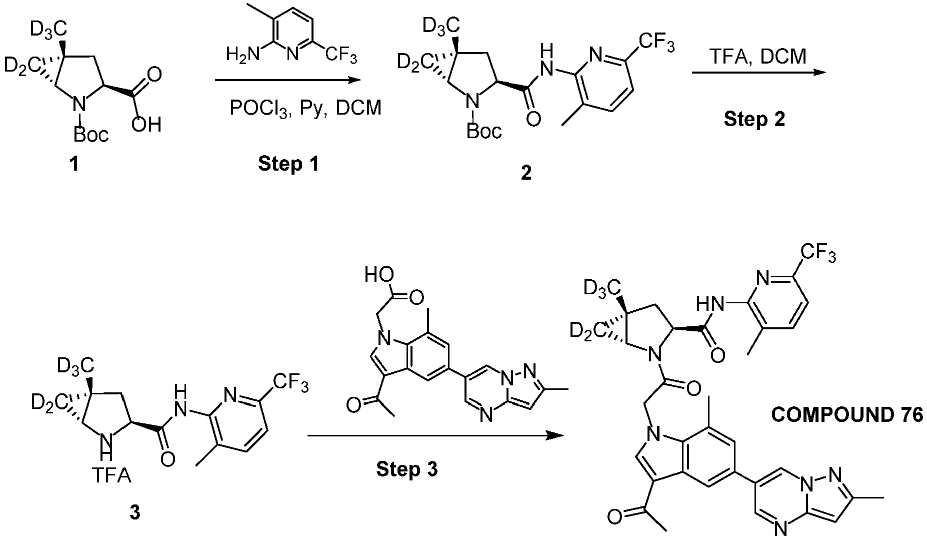

**Step 1:** To a solution of (1R,3S,5R)-2-(tert-butoxycarbonyl)-5-(methyl-d3)-2-azabicyclo[3.1.0]hexane-3-carboxylic-6,6-d2 acid (0.15 g, 0.62 mmol) in DCM is added POCl₃ (1.1 equiv ), Pyridine (5 equiv, 1.87 mmol) and 3-methyl-6-(trifluoromethyl)pyridin-2-amine (0.11 g, 0.62 mmol) and the reaction is stirred at 0 °C under N₂ atmosphere 6 hrs. The mixture is diluted with EtOAc and washed with water and brine, dried over anhydrous Na₂SO₄ and concentrated. The residue is purified by column chromatography on silica gel eluted with PE/acetone (PE/EtOAc=20:1 to 3:1) to give intermediate **2.**

**Step 2:** To a solution of tert-butyl (1R,3S,5R)-5-(methyl-d3)-3-((3-methyl-6-(trifluoromethyl)pyridin-2-yl)carbamoyl)-2-azabicyclo[3.1.0]hexane-2-carboxylate-6,6-d2 (160 mg, 0.4 mmol) in DCM (6 mL) is added TFA (3 mL), then the reaction is stirred at room temperature for 1 h. The mixture is concentrated, and the residue is washed with Et₂O to give **5,** which is directly used to the reaction.

**Step 3:** To a solution of 2-(3-acetyl-7-methyl-5-(2-methylpyrazolo[1,5-a]pyrimidin-6-yl)-1H-indol-1-yl)acetic acid (1 equiv) in DMF (10 vol) at 0 °C under an atmosphere nitrogen atmosphere is added (1R,3S,5R)-5-(methyl-d3)-N-(3-methyl-6-(trifluoromethyl)pyridin-2-yl)-2-azabicyclo[3.1.0]hexane-6,6-d2-3-carboxamideTFA salt (3, 1 equiv), HATU (2.1 equiv), and DIPEA (5 equiv). The reaction mixture is stirred at room temperature for 3 h and then quenched with water (30 vol). The resulting mixture is extracted with DCM. The organic layer is washed with brine, dried over anhydrous Na2SO4, filtered, and then concentrated. The residue is purified by column chromatography on silica gel (MeOH/DCM) to give (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-(methyl-d3)-2-azabicyclo[3.1.0]hexane-6,6-d2-3-carboxamide.

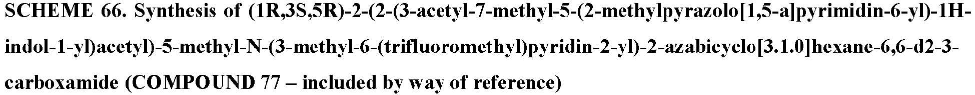

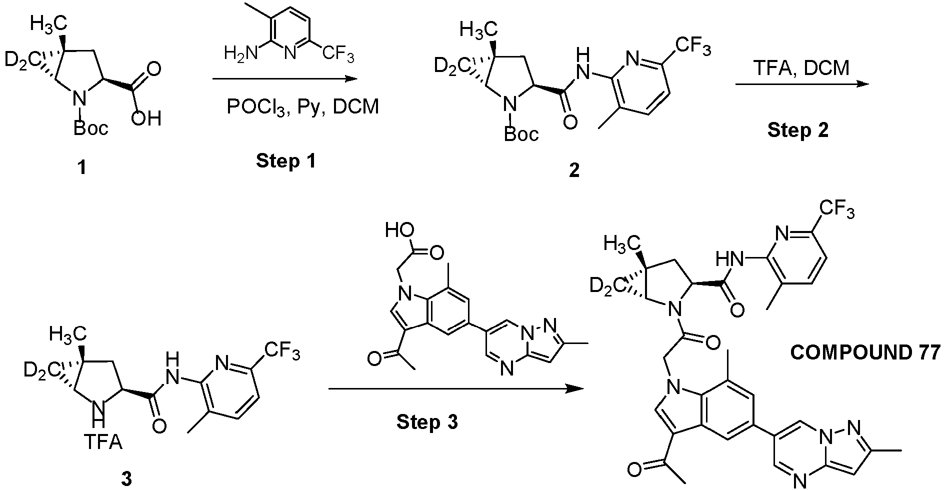

**Step 1:**To a solution of ((1R,3S,5R)-2-(tert-butoxycarbonyl)-5-methyl-2-azabicyclo[3.1.0]hexane-3-carboxylic-6,6-d2 acid (0.15 g, 0.62 mmol) in DCM is added POCl₃ (1.1 equiv ), Pyridine (5 equiv, 1.87 mmol) and 3-methyl-6-(trifluoromethyl)pyridin-2-amine (0.11 g, 0.62 mmol) and the reaction is stirred at 0 °C under N₂ atmosphere 6 hrs. The mixture is diluted with EtOAc and washed with water and brine, dried over anhydrous Na₂SO₄ and concentrated. The residue is purified by column chromatography on silica gel eluted with PE/acetone (PE/EtOAc=20:1 to 3:1) to give intermediate **2.**

**Step 2:** To a solution of tert-butyl (1R,3S,5R)-5-methyl-3-((3-methyl-6-(trifluoromethyl)pyridin-2-yl)carbamoyl)-2-azabicyclo[3.1.0]hexane-2-carboxylate-6,6-d2 (160 mg, 0.4 mmol) in DCM (6 mL) is added TFA (3 mL), then the reaction is stirred at room temperature for 1 h. The mixture is concentrated, and the residue is washed with Et₂O to give **8,** which is directly used to the reaction.

**Step 3:** To a solution of 2-(3-acetyl-7-methyl-5-(2-methylpyrazolo[1,5-a]pyrimidin-6-yl)-1H-indol-1-yl)acetic acid (1 equiv) in DMF (10 vol) at 0 °C under an atmosphere nitrogen atmosphere is added (1R,3S,5R)-5-methyl-N-(3-methyl-6-(trifluoromethyl)pyridin-2-yl)-2-azabicyclo[3.1.0]hexane-6,6-d2-3-carboxamide TFA salt (3, 1 equiv), HATU (2.1 equiv), and DIPEA (5 equiv). The reaction mixture is stirred at room temperature for 3 h and then quenched with water (30 vol). The resulting mixture is extracted with DCM. The organic layer is washed with brine, dried over anhydrous Na2SO4, filtered, and then concentrated. The residue is purified by column chromatography on silica gel (MeOH/DCM) to give (1R,3S,5R)-2-(2-(3-acetyl-7-methyl-5-(2-methylpyrazolo[1,5-a]pyrimidin-6-yl)-1H-indol-1-yl)acetyl)-5-methyl-N-(3-methyl-6-(trifluoromethyl)pyridin-2-yl)-2-azabicyclo[3.1.0]hexane-6,6-d2-3-carboxamide.

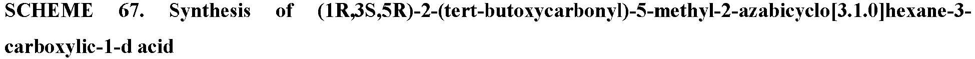

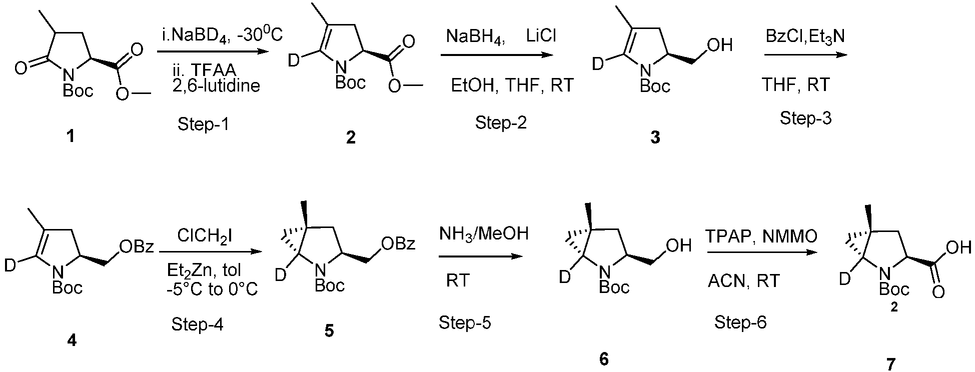

**Step 1: 1-(tert-Butyl) 2-methyl (S)-4-methyl-2,3-dihydro-1H-pyrrole-1,2-dicarboxylate-5-d (2):** To a solution of 1-(tert-butyl) 2-methyl (2S)-4-methyl-5-oxopyrrolidine-1,2-dicarboxylate (1 equiv) in dry methanol-d (10 vol) at -30 °C was added sodium borodeuteride (1.5 equiv). The reaction mixture was stirred at -15 °C for 5 hours. D₂O was added to the reaction mixture and the resulting mixture was extracted with ethyl acetate. The organic layer was separated, dried over anhydrous Na₂SO₄, filtered, concentrated, and the resulting residue was used as such for the next step.

To a solution of above residue in dry toluene (10 vol) at 0 °C was added 2,6-lutidine (1.5 equiv) and trifluoroacetic anhydride (1 equiv). The resultant mixture was stirred at 80 °C for 2 hours and cooled to 0 °C before being quenched with water. Water was added to the reaction mixture and the resulting mixture was extracted with ethyl acetate. The organic layer was separated, dried over anhydrous Na₂SO₄, filtered and concentrated. The residue was purified by column chromatography on silica gel using hexane/EtOAc to afford intermediate **2.**

**Step 2: tert-Butyl (S)-2-(hydroxymethyl)-4-methyl-2,3-dihydro-1H-pyrrole-1-carboxylate-5-d (3):** To a mixture of sodium borohydride (3 equiv) and lithium chloride (3 equiv) in EtOH (15 vol) at 0 °C was added a solution of intermediate **2** (1 equiv) in THF (15 vol). The reaction mixture was stirred at room temperature for 16 hours. After completion of the reaction, the reaction mixture was quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **3.**

**Step 3: tert-Butyl (S)-2-((benzoyloxy)methyl)-4-methyl-2,3-dihydro-1H-pyrrole-1-carboxylate-5-d (4):** To a solution of intermediate **3** (1 equiv) in THF (10 vol) at 0 °C under nitrogen atmosphere was added triethylamine (3 equiv) and benzoyl chloride (1.1 equiv). The reaction mixture was stirred at room temperature for 2 hours. After completion of the reaction, the reaction mixture was quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **4.**

**Step 4: tert-Butyl (1R,3S,5R)-3-((benzoyloxy)methyl)-5-methyl-2-azabicyclo[3.1.0]hexane-2-carboxylate-1-d (5):** To a solution of intermediate **4** (1 equiv) in dry toluene (20 vol) at - 25 °C was added diethylzinc (3 equiv, 1.1 M in toluene, Aldrich). The reaction mixture was stirred at - 25 °C for 1 hour. Chloroiodomethane (6 equiv) was added and the mixture was stirred at 0 °C to - 5 °C for 2 hours. After completion of the reaction, the reaction mixture was poured into saturated aqueous NaHCO₃. The resulting mixture was stirred at room temperature for 20 minutes, filtered through celite, and washed with EtOAc. The organic layer was separated, dried over anhydrous Na₂SO₄, filtered and concentrated. The residue was purified by column chromatography on silica gel using hexane/EtOAc to afford intermediate **5.**

**Step 5: tert-Butyl (1R,3S,5R)-3-(hydroxymethyl)-5-methyl-2-azabicyclo[3.1.0]hexane-2-carboxylate-1-d (6):** To a solution of intermediate **5** (1 equiv) in MeOH (5 vol) at - 30 °C was added saturated methanolic ammonia solution (30 vol). The reaction mixture was stirred at room temperature for 48 hours. After completion of the reaction, solvent was removed under reduced pressure to afford intermediate **6.**

**Step 6: (1R,3S,5R)-2-(tert-Butoxycarbonyl)-5-methyl-2-azabicyclo[3.1.0]hexane-3-carboxylic-1-d acid (7):** To a solution of intermediate **6** (1 equiv) in acetonitrile (20 vol) at 0 °C was added N-methylmorpholine-N-oxide (10 equiv) and tetrapropylammonium perruthenate (0.1 equiv). The reaction mixture was stirred at room temperature for 16 hours. After completion of the reaction, 5% LiOH was added to the resulting mixture (pH=10~12) and aqueous layer was washed with DCM (3 times). Aqueous layer was acidified with 1.5 N HCl (pH=2~3) and then extracted with DCM. The organic layer was separated, dried over anhydrous Na₂SO₄, filtered and concentrated to afford intermediate 7 which can be used in the synthesis of deuterated compounds of the present disclosure.

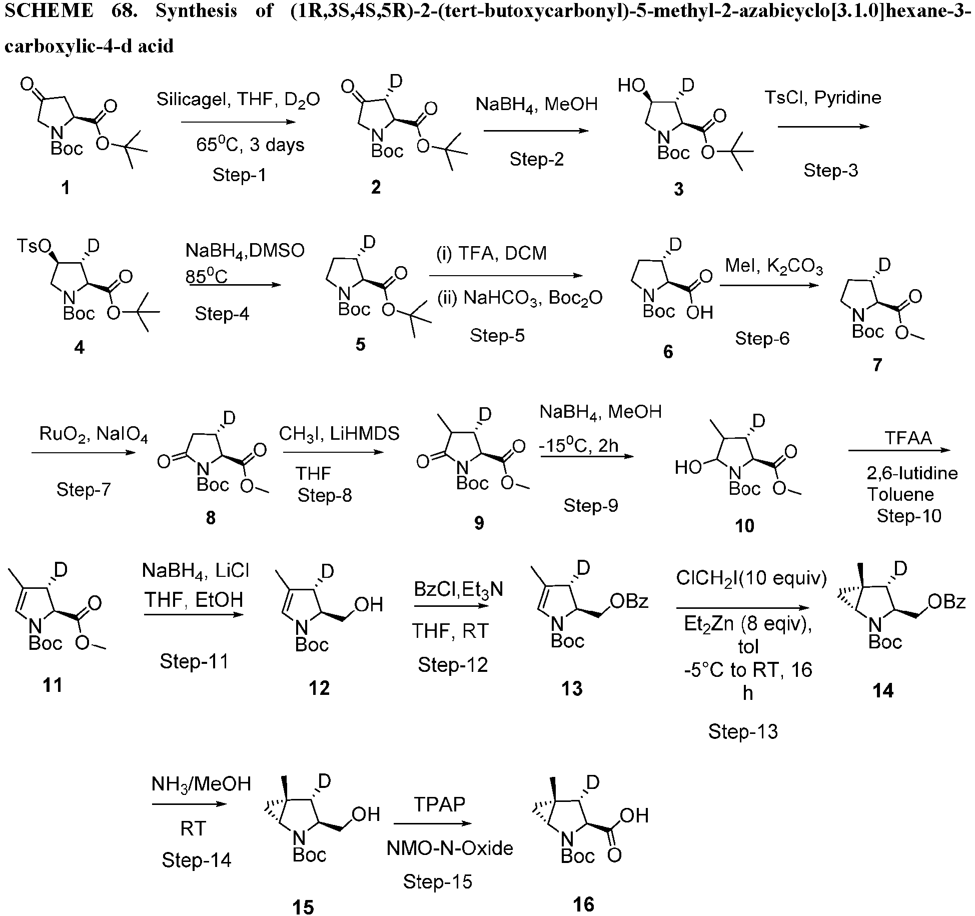

**Step** 1: **di-tert-Butyl (2S,3R)-4-oxopyrrolidine-1,2-dicarboxylate-3-d** (2): To a solution of di-tert-butyl (S)-4-oxopyrrolidine-1,2-dicarboxylate (1 equiv) in D₂O (3 vol) and THF (1 vol) was added silica gel (60 -120 mesh, prewashed with D₂O). The reaction mixture was stirred at 70 °C for 3 days. After completion of the reaction, the reaction mixture was filtered and concentrated, and the resulting residue (intermediate 2) was used as such for the next step.

**Step 2: di-tert-Butyl (2S,3R,4S)-4-hydroxypyrrolidine-1,2-dicarboxylate-3-d (3):** To a mixture of sodium borohydride (2 equiv) in MeOH (10 vol) and diethyl ether (13 vol) at 0 °C was added a solution of intermediate **2** (1 equiv). The reaction mixture was stirred at room temperature for 1 hour. After completion of the reaction, the reaction mixture was quenched with water. The resulting mixture was extracted with EtOAc. The organic layer was washed with brine, dried over anhydrous Na₂SO₄, filtered, and concentrated. The residue was purified by column chromatography on silica gel using hexane/EtOAc to afford intermediate **3.**

**Step 3: di-tert-Butyl (2S,3R,4S)-4-(tosyloxy)pyrrolidine-1,2-dicarboxylate-3-d (4):** To a solution of intermediate 3 (1 equiv) in pyridine (16.56 equiv) at 0 °C was added p-toluenesulfonyl chloride (3.37 equiv). The reaction mixture was stirred at room temperature for 3 hours. After completion of the reaction, the reaction mixture was quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **4.**

**Step 4: di-tert-Butyl (2S,3S)-pyrrolidine-1,2-dicarboxylate-3-d (5):** To a mixture of sodium borohydride (6.08 equiv) in DMSO (20 vol) was added a solution of intermediate **4** (1 equiv). The reaction mixture was stirred at 85 °C for 4 hours. After completion of the reaction, the reaction mixture was quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **5.**

**Step 5: (2S,3S)-1-(tert-Butoxycarbonyl)pyrrolidine-2-carboxylic-3-d acid (6):** To a solution of intermediate **5** (1 equiv) in DCM (10 vol) at 0 °C was added TFA (5 vol). The reaction mixture was stirred at 50 °C for 3 hours and concentrated to afford deboc residue. To a solution of deboc residue (1 equiv) in THF (10 vol) at 0 °C under nitrogen atmosphere was added NaHCO₃ (5 equiv). Boc anhydride (1.1 equiv) was added and the mixture was stirred at room temperature for 16 hours. After completion of the reaction, water was added, and the resulting mixture was extracted with ethyl acetate. The organic layers were separated, and the aqueous layer was acidified with 1.5N HCl (pH=5~6), extracted with EtOAc, dried over anhydrous Na₂SO₄, filtered and concentrated to afford intermediate **6.**

**Step 6: 1-(tert-Butyl) 2-methyl (2S,3S)-pyrrolidine-1,2-dicarboxylate-3-d (7):** To a solution of intermediate **6** (1 equiv) in DMF (10 vol) was added potassium carbonate (3.44 equiv) and methyl iodide (2 equiv). The reaction mixture was stirred at room temperature for 16 hours and then quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **7.**

**Step 7: 1-(tert-Butyl) 2-methyl (2S,3S)-5-oxopyrrolidine-1,2-dicarboxylate-3-d (8):** To a solution of intermediate 7 (1 equiv) in 10% NaIO₄ solution in EtOAc (15 vol) was added RuO₂ (0.14 equiv). The reaction mixture was stirred at room temperature for 4 hours and quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **8.**

**Step 8: 1-(tert-Butyl) 2-methyl (2S,3S)-4-methyl-5-oxopyrrolidine-1,2-dicarboxylate-3-d (9):** To a solution of intermediate **8** (1 equiv) in dry THF (10 vol) at -78 °C was added LiHMDS (1.03 equiv, 1.0 M in THF, Aldrich). The reaction mixture was stirred at -78 °C for 1 hour and then CH₃I (2 equiv) was added and the mixture was stirred at -78 °C for 2 hours. Water was added and the resulting mixture was extracted with ethyl acetate. The organic layer was separated, dried over anhydrous Na₂SO₄, filtered and concentrated. The residue was purified by column chromatography on silica gel using hexane/EtOAc to afford intermediate **9.**

**Step 9: 1-(tert-Butyl) 2-methyl (2S,3S)-5-hydroxy-4-methylpyrrolidine-1,2-dicarboxylate-3-d (10):** To a mixture of sodium borohydride (1.5 equiv) in MeOH (10 vol) was added a solution of intermediate **9** (1 equiv). The reaction mixture was stirred at -15 °C for 2 hours. After completion of the reaction, the reaction mixture was quenched with water and extracted with EtOAc. The organic layer was washed with brine, dried over anhydrous Na₂SO₄, filtered and concentrated. The resulting residue (intermediate **10**) was used as such for the next step.

**Step 10: 1-(tert-Butyl) 2-methyl (2S,3S)-4-methyl-2,3-dihydro-1H-pyrrole-1,2-dicarboxylate-3-d (11):** To a solution of intermediate **10** (1 equiv) in dry toluene (10 vol) at 0 °C was added 2,6-lutidine (1.5 equiv) and trifluoroacetic anhydride (1 equiv). The resultant mixture was stirred at 85 °C for 1 hour, cooled to 0 °C, and quenched with water. Water was added and the resulting mixture was extracted with ethyl acetate. The organic layer was separated, dried over anhydrous Na₂SO₄, filtered and concentrated. The residue was purified by column chromatography on silica gel using hexane/EtOAc to afford intermediate **11.**

**Step 11: tert-Butyl (2S,3S)-2-(hydroxymethyl)-4-methyl-2,3-dihydro-1H-pyrrole-1-carboxylate-3-d (12)** : To a mixture of sodium borohydride (3 equiv) and lithium chloride (3 equiv) in EtOH (15 vol) at 0 °C was added a solution of intermediate **11** (1 equiv) in THF (15 vol). The reaction mixture was stirred at room temperature for 16 hours. After completion of the reaction, the reaction mixture was quenched with water and extracted with EtOAc. The organic layer was washed with brine, dried over anhydrous Na₂SO₄, filtered and concentrated to afford intermediate **12.**

**Step 12: tert-Butyl (2S,3S)-2-((benzoyloxy)methyl)-4-methyl-2,3-dihydro-1H-pyrrole-1-carboxylate-3-d (13):** To a solution of intermediate **12** (1 equiv) in THF (10 vol) at 0 °C under nitrogen atmosphere was added triethylamine (3 equiv) and benzoyl chloride (1.5 equiv). The reaction mixture was stirred at room temperature for 16 hours. After completion of the reaction, the reaction mixture was quenched with water. The resulting mixture was extracted with EtOAc. The organic layer was washed with brine, dried over anhydrous Na₂SO₄, filtered and concentrated. The residue was purified by column chromatography on silica gel using hexane/EtOAc to afford intermediate **13.**

**Step 13: tert-Butyl (1R,3S,4S,5R)-3-((benzoyloxy)methyl)-5-methyl-2-azabicyclo[3.1.0]hexane-2-carboxylate-4-d (14):** To a solution of intermediate **13** (1 equiv) in dry toluene (10 vol) at - 25 °C was added diethylzinc (8 equiv, 1.1 M in toluene, Aldrich). The reaction mixture was stirred at - 25 °C for 30 minutes. Chloroiodomethane (10 equiv) was added and the mixture was stirred at 0 °C to - 5 °C and allowed to warm to room temperature for 16 hours. After completion of the reaction, the reaction mixture was poured into saturated aqueous NaHCO₃. The resulting mixture was stirred at room temperature for 20 minutes, filtered through celite, and then washed with EtOAc. The organic layer was separated, dried over anhydrous Na₂SO₄, filtered and concentrated. The residue was purified by repeated column chromatography on silica gel using hexane/EtOAc to afford intermediate **14.**

**Step 14: tert-Butyl (1R,3S,4S,5R)-3-(hydroxymethyl)-5-methyl-2-azabicyclo[3.1.0]hexane-2-carboxylate-4-d (15):** To a solution of intermediate **14** (1 equiv) in MeOH (10 vol) at - 30 °C was added saturated methanolic ammonia solution (20 vol). The reaction mixture was stirred at room temperature for 3 days. After completion of the reaction, solvent was removed under reduced pressure and the residue was purified by column chromatography on silica gel using hexane/EtOAc to afford intermediate **15.**

**Step 15: (1R,3S,4S,5R)-2-(tert-Butoxycarbonyl)-5-methyl-2-azabicyclo[3.1.0]hexane-3-carboxylic-4-d acid (16):** To a solution of intermediate **15** (1 equiv) in acetonitrile (20 vol) at 0 °C was added N-methylmorpholine-N-oxide (10 equiv) and tetrapropylammonium perruthenate (0.1 equiv). The reaction mixture was stirred at room temperature for 16 hours. After completion of the reaction, 5% LiOH was added to the resulting mixture (pH=10~12) and the aqueous layer was washed with DCM (3 times). Then aqueous layer was acidified with 1.5 N HCl (pH=2~3) and extracted with DCM. The organic layer was separated, dried over anhydrous Na₂SO₄, filtered and concentrated to afford intermediate **16,** which can be used to prepare deuterated compounds of the present disclosure.

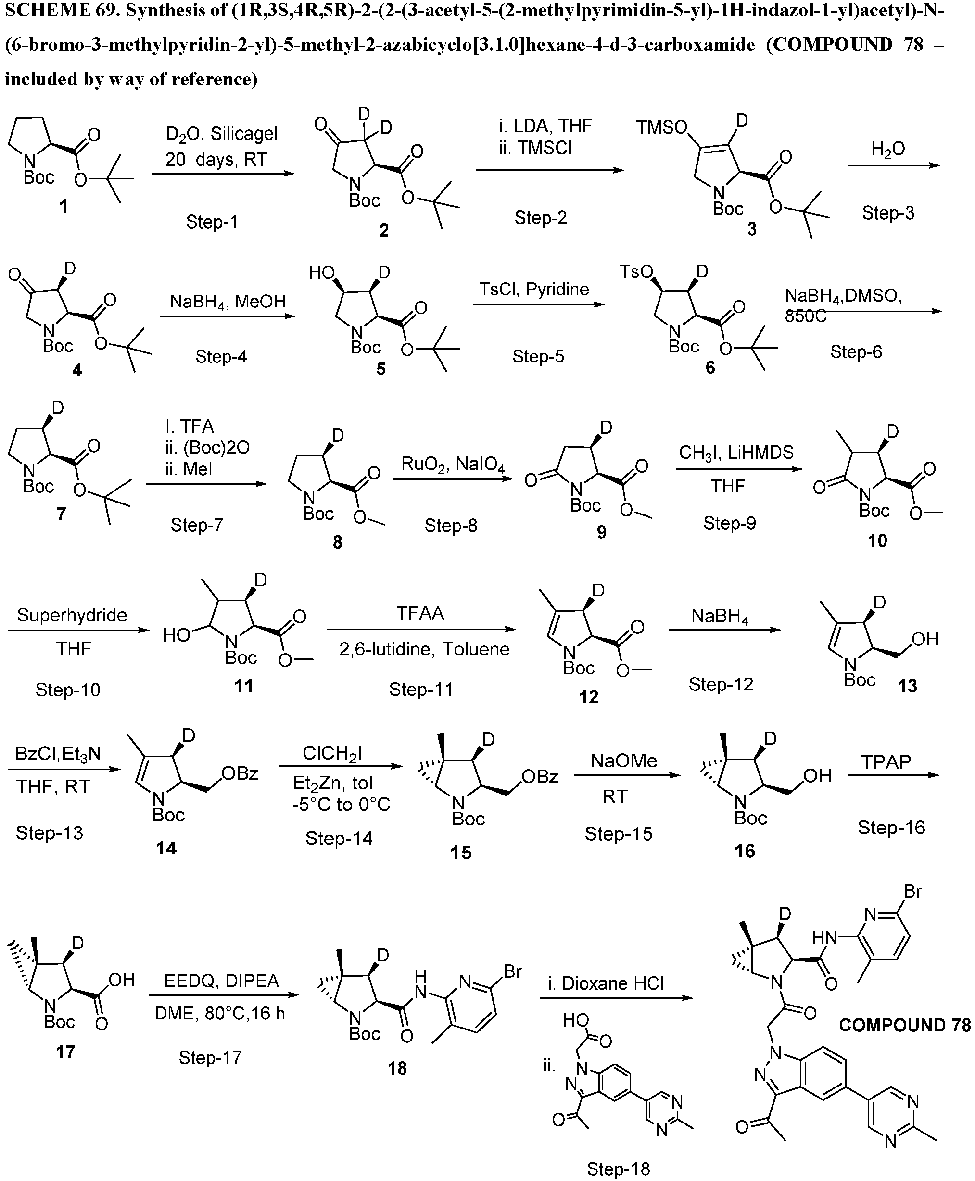

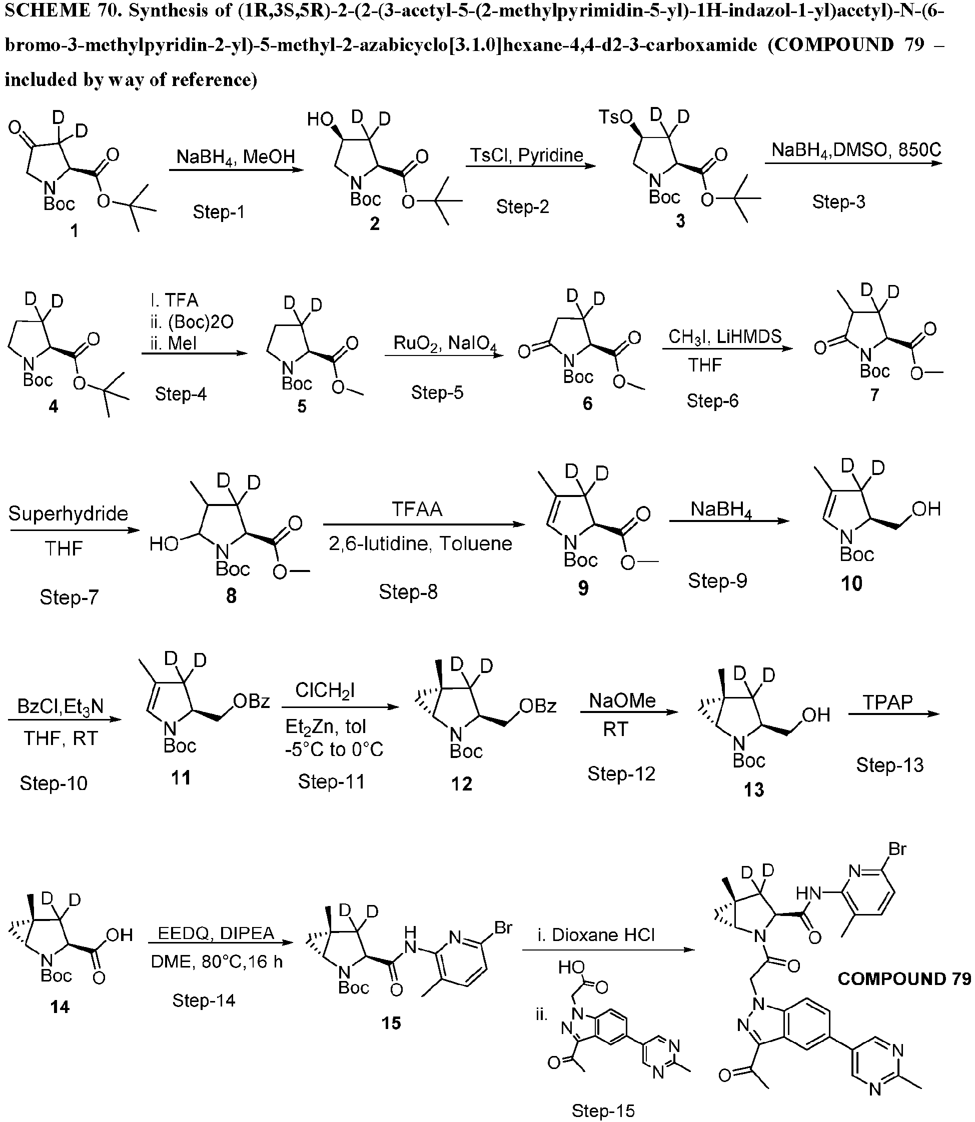

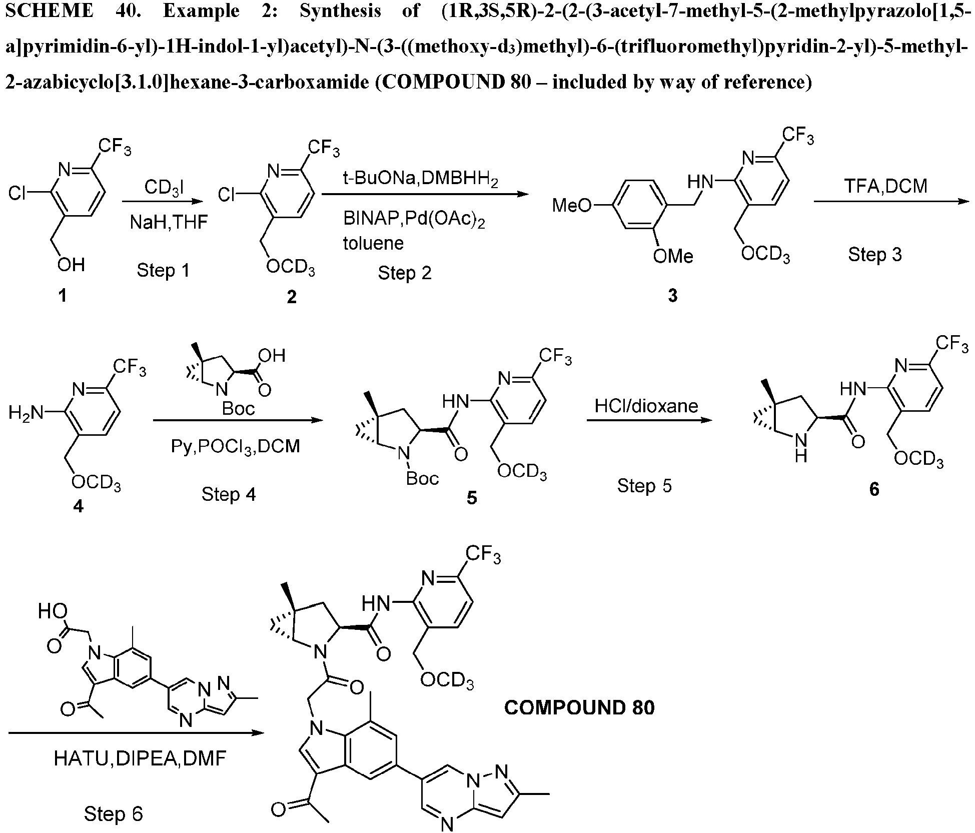

**Step 1:** To a solution of intermediate **1** (770 mg, 3.6 mmol) in anhydrous THF (8 mL) was added NaH (60%, 288 mg, 7.2 mmol) at 0 °C under N₂ atmosphere, and the mixture was stirred at 0 °C for 30 minutes. CD₃I (0.45 mL, 7.2 mmol) was added to the above mixture and the resulting mixture was stirred at room temperature for another 3 hours under N₂ atmosphere. The mixture was quenched with ice water and extracted with EtOAc twice. The organic layer was washed with brine, dried over anhydrous Na₂SO₄, filtered and concentrated to dryness under reduced pressure. The residue was purified by column chromatography on silica gel (eluted with PE: EtOAc = 6: 1 to 5: 1) to give intermediate **2** (428 mg, yield 52.1%) as light oil. LC/MS (ESI) m/z: 229 (M+H)⁺.

**Step 2:** To a mixture of intermediate **2** (428 mg, 1.88 mmol), t-BuONa (361 mg, 3.76 mmol) in toluene (8 mL) was added BINAP (237 mg, 0.38 mmol), Pd(OAc)₂ (85 mg, 0.38 mmol) and (2,4-dimethoxyphenyl)methanamine (472 mg, 2.82 mmol) at 0 °C. The mixture was stirred at 80°C overnight under N₂ atmosphere. The mixture was poured into ice water and extracted with EtOAc twice. The combined organic layers were washed with brine, dried over anhydrous Na₂SO₄, filtered and concentrated to dryness under reduced pressure. The residue was purified by flash chromatography on silica gel (PE: EtOAc = 40: 1 to 5: 1) give intermediate **3** (383 mg, yield 56.7%) as yellow oil. LC/MS (ESI) m/z: 360 (M+H)⁺.

**Step 3:** A solution of intermediate **3** (383 mg, 1.07 mmol) in DCM (4 mL) was added TFA (2 mL) and stirred at room temperature overnight. The mixture was concentrated to dryness under reduced pressure and the residue was poured into saturated aq. NaHCO₃ solution and extracted with DCM twice. The combined organic layers were washed with brine, dried over anhydrous Na₂SO₄ and concentrated to dryness to give intermediate **4** (223 mg, yield 100%) as a red solid, which was directly used in the next step without further purification LC/MS (ESI) m/z: 209 (M+H)⁺.

**Step 4:** To a mixture of intermediate **4** (223 mg, 1.1 mmol) and (1R,3S,5R)-2-(tert-butoxycarbonyl)-5-methyl-2-azabicyclo[3.1.0]hexane-3-carboxylic acid (265 mg, 1.1 mmol) in anhydrous DCM (4 mL) was added pyridine (0.44 mL, 5.5 mmol), followed by the addition of POCl₃ (0.11 mg, 1.2 mmol) at 0 °C. The reaction was stirred at room temperature for 1 hour before being poured into ice water and extracted with DCM twice. The combined organic layers were washed with brine, dried over anhydrous Na₂SO₄ and concentrated to dryness under reduced pressure. The residue was purified by flash chromatography on silica gel (PE: EtOAc = 10: 1 to 5:1) to give compound **5** (159 mg, yield 33.5%) as a white solid. LC/MS (ESI) m/z: 433 (M+H)⁺.

**Step 5:** A solution of intermediate **5** (159 mg, 0.37 mmol) in HCl/1,4-dioxane (3 mL, 4M) was stirred at room temperature for 2 hours before concentrated to dryness under reduced pressure to give intermediate **6** (154 mg, yield 100.0%) as a yellow solid. LC/MS (ESI) m/z: 333 (M+H)⁺.

**Step 6:** To a mixture of intermediate **6** (27 mg, 0.08 mmol) and 2-(3-acetyl-7-methyl-5-(2-methylpyrazolo[1,5-a]pyrimidin-6-yl)-1H-indol-1-yl)acetic acid (25 mg, 0.07 mmol) in DMF (2 mL) was added DIPEA (0.06 mL, 0.35 mmol) followed by HATU (42 mg, 0.11 mmol) at 0 °C. The mixture was stirred at room temperature for 2 hours before diluted with EtOAc and washed with saturated aq.NH₄Cl solution and brine, dried over anhydrous Na₂SO₄, filtered and concentrated to dryness under reduced pressure. The residue was purified by pre-HPLC (PE :EtOAc =1:3) to give **COMPOUND 80** (3.2 mg, yield 5.9%) as a white solid. ¹H NMR (400 MHz, CDCl₃) δ 8.87 (s, 1H), 8.68 (d, J = 3.2 Hz, 2H), 8.47 (s, 1H), 7.67 (d, J = 18 Hz, 2H), 7.35 (d, J = 8 Hz, 1H), 7.10 (s, 1H), 6.42 (s, 1H), 5.37 (d, J = 17.6 Hz, 1H), 5.25 (d, J = 17.6 Hz, 1H), 4.98 (s, 1H), 4.41 (d, J = 12.8 Hz, 1H), 4.25 (d, J = 12.8 Hz, 1H), 3.14 (s, 1H), 2.65 (s, 3H), 2.54 - 2.39 (m, 8H), 1.35 (s, 3H), 1.08 - 1.04 (m, 1H), 0.82 - 0.80 (m, 1H); LC/MS (ESI) m/z: 677 (M+H)⁺.

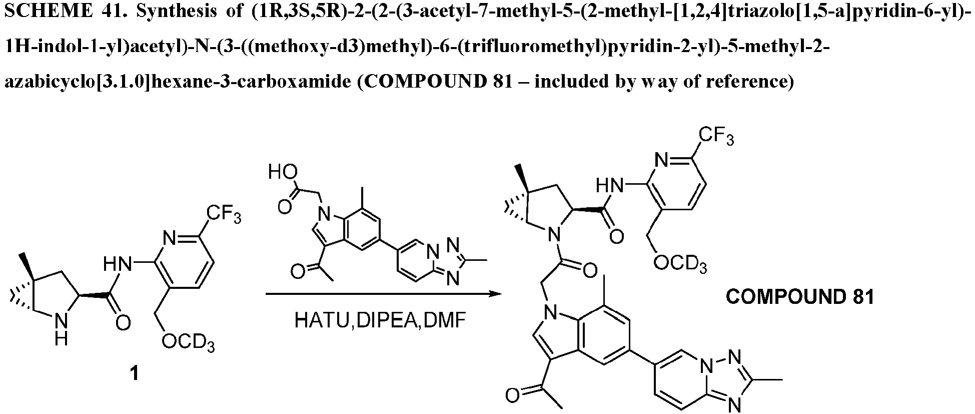

**Step 1:** To a mixture of intermediate **1** (30 mg, 0.09 mmol) and 2-(3-acetyl-7-methyl-5-(2-methyl-[1,2,4]triazolo[1,5-a]pyridin-6-yl)-1H-indol-1-yl)acetic acid (33 mg, 0.09 mmol) in DMF (2 mL) was added DIPEA (58 mg, 0.45 mmol), followed by HATU (53 mg, 0.14 mmol) at 0 °C. The mixture was stirred at room temperature for 2 hours before diluted with EtOAc and washed with saturated aq.NH₄Cl solution and brine, dried over anhydrous Na₂SO₄, filtered and concentrated to dryness under reduced pressure. The residue was purified by prep-HPLC (DCM: MeOH = 30:1) to give **COMPOUND 81** (3.9 mg, yield 6.4%) as a white solid. ¹H NMR (400 MHz, CDCl₃) δ 8.97 (s, 1H), 8.70 (d, J = 1.6 Hz, 1H), 8.53 (d, J = 1.6 Hz, 1H), 7.84 (dd, J = 9.2, 9.2 Hz, 1H), 7.79 - 7.69 (m, 3H), 7.42 (d, J = 7.6 Hz, 1H), 7.19 (s, 1H), 5.45 (d, J = 17.2 Hz, 1H), 5.33 (d, J = 17.6 Hz, 1H), 5.14 - 4.98 (m, 1H), 4.47 (d, J = 12.8 Hz, 1H), 4.32 (d, J = 12.8 Hz, 1H), 3.28 - 3.15 (m, 1H), 2.72 (s, 3H), 2.64 (s, 4H), 2.52 (s, 3H), 2.49 - 2.43 (m, 1H), 1.42 (s, 3H), 1.13 (t, J = 5.4 Hz, 1H), 0.88 (dd, J = 5.6, 5.2 Hz, 1H); LC/MS (ESI) m/z: 677 (M+H)⁺.

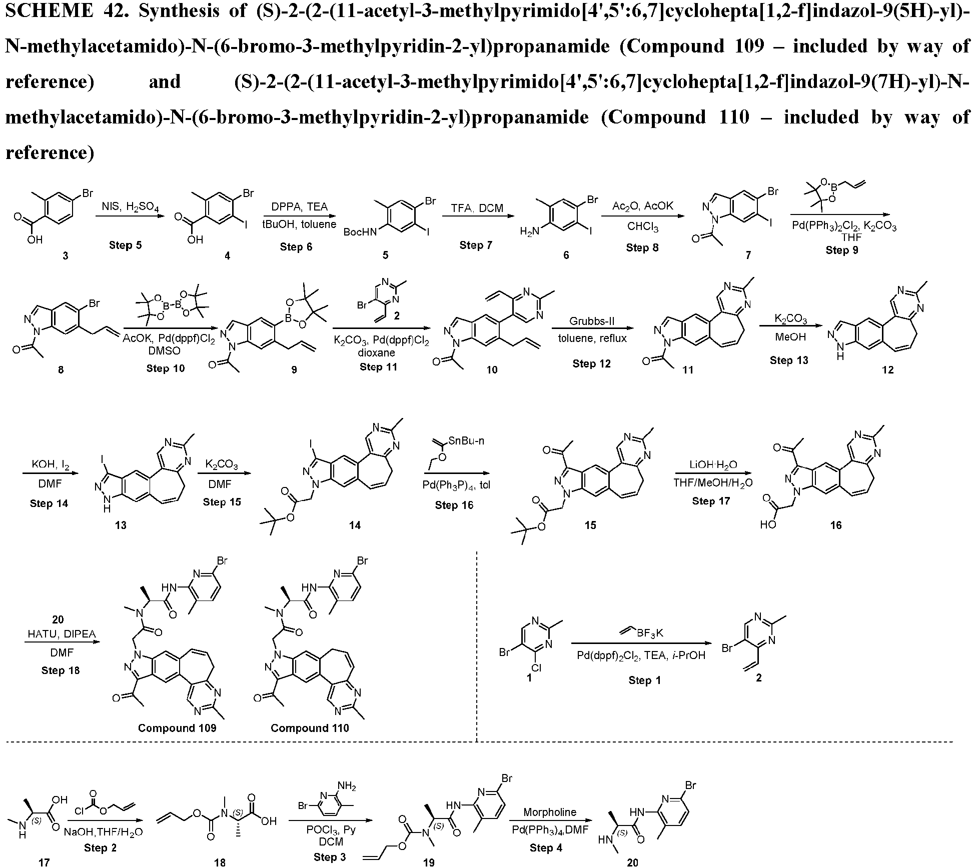

### Step 1: 5-bromo-2-methyl-4-vinylpyrimidine (2)

To a mixture of compound **1** (1.5 g, 7.20 mmol), potassium vinyltrifluoroborate (956 mg, 7.20 mmol) and TEA (2 mL, 14.4 mmol) in i-PrOH (30 mL) was added Pd(dppf)Cl₂ (263 mg, 0.36 mmol) at room temperature and the mixture was stirred under N₂ atmosphere at 80 °C for 16 hours. The reaction was cooled down to room temperature and concentrated to dryness under reduced pressure. The residue was purified by flash chromatography on silica gel (PE: EtOAc = 50: 1 to 30: 1) to give compound **2** (1.1 g, yield 76.4%) as a yellow oil. LC/MS(ESI) m/z: 199/201 (M+H)⁺.

### Step 2: N-((allyloxy)carbonyl)-N-methyl-L-alanine (18)

To a mixture of compound **17** (1 g, 9.70 mmol) and allyl chlorocarbonate (1.4 g, 11.64 mmol) in THF (4 mL) and H₂O (8 mL) was added NaOH (9.7 mL, 19.40 mmol, 2M in water) and the mixture was stirred at room temperature for 3 hours. The mixture was concentrated under reduced pressure and diluted with water. The mixture was washed with EtOAc and the aqueous layer was acidified by addition of 1N HCl solution. The mixture was extracted with EtOAc twice and the combined organic layers were dried over anhydrous Na₂SO₄, filtered and concentrated to dryness under reduced pressure to give compound **18** (1.38 g, 76.0 % yield) as a light oil. LC/MS (ESI)m/z: 188 (M+H)⁺.

### Step 3: allyl (S)-(1-((6-bromo-3-methylpyridin-2-yl)amino)-1-oxopropan-2-yl)(methyl)carbamate (19)

To a mixture of compound **18** (150 mg, 0.80 mmol) and 6-bromo-3-methylpyridin-2-amine (150 mg, 0.80 mmol) in DCM (5 mL) was added pyridine (317 mg, 4.0 mmol) and POCl₃ (135 mg, 0.88 mmol) at 0 °C and the mixture was stirred under N₂ atmosphere at room temperature for 2 hours. The mixture was poured into iced-water and extracted with DCM twice. The combined organic layers were washed with brine, dried over anhydrous Na₂SO₄, filtered and concentrated to dryness under reduced pressure. The residue was purified by flash chromatography on silica gel (PE: EtOAc= 10: 1 to 2: 1) to give compound **19** (190 mg, 66.56% yield) as a light-yellow oil. LC/MS (ESI) m/z: 356 (M+H)⁺.

### Step 4: (S)-N-(6-bromo-3-methylpyridin-2-yl)-2-(methylamino)propanamide (20)

To a solution compound **19** (130 mg, 0.37 mmol) in DMF (3 mL) was added Pd(PPh₃)₄ (84 mg, 0.073 mmol) and morpholine (64 mg, 0.73 mmol) at 0 °C and the mixture was stirred under N₂ atmosphere at room temperature for 2 hours. The mixture was diluted with EtOAc and water and the aqueous layer was extracted with EtOAc twice. The combined organic layers were washed with brine, dried over anhydrous Na₂SO₄, filtered and concentrated to dryness under reduced pressure. The residue was purified by prep-TLC (DCM: MeOH = 15: 1) to give compound **20** (50 mg, 50.3% yield) as a yellow solid. LC/MS (ESI) m/z: 272 (M+H)⁺.

### Step 5: 4-bromo-5-iodo-2-methylbenzoic acid (4)

To a solution of compound **3** (40 g, 186 mmol) in H₂SO₄ (160 mL) was added a solution of NIS (41.8 g, 186 mmol) in H₂SO₄ (160 mL) at 0 °C and the mixture was stirred at room temperature for 16 hours. The mixture was poured into ice-water (about 3.5 L) and extracted with EtOAc twice. The combined organic layers were washed with saturated aq. Na₂S₂O₃ solution and brine, dried over anhydrous Na₂SO₄, filtered and concentrated to dryness under reduced pressure. The residue was purified by crystallization with EtOAc to give compound **4** (43.8 g, yield 68.8%) as a white solid. LC/MS(ESI) m/z: 341/343 (M+H)⁺.

### Step 6: tert-butyl (4-bromo-5-iodo-2-methylphenyl)carbamate (5)

To a mixture of compound **4** (43 g, 126 mmol) and TEA (36 mL, 259 mmol) in toluene (440 mL) was added DPPA (33.4 mL, 155 mmol) at 0 °C and the mixture was stirred under N₂ atmosphere at 100 °C for 3 hours. The mixture was cooled to 10 °C and t-BuOH (440 mL) was added. The reaction was stirred at 100 °C for another 16 hours and filtered through Celite. The filter cake was washed with water and EtOAc. The filtrate was concentrated to dryness under reduced pressure and the aqueous layer was extracted with EtOAc twice. The combined organic layers were dried over anhydrous Na₂SO₄, filtered and concentrated to dryness under reduced pressure. The residue was purified by crystallization with EtOAc to give compound **5** (36.6 g, yield 70.5%) as a white solid. LC/MS(ESI) m/z: 412/414 (M+H)⁺.

### Step 7: 4-bromo-5-iodo-2-methylaniline (6)

To a solution of compound **5** (36.6 g, 88.8 mmol) in DCM (360 mL) was added TFA (360 mL) at 0 °C and the reaction was stirred at room temperature for 3 hours. The mixture was concentrated to dryness under reduced pressure, co-evaporated with DCM three times. The residue was purified by crystallization with EtOAc to give compound **6** (26.5 g, yield 95.6%) as a white solid. LC/MS(ESI) m/z: 312/314 (M+H)⁺.

### Step 8: 1-(5-bromo-6-iodo-1H-indazol-1-yl)ethan-1-one (7)

To a solution of compound **6** (26.5 g, 84.9 mmol) in CHCl₃ (300 mL) was added AcOK (11 g, 112 mmol) and Ac₂O (26.5 mL, 280 mmol) at 0 °C and the mixture was stirred at 10 °C for 1 hour. Isoamyl nitrite (25 mL, 186 mmol) was added and the reaction was stirred under N₂ atmosphere at 60 °C for 16 hours. The reaction was diluted with water and DCM and the mixture was filter through Celite. The filtrate was separated and the aqueous layer was extracted with DCM twice. The combined organic layers were washed with brine, dried over anhydrous Na₂SO₄, filtered and concentrated to dryness under reduced pressure. The residue was purified by crystallization with EtOAc to give compound 7 (12 g, yield 38.7%) as a brown solid. LC/MS(ESI) m/z: 365/367 (M+H)⁺.

### Step 9: 1-(6-allyl-5-bromo-1H-indazol-1-yl)ethan-1-one (8)

To a mixture of compound 7 (7 g, 19.2 mmol) and K₂CO₃ (5.3 g, 38.4 mmol) in THF (70 mL) was added 4,4,5,5-tetramethyl-2-(prop-2-en-1-yl)-1,3,2-dioxaborolane (3.87 g, 23.02 mmol) and Pd(PPh₃)₂Cl₃ (673 mg, 0.96 mmol) at 0 °C and the mixture stirred with pressure flask under N₂ atmosphere at 70 °C overnight. The mixture was diluted with EtOAc and washed with water and brine. The aqueous layer was extracted with EtOAc twice and the combined organic layers were dried over anhydrous Na₂SO₄, filtered and concentrated to dryness under reduced pressure. The residue was purified by flash chromatography on silica gel (PE: EtOAc = 100: 1) to give compound **8** (3 g, 56.0% yield) as a white solid. LC/MS(ESI) m/z: 279/281 (M+H)⁺.

### Step 10: 1-(6-allyl-5-(4,4,5,5-tetramethyl-1,3,2-dioxaborolan-2-yl)-1H-indazol-1-yl)ethan-1-one (9)

To a mixture of compound **8** (1.0 g, 3.58 mmol) and bis(pinacolato)diboron (1.0 g, 3.94 mmol) in 1,4-dioxane (20 mL) was added AcOK (1.06 g, 10.8 mmol) and Pd(dppf)Cl₂ (262 mg, 0.36 mmol) at 0 °C and the mixture was stirred under N₂ atmosphere at 90 °C for 3 hours. The mixture was cooled down to room temperature, poured into iced-water and extracted with EtOAc twice. The combined organic layers were washed with brine, dried over anhydrous Na₂SO₄, filtered and concentrated to dryness under reduced pressure. The residue was purified by flash chromatography on silica gel (PE: EtOAc = 200: 1 to 80: 1) to give compound **9** (1 g, yield 85.5%) as a white solid. LC/MS(ESI) m/z: 327 (M+H)⁺.

### Step 11: 1-(6-allyl-5-(2-methyl-4-vinylpyrimidin-5-yl)-1H-indazol-1-yl)ethan-1-one (10)

To a mixture of compound **9** (460 mg, 1.41 mmol) and 5-bromo-2-methyl-4-vinylpyrimidine (421 mg, 2.12 mmol) in THF (30 mL) and H₂O (3 mL) was added KF (410 mg, 7.05 mmol), Pd₂(dba)₃ (129 mg, 0.14 mmol) and S-phos (174 mg, 0.42 mmol) at 0 °C. The mixture was stirred under N₂ atmosphere at 70 °C overnight. The reaction mixture was cooled down to room temperature, diluted with water and extracted with EtOAc twice. The combined organic layers were washed with brine, dried over anhydrous Na₂SO₄, filtered and concentrated to dryness under reduced pressure. The residue was purified by flash chromatography on silica gel (PE: EtOAc = 20: 1 to 2: 1) to give compound **10** (265 mg, 59.0 % yield) as a yellow oil. LC/MS(ESI) m/z: 319 (M+H)⁺.

### Step 12: 1-(3-methylpyrimido[4',5':6,7]cyclohepta[1,2-f]indazol-9(5H)-yl)ethan-1-one (11)

To a solution of compound **10** (260 mg, 0.82 mmol) in degassed toluene (210 mL) was added Grubbs II catalyst (139 mg, 0.16 mmol) under N₂ atmosphere at room temperature and the mixture was stirred at 80°C overnight. The mixture was concentrated to dryness under reduced pressure and the residue was purified by flash chromatography on silica gel (PE: EtOAc = 10 : 1 to 2: 1) to give compound **11** (165 mg, 69.6 % yield) as a yellow solid. LC/MS (ESI) m/z: 291 (M+H)⁺.

### Step 13: 3-methyl-5,9-dihydropyrimido[4',5':6,7]cyclohepta[1,2-f]indazole (12)

To a solution of compound **11** (150 mg, 0.52 mmol) in MeOH (3 mL) was added K₂CO₃ (107 mg, 0.78 mmol) and the mixture was stirred at room temperature for 1 hour. The mixture was diluted with water and extracted with DCM twice. The combined organic layers were washed with brine, dried over anhydrous Na₂SO₄, filtered and concentrated to dryness under reduced pressure. The residue was purified by flash chromatography on silica gel (DCM: MeOH = 100: 1 to 80: 1) to give compound **12** (96 mg, 74.8% yield) as a yellow solid. LC/MS (ESI) m/z: 249 (M+H)⁺.

### Step 14: 11-iodo-3-methyl-5,9-dihydropyrimido[4',5':6,7]cyclohepta[1,2-f]indazole (13)

To a solution of compound **12** (96 mg, 0.39 mmol) in DMF (3 mL) was added KOH (49 mg, 0.87 mmol) and I₂ (147 mg, 0.58 mmol) in portions at 0 °C. The resulting mixture was stirred at room temperature for 2 hours. The mixture was diluted with water and extracted with EtOAc twice. The combined organic layers were washed with saturated aq. Na₂SO₃ solution and brine, dried over anhydrous Na₂SO₄ and concentrated to dryness under reduced pressure. The residue was purified by flash chromatography on silica gel (PE: EtOAc = 20: 1 to 1: 1) to give compound **13** (56 mg, 38.7% yield) as a yellow solid. LC/MS (ESI) m/z: 375 (M+H)⁺.

### Step 15: tert-butyl 2-(11-iodo-3-methylpyrimido[4',5':6,7]cyclohepta[1,2-f]indazol-9(5H)-yl)acetate (14)

To a solution of compound **13** (56 mg, 0.15 mmol) in DMF (2 mL) was added K₂CO₃ (62 mg, 0.45 mmol) and tert-butyl 2-bromoacetate (32 mg, 0.17 mmol) and the reaction mixture was stirred at room temperature overnight. The mixture was diluted with water and extracted with EtOAc twice. The combined organic layers were washed with brine, dried over anhydrous Na₂SO₄, filtered and concentrated to dryness under reduced pressure. The residue was purified by flash chromatography on silica gel (PE: EtOAc =10: 1 to 3: 1) to give compound **14** (60 mg, 82.1% yield) as a yellow solid. LC/MS (ESI) m/z: 489 (M+H)⁺.

### Step 16: tert-butyl 2-(11-acetyl-3-methylpyrimido[4',5':6,7]cyclohepta[1,2-f]indazol-9(5H)-yl)acetate (15)

To a mixture of compound **14** (60 mg, 0.12 mmol) and tributyl(1-ethoxyvinyl)stannane (58 mg, 0.16 mmol) in toluene (11 mL) was added Pd(PPh₃)₄ (14 mg, 0.01 mmol) and the mixture was stirred under N₂ atmosphere at 100 °C overnight. The mixture was concentrated to dryness and the residue was re-dissolved in THF (5 mL). 0.5 N aq. HCl solution (1 mL) was added to the mixture and the mixture was stirred at room temperature for 0.5 hour. The mixture was extracted with EtOAc twice and the combined organic layers were washed with brine, dried over anhydrous Na₂SO₄, filtered and concentrated to dryness under reduced pressure. The residue was purified by flash chromatography on silica gel (PE: EtOAc = 10: 1 to 2: 1) to give compound **15** (49 mg, 98.7 % yield) as a yellow solid. LC/MS (ESI)m/z: 405 (M+H)⁺.

### Step 17: 2-(11-acetyl-3-methylpyrimido[4',5':6,7]cyclohepta[1,2-f]indazol-9(5H)-yl)acetic acid (16)

To a solution of compound 5 (49 mg, 0.12 mmol) in MeOH (2 mL), THF (1 mL) and H₂O (1 mL) was added LiOH·H₂O (21 mg, 0.49 mmol) at 0 °C and the mixture was stirred at room temperature for 1 hour. The mixture was diluted with water and washed with Et₂O. The aqueous layer was acidified with 0.5 M aq. HCl solution and extracted with EtOAc twice. The combined organic layers were dried over anhydrous Na₂SO₄, filtered and concentrated to dryness under reduced pressure to give compound **16** (36 mg, 83.6% yield) as a yellow solid. LC/MS (ESI)m/z: 349 (M+H)⁺.

### Step 18: (S)-2-(2-(11-acetyl-3-methylpyrimido[4',5':6,7]cyclohepta[1,2-f]indazol-9(5H)-yl)-N-methylacetamido)-N-(6-bromo-3-methylpyridin-2-yl)propanamide (Compound 109) and (S)-2-(2-(11-acetyl-3-methylpyrimido[4',5':6,7]cyclohepta[1,2-flindazol-9(7H)-yl)-N-methylacetamido)-N-(6-bromo-3-methylpyridin-2-yl)propanamide (Compound 110)

To a mixture of compound **10** (30 mg, 0.086 mmol) and (S)-N-(6-bromo-3-methylpyridin-2-yl)-2-(methylamino)propanamide (23 mg, 0.086 mmol) in DMF (2 mL) was added DIPEA (56 mg, 0.43 mmol) and HATU (49 mg, 0.13 mmol) at 0 °C and the resulting mixture was stirred under N₂ atmosphere at room temperature for 2 hours. The mixture was diluted with EtOAc and washed with saturated aq. NH₄Cl solution and brine. The aqueous layer was extracted with EtOAc twice and the combined organic layers were dried over anhydrous Na₂SO₄, filtered and concentrated to dryness under reduced pressure. The residue was purified by prep-TLC (DCM: MeOH= 20: 1) and further purified by SFC to give **Compound 109** (3.5 mg, 6.7 % yield) and **Compound 110** (2.3 mg, 4.4 % yield) as a yellow solid.

**Compound 109** ¹H NMR (400 MHz, CDCl₃) δ 8.77 (s, 1H), 8.64 (s, 1H), 7.45 (s, 1H), 7.37 (d, *J* = 8.0 Hz, 1H), 7.25 (s, 1H), 6.79 (d, *J* = 10.8 Hz, 1H), 6.33 - 6.25 (m, 1H), 5.48 - 5.40 (m, 2H), 5.29 (d, *J* = 7.6 Hz, 1H), 3.26 (d, *J* = 7.2 Hz 2H), 3.21 (s, 3H), 2.78 (s, 3H), 2.73 (s, 3H), 2.06 (s, 3H), 1.51 (d, *J* = 6.8 Hz, 3H); LC/MS (ESI)m/z: 602/604 (M+H)⁺. **Compound 110** ¹H NMR (400 MHz, CDCl₃) δ 9.02 (s, 1H), 8.50 (s, 1H), 8.11 (s, 1H), 7.37 (d, *J* = 10.0 Hz, 2H), 6.67 - 6.55 (m, 2H), 5.45 - 5.37 (m, 2H), 5.33 - 5.29 (m, 1H), 3.37 - 3.21 (m, 2H), 3.18 (s, 3H), 2.83 (s, 3H), 2.69 (s, 3H), 2.05 (s, 3H), 1.50 (d, *J* = 6.8 Hz, 3H); LC/MS (ESI)m/z: 602/604 (M+H)⁺.

### EXAMPLE 2. NON-LIMITING EXAMPLES OF COMPOUNDS OF THE PRESENT DISCLOSURE

Table 1 shows illustrative Factor D inhibitors with characterizing data. The assay according to Example 3 was used to determine the IC₅₀'s of the compounds. Other standard factor D inhibition assays are also available. Three ***s are used to denote compounds with an IC₅₀ less than 1 micromolar; two **s indicate compound with an IC₅₀ between 1 micromolar and 10 micromolar, and one * denotes compounds with an IC₅₀ greater than 10 micromolar.

**Table 1. Non-limiting Examples of Compounds of the Present Disclosure (compounds marked * included by way of reference)**

| Cmp No | Structure | Name | IC₅₀ | RT min (Method A or B) | MS M+1 |
|---|---|---|---|---|---|
| 1* | 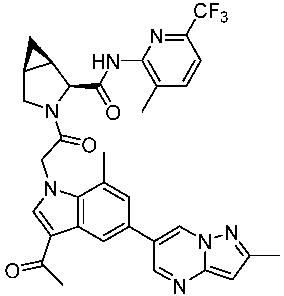 | (1R,2S,5S)-3-(2-(3-acetyl-7-methyl-5-(2-methylpyrazolo[1,5-a]pyrimidin-6-yl)-1H-indol-1-yl)acetyl)-N-(3-methyl-6-(trifluoromethyl)pyridin-2-yl)-3-azabicyclo[3.1.0]hexane-2-carboxamide | *** | 3.49 (B) | 632 |
| 2* | 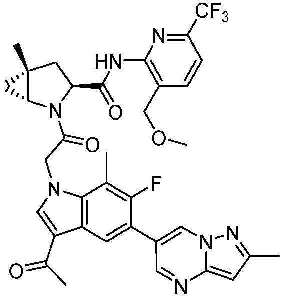 | (1R,3S,5R)-2-(2-(3-acetyl-6-fluoro-7-methyl-5-(2-methylpyrazolo[1,5-a]pyrimidin-6-yl)-1H-indol-1-yl)acetyl)-N-(3-(methoxymethyl)-6-(trifluoromethyl)pyridin-2-yl)-5-methyl-2-azabicyclo[3.1.0]hexane-3-carboxamide | *** | 3.62 (B) | 692 |
| 3* | 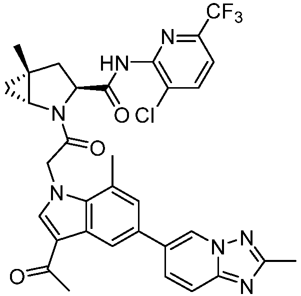 | (1R,3 S,5R)-2-(2-(3-acety 1-7-methyl-5-(2-methyl-[1,2,4]triazolo[1,5-a]pyridin-6-yl)-1H-indol-1-yl)acetyl)-N-(3-chloro-6-(trifluoromethyl)pyridin-2-yl)-5-methyl-2-azabicyclo[3.1.0]hexane-3-carboxamide | *** | 3.44 (B) | 664 |
| 4* | 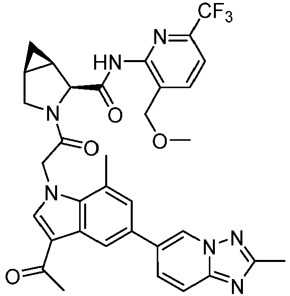 | (1R,2S,5S)-3-(2-(3-acetyl-7-methyl-5-(2-methyl-[1,2,4]triazolo[1,5-a]pyridin-6-yl)-1H-indol-1-yl)acetyl)-N-(3-(methoxymethyl)-6-(trifluoromethyl)pyridin-2-yl)-3-azabicyclo[3.1.0]hexane-2-carboxamide | *** | 3.39 (B) | 660 |
| 5* | 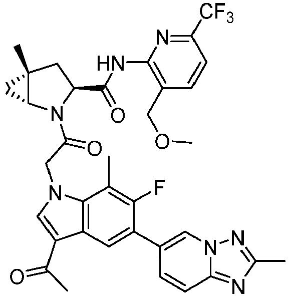 | (1R,3S,5R)-2-(2-(3-acety1-6-fluoro-7-methyl-5-(2-methyl-[1,2,4]triazolo[1,5-a]pyridin-6-yl)-1H-indol-1-yl)acetyl)-N-(3-(methoxymethyl)-6-(trifluoromethyl)pyridin-2-yl)-5-methyl-2-azabicyclo[3.1.0]hexane-3-carboxamide | *** | 3.7 (B) | 692 |
| 6* | 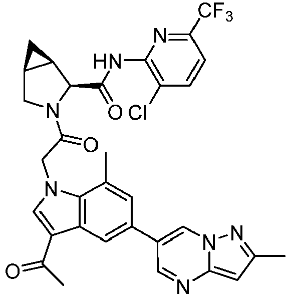 | (1R,2S,5S)-3-(2-(3-acetyl-7 - methyl-5-(2-methylpyrazolo[1,5-a]pyrimidin-6-yl)-1H-indol-1-yl)acetyl)-N-(3-chloro-6-(trifluoromethyl)pyridin-2-yl)-3-azabicyclo[3.1.0]hexane-2-carboxamide | *** | 1.77 (B) | 650 |
| 7* | 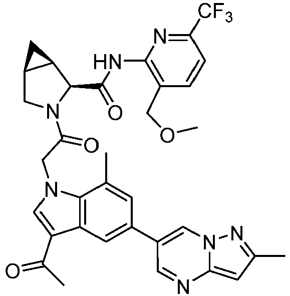 | (1R,2S,5S)-3-(2-(3-acetyl-7-methyl-5-(2-methylpyrazolo[1,5-a]pyrimidin-6-yl)-1H-indol-1-yl)acetyl)-N-(3-(methoxymethyl)-6-(trifluoromethyl)pyridin-2-yl)-3-azabicyclo[3.1.0]hexane-2-carboxamide | *** | 3.55 (B) | 660 |
| 8* | 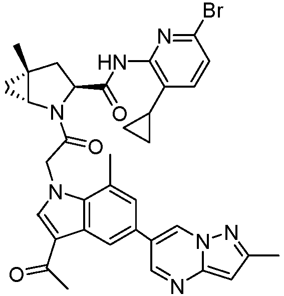 | (1R,3S,5R)-2-(2-(3-acetyl-7-methyl-5-(2-methylpyrazolo[1,5-a]pyrimidin-6-yl)-1H-indol-1-yl)acetyl)-N-(6-bromo-3-cyclopropylpyridin-2-yl)-5-methyl-2-azabicyclo[3.1.0]hexane-3-carboxamide | * | 3.49 (B) | 680 |
| 9* | 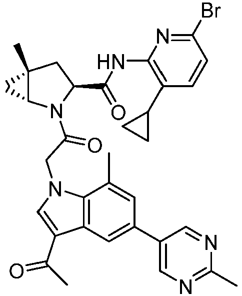 | (1R,3S,5R)-2-(2-(3-acety1-7-methyl-5-(2-methylpyrimidin-5-yl)-1H-indol-1-yl)acetyl)-N-(6-bromo-3-cyclopropylpyridin-2-yl)-5-methyl-2-azabicyclo[3.1.0]hexane-3-carboxamide | *** | 3.41 (B) | 642 |
| 10* | 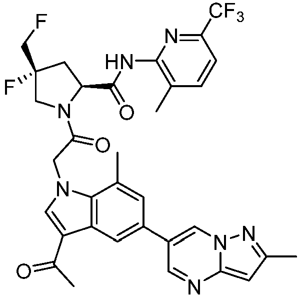 | (2S,4R)-1-(2-(3-acetyl-7-methyl-5-(2-methylpyrazolo[1,5-a]pyrimidin-6-yl)-1H-indol-1-yl)acetyl)-4-fluoro-4-(fluoromethyl)-N-(3-methyl-6-(trifluoromethyl)pyridin-2-yl)pyrrolidine-2-carboxamide | *** | 3.41 (B) | 668 |
| 11* | 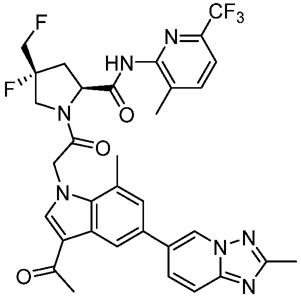 | (2S,4R)-1-(2-(3-acetyl-7-methyl-5-(2-methyl-[1,2,4]triazolo[1,5-a]pyridin-6-yl)-1H-indol-1-yl)acetyl)-4-fluoro-4-(fluoromethyl)-N-(3-methyl-6-(trifluoromethyl)pyridin-2-yl)pyrrolidine-2-carboxamide | *** | 32 (B) | 668 |
| 12* | 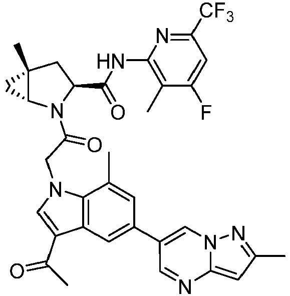 | (1R,3S,5R)-2-(2-(3-acety1-7-methyl-5-(2-methylpyrazolo[1,5-a]pyrimidin-6-yl)-1H-indol-1-yl)acetyl)-N-(4-fluoro-3-methyl-6-(trifluoromethyl)pyridin-2-yl)-5-methyl-2-azabicyclo[3.1.0]hexane-3-carboxamide | *** | 3.95 (B) | 662 |
| 13* | 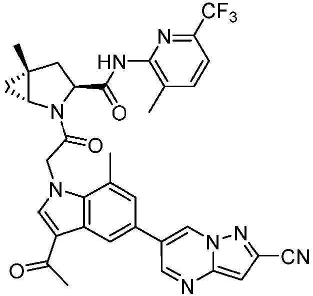 | (1R,3S,5R)-2-(2-(3-acetyl-5-(2-cyanopyrazolo[1,5-a]pyrimidin-6-yl)-7-methyl-1H-indol-1-yl)acetyl)-5-methyl-N-(3-methyl-6-(trifluoromethyl)pyridin-2-yl)-2-azabicyclo[3.1.0]hexane-3-carboxamide | *** | 3.79 (B) | 655 |
| 14* | 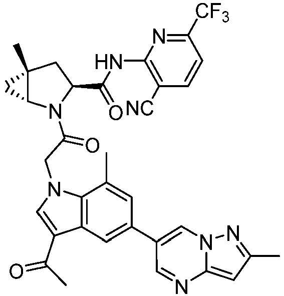 | (1R,3S,5R)-2-(2-(3-acetyl-7-methyl-5-(2-methylpyrazolo[1,5-a]pyrimidin-6-yl)-1H-indol-1-yl)acetyl)-N-(3-cyano-6-(trifluoromethyl)pyridin-2-yl)-5-methyl-2-azabicyclo[3.1.0]hexane-3-carboxamide | *** | 3.56 (B) | 655 |
| 15* | 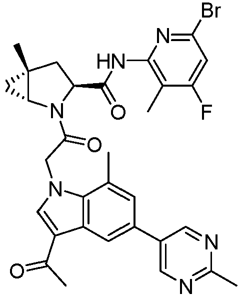 | (1R,3S,5R)-2-(2-(3-acetyl-7-methyl-5-(2-methylpyrimidin-5-yl)-1H-indol-1-yl)acetyl)-N-(6-bromo-4-fluoro-3-methylpyridin-2-yl)-5-methyl-2-azabicyclo[3.1.0]hexane-3-carboxamide | *** | 3.12 (B) | 633 |
| 16* | 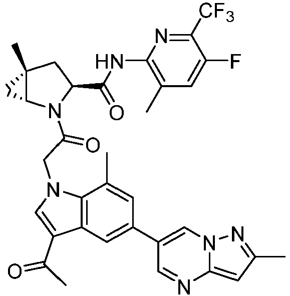 | (1R,3S,5R)-2-(2-(3-acetyl-7-methyl-5-(2-methylpyrazolo[1,5-a]pyrimidin-6-yl)-1H-indol-1-yl)acetyl)-N-(5-fluoro-3-methyl-6-(trifluoromethyl)pyridin-2-yl)-5-methyl-2-azabicyclo[3.1.0]hexane-3-carboxamide | *** | 2.55 (B) | 662 |
| 17* | 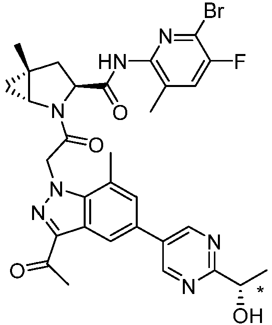 | (1R,3S,5R)-2-(2-(3-acetyl-5-(2-((S)-1-hydroxyethyl)pyrimidin-5-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide | *** | 3.35 (B) | 664 |
| 18* | 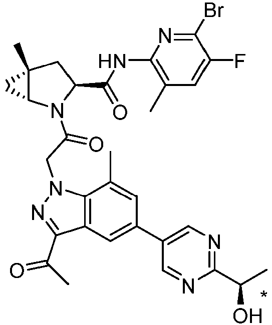 | (1R,3S,5R)-2-(2-(3-acetyl-5-(2-((R)-1-hydroxyethyl)pyrimidin-5-yl)-7-methyl-1H-indazol-1-yl)acetyl)-N-(6-bromo-5-fluoro-3-methylpyridin-2-yl)-5-methyl-2-azabicyclo[3.1.0]hexane-3-carboxamide | *** | 3.39 (B) | 664 |
| 19* | 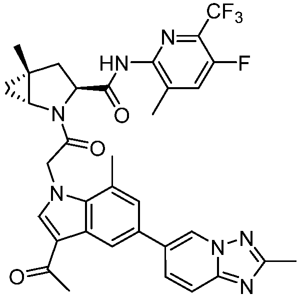 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(5-fluoro-3-methyl-6-(trifluoromethyl)pyridin-2-yl)-5-methyl-2-azabicyclo[3.1.0]hexane-3-carboxamide | *** | 3.44 (B) | 662 |
| 20* | 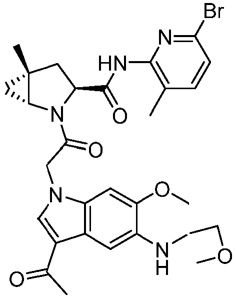 | (1R,3S,5R)-2-(2-(8'-acetyl-1'H-spiro[oxetane-3,2'-[1,4]oxazino[3,2-f]indol]-6'(3'H)-yl)acetyl)-N-(6-bromo-3-methylpyridin-2-yl)-5-methyl-2-azabicyclo[3.1.0]hexane-3-carboxamide | *** | 2.82 (B) | 608 |
| 21* | 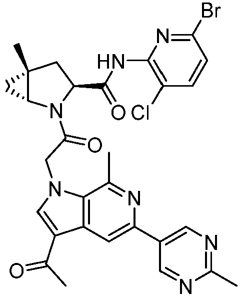 | (1R,3S,5R)-2-(2-(3-acetyl-7-methyl-5-(2-methylpyrimidin-5-yl)-1H-pyrrolo[2,3-c]pyridin-1-yl)acetyl)-N-(6-bromo-3-chloropyridin-2-yl)-5-methyl-2-azabicyclo[3.1.0]hexane-3-carboxamide | *** | 2.72 (B) | 636 |
| 22* | 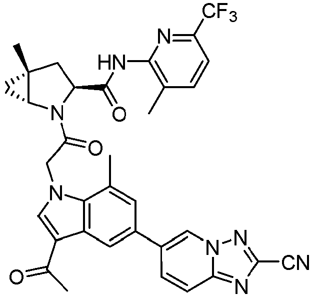 | (1R,3S,5R)-2-(2-(3-acetyl-5-(2-cyano-[1,2,4]triazolo[1,5-a]pyridin-6-yl)-7-methyl-1H-indol-1-yl)acetyl)-5-methyl-N-(3-methyl-6-(trifluoromethyl)pyridin-2-yl)-2-azabicyclo[3.1.0]hexane-3-carboxamide | *** | 3.62 (B) | 655 |
| 23 | 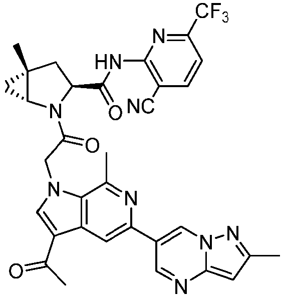 | (1R,3S,5R)-2-(2-(3-acetyl-7-methyl-5-(2-methylpyrazolo[1,5-a]pyrimidin-6-yl)-1H-pyrrolo[2,3-c]pyridin-1-yl)acetyl)-N-(3-cyano-6-(trifluoromethyl)pyridin-2-yl)-5-methyl-2-azabicyclo[3.1.0]hexane-3-carboxamide | *** | 2.61 (B) | 656 |
| 24 | 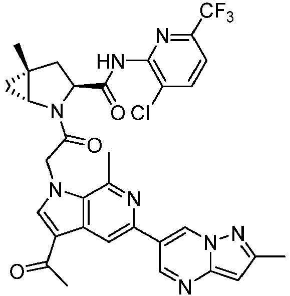 | (1R,3S,5R)-2-(2-(3-acetyl-7-methyl-5-(2-methylpyrazolo[1,5-a]pyrimidin-6-yl)-1H-pyrrolo[2,3-c]pyridin-1-yl)acetyl)-N-(3-chloro-6-(trifluoromethyl)pyridin-2-yl)-5-methyl-2-azabicyclo[3.1.0]hexane-3-carboxamide | *** | 2.87 (B) | 665 |
| 25* | 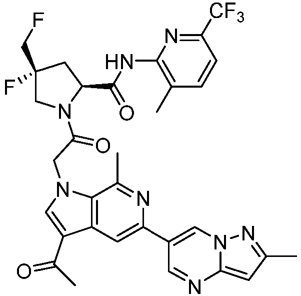 | (2S,4R)-1-(2-(3-acetyl-7-methyl-5-(2-methylpyrazolo[1,5-a]pyrimidin-6-yl)-1H-pyrrolo[2,3-c]pyridin-1-yl)acetyl)-4-fluoro-4-(fluoromethyl)-N-(3-methyl-6-(trifluoromethyl)pyridin-2-yl)pyrrolidine-2-carboxamide | *** | 2.65 (B) | 669 |
| 26* | 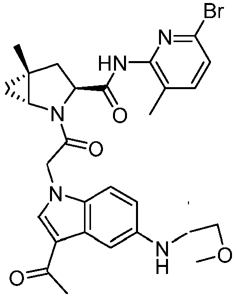 | (1R,3S,5R)-2-(2-(3-acetyl-5-((3-methyloxetan-3-yl)amino)-1H-indol-1-yl)acetyl)-N-(6-bromo-3-methylpyridin-2-yl)-5-methyl-2-azabicyclo[3.1.0]hexane-3-carboxamide | *** | 2.21 (B) | 594 |
| 27* | 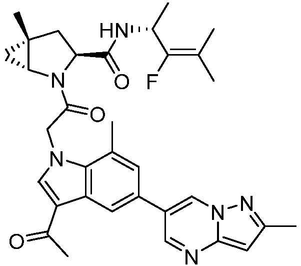 | (1R,3S,5R)-2-(2-(3-acetyl-7-methyl-5-(2-methylpyrazolo[1,5-a]pyrimidin-6-yl)-1H-indol-1-yl)acetyl)-N-((R)-3-fluoro-4-methylpent-3-en-2-yl)-5-methyl-2-azabicyclo[3.1.0]hexane-3-carboxamide | *** | 3.82 (B) | 585 |
| 28* | 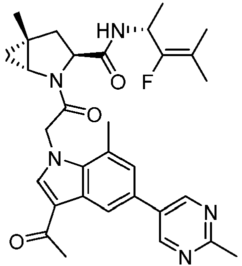 | (1R,3S,5R)-2-(2-(3-acetyl-7-methyl-5-(2-methylpyrimidin-5-yl)-1H-indol-1-yl)acetyl)-N-((R)-3-fluoro-4-methylpent-3-en-2-yl)-5-methyl-2-azabicyclo[3.1.0]hexane-3-carboxamide | *** | 3.16 (B) | 546 |
| 29 | 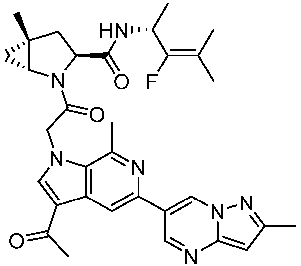 | (1R,3S,5R)-2-(2-(3-acetyl-7-methyl-5-(2-methylpyrazolo[1,5-a]pyrimidin-6-yl)-1H-pyrrolo[2,3-c]pyridin-1-yl)acetyl)-N-((R)-3-fluoro-4-methylpent-3-en-2-yl)-5-methyl-2-azabicyclo[3.1.0]hexane-3-carboxamide | *** | 2.98 (B) | 586 |
| 30* | 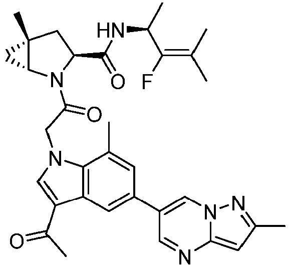 | (1R,3S,5R)-2-(2-(3-acetyl-7-methyl-5-(2-methylpyrazolo[1,5-a]pyrimidin-6-yl)-1H-indol-1-yl)acetyl)-N-((S)-3-fluoro-4-methylpent-3-en-2-yl)-5-methyl-2-azabicyclo[3.1.0]hexane-3-carboxamide | *** | 3.73 (B) | 585 |
| 31* | 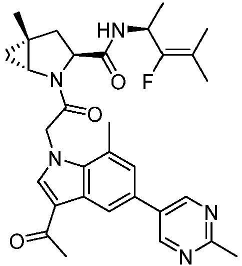 | (1R,3S,5R)-2-(2-(3-acetyl-7-methyl-5-(2-methylpyrimidin-5-yl)-1H-indol-1-yl)acetyl)-N-((S)-3-fluoro-4-methylpent-3-en-2-yl)-5-methyl-2-azabicyclo[3.1.0]hexane-3-carboxamide | *** | 3.45 (B) | 546 |
| 32 | 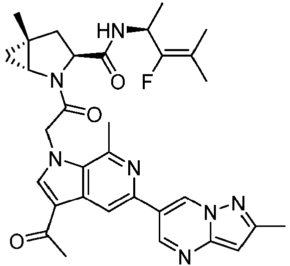 | (1R,3S,5R)-2-(2-(3-acetyl-7-methyl-5-(2-methylpyrazolo[1,5-a]pyrimidin-6-yl)-1H-pyrrolo[2,3-c]pyridin-1-yl)acetyl)-N-((S)-3-fluoro-4-methylpent-3-en-2-yl)-5-methyl-2-azabicyclo[3.1.0]hexane-3-carboxamide | *** | 2.92 (B) | 586 |
| 33* | 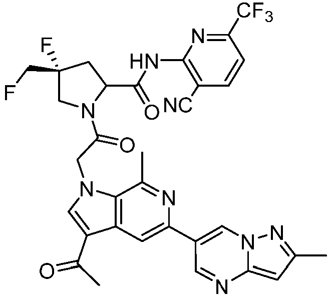 | (4R)-1-(2-(3-acetyl-7-methyl-5-(2-methylpyrazolo[1,5-a]pyrimidin-6-yl)-1H-pyrrolo[2,3-c]pyridin-1-yl)acetyl)-N-(3-cyano-6-(trifluoromethyl)pyridin-2-yl)-4-fluoro-4-(fluoromethyl)pyrrolidine-2-carboxamide | *** | 2.61 (B) | 680 |
| 34* | 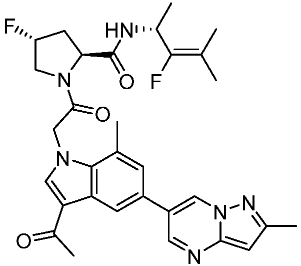 | (2S,4R)-1-(2-(3-acetyl-7-methyl-5-(2-methylpyrazolo[1,5-a]pyrimidin-6-yl)-1H-indol-1-yl)acetyl)-4-fluoro-N-((R)-3-fluoro-4-methylpent-3-en-2-yl)pyrrolidine-2-carboxamide | *** | 3.29 (B) | 577 |
| 35* | 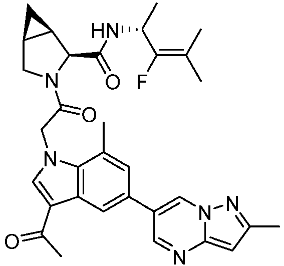 | (1R,2S,5S)-3-(2-(3-acetyl-7-methyl-5-(2-methylpyrazolo[1,5-a]pyrimidin-6-yl)-1H-indol-1-yl)acetyl)-N-((R)-3-fluoro-4-methylpent-3-en-2-yl)-3-azabicyclo[3.1.0]hexane-2-carboxamide | *** | 3.29 (B) | 571 |
| 36* | 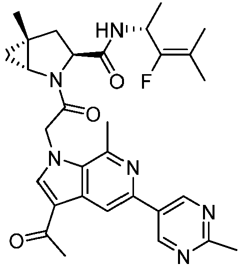 | (1R,3S,5R)-2-(2-(3-acetyl-7-methyl-5-(2-methylpyrimidin-5-yl)-1H-pyrrolo[2,3-c]pyridin-1-yl)acetyl)-N-((R)-3-fluoro-4-methylpent-3-en-2-yl)-5-methyl-2-azabicyclo[3.1.0]hexane-3-carboxamide | *** | 2.59 (B) | 547 |
| 37* | 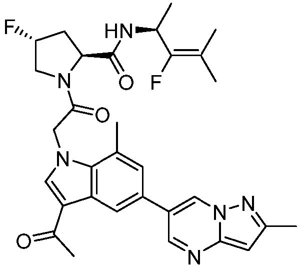 | (2S,4R)-1-(2-(3-acetyl-7-methyl-5-(2-methylpyrazolo[1,5-a]pyrimidin-6-yl)-1H-indol-1-yl)acetyl)-4-fluoro-N-((S)-3-fluoro-4-methylpent-3-en-2-yl)pyrrolidine-2-carboxamide | *** | 3.6 (B) | 577 |
| 38* | 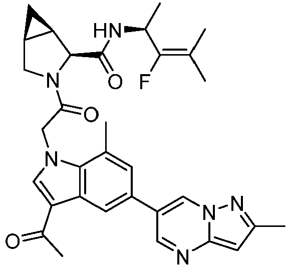 | (1R,2S,5S)-3-(2-(3-acetyl-7-methyl-5-(2-methylpyrazolo[1,5-a]pyrimidin-6-yl)-1H-indol-1-yl)acetyl)-N-((S)-3-fluoro-4-methylpent-3-en-2-yl)-3-azabicyclo[3.1.0]hexane-2-carboxamide | *** | 3.7 (B) | 571 |
| 39* | 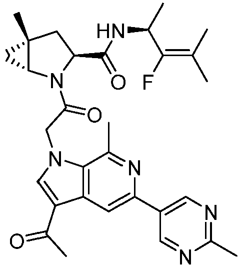 | (1R,3S,5R)-2-(2-(3-acetyl-7-methyl-5-(2-methylpyrimidin-5-yl)-1H-pyrrolo[2,3-c]pyridin-1-yl)acetyl)-N-((S)-3-fluoro-4-methylpent-3-en-2-yl)-5-methyl-2-azabicyclo[3.1.0]hexane-3-carboxamide | *** | 2.33 (B) | 547 |
| 40* | 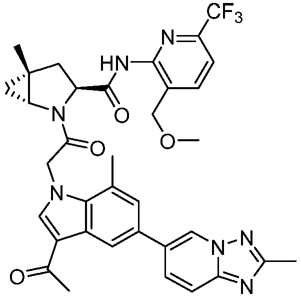 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(3-(methoxymethyl)-6-(trifluoromethyl)pyridin-2-yl)-5-methyl-2-azabicyclo[3.1.0]hexane-3-carboxamide | *** | 3.38 (B) | 674 |
| 41* | 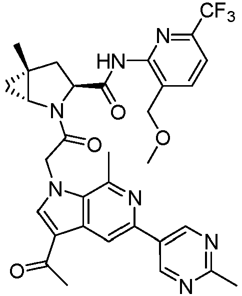 | (1R,3S,5R)-2-(2-(3-acetyl-7-methyl-5-(2-methylpyrimidin-5-yl)-1H-pyrrolo[2,3-c]pyridin-1-yl)acetyl)-N-(3-(methoxymethyl)-6-(trifluoromethyl)pyridin-2-yl)-5-methyl-2-azabicyclo[3.1.0]hexane-3-carboxamide | *** | 2.29 (B) | 636 |
| 42* | 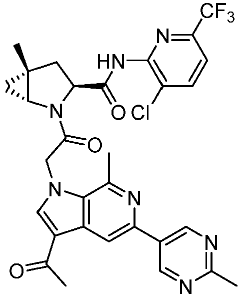 | (1R,3S,5R)-2-(2-(3-acetyl-7-methyl-5-(2-methylpyrimidin-5-yl)-1H-pyrrolo[2,3-c]pyridin-1-yl)acetyl)-N-(3-chloro-6-(trifluoromethyl)pyridin-2-yl)-5-methyl-2-azabicyclo[3.1.0]hexane-3-carboxamide | *** | 2.1 (B) | 626 |
| 43* | 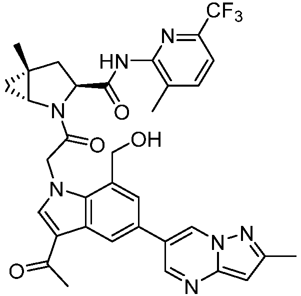 | (1R,3S,5R)-2-(2-(3-acetyl-7-(hydroxymethyl)-5-(2-methylpyrazolo[1,5-a]pyrimidin-6-yl)-1H-indol-1-yl)acetyl)-5-methyl-N-(3-methyl-6-(trifluoromethyl)pyridin-2-yl)-2-azabicyclo[3.1.0]hexane-3-carboxamide | *** | 3.21 (B) | 660 |
| 44* | 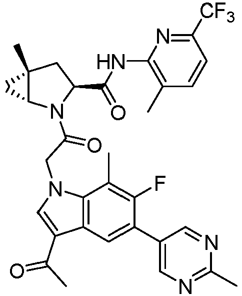 | (1R,3S,5R)-2-(2-(3-acetyl-6-fluoro-7-methyl-5-(2-methylpyrimidin-5-yl)-1H-indol-1-yl)acetyl)-5-methyl-N-(3-methyl-6-(trifluoromethyl)pyridin-2-yl)-2-azabicyclo[3.1.0]hexane-3-carboxamide | *** | 3.29 (B) | 623 |
| 45* | 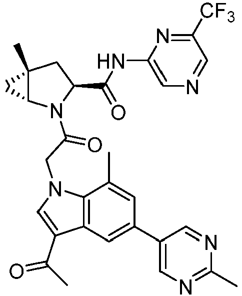 | (1R,3S,5R)-2-(2-(3-acetyl-7-methyl-5-(2-methylpyrimidin-5-yl)-1H-indol-1-yl)acetyl)-5-methyl-N-(6-(trifluoromethyl)pyrazin-2-yl)-2-azabicyclo[3.1.0]hexane-3-carboxamide | *** | 3.48 (B) | 592 |
| 46* | 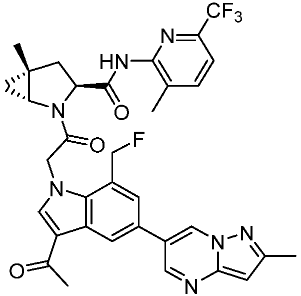 | (1R,3S,5R)-2-(2-(3-acetyl-7-(fluoromethyl)-5-(2-methylpyrazolo[1,5-a]pyrimidin-6-yl)-1H-indol-1-yl)acetyl)-5-methyl-N-(3-methyl-6-(trifluoromethyl)pyridin-2-yl)-2-azabicyclo[3.1.0]hexane-3-carboxamide | *** | 3.69 (B) | 662 |
| 47* | 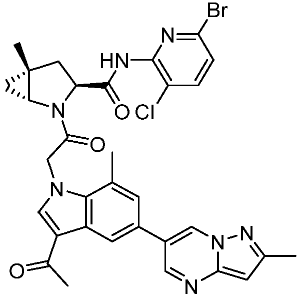 | (1R,3S,5R)-2-(2-(3-acetyl-7-methyl-5-(2-methylpyrazolo[1,5-a]pyrimidin-6-yl)-1H-indol-1-yl)acetyl)-N-(6-bromo-3-chloropyridin-2-yl)-5-methyl-2-azabicyclo[3.1.0]hexane-3-carboxamide | *** | 2.86 (B) | 674 |
| 48* | 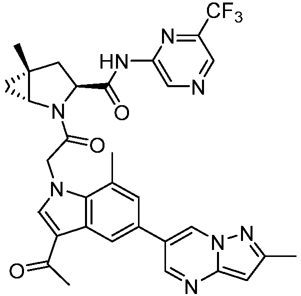 | (1R,3S,5R)-2-(2-(3-acetyl-7-methyl-5-(2-methylpyrazolo[1,5-a]pyrimidin-6-yl)-1H-indol-1-yl)acetyl)-5-methyl-N-(6-(trifluoromethyl)pyrazin-2-yl)-2-azabicyclo[3.1.0]hexane-3-carboxamide | *** | 0.4 (B) | 632 |
| 49* | 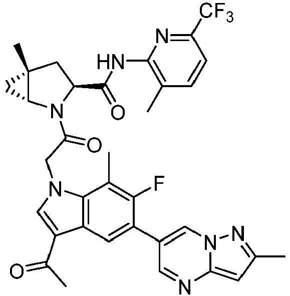 | (1R,3S,5R)-2-(2-(3-acetyl-6-fluoro-7-methyl-5-(2-methylpyrazolo[1,5-a]pyrimidin-6-yl)-1H-indol-1-yl)acetyl)-5-methyl-N-(3-methyl-6-(trifluoromethyl)pyridin-2-yl)-2-azabicyclo[3.1.0]hexane-3-carboxamide | *** | 2.73 (B) | 662 |
| 50* | 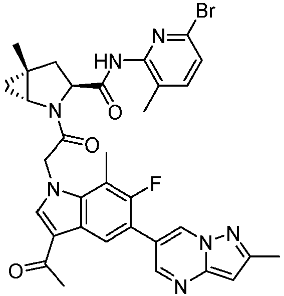 | (1R,3S,5R)-2-(2-(3-acetyl-6-fluoro-7-methyl-5-(2-methylpyrazolo[1,5-a]pyrimidin-6-yl)-1H-indol-1-yl)acetyl)-N-(6-bromo-3-methylpyridin-2-yl)-5-methyl-2-azabicyclo[3.1.0]hexane-3-carboxamide | *** | 3.79 (B) | 672 |
| 51* | 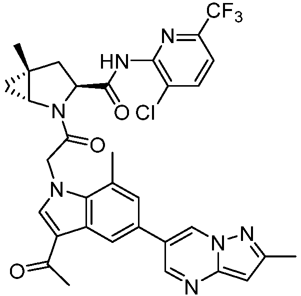 | (1R,3S,5R)-2-(2-(3-acetyl-7-methyl-5-(2-methylpyrazolo[1,5-a]pyrimidin-6-yl)-1H-indol-1-yl)acetyl)-N-(3-chloro-6-(trifluoromethyl)pyridin-2-yl)-5-methyl-2-azabicyclo[3.1.0]hexane-3-carboxamide | *** | 3.94 (B) | 664 |
| 52* | 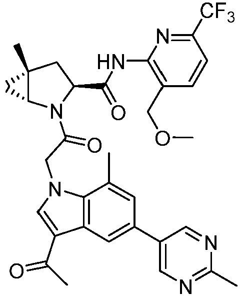 | (1R,3S,5R)-2-(2-(3-acetyl-7-methyl-5-(2-methylpyrimidin-5-yl)-1H-indol-1-yl)acetyl)-N-(3-(methoxymethyl)-6-(trifluoromethyl)pyridin-2-yl)-5-methyl-2-azabicyclo[3.1.0]hexane-3-carboxamide | *** | 2.47 (B) | 635 |
| 53* | 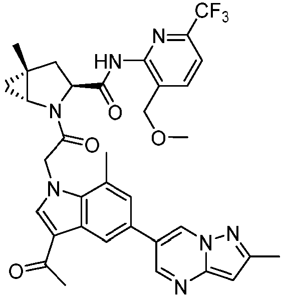 | (1R,3S,5R)-2-(2-(3-acetyl-7-methyl-5-(2-methylpyrazolo[1,5-a]pyrimidin-6-yl)-1H-indol-1-yl)acetyl)-N-(3-(methoxymethyl)-6-(trifluoromethyl)pyridin-2-yl)-5-methyl-2-azabicyclo[3.1.0]hexane-3-carboxamide | *** | 3.25 (B) | 674 |
| 54* | 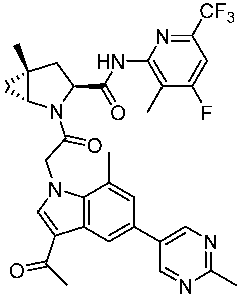 | (1R,3S,5R)-2-(2-(3-acetyl-7-methyl-5-(2-methylpyrimidin-5-yl)-1H-indol-1-yl)acetyl)-N-(4-fluoro-3-methyl-6-(trifluoromethyl)pyridin-2-yl)-5-methyl-2-azabicyclo[3.1.0]hexane-3-carboxamide | *** | 3.02 (B) | 623 |
| 55 | 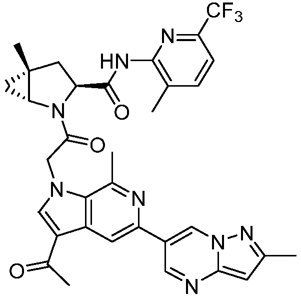 | (1R,3S,5R)-2-(2-(3-acetyl-7-methyl-5-(2-methylpyrazolo[1,5-a]pyrimidin-6-yl)-1H-pyrrolo[2,3-c]pyridin-1-yl)acetyl)-5-methyl-N-(3-methyl-6-(trifluoromethyl)pyridin-2-yl)-2-azabicyclo[3.1.0]hexane-3-carboxamide | *** | 2.63 (B) | 645 |
| 56* | 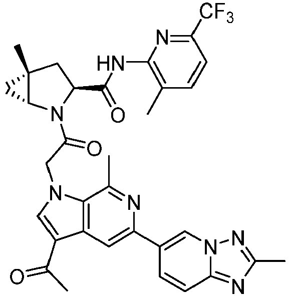 | (1R,3S,5R)-2-(2-(3-acetyl-7-methyl-5-(2-methyl-[1,2,4]triazolo[1,5-a]pyridin-6-yl)-1H-pyrrolo[2,3-c]pyridin-1-yl)acetyl)-5-methyl-N-(3-methyl-6-(trifluoromethyl)pyridin-2-yl)-2-azabicyclo[3.1.0]hexane-3-carboxamide | *** | 2.43 (B) | 645 |
| 57* | 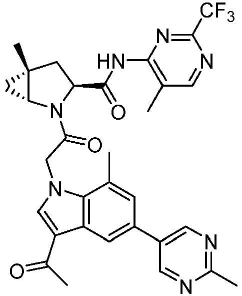 | (1R,3S,5R)-2-(2-(3-acetyl-7-methyl-5-(2-methylpyrimidin-5-yl)-1H-indol-1-yl)acetyl)-5-methyl-N-(5-methyl-2-(trifluoromethyl)pyrimidin-4-yl)-2-azabicyclo[3.1.0]hexane-3-carboxamide | *** | 3.15 (B) | 606 |
| 58* | 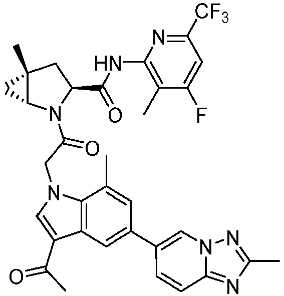 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(4-fluoro-3-methyl-6-(trifluoromethyl)pyridin-2-yl)-5-methyl-2-azabicyclo[3.1.0]hexane-3-carboxamide | *** | 3.27 (B) | 662 |
| 59* | 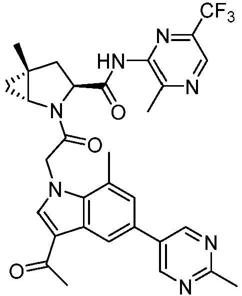 | (1R,3S,5R)-2-(2-(3-acetyl-7-methyl-5-(2-methylpyrimidin-5-yl)-1H-indol-1-yl)acetyl)-5-methyl-N-(3-methyl-6-(trifluoromethyl)pyrazin-2-yl)-2-azabicyclo[3.1.0]hexane-3-carboxamide | ** | 3.37 (B) | 606 |
| 60* | 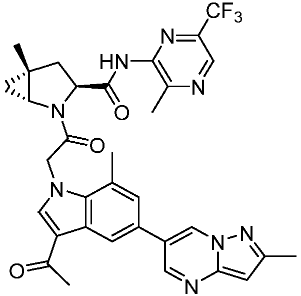 | (1R,3S,5R)-2-(2-(3-acetyl-7-methyl-5-(2-methylpyrazolo[1,5-a]pyrimidin-6-yl)-1H-indol-1-yl)acetyl)-5-methyl-N-(3-methyl-6-(trifluoromethyl)pyrazin-2-yl)-2-azabicyclo[3.1.0]hexane-3-carboxamide | *** | 3.75 (B) | 645 |
| 61* | 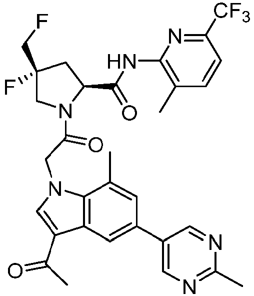 | (2S,4R)-1-(2-(3-acetyl-7-methyl-5-(2-methylpyrimidin-5-yl)-1H-indol-1-yl)acetyl)-4-fluoro-4-(fluoromethyl)-N-(3-methyl-6-(trifluoromethyl)pyridin-2-yl)pyrrolidine-2-carboxamide | *** | 2.99 (B) | 629 |
| 62* | 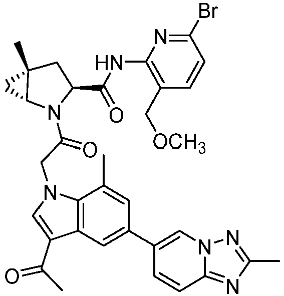 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(6-bromo-3-(methoxymethyl)pyridin-2-yl)-5-methyl-2-azabicyclo[3.1.0]hexane-3-carboxamide | *** | 1.85 (A) | 685 |
| 63* | 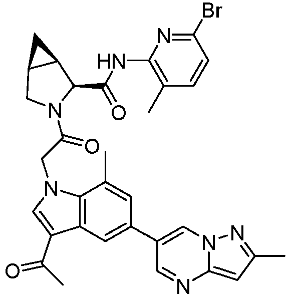 | (1R,2S,5S)-3-(2-(3-acetyl-7-methyl-5-(2-methylpyrazolo[1,5-a]pyrimidin-6-yl)-1H-indol-1-yl)acetyl)-N-(6-bromo-3-methylpyridin-2-yl)-3-azabicyclo[3.1.0]hexane-2-carboxamide | *** | 1.86 (A) | 642 |
| 64* | 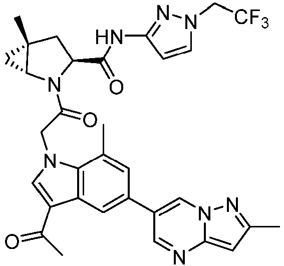 | (1R,3S,5R)-2-(2-(3-acetyl-7-methyl-5-(2-methylpyrazolo[1,5-a]pyrimidin-6-yl)-1H-indol-1-yl)acetyl)-5-methyl-N-(1-(2,2,2-trifluoroethyl)-1H-pyrazol-3-yl)-2-azabicyclo[3.1.0]hexane-3-carboxamide | *** | 1.95 (A) | 633 |
| 65* | 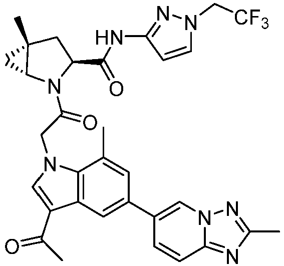 | (1R,3S,5R)-2-(2-(3-acetyl-7-methyl-5-(2-methyl-[1,2,4]triazolo[1,5-a]pyridin-6-yl)-1H-indol-1-yl)acetyl)-5-methyl-N-(1-(2,2,2-trifluoroethyl)-1H-pyrazol-3-yl)-2-azabicyclo[3.1.0]hexane-3-carboxamide | *** | 1.75 (A) | 633 |
| 66* | 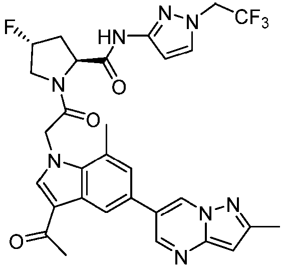 | (2S,4R)-1-(2-(3-acetyl-7-methyl-5-(2-methylpyrazolo[1,5-a]pyrimidin-6-yl)-1H-indol-1-yl)acetyl)-4-fluoro-N-(1-(2,2,2-trifluoroethyl)-1H-pyrazol-3-yl)pyrrolidine-2-carboxamide | *** | 1.68 (A) | 625 |
| 67* | 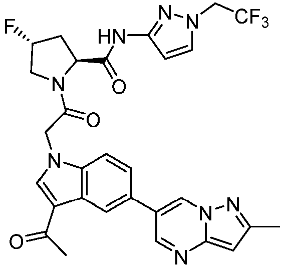 | (2S,4R)-1-(2-(3-acetyl-5-(2-methylpyrazolo[1,5-a]pyrimidin-6-yl)-1H-indol-1-yl)acetyl)-4-fluoro-N-(1-(2,2,2-trifluoroethyl)-1H-pyrazol-3-yl)pyrrolidine-2-carboxamide | *** | 1.57 (A) | 611 |
| 68* | 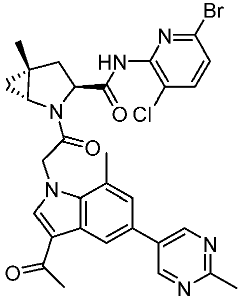 | (1R,3S,5R)-2-(2-(3-acetyl-7-methyl-5-(2-methylpyrimidin-5-yl)-1H-indol-1-yl)acetyl)-N-(6-bromo-3-chloropyridin-2-yl)-5-methyl-2-azabicyclo[3.1.0]hexane-3-carboxamide | *** | 1.81 (A) | 637 |
| 69* | 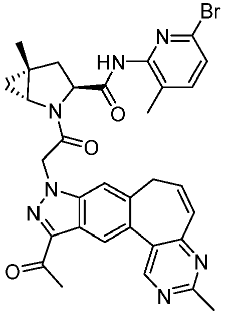 | (1R,3S,5R)-2-(2-(11-acetyl-3-methylpyrimido[4',5':6,7]cycloh epta[1,2-f]indazol-9(7H)-yl)acetyl)-N-(6-bromo-3-methylpyridin-2-yl)-5-methyl-2-azabicyclo[3.1.0]hexane-3-carboxamide | *** | 3.25 (B) | 640 |
| 70* | 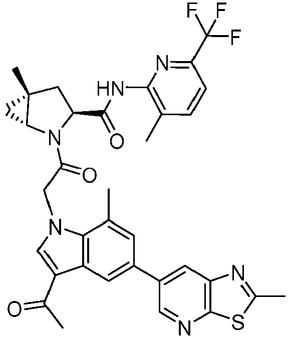 | (1R,3S,5R)-2-(2-(3-acetyl-7-methyl-5-(2-methylthiazolo[5,4-b]pyridin-6-yl)-1H-indol-1-yl)acetyl)-5-methyl-N-(3-methyl-6-(trifluoromethyl)pyridin-2-yl)-2-azabicyclo[3.1.0]hexane-3-carboxamide | *** | 2.26 (A) | 661 |
| 71* | 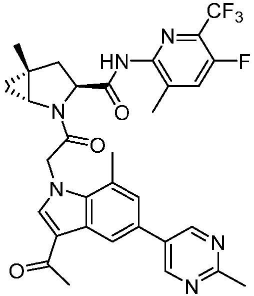 | (1R,3S,5R)-2-(2-(3-acetyl-7-methyl-5-(2-methylpyrimidin-5-yl)-1H-indol-1-yl)acetyl)-N-(5-fluoro-3-methyl-6-(trifluoromethyl)pyridin-2-yl)-5-methyl-2-azabicyclo[3.1.0]hexane-3-carboxamide | *** | 2.00 (A) | 623 |
| 72* | 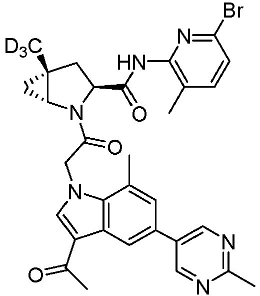 | (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-(methyl-d3)-2-azabicyclo[3.1.0]hexane-3-carboxamide | | | |
| 73* | 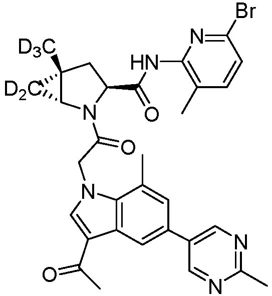 | (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-(methyl-d3)-2-azabicyclo[3.1.0]hexane-6,6-d2-3-carboxamide | | | |
| 74* | 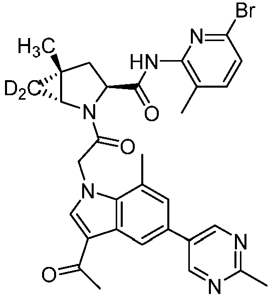 | (1R,3S,5R)-2-(2-(3-acetyl-7-methyl-5-(2-methylpyrimidin-5-yl)-1H-indol-1-yl)acetyl)-N-(6-bromo-3-methylpyridin-2-yl)-5-methyl-2-azabicyclo[3.1.0]hexane-6,6-d2-3-carboxamide | | | |
| 75* | 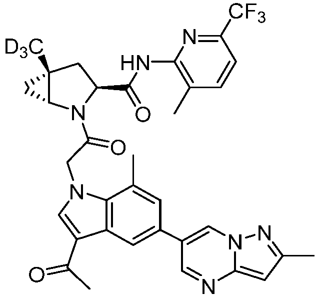 | (1R,3S,5R)-2-(2-(3-acetyl-7-methyl-5-(2-methylpyrazolo[1,5-a]pyrimidin-6-yl)-1H-indol-1-yl)acetyl)-5-(methyl-d3)-N-(3-methyl-6-(trifluoromethyl)pyridin-2-yl)-2-azabicyclo[3.1.0]hexane-3-carboxamide | | | |
| 76* | 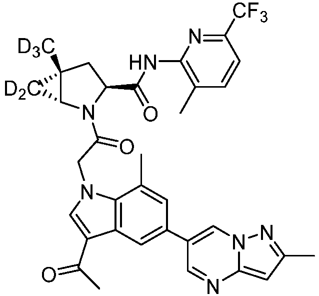 | (1R,3S,5R)-2-(2-(3-acetyl-7-methyl-5-(2-methylpyrazolo[1,5-a]pyrimidin-6-yl)-1H-indol-1-yl)acetyl)-5-(methyl-d3)-N-(3-methyl-6-(trifluoromethyl)pyridin-2-yl)-2-azabicyclo[3.1.0]hexane-6,6-d2-3-carboxamide | | | |
| 77* | 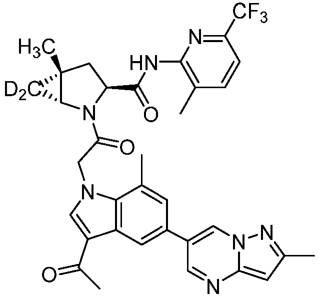 | (1R,3S,5R)-2-(2-(3-acetyl-7-methyl-5-(2-methylpyrazolo[1,5-a]pyrimidin-6-yl)-1H-indol-1-yl)acetyl)-5-methyl-N-(3-methyl-6-(trifluoromethyl)pyridin-2-yl)-2-azabicyclo[3.1.0]hexane-6,6-d2-3-carboxamide | | | |
| 78* | 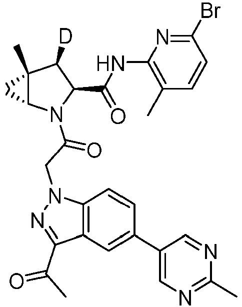 | (1R,3S,4R,5R)-2-(2-(3-acetyl-5-(2-methylpyrimidin-5-yl)-1H-indazol-1-yl)acetyl)-N-(6-bromo-3-methylpyridin-2-yl)-5-methyl-2-azabicyclo[3.1.0]hexane-4-d-3-carboxamide | | | |
| 79* | 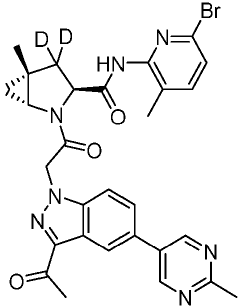 | (1R,3S,5R)-2-(2-(3-acetyl-5-(2-methylpyrimidin-5-yl)-1H-indazol-1-yl)acetyl)-N-(6-bromo-3-methylpyridin-2-yl)-5-methyl-2-azabicyclo[3.1.0]hexane-4,4-d2-3-carboxamide | | | |
| 80* | 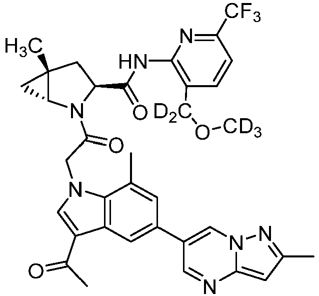 | (1R,3S,5R)-2-(2-(3-acetyl-7-methyl-5-(2-methylpyrazolo[1,5-a]pyrimidin-6-yl)-1H-indol-1-yl)acetyl)-N-(3-((methoxy-d3)methyl-d2)-6-(trifluoromethyl)pyridin-2-yl)-5-methyl-2-azabicyclo[3.1.0]hexane-3-carboxamide | *** | 2.71 (B) | 679 |
| 81* | 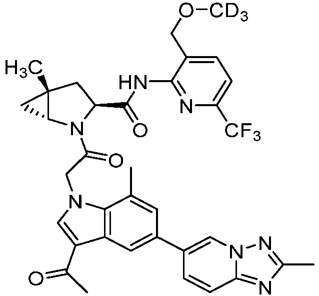 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(3-((methoxy-d3)methyl-d2)-6-(trifluoromethyl)pyridin-2-yl)-5-methyl-2-azabicyclo[3.1.0]hexane-3-carboxamide | *** | 3.39 (B) | 677 |
| 82* | 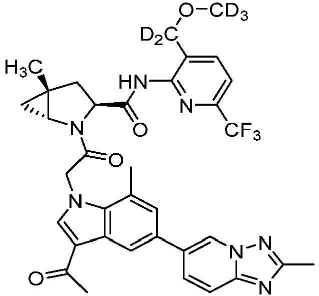 | (1R,3S,5R)-2-(2-(3-acetyl-7-methyl-5-(2-methyl-[1,2,4]triazolo[1,5-a]pyridin-6-yl)-1H-indol-1-yl)acetyl)-N-(3-((methoxy-d3)methyl-d2)-6-(trifluoromethyl)pyridin-2-yl)-5-methyl-2-azabicyclo[3.1.0]hexane-3-carboxamide | | 3.37 (B) | 677 |
| 92* | 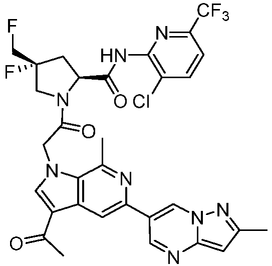 | (2S,4R)-1-(2-(3-acetyl-7-methyl-5-(2-methylpyrazolo[1,5-a]pyrimidin-6-yl)-1H-pyrrolo[2,3-c]pyridin-1-yl)acetyl)-N-(3-chloro-6-(trifluoromethyl)pyridin-2-yl)-4-fluoro-4-(fluoromethyl)pyrrolidine-2-carboxamide | *** | 1.74 (A) | 690 |
| 109* | 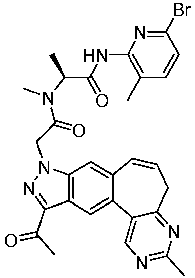 | (S)-2-(2-(11-acetyl-3-methylpyrimido[4',5':6,7]cycloh epta[1,2-f]indazol-9(5H)-yl)-N-methylacetamido)-N-(6-bromo-3-methylpyridin-2-yl)propanamide | *** | 1.95 (A) | 602 |
| 110* | 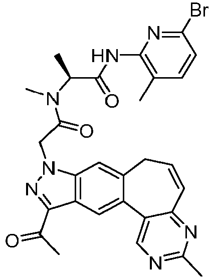 | (S)-2-(2-(11-acetyl-3-methylpyrimido[4',5':6,7]cycloh epta[1,2-f]indazol-9(7H)-yl)-N-methylacetamido)-N-(6-bromo-3-methylpyridin-2-yl)propanamide | *** | 1.89 (A) | 602 |

As used in compound 17 and 18 above an * denotes relative stereochemistry

### EXAMPLE 3. HUMAN FACTOR D ASSAY

Human Factor D (purified from human serum, Complement Technology, Inc.) at 80 nM final concentration is incubated with test compound at various concentrations for 5 min at room temperature in 50 mM Tris, 1M NaCl, pH 7.5. A synthetic substrate Z-L-Lys-SBzl and DTNB (Ellman's reagent) are added to final concentrations of 100 µM each. Absorbance at 405 nm (A₄₀₅) is recorded at 30 second intervals for 30 min using a microplate spectrophotometer. IC₅₀ values are calculated by nonlinear regression of complement Factor D reaction rates as a function of test compound concentration.

### EXAMPLE 4. HEMOLYSIS ASSAY

The hemolysis assay was previously described by G. Ruiz-Gomez, et al., J. Med. Chem. (2009) 52: 6042-6052. Prior to the assay, the optimum concentration of Normal Human Serum (NHS) needed to achieve 100% lysis of rabbit erythrocytes (RE) is determined by titration. In the assay, NHS (Complement Technology) is diluted in GVB⁰ Buffer (0.1 % gelatin, 5 mM Veronal, 145 mM NaCl, 0.025 % NaN₃, pH 7.3, Complement Technology) plus 10 mM Mg-EGTA and incubated with test compound at various concentrations for 15 min at 37°C. RE (Complement Technology) freshly suspended in GVB⁰ plus 10 mM Mg-EGTA are added to a final concentration of 1 x 10⁸ cells/mL and reactions are incubated for 30 min at 37°C. Positive control reactions (100% lysis) consist of GVB⁰ plus 10 mM Mg-EGTA with NHS and RE but without test compound; negative control reactions (0% lysis) consist of GVB⁰ plus 10 mM Mg-EGTA with RE only. Samples are centrifuged at 2000g for 3 min and supernatants collected. Absorbance at 405 nm (A₄₀₅) is recorded using a microplate spectrophotometer. IC₅₀ values are calculated by nonlinear regression from the percentage of hemolysis as a function of test compound concentration.

## Claims

1. A compound of Formula: 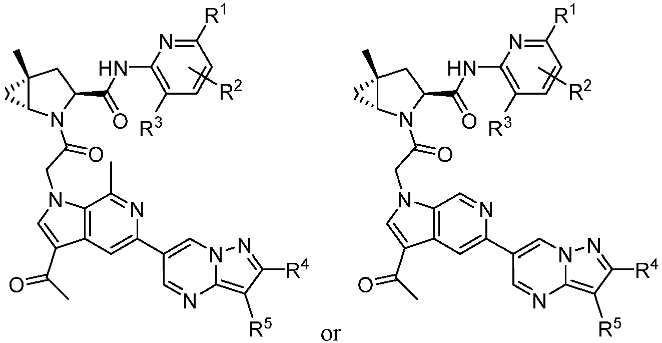
or a pharmaceutically acceptable salt thereof;
wherein:
R¹ is selected from the group consisting of halogen and C₁-C₂haloalkyl;
R² is selected from the group consisting of hydrogen, halogen, C₁-C₃alkyl, C₁-C₃haloalkyl, -COOR⁹, - CONR⁹R¹⁰, cyano, C₂-C₆alkanoyl, C₁-C₆alkoxy, -C₀-C₄alkylNR⁹R¹⁰, -C₀-C₄alkylOR⁹, and C₁-C₆haloalkoxy;
R³ is C₁-C₃alkyl, halogen, or C₁-C₃haloalkyl;
R⁴ and R⁵ are independently selected from the group consisting of hydrogen, halogen, C₁-C₃alkyl, C₁-C₃haloalkyl, -COOR⁹, -CONR⁹R¹⁰, cyano, C₂-C₆alkanoyl, C₁-C₆alkoxy, -C₀-C₄alkylNR⁹R¹⁰, -C₀-C₄alkylOR⁹, and C₁-C₆haloalkoxy; and
each R⁹ and R¹⁰ are independently selected from the group consisting of hydrogen and C₁-C₄alkyl.

2. The compound of claim 1, wherein the compound is: 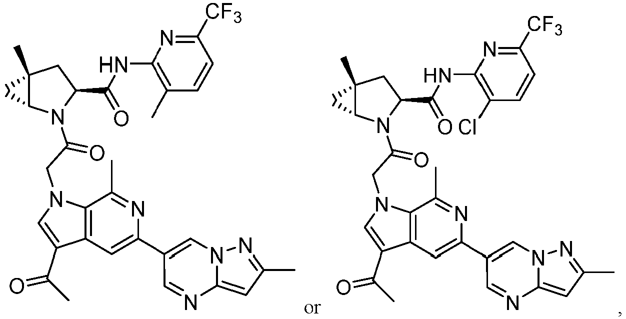 or a pharmaceutically acceptable salt thereof.

3. A compound selected from: 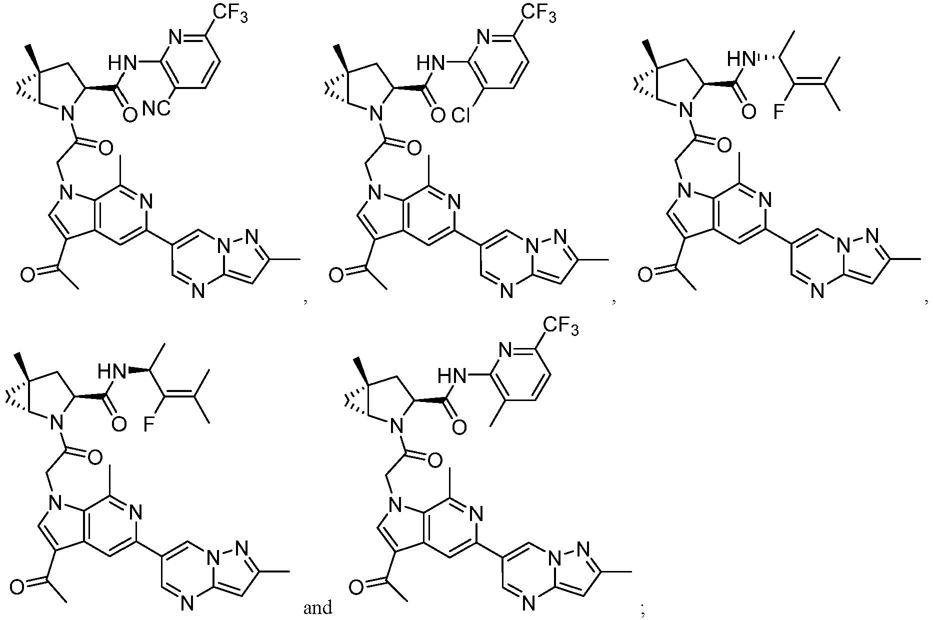 or a pharmaceutically acceptable salt thereof.

4. A pharmaceutical composition comprising a compound of any one of claims 1-3 or a pharmaceutically acceptable salt thereof, and a pharmaceutically acceptable carrier.

5. A compound according to any one of claims 1 to 3 or a pharmaceutically acceptable salt thereof, for use in treating a complement factor D mediated disorder in a subject in need thereof, wherein the disorder is selected from acute respiratory distress syndrome, age-related macular degeneration, arthritis, asthma, Alzheimer's dementia, amyotrophic lateral sclerosis, antibody-mediated transplant rejection, antineutrophil cytoplasm antibody-associated vasculitis, antiphospholipid syndrome, atypical or typical hemolytic uremic syndrome, cardiovascular disease, cold agglutinin disease, complement 3 glomerulopathy, chronic obstructive pulmonary disease, cirrhosis, Crohn's disease, C3 glomerulonephritis, diabetic retinopathy, dermatomyositis, dermatitis, epidermolysis bullosa acquisita, fatty liver, focal segmental glomerulosclerosis, geographic atrophy, glomerulonephritis, graft versus host disease, Guillain Barre syndrome, hemolytic anemia, hidradenitis suppurativa, IgA nephropathy, ischemia/reperfusion injury, liver failure, liver inflammation, lupus nephritis, membrane proliferative glomerulonephritis, multifocal motor neuropathy, multiple sclerosis, myasthenia gravis, neuromyelitis optica, nonalcoholic steatohepatitis, ocular disorder, ophthalmic disease, pancreatitis, paroxysmal nocturnal hemoglobinuria, pemphigoid, pemphigus vulgaris, pre-eclampsia, reduced glomerular filtration rate, renovascular disorder, respiratory disease, retinal detachment, rheumatoid arthritis, scleroderma, sepsis, Shiga toxin *E. coli*-related hemolytic uremic syndrome, spinal cord injury, sickle cell disease, traumatic brain injury, and ulcerative colitis.

6. A compound for use according to claim 5, wherein the subject is a human.

7. A compound for use according to claim 5 or 6, wherein the disorder is C3 glomerulopathy, C3 glomerulonephritis, or dense deposit disease.

8. A compound for use according to claim 5 or 6, wherein the disorder is an ophthalmic disorder, age-related macular degeneration, or geographic atrophy.

9. A compound for use according to claim 5 or 6, wherein the disorder is paroxysmal nocturnal hemoglobinuria.

10. A compound for use according to claim 5 or 6, wherein the disorder is sickle cell disease.

11. A compound for use according to claim 5 or 6, wherein the disorder is lupus nephritis or IgA nephropathy.

12. A compound for use according to claim 5 or 6, wherein the disorder is renovascular disorder.

## Patentansprüche

1. Verbindung der Formel: 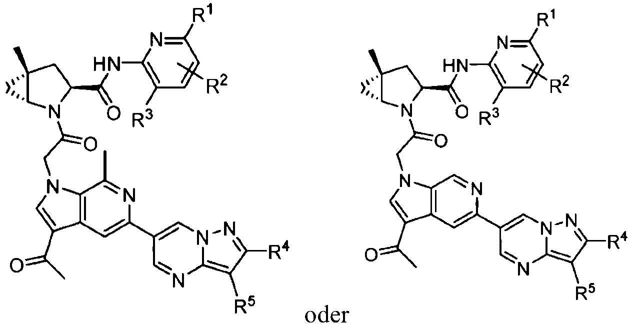
oder ein pharmazeutisch unbedenkliches Salz davon;
wobei:
R¹ aus der Gruppe ausgewählt ist, bestehend aus Halogen und C₁-C₂-Haloalkyl;
R² aus der Gruppe ausgewählt ist, bestehend aus Wasserstoff, Halogen, C₁-C₃-Alkyl, C₁-C₃-Haloalkyl, -COOR⁹, -CONR₉R¹⁰, Cyan, C₂-C₆-Alkanoyl, C₁-C₆-Alkoxy, -C₀-C₄-AlkylNR⁹R¹⁰, -C₀-C₄-AlkylOR⁹ und C₁-C₆-Haloalkoxy;
R³ C₁-C₃-Alkyl, Halogen oder C₁-C₃-Haloalkyl ist;
R⁴ und R⁵ unabhängig voneinander aus der Gruppe ausgewählt sind, bestehend aus Wasserstoff, Halogen, C₁-C₃-Alkyl, C₁-C₃-Haloalkyl, -COOR⁹, -CONR⁹R¹⁰, Cyan, C₂-C₆-Alkanoyl, C₁-C₆-Alkoxy, -C₀-C₄-AlkylNR⁹R¹⁰, -C₀-C₄-AlkylOR⁹ und C₁-C₆-Haloalkoxy; und
jedes R⁹ und R¹⁰ unabhängig voneinander aus der Gruppe ausgewählt sind, bestehend aus Wasserstoff und C₁-C₄-Alkyl.

2. Verbindung nach Anspruch 1, wobei die Verbindung ist: 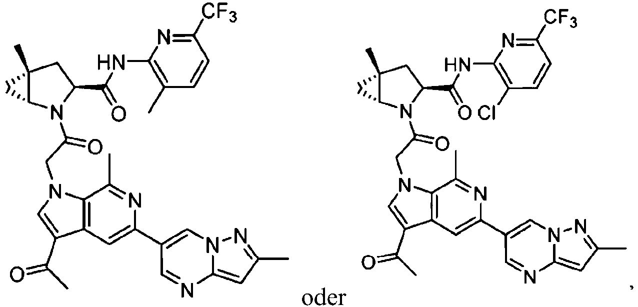 oder ein pharmazeutisch unbedenkliches Salz davon.

3. Verbindung, ausgewählt aus: 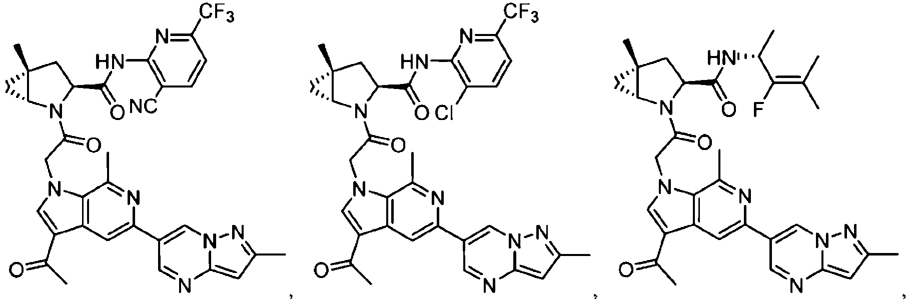 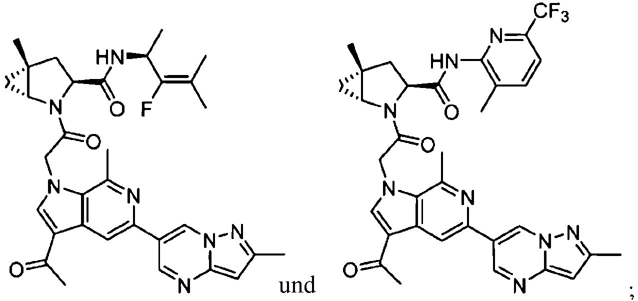 oder einem pharmazeutisch unbedenklichen Salz davon.

4. Pharmazeutische Zusammensetzung, umfassend eine Verbindung nach einem der Ansprüche 1-3 oder ein pharmazeutisch unbedenkliches Salz davon und einen pharmazeutisch unbedenklichen Träger.

5. Verbindung nach einem der Ansprüche 1-3 oder ein pharmazeutisch unbedenkliches Salz davon für die Verwendung bei der Behandlung einer durch Komplementfaktor D vermittelten Erkrankung bei einem Subjekt, das dies benötigt, wobei die Erkrankung aus akutem Atemnotsyndrom, altersbedingter Makuladegeneration, Arthritis, Asthma, Alzheimer-Demenz, amyotropher Lateralsklerose, antikörpervermittelter Transplantatabstoßung, antineutrophiler zytoplasmatischer Antikörper-assoziierter Vaskulitis, Antiphospholipid-Syndrom, atypischem oder typischem hämolytisch-urämischem Syndrom, Herz-Kreislauf-Krankheit, Kaltagglutinin-Krankheit, Komplement-3-Glomerulopathie, chronisch obstruktiver Lungenerkrankung, Zirrhose, Morbus Crohn, C3-Glomerulonephritis, diabetischer Retinopathie, Dermatomyositis, Dermatitis, Epidermolysis bullosa acquisita, Fettleber, fokaler segmentaler Glomerulosklerose, geografischer Atrophie, Glomerulonephritis, Transplantat-gegen-Wirt-Reaktion, Guillain-Barré-Syndrom, hämolytischer Anämie, Hidradenitis suppurativa, IgA-Nephropathie, Ischämie-/Reperfusionsschaden, Leberversagen, Leberentzündung, Lupus nephritis, membranoproliferativer Glomerulonephritis, multifokaler motorischer Neuropathie, Multipler Sklerose, Myasthenia gravis, Neuromyelitis optica, nichtalkoholischer Steatohepatitis, Augenerkrankung, ophthalmologischer Krankheit, Pankreatitis, paroxysmaler nächtlicher Hämoglobinurie, Pemphigoid, Pemphigus vulgaris, Präeklampsie, reduzierter glomerulärer Filtrationsrate, renovaskulärer Erkrankung, Atemwegserkrankung, Netzhautablösung, rheumatoider Arthritis, Sklerodermie, Sepsis, durch Shiga-Toxin bildende *E. coli* verwandtem hämolytisch-urämischem Syndrom, Rückenmarksverletzung, Sichelzellenanämie, traumatischer Hirnverletzung und Colitis ulcerosa.

6. Verbindung für die Verwendung nach Anspruch 5, wobei das Subjekt ein Mensch ist.

7. Verbindung für die Verwendung nach Anspruch 5 oder 6, wobei die Erkrankung eine C3-Glomerulopathie, eine C3-Glomerulonephritis oder eine Dense-Deposit-Krankheit ist.

8. Verbindung für die Verwendung nach Anspruch 5 oder 6, wobei die Erkrankung eine Augenerkrankung, eine altersbedingte Makuladegeneration oder eine geografische Atrophie ist.

9. Verbindung für die Verwendung nach Anspruch 5 oder 6, wobei die Erkrankung paroxysmale nächtliche Hämoglobinurie ist.

10. Verbindung für die Verwendung nach Anspruch 5 oder 6, wobei die Erkrankung Sichelzellenanämie ist.

11. Verbindung für die Verwendung nach Anspruch 5 oder 6, wobei die Erkrankung Lupus nephritis oder IgA-Nephropathie ist.

12. Verbindung für die Verwendung nach Anspruch 5 oder 6, wobei die Erkrankung eine renovaskuläre Erkrankung ist.

## Revendications

1. Composé de formule : 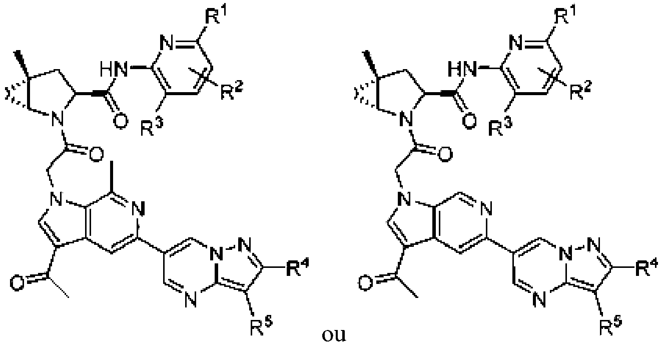
ou sel pharmaceutiquement acceptable de celui-ci ;
où :
R¹ est sélectionné dans le groupe constitué par halogène et halogénoalkyle en C₁-C₂ ;
R² est sélectionné dans le groupe constitué par hydrogène, halogène, alkyle en C₁-C₃, halogénoalkyle en C₁₋C₃, -COOR⁹, -CONR⁹R¹⁰, cyano, alcanoyle en C₂-C₆, alcoxy en C₁-C₆, - alkyl en C₀-C₄NR⁹R¹⁰, -alkyl en C₀-C₄OR⁹, et halogénoalcoxy en C₁-C₆ ;
R³ est alkyle en C₁-C₃, halogène, ou halogénoalkyle en C₁-C₃ ;
R⁴ et R⁵ sont sélectionnés indépendamment dans le groupe constitué par hydrogène, halogène, alkyle en C₁-C₃, halogénoalkyle en C₁-C₃, -COOR⁹, -CONR⁹R¹⁰, cyano, alcanoyle en C₂-C₆, alcoxy en C₁-C₆, -alkyl en C₀-C₄NR⁹R¹⁰, -alkyl en C₀₋C₄OR⁹, et halogénoalcoxy en C₁-C₆ ; et
chaque R⁹ et chaque R¹⁰ sont sélectionnés indépendamment dans le groupe constitué par hydrogène et alkyle en C₁-C₄.

2. Composé selon la revendication 1, dans lequel le composé est : 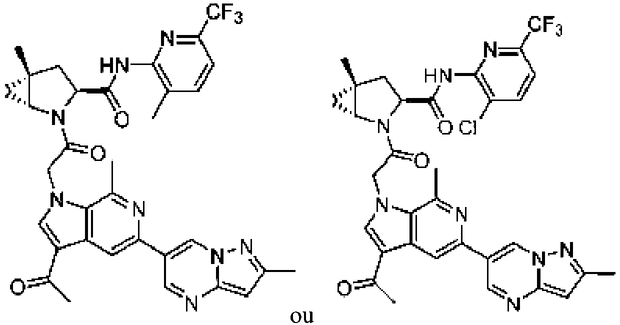 ou sel pharmaceutiquement acceptable de celui-ci.

3. Composé sélectionné parmi : 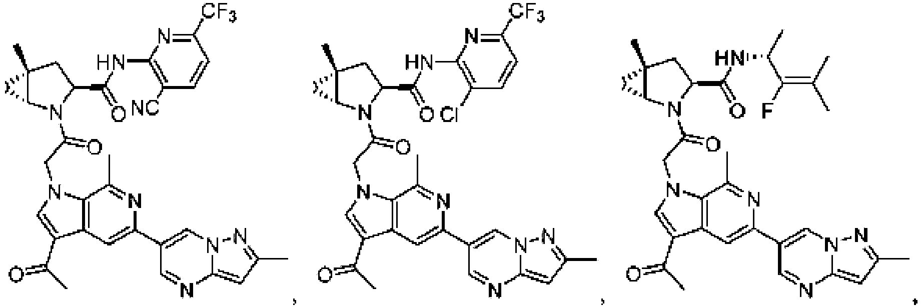 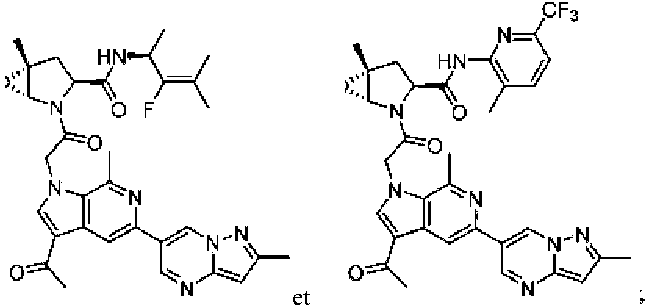 ou sel pharmaceutiquement acceptable de celui-ci.

4. Composition pharmaceutique comprenant un composé de l'une quelconque des revendications 1 à 3 ou un sel pharmaceutiquement acceptable de celui-ci, et un support pharmaceutiquement acceptable.

5. Composé selon l'une quelconque des revendications 1 à 3 ou sel pharmaceutiquement acceptable de celui-ci, pour une utilisation dans le traitement d'un trouble médié par le facteur D du complément chez un sujet qui en a besoin, dans lequel le trouble est sélectionné parmi le syndrome de détresse respiratoire aiguë, la dégénérescence maculaire liée à l'âge, l'arthrite, l'asthme, la maladie d'Alzheimer, la sclérose latérale amyotrophique, le rejet de greffe médié par anticorps, la vascularite associée aux anticorps anticytoplasme de neutrophiles, le syndrome des antiphospholipides, le syndrome hémolytique et urémique atypique ou typique, une maladie cardiovasculaire, la maladie des agglutinines froides, la glomérulopathie à C3, la bronchopneumopathie chronique obstructive, la cirrhose, la maladie de Crohn, la glomérulonéphrite à C3, la rétinopathie diabétique, la dermatomyosite, la dermatite, l'épidermolyse bulleuse acquise, la stéatose hépatique, la glomérulosclérose segmentaire et focale, l'atrophie géographique, la glomérulonéphrite, la maladie greffon contre hôte, le syndrome de Guillain-Barré, l'anémie hémolytique, l'hidradénite suppurée, la néphropathie à IgA, la lésion d'ischémie-reperfusion, l'insuffisance hépatique, l'inflammation du foie, la néphrite lupique, la glomérulonéphrite proliférative membranaire, la neuropathie motrice multifocale, la sclérose en plaques, la myasthénie grave, la neuromyélite optique, la stéatohépatite non alcoolique, un trouble oculaire, une maladie ophtalmique, la pancréatite, l'hémoglobinurie paroxystique nocturne, la pemphigoïde, le pemphigus vulgaire, la prééclampsie, la diminution du débit de filtration glomérulaire, un trouble rénovasculaire, une maladie respiratoire, le décollement de rétine, la polyarthrite rhumatoïde, la sclérodermie, la septicémie, le syndrome hémolytique et urémique associé à *E. coli* productrice de toxine Shiga, une lésion de la moelle épinière, la drépanocytose, le traumatisme crânien, et la rectocolite hémorragique.

6. Composé pour l'utilisation selon la revendication 5, dans lequel le sujet est un être humain.

7. Composé pour l'utilisation selon la revendication 5 ou 6, dans lequel le trouble est la glomérulopathie C3, la glomérulonéphrite C3, ou une maladie à dépôts denses.

8. Composé pour l'utilisation selon la revendication 5 ou 6, dans lequel le trouble est un trouble ophtalmique, la dégénérescence maculaire liée à l'âge, ou l'atrophie géographique.

9. Composé pour l'utilisation selon la revendication 5 ou 6, dans lequel le trouble est l'hémoglobinurie paroxystique nocturne.

10. Composé pour l'utilisation selon la revendication 5 ou 6, dans lequel le trouble est la drépanocytose.

11. Composé pour l'utilisation selon la revendication 5 ou 6, dans lequel le trouble est la néphrite lupique ou la néphropathie à IgA.

12. Composé pour l'utilisation selon la revendication 5 ou 6, dans lequel le trouble est un trouble rénovasculaire.
